(19) Europäisches Patentamt / European Patent Office / Office européen des brevets

(11) EP 4 130 270 A1

(12) EUROPEAN PATENT APPLICATION
published in accordance with Art. 153(4) EPC

(43) Date of publication:
**08.02.2023 Bulletin 2023/06**

(21) Application number: **21782017.4**

(22) Date of filing: **01.04.2021**

(51) International Patent Classification (IPC):
***C12N 15/13*** *(1990.01)* ***A61K 39/395*** *(1980.01)*
***A61P 35/00*** *(2000.01)* ***A61P 37/02*** *(2000.01)*
***A61P 37/08*** *(2000.01)* ***A61P 43/00*** *(2000.01)*
***C07K 16/00*** *(1995.01)* ***C07K 16/18*** *(1995.01)*
***C12N 1/15*** *(1990.01)* ***C12N 1/19*** *(1990.01)*
***C12N 1/21*** *(1990.01)* ***C12N 5/10*** *(1990.01)*
***C12N 15/63*** *(1990.01)*

(52) Cooperative Patent Classification (CPC):
**A61K 39/395; A61P 35/00; A61P 37/02; A61P 37/08; A61P 43/00; C07K 16/00; C07K 16/18; C12N 5/10; C12N 15/63; C12N 15/74; C12N 15/80; C12N 15/81**

(86) International application number:
**PCT/JP2021/014181**

(87) International publication number:
**WO 2021/201236 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.04.2020 JP 2020066313**
**29.10.2020 JP 2020181493**

(71) Applicant: **Kyowa Kirin Co., Ltd.**
**Tokyo 100-0004 (JP)**

(72) Inventors:
- **NIWA Rinpei**
**Tokyo 100-0004 (JP)**
- **USAMI Katsuaki**
**Tokyo 100-0004 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) ANTIBODY COMPOSITION

(57) An object of the present invention is to provide an antibody composition, which exhibits an effector function more specifically for a target cell coexpressing two types of antigens that are different from each other and damages the target cell, and also can maintain affinity for the individual target antigens sufficiently high. The present invention relates to an antibody composition against a first antigen and a second antigen that are different from each other, including a first IgG half-molecule and a second IgG half-molecule, and relates to the first IgG half-molecule and the second IgG half-molecule constituting the antibody composition.



Processed by Luminess, 75001 PARIS (FR)

(Cont. next page)

FIG. 3

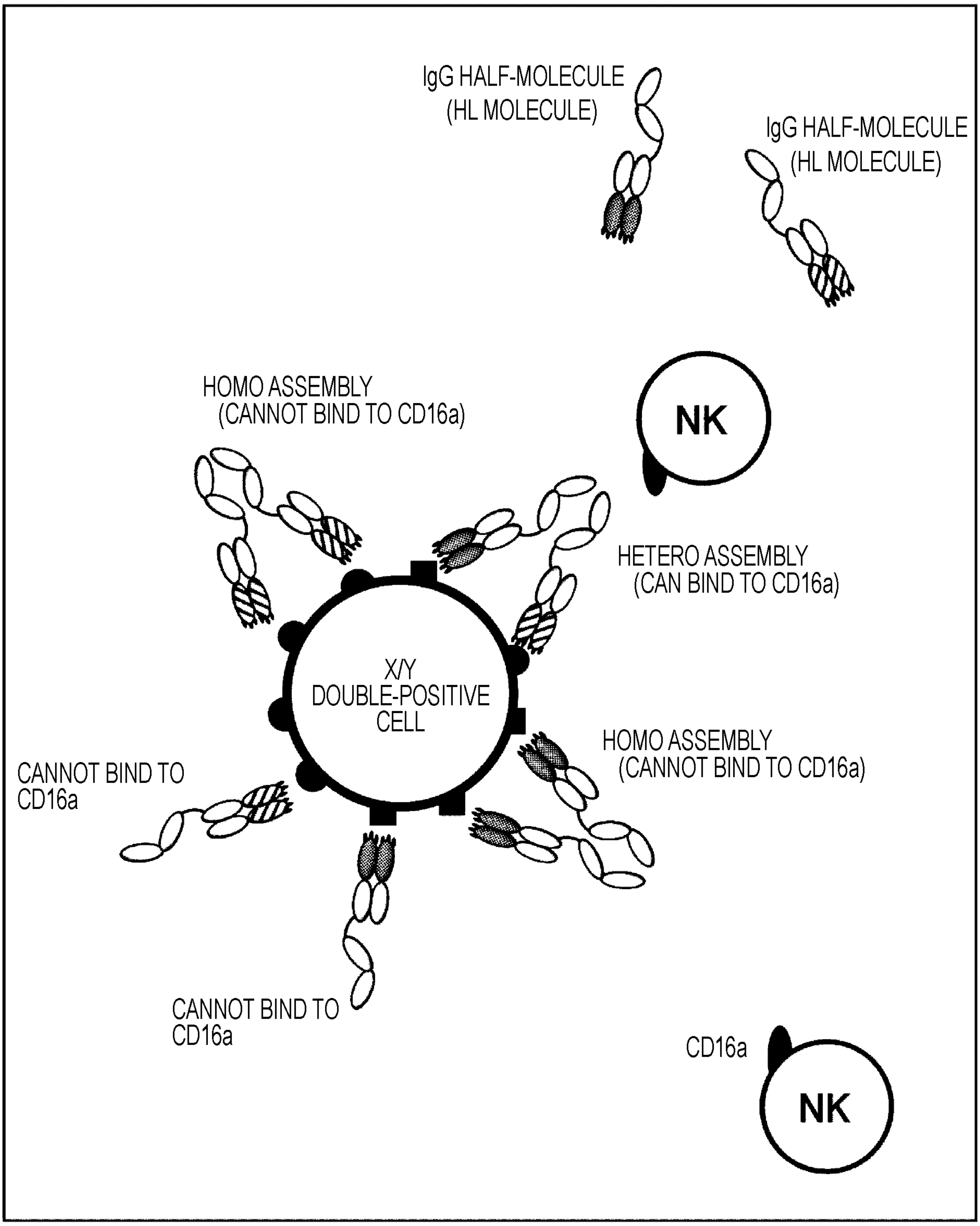
IgG HALF-MOLECULE
(HL MOLECULE)
IgG HALF-MOLECULE
(HL MOLECULE)
HOMO ASSEMBLY
(CANNOT BIND TO CD16a)
NK
HETERO ASSEMBLY
(CAN BIND TO CD16a)
X/Y
DOUBLE-POSITIVE
CELL
HOMO ASSEMBLY
(CANNOT BIND TO CD16a)
CANNOT BIND TO
CD16a
CANNOT BIND TO
CD16a
CD16a
NK

## Description

### TECHNICAL FIELD

The present invention relates to an antibody composition and a method for producing the same, an IgG half-molecule, and a kit including the IgG half-molecule.

### BACKGROUND ART

It is known that antibody preparations approved so far have various action mechanisms (Non-Patent Literature 1). Representative examples thereof include effector functions possessed by an IgG class antibody molecule such as a neutralization activity of inhibiting binding of a ligand for a growth factor or the like and a receptor, an agonistic activity of activating a bound receptor, an antibody-dependent cellular cytotoxicity (hereinafter ADCC) activity, and a complement-dependent cytotoxicity (hereinafter CDC) activity. Among these, from a biomarker analysis of a clinical study of rituximab or trastuzumab, the ADCC activity is suggested to be a clinically important action mechanism of an antibody preparation (Non-Patent Literatures 2 and 3).

An antibody is a tetrameric protein of about 150 kDa composed of a total of four molecules of polypeptide chains: two molecules of immunoglobulin heavy chain (H chain) and two molecules of immunoglobulin light chain (L chain). As shown in Fig. 1, an antibody is divided into a variable region (V) including a complementarity-determining region (CDR) that is directly involved in antigen binding and has a different amino acid sequence for each antibody clone, and a constant region including an Fc region (hereinafter also abbreviated as Fc) that controls the above-mentioned effector function or a blood half-life. The Fc includes CH1 to CH3 domains and a hinge domain.

A human antibody is classified into 5 classes: IgG, IgA, IgM, IgD, and IgE having different functions according to the sequence of the H chain constant region. Further, a human IgG class is divided into 4 subclasses from IgG1 to IgG4.

Among these, an antibody of the IgG1 subclass is known to have the highest ADCC activity and CDC activity (Non-Patent Literature 4), and many antibody preparations including rituximab and trastuzumab are IgG1.

On the other hand, the IgG4 subclass is known to have characteristics such that the effector function is weak as compared with other subclasses, and further it has an amino acid sequence of an intrinsic hinge domain, and reversible association and dissociation of two H chains in the body called "Fab arm exchange" occurs due to a weak interaction between two CH3 domains as compared with other subclasses (Non-Patent Literatures 5 and 6).

The ADCC activity is a mechanism of cytotoxicity caused through the expression of a molecule such as perforin, granzyme, or Fas as a result of activation of a natural killer cell (hereinafter NK cell) or the like by recognizing Fc of an IgG-type antibody bound to a membrane antigen on a cancer cell surface via a type of an Fc receptor, FcγRIIIa (hereinafter also abbreviated as CD16a) (Non-Patent Literature 1).

By X-ray crystallography, the binding mode between CD 16a and human IgG1 has been revealed (Non-Patent Literatures 7 and 8). The CD16a-binding domain on Fc of IgG1 is present at two sites due to the point symmetry of the structure of Fc, and in fact, Fc and IgG bind at a number ratio (stoichiometry) of 1:1. Then, in the two CH2 domains constituting Fc, contact is made in regions (hereinafter CD16a-binding domains) that are different from each other.

Specifically, CD16a interacts with L235, G236, G237, P238, S239, D265, V266, S267, H268, E269, E294, Q295, Y296, N297, S298, T299, R301, N325, A327, I332, or the like on one of the CH2 domains of IgG1. On the other hand, CD16a simultaneously interacts with L235, G236, G237, K326, A327, L328, P329, A330, or the like on the other CH2 domain of IgG1 (the number represents the position of an amino acid on the CH2 domain according to EU numbering) (Non-Patent Literatures 7, 8, 9, and 10).

It is possible to enhance an ADCC activity by artificially altering the CH2 domain of an antibody preparation to increase the binding ability to CD 16a. In fact, there are many known examples of enhancing an ADCC activity by altering an amino acid of the CH2 domain (Non-Patent Literature 11). It is known that an IgG1 antibody in which different amino acid alterations are added to the CH2 domain of one H chain of IgG1 and the CH2 domain of the other H chain, and the two H chains are bound through a disulfide bond has a high ADCC activity (Patent Literature 1).

Further, it is also known that an ADCC activity can be enhanced by altering a sugar chain of an N-linked complex sugar chain that is bound to Fc (Non-Patent Literature 12). Particularly, the technique for enhancing an ADCC activity by a sugar chain alteration is applied to approved antibody preparations such as mogamulizumab (Non-Patent Literature 13) and obinutuzumab (Non-Patent Literature 14).

A bispecific antibody is an artificially altered antibody molecule configured to be able to bind to two different types of antigens unlike natural antibodies, and many molecular forms have been reported (Non-Patent Literature 15).

A schematic diagram of a structure of a bispecific antibody is shown in Fig. 2A. As an application of the bispecific antibody to a medicine, for example, damage to a cancer cell by binding to the cancer cell and CD3 of a T cell surface (hereinafter, a CD3 bispecific antibody) so as to crosslink both, and enhancement of a drug efficacy by neutralizing two types of functional molecules are exemplified (Non-Patent Literature 15).

In a treatment of a cancer or an autoimmune disease, in order to remove a cancer cell that is an etiologic cell or a self-reactive lymphocyte, an antibody preparation exhibiting an effector function of an IgG-type antibody or a T cell recruiting function of a CD3 bispecific antibody is used.

However, such an etiologic cell is originally derived from a normal cell, and it is generally rare that an etiologic cell can be accurately distinguished from a normal cell by a single surface marker molecule. Therefore, the removal of an etiologic cell utilizing an effector function or the like also attacks a normal cell expressing the same antigen molecule, and may result in adverse effects in many cases.

For example, CD20 that is a target antigen of rituximab to be used in a treatment of lymphomas or various autoimmune diseases is expressed in a normal B cell, and HER2 that is a target antigen of trastuzumab to be used in a treatment of breast cancer is expressed in a cardiomyocyte. Therefore, there are concerns about adverse effects by disrupting a normal cell with such an antibody preparation.

On the other hand, Mazor et al. have reported a case where a bispecific antibody having reduced affinity for individual target antigens exhibits a relatively strong effector function for a double-positive cell (Non-Patent Literature 16).

Further, there has been a report on a combination of antigen-binding molecules including a first antigen-binding molecule having a first antigen-binding domain that binds to a first antigen and a first polypeptide including either one or both of first CH2 and first CH3, and a second antigen-binding molecule having a second antigen-binding domain that binds to a second antigen and a second polypeptide including either one or both of second CH2 and second CH3, wherein the first antigen-binding molecule and the second antigen-binding molecule are not bound through a covalent bond, and are more likely to form a heterodimer than a homodimer when mixed in a liquid (Patent Literature 2).

CITATION LIST

PATENT LITERATURE

Patent Literature 1: WO 2013/002362
Patent Literature 2: WO 2018/155611


NON-PATENT LITERATURE

Non-Patent Literature 1: Carter P. Nat Rev Cancer 2001; 1: 118-29
Non-Patent Literature 2: Cartron G, Dacheux L, Salles G, et al. Blood 2002; 99: 754-8
Non-Patent Literature 3: Weng WK, Levy R. J Clin Oncol 2003; 21: 3940-7
Non-Patent Literature 4: Birch, J. R., Lennox, E. S. (Eds.), Monoclonal Antibodies: Principles and Applications, Wiley-Liss, Inc., New York, p. 45 (1995)
Non-Patent Literature 5: Aalberse RC and Schuurman J, Immunology 2002; 105: 9-19
Non-Patent Literature 6: Labrijn AF, Nat Biotechnol 2009; 27: 767-71
Non-Patent Literature 7: Sondermann P, Nature 2000; 406: 267-73
Non-Patent Literature 8: Radaev S, J Biol Chem 2001; 276: 16469-77
Non-Patent Literature 9: Ferrara C, Proc Natl Acad Sci 2011; 108; 12669-74
Non-Patent Literature 10: Mizushima T, Genes Cells 2011; 16: 1071-80
Non-Patent Literature 11: Strohl WR, Curr Opin Biotechnol 2009; 20: 685-91
Non-Patent Literature 12: Niwa R, J Pharm Sci 2015; 930-41
Non-Patent Literature 13: Beck A, mAbs 2012; 4: 419-25
Non-Patent Literature 14: Goede V, N Engl J Med 2014; 370: 1101-10
Non-Patent Literature 15: Byrne H, Trends Biotechnol 2013; 31: 621-32
Non-Patent Literature 16: Mazor Y, MAbs 2015; 7: 377-89


SUMMARY OF INVENTION

TECHNICAL PROBLEM

As one method for solving the above-mentioned adverse effects, a technique using a bispecific antibody that exhibits an effector function only when it recognizes two different types of antigens for removing an etiologic cell with higher selectivity is conceivable.

However, as shown in Fig. 2B, when a target cell is attacked using an effector function by a normal bispecific antibody, there is a concern that the bispecific antibody may bind to and attack not only a target cell coexpressing two types of antigens (hereinafter also abbreviated as double-positive cell), but also a cell expressing only one type of target antigen (hereinafter also abbreviated as single-positive cell). Further, a technique for an antibody that exhibits an effector function specifically for a double-positive cell and damages the cell regardless of affinity for individual target antigens has not been known.

Therefore, the present inventors aim to provide an antibody composition which exhibits an effector function more specifically for a target cell coexpressing two types of antigens that are different from each other and damages the target cell.

SOLUTION TO PROBLEM

The present inventors conceived that the above problem can be solved by an antibody composition having the following elements [1] to [3] that are different from a normal human IgG1 antibody in a constant region of an antibody molecule as shown in Fig. 3.

[1] It is a mixture of antibody half-molecules (first and second IgG half-molecules) having antigen binding sites for a first antigen (antigen molecule X) and a second antigen (antigen molecule Y) that are different from each other. That is, it is a mixture of "HL molecules" in which a covalent bond formed by an inter-H chain disulfide bond is not present between the first IgG half-molecule and the second IgG half-molecule.

[2] The HL molecules against each of the antigen molecules X and Y bind to the surface of a target cell (X/Y double-positive cell) expressing both the antigen molecules X and Y and thereafter are associated with each other to form an H2L2 molecule in the same manner as normal IgG and form a CD16a-binding domain, and thus cause an antibody activity.

[3] A CD16a-binding domain is not formed even if the HL molecules against the antigen molecule X or Y are associated with each other on a cell surface to form a homo assembly so that an antibody activity is not caused against a cell expressing only a single antigen molecule.

Based on the above conception, the present inventors found the following (1) to (3) and thus completed the present invention.

(1) With respect to the above [1], as shown in Fig. 4, a part or the whole of a hinge domain of an IgG half-molecule is altered so as not to form a disulfide bond between H chains in the hinge domain by substitution or deletion or modification.

(2) As shown in Fig. 5, CD16a comes into contact with two CH2 domains (in Fig. 5, CH2-A and CH2-B) in Fc at different sites (a region 1 of CH2-A and a region 2 of CH2-B), respectively.

Therefore, when each of the region 2 of CH2-A and the region 1 of CH2-B that are not used for binding are "disrupted" by alteration or the like of an amino acid to attenuate the binding activity, in a homo assembly of HL molecules having such altered CH2-A, only the region 2 of CH2-A or in a homo assembly of HL molecules having such altered CH2-B, only the region 1 of CH2-B is not present. Therefore, to such a homo assembly, CD16a cannot bind with sufficient affinity.

On the other hand, according to a combination of HL molecules having such altered CH2-A and altered CH2-B as constituent elements (hetero assembly), an antibody composition satisfying the above conditions [2] and [3] is obtained.

(3) According to the hetero assembly formed from the IgG half-molecules in which a specific amino acid alteration is introduced into a CD16a-binding domain or another Fc region, an antibody composition having an enhanced effector function for the X/Y double-positive cell and/or exhibiting controlled biokinetics is obtained.

That is, the present invention relates to the following.

1. An antibody composition, which is an antibody composition against a first antigen and a second antigen that are different from each other, including a first IgG half-molecule and a second IgG half-molecule, wherein the following (1A) to (6A) are satisfied:

(1A) each of the first IgG half-molecule and the second IgG half-molecule is composed of one immunoglobulin light chain (hereinafter abbreviated as L chain) and one immunoglobulin heavy chain (hereinafter abbreviated as H chain), and the H chain includes an H chain variable region, a hinge domain, a CH1 domain, a CH2 domain, and a CH3 domain, and has a first Fcγ receptor IIIA (hereinafter CD16a)-binding domain and a second CD 16a-binding domain that are different from each other in the CH2 domain,
(2A) each of the first IgG half-molecule and the second IgG half-molecule includes amino acid residue substitutions of C226A and C229A numbered according to the EU index,
(3A) the first IgG half-molecule includes an antigen-binding domain that binds to the first antigen, and includes an amino acid residue substitution of D265A numbered according to the EU index in the first CD16a-binding domain,
(4A) the second IgG half-molecule includes an antigen-binding domain that binds to the second antigen, and includes an amino acid residue substitution of P329Y numbered according to the EU index in the second CD16a-binding domain,
(5A) each of the first IgG half-molecule and the second IgG half-molecule includes amino acid residue substitutions of (a) S239D and K326T numbered according to the EU index, or
each of the first IgG half-molecule and the second IgG half-molecule includes amino acid residue substitutions of (b) S239D, S298A, E333A, L242C, and K334C numbered according to the EU index, and
(6A) at least one of the first IgG half-molecule and the second IgG half-molecule includes an amino acid residue substitution in the CH3 domain as an alteration for attenuating an inter-CH3 domain interaction as compared with an inter-CH3 domain interaction of the IgG1 subclass.

2. The antibody composition according to the above 1, wherein the antibody composition exhibits an effector function specifically for a target cell coexpressing the first antigen and the second antigen and damages the target cell as compared with an effector function for a target cell expressing only the first antigen and a target cell expressing only the second antigen.
3. The antibody composition according to the above 1 or 2, wherein each of the first IgG half-molecule and the second IgG half-molecule includes at least one amino acid residue substitution selected from Y349A, L351A, T366A, L368A, D399A, F405A, Y407A, K409A, and K409R numbered according to the EU index as the alteration for attenuating the inter-CH3 domain interaction as compared with the inter-CH3 domain interaction of the IgG1 subclass.
4. The antibody composition according to the above 3, wherein each of the first IgG half-molecule and the second IgG half-molecule includes an amino acid residue substitution of K409R numbered according to the EU index as the alteration for attenuating the inter-CH3 domain interaction as compared with the inter-CH3 domain interaction of the IgG1 subclass.
5. The antibody composition according to any one of the above 1 to 4, wherein each of the first IgG half-molecule and the second IgG half-molecule includes the amino acid residue substitutions of (a) S239D and K326T.
6. The antibody composition according to any one of the above 1 to 5, wherein

an H chain constant region (also abbreviated as CH) of the first IgG half-molecule contains an amino acid sequence represented by SEQ ID NO: 248, and
a CH of the second IgG half-molecule contains an amino acid sequence represented by SEQ ID NO: 252.

7. The antibody composition according to any one of the above 1 to 6, wherein a ratio of sugar chains in which fucose is not bound to N-acetylglucosamine at a reducing end of the sugar chain among the total N-glycoside-linked type sugar chains bound to an Fc region in the first IgG half-molecule and the second IgG half-molecule is 20% or more.
8. The antibody composition according to any one of the above 1 to 7, wherein the immunoglobulin subclass of the first IgG half-molecule and the second IgG half-molecule is IgG 1.
9. A first IgG half-molecule, which is a first IgG half-molecule characterized by being associated with a second IgG half-molecule, wherein the first IgG half-molecule and the second IgG half-molecule satisfy the following (1B) to (7B):

(1B) the first IgG half-molecule and the second IgG half-molecule form an antibody composition against a first antigen and a second antigen that are different from each other,
(2B) each of the first IgG half-molecule and the second IgG half-molecule is composed of one L chain and one H chain, and the H chain includes an H chain variable region, a hinge domain, a CH1 domain, a CH2 domain, and a CH3 domain, and has a first CD16a-binding domain and a second CD16a-binding domain that are different from each other in the CH2 domain,
(3B) each of the first IgG half-molecule and the second IgG half-molecule includes amino acid residue substitutions of C226A and C229A numbered according to the EU index,

(4B) the first IgG half-molecule includes an antigen-binding domain that binds to the first antigen, and includes an amino acid residue substitution of D265A numbered according to the EU index in the first CD16a-binding domain,

(5B) the second IgG half-molecule includes an antigen-binding domain that binds to the second antigen, and includes an amino acid residue substitution of P329Y numbered according to the EU index in the second CD16a-binding domain,

(6B) each of the first IgG half-molecule and the second IgG half-molecule includes amino acid residue substitutions of (a) S239D and K326T numbered according to the EU index, or

each of the first IgG half-molecule and the second IgG half-molecule includes amino acid residue substitutions of (b) S239D, S298A, E333A, L242C, and K334C numbered according to the EU index, and

(7B) at least one of the first IgG half-molecule and the second IgG half-molecule includes an amino acid residue substitution in the CH3 domain as an alteration for attenuating an inter-CH3 domain interaction as compared with an inter-CH3 domain interaction of the IgG1 subclass.

10. A second IgG half-molecule, which is a second IgG half-molecule characterized by being associated with a first IgG half-molecule, wherein the first IgG half-molecule and the second IgG half-molecule satisfy the following (1C) to (7C):

(1C) the first IgG half-molecule and the second IgG half-molecule form an antibody composition against a first antigen and a second antigen that are different from each other,

(2C) each of the first IgG half-molecule and the second IgG half-molecule is composed of one L chain and one H chain, and the H chain includes an H chain variable region, a hinge domain, a CH1 domain, a CH2 domain, and a CH3 domain, and has a first CD16a-binding domain and a second CD16a-binding domain that are different from each other in the CH2 domain,

(3C) each of the first IgG half-molecule and the second IgG half-molecule includes amino acid residue substitutions of C226A and C229A numbered according to the EU index,

(4C) the first IgG half-molecule includes an antigen-binding domain that binds to the first antigen, and includes an amino acid residue substitution of D265A numbered according to the EU index in the first CD16a-binding domain,

(5C) the second IgG half-molecule includes an antigen-binding domain that binds to the second antigen, and includes an amino acid residue substitution of P329Y numbered according to the EU index in the second CD16a-binding domain,

(6C) each of the first IgG half-molecule and the second IgG half-molecule includes amino acid residue substitutions of (a) S239D and K326T numbered according to the EU index, or

each of the first IgG half-molecule and the second IgG half-molecule includes amino acid residue substitutions of (b) S239D, S298A, E333A, L242C, and K334C numbered according to the EU index, and

(7C) at least one of the first IgG half-molecule and the second IgG half-molecule includes an amino acid residue substitution in the CH3 domain as an alteration for attenuating an inter-CH3 domain interaction as compared with an inter-CH3 domain interaction of the IgG1 subclass.

11. A DNA encoding the following amino acid sequence a) or b):

a) an amino acid sequence of the first IgG half-molecule according to the above 9 or
b) an amino acid sequence of the second IgG half-molecule according to the above 10.

12. A recombinant vector, containing at least one of the DNA encoding the amino acid sequence a) and the DNA encoding the amino acid sequence b) according to the above 11.

13. A transformant, in which the recombinant vector according to the above 12 is introduced.

14. A kit, including the first IgG half-molecule according to the above 9 and the second IgG half-molecule according to the above 10.

15. A method for inducing an effector function specifically for a target cell coexpressing the first antigen and the second antigen as compared with an effector function for a target cell expressing only the first antigen and a target cell expressing only the second antigen using the antibody composition according to any one of the above 1 to 8.

16. A pharmaceutical composition, containing the antibody composition according to any one of the above 1 to 8.

17. The pharmaceutical composition according to the above 16, for use in a treatment of a cancer, an autoimmune disease, or an allergic disease.

18. Use of the antibody composition according to any one of the above 1 to 8 for producing a pharmaceutical composition for a treatment of a cancer, an autoimmune disease, or an allergic disease.

19. Use of the antibody composition according to any one of the above 1 to 8 for treating a cancer, an autoimmune disease, or an allergic disease.
20. The antibody composition according to any one of the above 1 to 8 for use in a treatment of a cancer, an autoimmune disease, or an allergic disease.
21. A method for treating a cancer, an autoimmune disease, or an allergic disease, including administering an effective amount of the antibody composition according to any one of the above 1 to 8 to a subject.
22. A first IgG half-molecule, which is a first IgG half-molecule for use in combination with a second IgG half-molecule, wherein the first IgG half-molecule and the second IgG half-molecule satisfy the following (1B) to (7B):

(1B) the first IgG half-molecule and the second IgG half-molecule form an antibody composition against a first antigen and a second antigen that are different from each other,
(2B) each of the first IgG half-molecule and the second IgG half-molecule is composed of one L chain and one H chain, and the H chain includes an H chain variable region, a hinge domain, a CH1 domain, a CH2 domain, and a CH3 domain, and has a first CD16a-binding domain and a second CD16a-binding domain that are different from each other in the CH2 domain,
(3B) each of the first IgG half-molecule and the second IgG half-molecule includes amino acid residue substitutions of C226A and C229A numbered according to the EU index,
(4B) the first IgG half-molecule includes an antigen-binding domain that binds to the first antigen, and includes an amino acid residue substitution of D265A numbered according to the EU index in the first CD16a-binding domain,
(5B) the second IgG half-molecule includes an antigen-binding domain that binds to the second antigen, and includes an amino acid residue substitution of P329Y numbered according to the EU index in the second CD16a-binding domain,
(6B) each of the first IgG half-molecule and the second IgG half-molecule includes amino acid residue substitutions of (a) S239D and K326T numbered according to the EU index, or
each of the first IgG half-molecule and the second IgG half-molecule includes amino acid residue substitutions of (b) S239D, S298A, E333A, L242C, and K334C numbered according to the EU index, and
(7B) at least one of the first IgG half-molecule and the second IgG half-molecule includes an amino acid residue substitution in the CH3 domain as an alteration for attenuating an inter-CH3 domain interaction as compared with an inter-CH3 domain interaction of the IgG1 subclass.

23. A second IgG half-molecule, which is a second IgG half-molecule for use in combination with a first IgG half-molecule, wherein the first IgG half-molecule and the second IgG half-molecule satisfy the following (1C) to (7C):

(1C) the first IgG half-molecule and the second IgG half-molecule form an antibody composition against a first antigen and a second antigen that are different from each other,
(2C) each of the first IgG half-molecule and the second IgG half-molecule is composed of one L chain and one H chain, and the H chain includes an H chain variable region, a hinge domain, a CH1 domain, a CH2 domain, and a CH3 domain, and has a first CD16a-binding domain and a second CD16a-binding domain that are different from each other in the CH2 domain,
(3C) each of the first IgG half-molecule and the second IgG half-molecule includes amino acid residue substitutions of C226A and C229A numbered according to the EU index,
(4C) the first IgG half-molecule includes an antigen-binding domain that binds to the first antigen, and includes an amino acid residue substitution of D265A numbered according to the EU index in the first CD16a-binding domain,
(5C) the second IgG half-molecule includes an antigen-binding domain that binds to the second antigen, and includes an amino acid residue substitution of P329Y numbered according to the EU index in the second CD16a-binding domain,
(6C) each of the first IgG half-molecule and the second IgG half-molecule includes amino acid residue substitutions of (a) S239D and K326T numbered according to the EU index, or
each of the first IgG half-molecule and the second IgG half-molecule includes amino acid residue substitutions of (b) S239D, S298A, E333A, L242C, and K334C numbered according to the EU index, and
(7C) at least one of the first IgG half-molecule and the second IgG half-molecule includes an amino acid residue substitution in the CH3 domain as an alteration for attenuating an inter-CH3 domain interaction as compared with an inter-CH3 domain interaction of the IgG1 subclass.

24. The first or second IgG half-molecule contained in the antibody composition according to any one of the above 1 to 8.

25. A method for inducing an effector function specifically for a double-positive cell expressing the first and second antigens using the IgG half-molecule according to the above 9, 10, or 24.

26. The antibody composition according to any one of the above 1 to 8, wherein the first antigen is CADM1 and the second antigen is CCR4.

27. The antibody composition according to any one of the above 1 to 8, wherein the first antigen is EGFR and the second antigen is HER2.

28. The antibody composition according to any one of the above 1 to 8, wherein the first antigen is CD52 and the second antigen is CD70.

29. The antibody composition according to any one of the above 1 to 8, wherein the first antigen is CD2 and the second antigen is CD70.

30. The antibody composition according to any one of the above 1 to 8, wherein the first antigen is CD19 and the second antigen is CD70.

31. The antibody composition according to any one of the above 1 to 8, wherein the first antigen is CD2 and the second antigen is CD40 ligand.

32. The antibody composition according to any one of the above 1 to 8, wherein the first antigen is PD-L1 and the second antigen is one selected from CD19, CD30, CCR4, CD20, CD22, and CD79b.

33. The antibody composition according to any one of the above 1 to 8, wherein the first antigen is CD8 and the second antigen is CCR4.

34. The antibody composition according to any one of the above 1 to 8, wherein the first antigen is CTLA-4 and the second antigen is one selected from CD4, CCR4, and GITR.

35. The antibody composition according to any one of the above 1 to 8, wherein the first antigen is TIGIT and the second antigen is one selected from CD4, CCR4, and GITR.

36. The antibody composition according to any one of the above 1 to 8, wherein the first antigen is PD-1 and the second antigen is one selected from CD4, CCR4, and GITR.

37. The antibody composition according to any one of the above 1 to 8, wherein the first antigen is OX40 and the second antigen is one selected from CD127, CD26, CD70, and CD15s.

38. The antibody composition according to any one of the above 1 to 8, wherein the first antigen is 4-1BB and the second antigen is one selected from CD127, CD26, CD70, and CD15s.

39. The antibody composition according to any one of the above 1 to 8, wherein the first antigen is GITR and the second antigen is one selected from CD127, CD26, CD70, and CD15s.

40. The antibody composition according to any one of the above 1 to 8, wherein the first antigen is CD40 ligand and the second antigen is one selected from CD127, CD26, CD70, and CD15s.

41. The antibody composition according to any one of the above 1 to 8, wherein the first antigen is CD4 and the second antigen is CD69.

42. A pharmaceutical composition for use in a treatment of ATL, containing the antibody composition according to the above 26.

43. A pharmaceutical composition for use in a treatment of at least one of gastric cancer and breast cancer, containing the antibody composition according to the above 27.

44. A pharmaceutical composition for use in a treatment of Sjogren's syndrome, containing the antibody composition according to the above 31.

45. A pharmaceutical composition for use in a treatment of at least one of lymphoma and leukemia, containing the antibody composition according to the above 32.

46. A pharmaceutical composition for use in a treatment of a cancer, containing the antibody composition according to any one of the above 33 to 36.

47. A pharmaceutical composition for use in a treatment of an autoimmune disease, containing the antibody composition according to any one of the above 37 to 40.

48. A pharmaceutical composition for use in a treatment of ANCA-associated glomerulonephritis, containing the antibody composition according to the above 41.

49. Use of the antibody composition according to the above 26 for producing a pharmaceutical composition for treating ATL.

50. Use of the antibody composition according to the above 27 for producing a pharmaceutical composition for treating at least one of gastric cancer and breast cancer.

51. Use of the antibody composition according to the above 31 for producing a pharmaceutical composition for treating Sjogren's syndrome.

52. Use of the antibody composition according to the above 32 for producing a pharmaceutical composition for treating at least one of lymphoma and leukemia.

53. Use of the antibody composition according to any one of the above 33 to 36 for producing a pharmaceutical composition for treating a cancer.

54. Use of the antibody composition according to any one of the above 37 to 40 for producing a pharmaceutical composition for treating an autoimmune disease.
55. Use of the antibody composition according to the above 41 for producing a pharmaceutical composition for treating ANCA-associated glomerulonephritis.
56. Use of the antibody composition according to the above 26 for treating ATL.
57. Use of the antibody composition according to the above 27 for treating at least one of gastric cancer and breast cancer.
58. Use of the antibody composition according to the above 31 for treating Sjogren's syndrome.
59. Use of the antibody composition according to the above 32 for treating at least one of lymphoma and leukemia.
60. Use of the antibody composition according to any one of the above 33 to 36 for treating a cancer.
61. Use of the antibody composition according to any one of the above 37 to 40 for treating an autoimmune disease.
62. Use of the antibody composition according to the above 41 for treating ANCA-associated glomerulonephritis.
63. The antibody composition according to the above 26 for use in a treatment of ATL.
64. The antibody composition according to the above 27 for use in a treatment of at least one of gastric cancer and breast cancer.
65. The antibody composition according to the above 31 for use in a treatment of Sjogren's syndrome.
66. The antibody composition according to the above 32 for use in a treatment of at least one of lymphoma and leukemia.
67. The antibody composition according to any one of the above 33 to 36 for use in a treatment of a cancer.
68. The antibody composition according to any one of the above 37 to 40 for use in a treatment of an autoimmune disease.
69. The antibody composition according to the above 41 for use in a treatment of ANCA-associated glomerulonephritis.
70. A method for treating ATL, including administering an effective amount of the antibody composition according to the above 26 to a subject.
71. A method for treating at least one of gastric cancer and breast cancer, including administering an effective amount of the antibody composition according to the above 27 to a subject.
72. A method for treating Sjogren's syndrome, including administering an effective amount of the antibody composition according to the above 31 to a subject.
73. A method for treating at least one of lymphoma and leukemia, including administering an effective amount of the antibody composition according to the above 32 to a subject.
74. A method for treating a cancer, including administering an effective amount of the antibody composition according to any one of the above 33 to 36 to a subject.
75. A method for treating an autoimmune disease, including administering an effective amount of the antibody composition according to any one of the above 37 to 40 to a subject.
76. A method for treating ANCA-associated glomerulonephritis, including administering an effective amount of the antibody composition according to the above 41 to a subject.
77. The antibody composition according to any one of the above 1 to 8, wherein the first antigen is PD-1 and the second antigen is CD3.
78. The antibody composition according to any one of the above 1 to 8, wherein the first antigen is PD-1 and the second antigen is CD4.
79. The antibody composition according to any one of the above 1 to 8, wherein the first antigen is integrin $\alpha4\beta7$ and the second antigen is one selected from CCR6, CXCR3, CD161, and CD127.
80. The antibody composition according to any one of the above 1 to 8, wherein the first antigen is integrin $\alpha4\beta7$ and the second antigen is CD40 ligand.
81. The antibody composition according to any one of the above 1 to 8, wherein the first antigen is integrin $\alpha4\beta1$ and the second antigen is CD40 ligand.
82. The antibody composition according to any one of the above 1 to 8, wherein the first antigen is CXCR5 and the second antigen is CD127.
83. The antibody composition according to any one of the above 1 to 8, wherein the first antigen is a TPO receptor (c-mpl) and the second antigen is CD34 or CD123.
84. The antibody composition according to any one of the above 1 to 8, wherein the first antigen is CXCR3 and the second antigen is CD3.
85. The antibody composition according to any one of the above 1 to 8, wherein the first antigen is CXCR3 and the second antigen is CD127 or CD40 ligand.
86. The antibody composition according to any one of the above 1 to 8, wherein the first antigen is CCR4 and the second antigen is CD127 or CD40 ligand.
87. The antibody composition according to any one of the above 1 to 8, wherein the first antigen is CCR6 and the

second antigen is CD127 or CD40 ligand.

88. The antibody composition according to any one of the above 1 to 8, wherein the first antigen is CRTH2 and the second antigen is CD127 or CD40 ligand.

89. The antibody composition according to any one of the above 1 to 8, wherein the first antigen is CRTH2 and the second antigen is CCR4.

90. The antibody composition according to any one of the above 1 to 8, wherein the first antigen is CRTH2 and the second antigen is ST2.

91. The antibody composition according to any one of the above 1 to 8, wherein the first antigen is CRTH2 and the second antigen is CCR6 or CCR3.

92. The antibody composition according to any one of the above 1 to 8, wherein the first antigen is CD207 and the second antigen is CD11b or CD1a.

93. The antibody composition according to any one of the above 1 to 8, wherein the first antigen is CD123 and the second antigen is HLA-DR or ASCT2.

94. The antibody composition according to any one of the above 1 to 8, wherein the first antigen is CSF1R and the second antigen is CD14.

95. The antibody composition according to any one of the above 1 to 8, wherein the first antigen is CSF1R and the second antigen is CD33.

96. The antibody composition according to any one of the above 1 to 8, wherein the first antigen is CD19 and the second antigen is CD38.

97. The antibody composition according to any one of the above 1 to 8, wherein the first antigen is CD3 and the second antigen is IL-23R.

98. The antibody composition according to any one of the above 1 to 8, wherein the first antigen is CD3 and the second antigen is CX3CR1.

99. The antibody composition according to any one of the above 1 to 8, wherein the first antigen is CXCR4 and the second antigen is type 1 collagen.

100. The antibody composition according to any one of the above 1 to 8, wherein the first antigen is CXCR4 and the second antigen is CD14.

101. The antibody composition according to any one of the above 1 to 8, wherein the first antigen is CXCR4 and the second antigen is CD16.

102. The antibody composition according to any one of the above 1 to 8, wherein the first antigen is CLEC10A and the second antigen is CD14 or CD16.

103. The antibody composition according to any one of the above 1 to 8, wherein the first antigen is CD70 and the second antigen is CD38.

104. The antibody composition according to any one of the above 1 to 8, wherein the first antigen is CD70 and the second antigen is one selected from CD4, CD127, and TIM-1.

105. The antibody composition according to any one of the above 1 to 8, wherein the first antigen is CD4 and the second antigen is TIM-1.

106. The antibody composition according to any one of the above 1 to 8, wherein the first antigen is CD70 and the second antigen is CD52.

107. The antibody composition according to any one of the above 1 to 8, wherein the first antigen is CB1 and the second antigen is AT1R.

108. The antibody composition according to any one of the above 1 to 8, wherein the first antigen is CD4 or PD-1 and the second antigen is CD153.

109. The antibody composition according to any one of the above 1 to 8, wherein the first antigen is FcεRI and the second antigen is CD34 or C-KIT.

110. The antibody composition according to any one of the above 1 to 8, wherein the first antigen is CD34 and the second antigen is CD203 or MRGPRX2.

111. The antibody composition according to any one of the above 1 to 8, wherein the first antigen is CD52 and the second antigen is CD127.

112. The antibody composition according to any one of the above 1 to 8, wherein the first antigen is CD69 and the second antigen is CD21.

113. The antibody composition according to any one of the above 1 to 8, wherein the first antigen is CD 106 and the second antigen is one selected from CD11c, CD 19, CD21, and CD72.

114. The antibody composition according to any one of the above 1 to 8, wherein the first antigen is 4-1BB and the second antigen is one selected from CD11c, CD19, CD21, and CD72.

115. The antibody composition according to any one of the above 1 to 8, wherein the first antigen is BLT1 and the second antigen is CD49b.

116. The antibody composition according to any one of the above 1 to 8, wherein the first antigen is CD226 and the

second antigen is CD8.

117. The antibody composition according to any one of the above 1 to 8, wherein the first antigen is CXCR3 and the second antigen is one selected from CD8, CD49a, IL-15R, and NKG2D.

118. The antibody composition according to any one of the above 1 to 8, wherein the first antigen is CD49a and the second antigen is one selected from CD8, IL-15R, and NKG2D.

119. The antibody composition according to any one of the above 1 to 8, wherein the first antigen is IL-15R and the second antigen is CD8 or NKG2D.

120. The antibody composition according to any one of the above 1 to 8, wherein the first antigen is NKG2D and the second antigen is CD8.

121. The antibody composition according to any one of the above 1 to 8, wherein the first antigen is CD177 and the second antigen is PR3.

122. The antibody composition according to any one of the above 1 to 8, wherein the first antigen is CD4 and the second antigen is CD127.

123. The antibody composition according to any one of the above 1 to 8, wherein the first antigen is CD40 ligand and the second antigen is IL-6.

124. The antibody composition according to any one of the above 1 to 8, wherein the first antigen is IL-17R and the second antigen is membrane-associated TNF.

125. The antibody composition according to any one of the above 1 to 8, wherein the first antigen is IL-23R and the second antigen is membrane-associated CCR6.

126. Use of the antibody composition according to the above 77 for producing a pharmaceutical composition for treating rheumatoid arthritis.

127. Use of the antibody composition according to the above 78 for producing a pharmaceutical composition for treating celiac disease.

128. Use of the antibody composition according to the above 79 for producing a pharmaceutical composition for treating an inflammatory bowel disease.

129. Use of the antibody composition according to the above 80 for producing a pharmaceutical composition for treating at least one of multiple sclerosis and an inflammatory bowel disease.

130. Use of the antibody composition according to the above 81 for producing a pharmaceutical composition for treating at least one of multiple sclerosis and an inflammatory bowel disease.

131. Use of the antibody composition according to the above 82 for producing a pharmaceutical composition for treating an allergic disease.

132. Use of the antibody composition according to the above 83 for producing a pharmaceutical composition for treating primary myelofibrosis.

133. Use of the antibody composition according to the above 84 for producing a pharmaceutical composition for treating at least one selected from an autoimmune disease, arteriosclerosis, and an ischemic heart disease.

134. Use of the antibody composition according to the above 85 for producing a pharmaceutical composition for treating systemic lupus erythematosus.

135. Use of the antibody composition according to the above 86 for producing a pharmaceutical composition for treating an allergic disease.

136. Use of the antibody composition according to the above 87 for producing a pharmaceutical composition for treating psoriasis.

137. Use of the antibody composition according to the above 88 for producing a pharmaceutical composition for treating an allergic disease.

138. Use of the antibody composition according to the above 89 for producing a pharmaceutical composition for treating at least one selected from asthma, eosinophilic sinusitis, and atopic dermatitis.

139. Use of the antibody composition according to the above 90 for producing a pharmaceutical composition for treating at least one of asthma and eosinophilic sinusitis.

140. Use of the antibody composition according to the above 91 for producing a pharmaceutical composition for treating an allergic disease.

141. Use of the antibody composition according to the above 92 for producing a pharmaceutical composition for treating langerhans cell histiocytosis (LCH).

142. Use of the antibody composition according to the above 93 for producing a pharmaceutical composition for treating systemic lupus erythematosus.

143. Use of the antibody composition according to the above 94 for producing a pharmaceutical composition for treating idiopathic pulmonary fibrosis.

144. Use of the antibody composition according to the above 95 for producing a pharmaceutical composition for treating a cancer.

145. Use of the antibody composition according to the above 96 or 97 for producing a pharmaceutical composition

for treating systemic lupus erythematosus.

146. Use of the antibody composition according to the above 98 for producing a pharmaceutical composition for treating at least one selected from an autoimmune disease, arteriosclerosis, and an ischemic heart disease.

147. Use of the antibody composition according to the above 99 for producing a pharmaceutical composition for treating at least one selected from various fibrotic diseases such as idiopathic pulmonary fibrosis, systemic sclerosis, and hepatic cirrhosis.

148. Use of the antibody composition according to the above 100 for producing a pharmaceutical composition for treating idiopathic pulmonary fibrosis.

149. Use of the antibody composition according to the above 101 for producing a pharmaceutical composition for treating at least one selected from neutrophilic asthma, chronic obstructive pulmonary disease, and Alzheimer's disease.

150. Use of the antibody composition according to the above 102 for producing a pharmaceutical composition for treating at least one selected from Crohn's disease, rheumatoid arthritis, and asthma.

151. Use of the antibody composition according to the above 103 for producing a pharmaceutical composition for treating at least one of multiple sclerosis and neuromyelitis optica.

152. Use of the antibody composition according to the above 104 or 105 for producing a pharmaceutical composition for treating systemic lupus erythematosus.

153. Use of the antibody composition according to the above 106 for producing a pharmaceutical composition for treating multiple sclerosis.

154. Use of the antibody composition according to the above 107 for producing a pharmaceutical composition for treating hepatic cirrhosis.

155. Use of the antibody composition according to the above 108 for producing a pharmaceutical composition for treating systemic lupus erythematosus.

156. Use of the antibody composition according to the above 109 for producing a pharmaceutical composition for treating a mast cell activation syndrome (MCAS) including at least one selected from urticaria, a food allergy, and mastocytosis.

157. Use of the antibody composition according to the above 110 for producing a pharmaceutical composition for treating a mast cell activation syndrome (MCAS) including at least one selected from urticaria, a food allergy, and mastocytosis.

158. Use of the antibody composition according to the above 111 for producing a pharmaceutical composition for treating multiple sclerosis.

159. Use of the antibody composition according to the above 112 for producing a pharmaceutical composition for treating at least one selected from multiple sclerosis, type 1 diabetes mellitus, and rheumatoid arthritis.

160. Use of the antibody composition according to the above 113 for producing a pharmaceutical composition for treating at least one selected from secondary progressive multiple sclerosis (SPMS), rheumatoid arthritis for which anti-TNF therapy is not effective, transplantation, and systemic lupus erythematosus.

161. Use of the antibody composition according to the above 114 for producing a pharmaceutical composition for treating at least one selected from secondary progressive multiple sclerosis (SPMS), rheumatoid arthritis for which anti-TNF therapy is not effective, transplantation, and systemic lupus erythematosus.

162. Use of the antibody composition according to the above 115 for producing a pharmaceutical composition for treating asthma.

163. Use of the antibody composition according to the above 116 for producing a pharmaceutical composition for treating systemic scleroderma.

164. Use of the antibody composition according to the above 117 for producing a pharmaceutical composition for treating at least one of vitiligo and psoriasis.

165. Use of the antibody composition according to the above 118 for producing a pharmaceutical composition for treating at least one of vitiligo and psoriasis.

166. Use of the antibody composition according to the above 119 for producing a pharmaceutical composition for treating at least one of vitiligo and psoriasis.

167. Use of the antibody composition according to the above 120 for producing a pharmaceutical composition for treating at least one of vitiligo and psoriasis.

168. Use of the antibody composition according to the above 121 for producing a pharmaceutical composition for treating at least one of ANCA (antineutrophil cytoplasmic antibody)-associated vasculitis and systemic lupus erythematosus.

169. Use of the antibody composition according to the above 122 for producing a pharmaceutical composition for treating a T cell-dependent immune-related disease.

170. Use of the antibody composition according to the above 123 for producing a pharmaceutical composition for treating an immune-related disease.

171. Use of the antibody composition according to the above 124 for producing a pharmaceutical composition for treating psoriatic arthritis.
172. Use of the antibody composition according to the above 125 for producing a pharmaceutical composition for treating an autoimmune disease including at least one of Sjogren's syndrome and psoriasis.
173. Use of the antibody composition according to the above 77 for treating rheumatoid arthritis.
174. Use of the antibody composition according to the above 78 for treating celiac disease.
175. Use of the antibody composition according to the above 79 for treating an inflammatory bowel disease.
176. Use of the antibody composition according to the above 80 for treating at least one of multiple sclerosis and an inflammatory bowel disease.
177. Use of the antibody composition according to the above 81 for treating at least one of multiple sclerosis and an inflammatory bowel disease.
178. Use of the antibody composition according to the above 82 for treating an allergic disease.
179. Use of the antibody composition according to the above 83 for treating primary myelofibrosis.
180. Use of the antibody composition according to the above 84 for treating at least one selected from an autoimmune disease, arteriosclerosis, and an ischemic heart disease.
181. Use of the antibody composition according to the above 85 for treating systemic lupus erythematosus.
182. Use of the antibody composition according to the above 86 for treating an allergic disease.
183. Use of the antibody composition according to the above 87 for treating psoriasis.
184. Use of the antibody composition according to the above 88 for treating an allergic disease.
185. Use of the antibody composition according to the above 89 for treating at least one selected from asthma, eosinophilic sinusitis, and atopic dermatitis.
186. Use of the antibody composition according to the above 90 for treating at least one of asthma and eosinophilic sinusitis.
187. Use of the antibody composition according to the above 91 for treating an allergic disease.
188. Use of the antibody composition according to the above 92 for treating langerhans cell histiocytosis (LCH).
189. Use of the antibody composition according to the above 93 for treating systemic lupus erythematosus.
190. Use of the antibody composition according to the above 94 for treating idiopathic pulmonary fibrosis.
191. Use of the antibody composition according to the above 95 for treating a cancer.
192. Use of the antibody composition according to the above 96 or 97 for treating systemic lupus erythematosus.
193. Use of the antibody composition according to the above 98 for treating at least one selected from an autoimmune disease, arteriosclerosis, and an ischemic heart disease.
194. Use of the antibody composition according to the above 99 for treating a fibrotic disease including at least one selected from idiopathic pulmonary fibrosis, systemic sclerosis, and hepatic cirrhosis.
195. Use of the antibody composition according to the above 100 for treating idiopathic pulmonary fibrosis.
196. Use of the antibody composition according to the above 101 for treating at least one selected from neutrophilic asthma, chronic obstructive pulmonary disease, and Alzheimer's disease.
197. Use of the antibody composition according to the above 102 for treating at least one selected from Crohn's disease, rheumatoid arthritis, and asthma.
198. Use of the antibody composition according to the above 103 for treating at least one of multiple sclerosis and neuromyelitis optica.
199. Use of the antibody composition according to the above 104 or 105 for treating systemic lupus erythematosus.
200. Use of the antibody composition according to the above 106 for treating multiple sclerosis.
201. Use of the antibody composition according to the above 107 for treating hepatic cirrhosis.
202. Use of the antibody composition according to the above 108 for treating systemic lupus erythematosus.
203. Use of the antibody composition according to the above 109 for treating a mast cell activation syndrome (MCAS) including at least one selected from urticaria, a food allergy, and mastocytosis.
204. Use of the antibody composition according to the above 110 for treating a mast cell activation syndrome (MCAS) including at least one selected from urticaria, a food allergy, and mastocytosis.
205. Use of the antibody composition according to the above 111 for treating multiple sclerosis.
206. Use of the antibody composition according to the above 112 for treating at least one selected from multiple sclerosis, type 1 diabetes mellitus, and rheumatoid arthritis.
207. Use of the antibody composition according to the above 113 for treating at least one selected from secondary progressive multiple sclerosis (SPMS), rheumatoid arthritis for which anti-TNF therapy is not effective, transplantation, and systemic lupus erythematosus.
208. Use of the antibody composition according to the above 114 for treating at least one selected from secondary progressive multiple sclerosis (SPMS), rheumatoid arthritis for which anti-TNF therapy is not effective, transplantation, and systemic lupus erythematosus.
209. Use of the antibody composition according to the above 115 for treating asthma.

210. Use of the antibody composition according to the above 116 for treating systemic scleroderma.
211. Use of the antibody composition according to the above 117 for treating at least one of vitiligo and psoriasis.
212. Use of the antibody composition according to the above 118 for treating at least one of vitiligo and psoriasis.
213. Use of the antibody composition according to the above 119 for treating at least one of vitiligo and psoriasis.
214. Use of the antibody composition according to the above 120 for treating at least one of vitiligo and psoriasis.
215. Use of the antibody composition according to the above 121 for treating at least one of ANCA-associated vasculitis and systemic lupus erythematosus.
216. Use of the antibody composition according to the above 122 for treating a T cell-dependent immune-related disease.
217. Use of the antibody composition according to the above 123 for treating an immune-related disease.
218. Use of the antibody composition according to the above 124 for treating psoriatic arthritis.
219. Use of the antibody composition according to the above 125 for treating an autoimmune disease including at least one of Sjogren's syndrome and psoriasis.
220. The antibody composition according to the above 77 for use in a treatment of rheumatoid arthritis.
221. The antibody composition according to the above 78 for use in a treatment of celiac disease.
222. The antibody composition according to the above 79 for use in a treatment of an inflammatory bowel disease.
223. The antibody composition according to the above 80 for use in a treatment of at least one of multiple sclerosis and an inflammatory bowel disease.
224. The antibody composition according to the above 81 for use in a treatment of at least one of multiple sclerosis and an inflammatory bowel disease.
225. The antibody composition according to the above 82 for use in a treatment of an allergic disease.
226. The antibody composition according to the above 83 for use in a treatment of primary myelofibrosis.
227. The antibody composition according to the above 84 for use in a treatment of at least one selected from an autoimmune disease, arteriosclerosis, and an ischemic heart disease.
228. The antibody composition according to the above 85 for use in a treatment of systemic lupus erythematosus.
229. The antibody composition according to the above 86 for use in a treatment of an allergic disease.
230. The antibody composition according to the above 87 for use in a treatment of psoriasis.
231. The antibody composition according to the above 88 for use in a treatment of an allergic disease.
232. The antibody composition according to the above 89 for use in a treatment of at least one selected from asthma, eosinophilic sinusitis, and atopic dermatitis.
233. The antibody composition according to the above 90 for use in a treatment of at least one of asthma and eosinophilic sinusitis.
234. The antibody composition according to the above 91 for use in a treatment of an allergic disease.
235. The antibody composition according to the above 92 for use in a treatment of langerhans cell histiocytosis (LCH).
236. The antibody composition according to the above 93 for use in a treatment of systemic lupus erythematosus.
237. The antibody composition according to the above 94 for use in a treatment of idiopathic pulmonary fibrosis.
238. The antibody composition according to the above 95 for use in a treatment of a cancer.
239. The antibody composition according to the above 96 or 97 for use in a treatment of systemic lupus erythematosus.
240. The antibody composition according to the above 98 for use in a treatment of at least one selected from an autoimmune disease, arteriosclerosis, and an ischemic heart disease.
241. The antibody composition according to the above 99 for use in a treatment of a fibrotic disease including at least one selected from idiopathic pulmonary fibrosis, systemic sclerosis, and hepatic cirrhosis.
242. The antibody composition according to the above 100 for use in a treatment of idiopathic pulmonary fibrosis.
243. The antibody composition according to the above 101 for use in a treatment of at least one selected from neutrophilic asthma, chronic obstructive pulmonary disease, and Alzheimer's disease.
244. The antibody composition according to the above 102 for use in a treatment of at least one selected from Crohn's disease, rheumatoid arthritis, and asthma.
245. The antibody composition according to the above 103 for use in a treatment of at least one of multiple sclerosis and neuromyelitis optica.
246. The antibody composition according to the above 104 or 105 for use in a treatment of systemic lupus erythematosus.
247. The antibody composition according to the above 106 for use in a treatment of multiple sclerosis.
248. The antibody composition according to the above 107 for use in a treatment of hepatic cirrhosis.
249. The antibody composition according to the above 108 for use in a treatment of systemic lupus erythematosus.
250. The antibody composition according to the above 109 for use in a treatment of a mast cell activation syndrome (MCAS) including at least one selected from urticaria, a food allergy, and mastocytosis.
251. The antibody composition according to the above 110 for use in a treatment of a mast cell activation syndrome (MCAS) including at least one selected from urticaria, a food allergy, and mastocytosis.

252. The antibody composition according to the above 111 for use in a treatment of multiple sclerosis.

253. The antibody composition according to the above 112 for use in a treatment of at least one selected from multiple sclerosis, type 1 diabetes mellitus, and rheumatoid arthritis.

254. The antibody composition according to the above 113 for use in a treatment of at least one selected from secondary progressive multiple sclerosis (SPMS), rheumatoid arthritis for which anti-TNF therapy is not effective, transplantation, and systemic lupus erythematosus.

255. The antibody composition according to the above 114 for use in a treatment of at least one selected from secondary progressive multiple sclerosis (SPMS), rheumatoid arthritis for which anti-TNF therapy is not effective, transplantation, and systemic lupus erythematosus.

256. The antibody composition according to the above 115 for use in a treatment of asthma.

257. The antibody composition according to the above 116 for use in a treatment of systemic scleroderma.

258. The antibody composition according to the above 117 for use in a treatment of at least one of vitiligo and psoriasis.

259. The antibody composition according to the above 118 for use in a treatment of at least one of vitiligo and psoriasis.

260. The antibody composition according to the above 119 for use in a treatment of at least one of vitiligo and psoriasis.

261. The antibody composition according to the above 120 for use in a treatment of at least one of vitiligo and psoriasis.

262. The antibody composition according to the above 121 for use in a treatment of at least one of ANCA-associated vasculitis and systemic lupus erythematosus.

263. The antibody composition according to the above 122 for use in a treatment of a T cell-dependent immune-related disease.

264. The antibody composition according to the above 123 for use in a treatment of an immune-related disease.

265. The antibody composition according to the above 124 for use in a treatment of psoriatic arthritis.

266. The antibody composition according to the above 125 for use in a treatment of an autoimmune disease including at least one of Sjogren's syndrome and psoriasis.

267. A method for treating rheumatoid arthritis, including administering an effective amount of the antibody composition according to the above 77 to a subject.

268. A method for treating celiac disease, including administering an effective amount of the antibody composition according to the above 78 to a subject.

269. A method for treating an inflammatory bowel disease, including administering an effective amount of the antibody composition according to the above 79 to a subject.

270. A method for treating at least one of multiple sclerosis and an inflammatory bowel disease, including administering an effective amount of the antibody composition according to the above 80 to a subject.

271. A method for treating at least one of multiple sclerosis and an inflammatory bowel disease, including administering an effective amount of the antibody composition according to the above 81 to a subject.

272. A method for treating an allergic disease, including administering an effective amount of the antibody composition according to the above 82 to a subject.

273. A method for treating primary myelofibrosis, including administering an effective amount of the antibody composition according to the above 83 to a subject.

274. A method for treating at least one selected from an autoimmune disease, arteriosclerosis, and an ischemic heart disease, including administering an effective amount of the antibody composition according to the above 84 to a subject.

275. A method for treating systemic lupus erythematosus, including administering an effective amount of the antibody composition according to the above 85 to a subject.

276. A method for treating an allergic disease, including administering an effective amount of the antibody composition according to the above 86 to a subject.

277. A method for treating psoriasis, including administering an effective amount of the antibody composition according to the above 87 to a subject.

278. A method for treating an allergic disease, including administering an effective amount of the antibody composition according to the above 88 to a subject.

279. A method for treating at least one selected from asthma, eosinophilic sinusitis, and atopic dermatitis, including administering an effective amount of the antibody composition according to the above 89 to a subject.

280. A method for treating at least one of asthma and eosinophilic sinusitis, including administering an effective amount of the antibody composition according to the above 90 to a subject.

281. For production of a pharmaceutical composition for treating an allergic disease, including administering an effective amount of the antibody composition according to the above 91 to a subject.

282. A method for treating langerhans cell histiocytosis (LCH), including administering an effective amount of the antibody composition according to the above 92 to a subject.

283. A method for treating systemic lupus erythematosus, including administering an effective amount of the antibody composition according to the above 93 to a subject.

284. A method for treating idiopathic pulmonary fibrosis, including administering an effective amount of the antibody composition according to the above 94 to a subject.
285. A method for treating a cancer, including administering an effective amount of the antibody composition according to the above 95 to a subject.
286. A method for treating systemic lupus erythematosus, including administering an effective amount of the antibody composition according to the above 96 or 97 to a subject.
287. A method for treating at least one selected from an autoimmune disease, arteriosclerosis, and an ischemic heart disease, including administering an effective amount of the antibody composition according to the above 98 to a subject.
288. A method for treating a fibrotic disease including at least one selected from idiopathic pulmonary fibrosis, systemic sclerosis, and hepatic cirrhosis, including administering an effective amount of the antibody composition according to the above 99 to a subject.
289. A method for treating idiopathic pulmonary fibrosis, including administering an effective amount of the antibody composition according to the above 100 to a subject.
290. A method for treating at least one selected from neutrophilic asthma, chronic obstructive pulmonary disease, and Alzheimer's disease, including administering an effective amount of the antibody composition according to the above 101 to a subject.
291. A method for treating at least one selected from Crohn's disease, rheumatoid arthritis, and asthma, including administering an effective amount of the antibody composition according to the above 102 to a subject.
292. A method for treating at least one of multiple sclerosis and neuromyelitis optica, using the antibody composition according to the above 103.
293. A method for treating systemic lupus erythematosus, including administering an effective amount of the antibody composition according to the above 104 or 105 to a subject.
294. A method for treating multiple sclerosis, including administering an effective amount of the antibody composition according to the above 106 to a subject.
295. A method for treating hepatic cirrhosis, including administering an effective amount of the antibody composition according to the above 107 to a subject.
296. A method for treating systemic lupus erythematosus, including administering an effective amount of the antibody composition according to the above 108 to a subject.
297. A method for treating a mast cell activation syndrome (MCAS) including at least one selected from urticaria, a food allergy, and mastocytosis, including administering an effective amount of the antibody composition according to the above 109 to a subject.
298. A method for treating a mast cell activation syndrome (MCAS) including at least one selected from urticaria, a food allergy, and mastocytosis, including administering an effective amount of the antibody composition according to the above 110 to a subject.
299. A method for treating multiple sclerosis, including administering an effective amount of the antibody composition according to the above 111 to a subject.
300. A method for treating at least one selected from multiple sclerosis, type 1 diabetes mellitus, and rheumatoid arthritis including administering an effective amount of the antibody composition according to the above 112 to a subject.
301. A method for treating at least one selected from secondary progressive multiple sclerosis (SPMS), rheumatoid arthritis for which anti-TNF therapy is not effective, transplantation, and systemic lupus erythematosus, including administering an effective amount of the antibody composition according to the above 113 to a subject.
302. A method for treating at least one selected from secondary progressive multiple sclerosis (SPMS), rheumatoid arthritis for which anti-TNF therapy is not effective, transplantation, and systemic lupus erythematosus, including administering an effective amount of the antibody composition according to the above 114 to a subject.
303. A method for treating asthma, including administering an effective amount of the antibody composition according to the above 115 to a subject.
304. A method for treating systemic scleroderma, including administering an effective amount of the antibody composition according to the above 116 to a subject.
305. A method for treating at least one of vitiligo and psoriasis, including administering an effective amount of the antibody composition according to the above 117 to a subject.
306. A method for treating at least one of vitiligo and psoriasis, including administering an effective amount of the antibody composition according to the above 118 to a subject.
307. A method for treating at least one of vitiligo and psoriasis, including administering an effective amount of the antibody composition according to the above 119 to a subject.
308. A method for treating at least one of vitiligo and psoriasis, including administering an effective amount of the antibody composition according to the above 120 to a subject.

309. A method for treating at least one of ANCA-associated vasculitis and systemic lupus erythematosus, including administering an effective amount of the antibody composition according to the above 121 to a subject.
310. A method for treating a T cell-dependent immune-related disease, including administering an effective amount of the antibody composition according to the above 122 to a subject.
311. A method for treating an immune-related disease, including administering an effective amount of the antibody composition according to the above 123 to a subject.
312. A method for treating psoriatic arthritis, including administering an effective amount of the antibody composition according to the above 124 to a subject.
313. A method for treating an autoimmune disease including at least one of Sjogren's syndrome and psoriasis, including administering an effective amount of the antibody composition according to the above 125 to a subject.
314. The antibody composition according to any one of the above 1 to 8, wherein the first antigen is CD64 and the second antigen is one selected from CD206, CD163, and CD68.
315. The antibody composition according to any one of the above 1 to 8, wherein the first antigen is CD163 and the second antigen is CD206 or CD68.
316. The antibody composition according to any one of the above 1 to 8, wherein the first antigen is CD206 and the second antigen is CD68.
317. The antibody composition according to any one of the above 1 to 8, wherein the first antigen is CD14 and the second antigen is one selected from CD48, CD84, CD97, and CD305.
318. The antibody composition according to any one of the above 1 to 8, wherein the first antigen is CD15 and the second antigen is one selected from CD48, CD84, CD97, and CD305.
319. The antibody composition according to any one of the above 1 to 8, wherein the first antigen is CD33 and the second antigen is one selected from CD48, CD84, CD97, and CD305.
320. The antibody composition according to any one of the above 1 to 8, wherein the first antigen is CD11b and the second antigen is one selected from CD48, CD84, CD97, and CD305.
321. The antibody composition according to any one of the above 1 to 8, wherein the first antigen is CCR4 and the second antigen is one selected from CADM1, CD30, and CD70.
322. The antibody composition according to any one of the above 1 to 8, wherein the first antigen is CD38 and the second antigen is CD138 or BCMA.
323. The antibody composition according to any one of the above 1 to 8, wherein the first antigen is BCMA and the second antigen is CD56 or CS1.
324. The antibody composition according to any one of the above 1 to 8, wherein the first antigen is CD40 ligand and the second antigen is one selected from CD36, CD62P, and CD63.
325. The antibody composition according to any one of the above 1 to 8, wherein the first antigen is TIM-3 and the second antigen is one selected from CD123, CD33, CD47, CD70, and CLEC12A.
326. The antibody composition according to any one of the above 1 to 8, wherein the first antigen is CD123 and the second antigen is one selected from CD33, CD47, CD70, and CLEC12A.
327. The antibody composition according to any one of the above 1 to 8, wherein the first antigen is CD5 and the second antigen is CD23.
328. The antibody composition according to any one of the above 1 to 8, wherein the first antigen is CD10 or CD5 and the second antigen is CD20.
329. The antibody composition according to any one of the above 1 to 8, wherein the first antigen is CD40 and the second antigen is one selected from CD80, CD86, ICOS ligand, 4-1BB ligand, OX40 ligand, CD70, GITR, PD-L1, PD-L2, B7-DC, B7H3, B7H4, B7H5, B7H6, and B7H7.
330. The antibody composition according to any one of the above 1 to 8, wherein the first antigen is PTPRS and the second antigen is one selected from IL-21R, CD38, and CD32a.
331. The antibody composition according to any one of the above 1 to 8, wherein the first antigen is OX40 and the second antigen is CD127 or CD40 ligand.
332. The antibody composition according to any one of the above 1 to 8, wherein the first antigen is OX40 and the second antigen is CD8 or NKG2D.
333. The antibody composition according to any one of the above 1 to 8, wherein the first antigen is OX40 and the second antigen is CD226.
334. The antibody composition according to any one of the above 1 to 8, wherein the first antigen is OX40 and the second antigen is CRTH2.
335. The antibody composition according to any one of the above 1 to 8, wherein the first antigen is CRTH2 and the second antigen is any one selected from CD2, CD7, and CD45.
336. A pharmaceutical composition for use in a treatment of a cancer, containing the antibody composition according to any one of the above 314 to 320.
337. A pharmaceutical composition for use in a treatment of leukemia, containing the antibody composition according

to any one of the above 321 to 328.
338. A pharmaceutical composition for use in a treatment of lymphoma, containing the antibody composition according to any one of the above 321 to 328.
339. A pharmaceutical composition for use in a treatment of an inflammatory disease, containing the antibody composition according to the above 329.
340. A pharmaceutical composition for use in a treatment of an allergic disease, containing the antibody composition according to any one of the above 331, 334, and 335.
341. A pharmaceutical composition for use in a treatment of at least one of vitiligo and psoriasis, containing the antibody composition according to the above 332.
342. A pharmaceutical composition for use in a treatment of scleroderma, containing the antibody composition according to the above 333.
343. Use of the antibody composition according to any one of the above 314 to 320 for producing a pharmaceutical composition for treating a cancer.
344. Use of the antibody composition according to any one of the above 321 to 328 for producing a pharmaceutical composition for treating leukemia.
345. Use of the antibody composition according to any one of the above 321 to 328 for producing a pharmaceutical composition for treating lymphoma.
346. Use of the antibody composition according to the above 329 for producing a pharmaceutical composition for treating an inflammatory disease.
347. Use of the antibody composition according to any one of the above 331, 334, and 335 for producing a pharmaceutical composition for treating an allergic disease.
348. Use of the antibody composition according to the above 332 for producing a pharmaceutical composition for treating at least one of vitiligo and psoriasis.
349. Use of the antibody composition according to the above 333 for producing a pharmaceutical composition for treating scleroderma.
350. Use of the antibody composition according to any one of the above 314 to 320 for treating a cancer.
351. Use of the antibody composition according to any one of the above 321 to 328 for treating leukemia.
352. Use of the antibody composition according to any one of the above 321 to 328 for treating lymphoma.
353. Use of the antibody composition according to the above 329 for treating an inflammatory disease.
354. Use of the antibody composition according to any one of the above 331, 334, and 335 for treating an allergic disease.
355. Use of the antibody composition according to the above 332 for treating at least one of vitiligo and psoriasis.
356. Use of the antibody composition according to the above 333 for treating scleroderma.
357. The antibody composition according to any one of the above 314 to 320 for use in a treatment of a cancer.
358. The antibody composition according to any one of the above 321 to 328 for use in a treatment of leukemia.
359. The antibody composition according to any one of the above 321 to 328 for use in a treatment of lymphoma.
360. The antibody composition according to the above 329 for use in a treatment of an inflammatory disease.
361. The antibody composition according to any one of the above 331, 334, and 335 for use in a treatment of an allergic disease.
362. The antibody composition according to the above 332 for use in a treatment of at least one of vitiligo and psoriasis.
363. The antibody composition according to the above 333 for use in a treatment of scleroderma.
364. A method for treating a cancer, including administering an effective amount of the antibody composition according to any one of the above 314 to 320 to a subject.
365. A method for treating leukemia, including administering an effective amount of the antibody composition according to any one of the above 321 to 328 to a subject.
366. A method for treating lymphoma, including administering an effective amount of the antibody composition according to any one of the above 321 to 328 to a subject.
367. A method for treating an inflammatory disease, including administering an effective amount of the antibody composition according to the above 329 to a subject.
368. A method for treating an allergic disease, including administering an effective amount of the antibody composition according to any one of the above 331, 334, and 335 to a subject.
369. A method for treating at least one of vitiligo and psoriasis, including administering an effective amount of the antibody composition according to the above 332 to a subject.
370. A method for treating scleroderma, including administering an effective amount of the antibody composition according to the above 333 to a subject.
371. A pharmaceutical composition for use in a treatment of rheumatoid arthritis, containing the antibody composition according to the above 77.
372. A pharmaceutical composition for use in a treatment of celiac disease, containing the antibody composition

according to the above 78.

373. A pharmaceutical composition for use in a treatment of an inflammatory bowel disease, containing the antibody composition according to the above 79.

374. A pharmaceutical composition for use in a treatment of at least one of multiple sclerosis and an inflammatory bowel disease, containing the antibody composition according to the above 80.

375. A pharmaceutical composition for use in a treatment of at least one of multiple sclerosis and an inflammatory bowel disease, containing the antibody composition according to the above 81.

376. A pharmaceutical composition for use in a treatment of an allergic disease, containing the antibody composition according to the above 82.

377. A pharmaceutical composition for use in a treatment of primary myelofibrosis, containing the antibody composition according to the above 83.

378. A pharmaceutical composition for use in a treatment of at least one selected from an autoimmune disease, arteriosclerosis, and an ischemic heart disease, containing the antibody composition according to the above 84.

379. A pharmaceutical composition for use in a treatment of systemic lupus erythematosus, containing the antibody composition according to the above 85.

380. A pharmaceutical composition for use in a treatment of an allergic disease, containing the antibody composition according to the above 86.

381. A pharmaceutical composition for use in a treatment of psoriasis, containing the antibody composition according to the above 87.

382. A pharmaceutical composition for use in a treatment of an allergic disease, containing the antibody composition according to the above 88.

383. A pharmaceutical composition for use in a treatment of at least one selected from asthma, eosinophilic sinusitis, and atopic dermatitis, containing the antibody composition according to the above 89.

384. A pharmaceutical composition for use in a treatment of at least one of asthma and eosinophilic sinusitis, containing the antibody composition according to the above 90.

385. A pharmaceutical composition for use in a treatment of an allergic disease, containing the antibody composition according to the above 91.

386. A pharmaceutical composition for use in a treatment of langerhans cell histiocytosis (LCH), containing the antibody composition according to the above 92.

387. A pharmaceutical composition for use in a treatment of systemic lupus erythematosus, containing the antibody composition according to the above 93.

388. A pharmaceutical composition for use in a treatment of idiopathic pulmonary fibrosis, containing the antibody composition according to the above 94.

389. A pharmaceutical composition for use in a treatment of a cancer, containing the antibody composition according to the above 95.

390. A pharmaceutical composition for use in a treatment of systemic lupus erythematosus, containing the antibody composition according to the above 96 or 97.

391. A pharmaceutical composition for use in a treatment of at least one selected from an autoimmune disease, arteriosclerosis, and an ischemic heart disease, containing the antibody composition according to the above 98.

392. A pharmaceutical composition for use in a treatment of a fibrotic disease including at least one selected from idiopathic pulmonary fibrosis, systemic sclerosis, and hepatic cirrhosis, containing the antibody composition according to the above 99.

393. A pharmaceutical composition for use in a treatment of idiopathic pulmonary fibrosis, containing the antibody composition according to the above 100.

394. A pharmaceutical composition for use in a treatment of at least one selected from neutrophilic asthma, chronic obstructive pulmonary disease, and Alzheimer’s disease, containing the antibody composition according to the above 101.

395. A pharmaceutical composition for use in a treatment of at least one selected from Crohn’s disease, rheumatoid arthritis, and asthma, containing the antibody composition according to the above 102.

396. A pharmaceutical composition for use in a treatment of at least one of multiple sclerosis and neuromyelitis optica, containing the antibody composition according to the above 103.

397. A pharmaceutical composition for use in a treatment of systemic lupus erythematosus, containing the antibody composition according to the above 104 or 105.

398. A pharmaceutical composition for use in a treatment of multiple sclerosis, containing the antibody composition according to the above 106.

399. A pharmaceutical composition for use in a treatment of hepatic cirrhosis, containing the antibody composition according to the above 107.

400. A pharmaceutical composition for use in a treatment of systemic lupus erythematosus, containing the antibody

composition according to the above 108.

401. A pharmaceutical composition for use in a treatment of a mast cell activation syndrome (MCAS) including at least one selected from urticaria, a food allergy, and mastocytosis, containing the antibody composition according to the above 109.

402. A pharmaceutical composition for use in a treatment of a mast cell activation syndrome (MCAS) including at least one selected from urticaria, a food allergy, and mastocytosis, containing the antibody composition according to the above 110.

403. A pharmaceutical composition for use in a treatment of multiple sclerosis, containing the antibody composition according to the above 111.

404. A pharmaceutical composition for use in a treatment of at least one selected from multiple sclerosis, type 1 diabetes mellitus, and rheumatoid arthritis, containing the antibody composition according to the above 112.

405. A pharmaceutical composition for use in a treatment of at least one selected from secondary progressive multiple sclerosis (SPMS), rheumatoid arthritis for which anti-TNF therapy is not effective, transplantation, and systemic lupus erythematosus, containing the antibody composition according to the above 113.

406. A pharmaceutical composition for use in a treatment of at least one selected from secondary progressive multiple sclerosis (SPMS), rheumatoid arthritis for which anti-TNF therapy is not effective, transplantation, and systemic lupus erythematosus, containing the antibody composition according to the above 114.

407. A pharmaceutical composition for use in a treatment of asthma, containing the antibody composition according to the above 115.

408. A pharmaceutical composition for use in a treatment of systemic scleroderma, containing the antibody composition according to the above 116.

409. A pharmaceutical composition for use in a treatment of at least one of vitiligo and psoriasis, containing the antibody composition according to the above 117.

410. A pharmaceutical composition for use in a treatment of at least one of vitiligo and psoriasis, containing the antibody composition according to the above 118.

411. A pharmaceutical composition for use in a treatment of at least one of vitiligo and psoriasis, containing the antibody composition according to the above 119.

412. A pharmaceutical composition for use in a treatment of at least one of vitiligo and psoriasis, containing the antibody composition according to the above 120.

413. A pharmaceutical composition for use in a treatment of at least one of ANCA-associated vasculitis and systemic lupus erythematosus, containing the antibody composition according to the above 121.

414. A pharmaceutical composition for use in a treatment of a T cell-dependent immune-related disease, containing the antibody composition according to the above 122.

415. A pharmaceutical composition for use in a treatment of an immune-related disease, containing the antibody composition according to the above 123.

416. A pharmaceutical composition for use in a treatment of psoriatic arthritis, containing the antibody composition according to the above 124.

417. A pharmaceutical composition for use in a treatment of an autoimmune disease including at least one of Sjogren's syndrome and psoriasis, containing the antibody composition according to the above 125.

## ADVANTAGEOUS EFFECTS OF INVENTION

The antibody composition of the present invention includes first and second IgG half-molecules that are two types of IgG half-molecules, which have antigen-binding domains for two types of antigens that are different from each other, and in which a CD16a-binding activity in CD16a-binding domains that are different from each other is attenuated. Accordingly, even if an antibody structure of a homo assembly is constituted by the first IgG half-molecules or the second IgG half-molecules, it cannot bind to CD 16a, and therefore cannot exhibit an activity of an antibody. On the other hand, when an antibody structure of a hetero assembly is constituted by the first and second IgG half-molecules, it can bind to CD 16a through the second CD16a-binding domain in the first IgG half-molecule and the first CD16a-binding domain in the second IgG half-molecule.

Further, a hinge domain in the first and second IgG half-molecules is altered so as not to form a disulfide bond. Accordingly, an inter-H chain disulfide bond is not formed between the first IgG half-molecule and the second IgG half-molecule. Therefore, when the first and second IgG half-molecules are mixed, it becomes possible to make the first and second IgG half-molecules exist in an equilibrium state of an assembly or half-molecules. Accordingly, the antibody composition of the present invention can exhibit an effector function more specifically for a double-positive cell and damage the cell as compared with a single-positive cell.

In addition, in the antibody composition of the present invention, a specific amino acid alteration is introduced into a CD16a-binding domain or another Fc region in the first and second IgG half-molecules. Due to this, the hetero

assembly formed by the first and second IgG half-molecules exhibits an enhanced effector function for a double-positive cell and/or controlled biokinetics.

BRIEF DESCRIPTION OF DRAWINGS

[Fig. 1] Fig. 1 is a schematic diagram showing the structures of an antibody, VHH-Fc, and scFv-Fc.

[Fig. 2A] Fig. 2A shows a schematic diagram of the structure of a general bispecific antibody.

[Fig. 2B] Fig. 2B shows a schematic diagram of a binding mode by a general bispecific antibody.

[Fig. 3] Fig. 3 shows a schematic diagram of an embodiment of a binding mode by an antibody composition of the present invention.

[Fig. 4] Fig. 4 shows a schematic diagram of an embodiment of the structure of the antibody composition of the present invention.

[Fig. 5] Fig. 5 shows a schematic diagram of a binding mode between normal human IgG1 and CD16a.

[Fig. 6] Fig. 6 shows a schematic diagram of a binding mode between the antibody composition of the present invention and CD 16a.

[Fig. 7] Fig. 7 is a schematic diagram showing a candidate site of an amino acid alteration on a format of normal human IgG1.

[Fig. 8] Fig. 8 is a view showing the ADCC activity of human IgG1 anti-CCR6 antibodies in which a CD16a-binding domain was "disrupted".

[Fig. 9] Fig. 9 is a schematic diagram of a monovalent antibody for evaluating an ADCC activity by asymmetrically introducing the alteration into only each of CH2-A and CH2-B.

[Fig. 10] Fig. 10 is a view showing the ADCC activity of CD16a-binding asymmetrically altered monovalent antibodies.

[Fig. 11] Fig. 11 is a schematic diagram of an IgG1114_AA_AAA_D265A (/P329Y)-type IgG half-molecule used in Example 3.

[Fig. 12] Fig. 12 shows the results of evaluating the purification degree by SDS-PAGE of anti-CD4 antibody half-molecules and anti-CD70 antibody half-molecules.

[Fig. 13] Fig. 13 shows the results of measuring the expression of CD4 antigen and CD70 antigen in CD4 single-positive cells, CD70 single-positive cells, and CD4/CD70 double-positive cells.

[Fig. 14] Fig. 14 is a view showing the ADCC activity of anti-CD4 IgG1 and anti-CD70 IgG1 and half-molecules against CD4 single-positive cells, CD70 single-positive cells, and CD4/CD70 double-positive cells.

[Fig. 15A] Fig. 15A is a view showing the ADCC activity when adding each half-molecule against CD4/CD70 double-positive cells (TL-Oml), CD70 single-positive cells (MT-1), and CD4 single-positive cells (CD4/EL-4).

[Fig. 15B] Fig. 15B is a view showing the ADCC activity when adding each half-molecule against CD4/CD70 double-positive cells (TL-Oml), CD70 single-positive cells (MT-1), and CD4 single-positive cells (CD4/EL-4).

[Fig. 15C] Fig. 15C is a view showing the ADCC activity when adding each half-molecule against CD4/CD70 double-positive cells (TL-Oml), CD70 single-positive cells (MT-1), and CD4 single-positive cells (CD4/EL-4).

[Fig. 15D] Fig. 15D is a view showing the ADCC activity when adding each half-molecule against CD4/CD70 double-positive cells (TL-Oml), CD70 single-positive cells (MT-1), and CD4 single-positive cells (CD4/EL-4).

[Fig. 15E] Fig. 15E is a view showing the ADCC activity when adding each half-molecule against CD4/CD70 double-positive cells (TL-Om1), CD70 single-positive cells (MT-1), and CD4 single-positive cells (CD4/EL-4).

[Fig. 15F] Fig. 15F is a view showing the ADCC activity when adding each half-molecule against CD4/CD70 double-positive cells (TL-Oml), CD70 single-positive cells (MT-1), and CD4 single-positive cells (CD4/EL-4).

[Fig. 15G] Fig. 15G is a view showing the ADCC activity when adding each half-molecule against CD4/CD70 double-positive cells (TL-Oml), CD70 single-positive cells (MT-1), and CD4 single-positive cells (CD4/EL-4).

[Fig. 15H] Fig. 15H is a view showing the ADCC activity when adding each half-molecule, anti-CD4 IgG1, or anti-CD70 IgG1 against CD4/CD70 double-positive cells (TL-Om1), CD70 single-positive cells (MT-1), and CD4 single-positive cells (CD4/EL-4).

[Fig. 16A] Fig. 16A is a view showing the ADCC activity when adding each half-molecule against CD4/CD70 double-positive cells (TL-Oml), CD70 single-positive cells (MT-1), and CD4 single-positive cells (CD4/EL-4). The name of the amino acid alteration introduced into the CH3 domain of the added half-molecule is shown above each graph.

[Fig. 16B] Fig. 16B is a view showing the ADCC activity when adding each half-molecule, anti-CD4 IgG1, or anti-CD70 IgG1 against CD4/CD70 double-positive cells (TL-Om1), CD70 single-positive cells (MT-1), and CD4 single-positive cells (CD4/EL-4). The abbreviation of the added antibody (in the case of the half-molecule, the name of the amino acid alteration introduced into the CH3 domain) is shown above each graph.

[Fig. 17] Fig. 17 is a view showing the changes in serum antibody concentration when 1 mg/kg of an anti-DNP antibody (wild type) or each mixture of half-molecules was administered to mice.

[Fig. 18A] Fig. 18A is a view showing the ADCC activity in an immunoglobulin-added system when adding each half-molecule against CD4/CD70 double-positive cells (TL-Om1), CD70 single-positive cells (MT-1), and CD4 single-positive cells (CD4/EL-4). As the abbreviation of the added half-molecule, the name of the CD16a-binding enhancing amino acid alteration is shown above each graph.

[Fig. 18B] Fig. 18B is a view showing the ADCC activity in an immunoglobulin-added system when adding each half-molecule against CD4/CD70 double-positive cells (TL-Om1), CD70 single-positive cells (MT-1), and CD4 single-positive cells (CD4/EL-4). As the abbreviation of the added half-molecule, the name of the CD16a-binding enhancing amino acid alteration is shown above each graph.

[Fig. 18C] Fig. 18C is a view showing the ADCC activity in an immunoglobulin-added system when adding a half-molecule, anti-CD4 IgG1, or anti-CD70 IgG1 against CD4/CD70 double-positive cells (TL-Oml), CD70 single-positive cells (MT-1), and CD4 single-positive cells (CD4/EL-4). The abbreviation of the added antibody (in the case of the half-molecule, the name of the CD16a-binding enhancing amino acid alteration) is shown above each graph.

[Fig. 19A] Fig. 19A is a view showing the ADCC activity (cell viability) of each half-molecule in a human blood reconstitution system. The horizontal axis represents the concentration of the added antibody (in the case of the half-molecule, the total concentration of the mixture), and the vertical axis represents the existence ratio (%) of each target cell (shown above the graph) in the total CD3-positive T cells or the total lymphocytes when no antibody was added.

[Fig. 19B] Fig. 19B is a view showing the ADCC activity (cell viability) of each half-molecule in a human blood reconstitution system. The horizontal axis represents the concentration of the antibody (in the case of the half-molecule, the total concentration of the mixture), and the vertical axis represents the existence ratio (%) of each target cell (shown above the graph) in the total CD3-positive T cells or the total lymphocytes when no antibody was added.

[Fig. 20] Fig. 20 is a view showing the ADCC activity when adding each half-molecule, anti-CD4 IgG1, or anti-CCR4 IgG1 against CD4/CCR4 double-positive cells (TL-Om1), CCR4 single-positive cells (MT-1), and CD4 single-positive cells (CD4/EL-4).

[Fig. 21] Fig. 21 is a view showing the ADCC activity when adding each half-molecule, anti-CD4 IgG1, or anti-CCR4 IgG1 against CD4/CCR4 double-positive cells (HH), CCR4 single-positive cells (L428), and CD4 single-positive cells (TALL1).

[Fig. 22] Fig. 22 is a view showing the ADCC activity when adding each half-molecule, anti-CCR4 IgG1, or anti-CD70 IgG1 against CCR4/CD70 double-positive cells (L428), CCR4 single-positive cells (PEER), and CD70 single-positive cells (SUP-M2).

[Fig. 23] Fig. 23 is a view showing the binding activity to CD16a of each half-molecule and an anti-DNA antibody.

## DESCRIPTION OF EMBODIMENTS

### 1. Structure of Antibody Composition

In the present invention, an antibody molecule is also referred to as an immunoglobulin (hereinafter referred to as Ig), and a human antibody is classified into isotypes of IgA1, IgA2, IgD, IgE, IgG1, IgG2, IgG3, IgG4, and IgM according to a difference in molecular structure. IgG1, IgG2, IgG3, and IgG4 having relatively high homology of an amino acid sequence are also collectively referred to as IgG.

An antibody molecule is constituted by polypeptides called heavy chain (hereinafter referred to as H chain) and light chain (hereinafter referred to as L chain). An antibody is a tetrameric protein composed of two H chains and two L chains.

Further, the H chain is constituted by each region of an H chain variable region (also referred to as VH) and an H chain constant region (also referred to as CH) from the N terminal side and the L chain is constituted by each region of an L chain variable region (also referred to as VL) and an L chain constant region (also referred to as CL) from the N terminal side. In the CH, $\alpha$, $\delta$, $\varepsilon$, $\gamma$ and $\mu$ chains are known for each subclass. In the CL, $\lambda$ and $\kappa$ are known.

A domain is a functional structural unit that constitutes each polypeptide of an antibody molecule. Further, an Fc region (Fc) in the present invention refers to a partial sequence and a partial structure of an H chain constant region composed of a hinge domain, a CH2 domain, and a CH3 domain.

The CH is constituted by each domain of a CH1 domain, a hinge domain, a CH2 domain, and a CH3 domain from the N terminal side. The CH1 domain, the hinge domain, the CH2 domain, the CH3 domain, and the Fc in the present invention can be specified by the amino acid residue number from the N terminus according to the EU index [Kabat et al., Sequences of Proteins of Immunological Interest, US Dept. Health and Human Services (1991)].

Specifically, CH1 is specified as an amino acid sequence at positions 118 to 215 according to the EU index, the hinge is specified as an amino acid sequence at positions 216 to 230 according to the EU index, CH2 is specified as an amino acid sequence at positions 231 to 340 according to the EU index, and CH3 is specified as an amino acid

sequence at positions 341 to 447 according to the EU index.

As the IgG in the present invention, an artificial subspecies of an altered molecule that includes at least an antigen-binding domain and Fc and has a similar function to IgG is also included. Specifically, an altered molecule obtained by substitution, deletion or addition, or modification of an amino acid residue of IgG, and further an adduct of a polypeptide or a domain, and the like are included. Further, those obtained by substitution of a part or the whole of an antigen-binding site composed of VH, VL, or Fab of IgG with another antigen-binding domain are also included. Specifically, single-chain Fv (scFv)-Fc, a variable domain of heavy chain of heavy chain antibody (VHH)-Fc shown in Fig. 1, and the like are also included (Brinkmann U et al., MABS 2017, 9: 182-212; Fernandes CFC et al., Frontiers in Immunology 2017, 8: 653).

As the antibody in the present invention, other than a monoclonal antibody obtained from a hybridoma, a recombinant antibody produced by a genetic recombination technique is also included. As the recombinant antibody, a chimeric antibody obtained by binding a human antibody constant region to a non-human antibody variable region, a humanized antibody, and a human antibody produced using a human antibody-producing animal, or the like are included.

The chimeric antibody can be produced by obtaining cDNAs encoding VH and VL from a hybridoma that produces a monoclonal antibody and is derived from a non-human animal cell, inserting each of the cDNAs into an expression vector for an animal cell having DNAs encoding CH and CL of a human antibody, thereby constructing a human chimeric antibody expression vector, and introducing the vector into an animal cell and expressing the antibody.

The humanized antibody refers to an antibody produced by inserting complementarity determining regions (hereinafter abbreviated as CDRs) of the H chain and the L chain of a non-human antibody variable region into a framework region (hereinafter abbreviated as FR) of a human antibody variable region.

The humanized antibody (or a CDR-grafted antibody) can be produced by the following method. A cDNA encoding the amino acid sequence of a CDR of VH of a non-human animal antibody and the amino acid sequence of VH composed of the amino acid sequence of FR of VH of an arbitrary human antibody, and a cDNA encoding the amino acid sequence of a CDR of VL of a non-human animal antibody and the amino acid sequence of VL composed of the amino acid sequence of FR of VL of an arbitrary human antibody are constructed. Each of the cDNAs is inserted into an expression vector for an animal cell having DNAs encoding CH and CL of a human antibody to construct a humanized antibody expression vector, and introducing the vector into an animal cell and expressing the antibody, whereby the humanized antibody can be produced.

The human antibody originally refers to an antibody that can be naturally present in the human body or an antibody composed of an amino acid sequence encoded by a human gene, but also includes antibodies that are obtained from human antibody phage libraries, cloning of immortalized human peripheral blood lymphocytes, or human antibody-producing transgenic animals, which are produced by recent advancement of genetic engineering, cellular engineering, and developmental engineering technologies, and the like.

The human antibody can be obtained by immunizing a mouse having a human immunoglobulin gene (Tomizuka K. et al., Proc Natl Acad Sci USA. 97, 722-7, 2000) with a desired antigen. Further, the human antibody can be obtained without immunization by selecting a human antibody having a desired binding activity using a phage display library obtained by amplifying antibody genes from human-derived B cells (Winter G. et al., Annu Rev Immunol. 12: 433-55, 1994).

Further, the human antibody can be obtained by producing a cell that produces a human antibody having a desired binding activity by immortalizing a human B cell using EB virus (Rosen A. et al., Nature 267, 52-54, 1977).

As for the antibody that is present in the human body, for example, a lymphocyte isolated from human peripheral blood is infected with EB virus or the like so as to immortalize it, followed by cloning, whereby a lymphocyte that produces the antibody can be cultured, and the antibody can be purified from the culture.

The human antibody phage library is a library of phages in which an antibody fragment such as Fab or scFv is expressed on the surface thereof by inserting an antibody gene prepared from a human B cell into a phage gene. It is possible to collect phages that express an antibody fragment having a desired antigen-binding activity from the library using a binding activity to a substrate onto which an antigen is immobilized as an index. The antibody fragment further can also be converted into a human antibody molecule composed of two complete H chains and two complete L chains using a genetic engineering technique.

The human antibody-producing transgenic animal refers to an animal in which a human antibody gene is incorporated into the chromosome of a host animal. Specifically, the human antibody-producing transgenic animal can be produced by introducing a human antibody gene into a mouse ES cell, implanting the ES cell to an early embryo of another mouse, and then allowing the embryo to develop.

As for a method for producing a human antibody from a human antibody-producing transgenic animal, a human antibody-producing hybridoma is obtained by a general method for producing a hybridoma performed for mammals other than humans, and cultured, whereby a human antibody can be produced and accumulated in the culture.

The amino acid sequences of VH and VL may be any of the amino acid sequences of VH and VL of a human antibody, the amino acid sequences of VH and VL of a non-human animal antibody, the amino acid sequences of a humanized antibody in which a CDR of a non-human animal antibody is implanted to a framework of a human antibody,

and the amino acid sequences of VH and VL derived from a human antibody.

Specifically, the amino acid sequences of VH and VL of a non-human animal antibody, a humanized antibody, and a human antibody that a hybridoma or an antibody-producing cell produces, and the like are exemplified.

The amino acid sequence of CL may be either the amino acid sequence of a human antibody or the amino acid sequence of a non-human animal antibody, but is preferably the amino acid sequence of Cκ or Cλ of a human antibody.

The CH may be any as long as it belongs to an immunoglobulin, but preferably, any of γ1 (IgG1), γ2 (IgG2), γ3 (IgG3), and γ4 (IgG4) subclasses belonging to the human IgG class can be used.

The "antigen-binding domain" may be a binding protein recombined by utilizing a binding domain of a known binding molecule such as an antibody, a ligand, a receptor, or the like, and specific examples include a recombinant protein including a CDR of an antibody that binds to each antigen, an antibody variable region including a CDR, a recombinant protein including an antibody variable region and a binding domain of a ligand that binds to each antigen, and the like. Among these, the antigen-binding domain is preferably an antibody variable region in the present invention.

The antibody composition of the present invention is an antibody composition against a first antigen and a second antigen that are different from each other, including a first IgG half-molecule and a second IgG half-molecule, and has the following properties 1) to 6).

1) Each of the first IgG half-molecule and the second IgG half-molecule is composed of one L chain and one H chain, and the H chain includes an H chain variable region, a hinge domain, and CH1, CH2, and CH3 domains, and has first and second CD16a-binding domains that are different from each other in the CH2 domain.
2) The hinge domain of each of the first IgG half-molecule and the second IgG half-molecule includes an alteration of a substitution or a deletion of a part or the whole or a modification so as not to form an inter-H chain disulfide bond between the first IgG half-molecule and the second IgG half-molecule.
3) The first IgG half-molecule includes an antigen-binding domain that binds to the first antigen, and includes an alteration for attenuating a CD 16a-binding activity in the first CD 16a-binding domain.
4) The second IgG half-molecule includes an antigen-binding domain that binds to the second antigen, and includes an alteration for attenuating a CD 16a-binding activity in the second CD16a-binding domain.
5) Each of the first IgG half-molecule and the second IgG half-molecule includes an alteration for enhancing a CD16a-binding activity.
6) At least one of the first IgG half-molecule and the second IgG half-molecule includes an alteration for attenuating an inter-CH3 domain interaction as compared with an inter-CH3 domain interaction of the IgG1 subclass.

The "antibody composition against a first antigen and a second antigen that are different from each other" denotes a composition containing an IgG half-molecule against a first antigen and an IgG half-molecule against a second antigen.

As a possible state of the IgG half-molecule in the antibody composition of the present invention, for example, a monomeric half-molecule and an assembly composed of half-molecules are exemplified. Examples of the half-molecule include the first IgG half-molecule and the second IgG half-molecule. Examples of the assembly include an assembly of the first IgG half-molecules, an assembly of the second IgG half-molecules, and an assembly of the first IgG half-molecule and the second IgG half-molecule. Specifically, the first and second half-molecules contained in the antibody composition may be in a state of equilibrium between an assembly composed of the first IgG half-molecules and an assembly composed of the second IgG half-molecules, and an assembly composed of the first IgG half-molecule and the second IgG half-molecule.

The "antibody composition against a first antigen and a second antigen that are different from each other" of the present invention also includes an antibody composition containing an IgG half-molecule against a first antigenic determinant (epitope) and an IgG half-molecule against a second antigenic determinant (epitope) in the same antigen.

The "IgG half-molecule" is a dimeric protein composed of one L chain and one H chain, and the H chain includes an H chain variable region, CH1 to CH3 domains, and a hinge domain. In the CH2 domain of the H chain of the IgG half-molecule, two CD16a-binding domains (first and second CD16a-binding domains) that are different from each other are present.

Further, the "IgG half-molecule" also includes a half-molecule of an artificial subspecies of altered molecule that includes an antigen-binding domain and Fc and has a similar function to IgG. Specifically, a half-molecule of an altered molecule obtained by substitution, deletion or addition, or modification of an amino acid of IgG, and further a half-molecule of an adduct of a polypeptide or a domain, and the like are included. Further, half-molecules of those obtained by substitution of a part or the whole of an antigen-binding site composed of VH, VL, or Fab of IgG with another antigen-binding domain, specifically, half-molecules of single-chain Fv (scFv)-Fc and VHH-Fc shown in Fig. 1, and the like are also included.

The "CD16a-binding domain" refers to a domain that is present in Fc of IgG and binds to CD16a. The CD16a-binding domain on Fc of IgG1 is present at two sites due to the point symmetry of the structure of Fc, and in the CH2 domain constituting Fc and composed of two polypeptide chains, contact with CD 16a is made in regions that are different from each other (see Fig. 5).

Examples of the first CD16a-binding domain include a domain including at least one selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index.

When the immunoglobulin subclass of the CH2 domain is IgG1, examples of the first CD16a-binding domain include a domain including at least one selected from amino acid residues of Leu at position 235, Gly at position 236, Gly at position 237, Pro at position 238, Ser at position 239, Asp at position 265, Val at position 266, Ser at position 267, His at position 268, Glu at position 269, Glu at position 294, Gln at position 295, Tyr at position 296, Asn at position 297, Ser at position 298, Thr at position 299, Arg at position 301, Asn at position 325, Ala at position 327, and Ile at position 332 numbered according to the EU index.

Examples of the second CD16a-binding domain include a domain including at least one selected from amino acid residues at position 235, position 236, position 237, position 326, position 327, position 328, position 329, and position 330 numbered according to the EU index.

When the immunoglobulin subclass of the CH2 domain is IgG1, examples of the second CD16a-binding domain include a domain including at least one selected from amino acid residues of Leu at position 235, Gly at position 236, Gly at position 237, Lys at position 326, Ala at position 327, Leu at position 328, Pro at position 329, and Ala at position 330 numbered according to the EU index.

The "CD16a-binding activity" refers to an activity of binding of Fc of IgG to CD 16a. The CD16a-binding activity of the IgG half-molecule can be confirmed by combining two IgG half-molecules to form IgG, and thereafter allowing the IgG to react with a recombinant CD16a protein, and measuring the binding activity (US Patent Application Publication No. 2004/0259150).

As one aspect of the method for measuring the CD16a-binding activity of the IgG half-molecule, for example, the binding activity to CD16a expressed on a cell membrane can be measured by a fluorescent antibody method (Cancer Immunol. Immunother, 36, 373, 1993), or the like. Further, for example, the binding activity to a purified CD16a protein can be measured according to an immunological quantitative method such as Western staining, RIA (radioimmunoassay), VIA (viroimmunoassay), EIA (enzymoimmunoassay), FIA (fluoroimmunoassay), or MIA (metalloimmunoassay) described in literature [Monoclonal Antibodies: Principles and Applications, Wiley-Liss, Inc., 1995; Enzyme Immunoassay, 3rd Ed., IGAKU-SHOIN Ltd. (1987); Enzyme Antibody Technique, Revised Edition, Gakusai Kikaku (1985)], or the like.

Specifically, for example, when the binding activity to the purified CD16a protein is quantified by EIA, the quantification can be carried out as follows. FcγRIIIa is immobilized on a plastic plate for EIA, and is allowed to react with a sample containing the antibody composition. Subsequently, the amount of the antibody composition bound using an appropriate secondary antibody is measured.

The binding activity to the purified CD16a protein can also be measured by measurement using a biosensor [for example, BIAcore (manufactured by Biacore, Inc.)] [J. Immunol. Methods, 200, 121 (1997)] or by an isothermal titration calorimetry method [Proc. Natl. Acad. Sci. U. S. A., 97, 9026 (2000)], or the like.

Further, the CD16a-binding activity of the IgG half-molecule can also be confirmed by allowing the Fc of the IgG half-molecule to have the below-mentioned effector function (ADCC activity or the like), constituting IgG in which two molecules of the IgG half-molecule are combined, and measuring the effector function of the IgG.

The "effector function" refers to an antibody-dependent function caused via Fc of an antibody. As the effector function, for example, an ADCC activity, a CDC activity, or antibody-dependent phagocytosis (antibody-dependent cellular phagocytosis activity: ADCP activity) by a phagocyte such as a macrophage or a dendritic cell is exemplified. As a method for measuring the effector function, for example, an ADCC activity and a CDC activity can be measured using a method described in Cancer Immunol. Immunother., 36, 373 (1993).

As one aspect of the method for measuring an ADCC activity or a CDC activity, for example, the following method is exemplified. Specifically, for example, a method described later in Examples is exemplified.

1) As an effector cell, a human peripheral blood mononuclear cell (PBMC) or a cell line made to stably express human CD16a by gene transfer of human CD 16a, and a target cell are prepared using a culture medium (for example, RPMI medium). In the case of measurement of a CDC activity, a solution obtained by diluting a human complement protein to an appropriate concentration is mixed with a target cell and adjusted.
2) Into a cell culture container (for example, 96-well plate), an antibody solution, the target cells, and the effector cells are dispensed. The number ratio of the effector cells and the target cells (E/T ratio) is made constant.
3) The resultant is left to stand for about 2 to 6 hours in a $CO_2$ incubator. At the same time, a solubilizing solution (for example, an aqueous solution containing an acid, an alkali, a surfactant, or the like) is added to a 100% reaction well, and the target cells are completely lysed. After the reaction container is centrifuged, the supernatant is collected

and dispensed into an ELISA plate. A coloring solution is applied thereto to cause a reaction, and thereafter, a stopping solution is added thereto, and an absorbance (A450) is measured using a plate reader.
4) The ADCC activity (%) and the CDC activity (%) are calculated using the following formula.

$$\text{ADCC activity (\%) or CDC activity (\%)} = 100 \times (S - E - T) / (Max - T)$$

$$S = \text{absorbance of sample reaction well - absorbance of culture medium well}$$

$$E = \text{absorbance of effector well - absorbance of culture medium well}$$

$$T = \text{absorbance of target well - absorbance of culture medium well}$$

$$Max = \text{100\% reaction well - 100\% reaction control well}$$

As a kit for ADCC measurement, a known kit can be used, and for example, CytoTox 96(R) Non-Radioactive Cytotoxicity Assay (Promega) is exemplified.

The ADCC activity refers to an activity in which an antibody bound to an antigen on a target cell binds to an Fc receptor of an immune cell via Fc of the antibody so as to activate the immune cell (a natural killer cell or the like) and damage the target cell.

The Fc receptor (hereinafter sometimes referred to as FcR) is a receptor that binds to Fc of an antibody, and the binding of the antibody induces various effector functions.

The FcR corresponds to the subclass of an antibody, and IgG, IgE, IgA, and IgM bind specifically to FcγR, FcεR, FcαR, and FcμR, respectively. Further, in the FcγR, there exist FcγRI (CD64), FcγRII (CD32), and FcγRIII (CD16) subtypes, and there exist FcγRIA, FcγRIB, FcγRIC, FcγRIIA, FcγRIIB, FcγRIIC, FcγRIIIA (CD16a), and FcγRIIIB isoforms, respectively. The different types of FcyRs are present on different cells [Annu. Rev. Immunol. 9: 457-492 (1991)].

In humans, FcyRIIIB is expressed specifically in neutrophils, and FcyRIIIA is expressed in monocytes, natural killer cells (NK cells), and some T cells. Binding of the antibody via FcyRIIIA induces NK cell-dependent ADCC.

The CDC activity refers to an activity in which an antibody bound to an antigen on a target cell activates a series of cascades (complement activation pathways) composed of complement-related protein groups in the blood, and damages the target cell. In addition, a protein fragment generated by the activation of the complement can induce the migration and activation of an immune cell.

The cascade of CDC activity starts by forming a C1 complex through binding of C1q having a binding domain for Fc of an antibody to Fc, and its binding to C1r and C1s that are two serine proteases.

The state where "the CD16a-binding activity in the CD16a-binding domain is attenuated" refers to a state where the CD16a-binding activity and/or the effector function (ADCC activity or the like) of IgG obtained by combining two molecules of the IgG half-molecule to which an alteration for attenuating the CD16a-binding activity in the first and/or second CD16a-binding domain in Fc is added is attenuated as compared with the CD16a-binding activity and/or the effector function of IgG obtained by combining two molecules of the IgG half-molecule before the alteration.

As one aspect of the state where the CD16a-binding activity in the CD16a-binding domain is attenuated, for example, a state where the CD16a-binding activity of IgG obtained by combining two molecules of the IgG half-molecule to which the alteration is added is preferably 60% or less, more preferably 50% or less, further more preferably, in the following order, 40% or less, 30% or less, 20% or less, and 10% or less when the CD16a-binding activity of IgG obtained by combining two molecules of the IgG half-molecule before the alteration is taken as 100% is exemplified.

As one aspect of the state where the CD16a-binding activity in the CD16a-binding domain is attenuated, for example, a state where the ADCC activity of IgG obtained by combining two molecules of the IgG half-molecule to which the alteration is added is preferably 80% or less, more preferably 70% or less, further more preferably, in the following order, 60% or less, 50% or less, 40% or less, and 30% or less when the ADCC activity of IgG obtained by combining two molecules of the IgG half-molecule before the alteration is taken as 100% is exemplified.

The CD16a-binding activity in the CD16a-binding domain is attenuated by alteration in the CD16a-binding domain. The "alteration" in the present description refers to alteration of an amino acid residue from the wild-type amino acid sequence, and means, for example, substitution, deletion, or addition, or modification of an amino acid residue. In the present description, substitution of an amino acid residue is also abbreviated as amino acid residue substitution. The alteration for attenuating the CD16a-binding activity in the CD16a-binding domain in the present description is not

particularly limited by the type of antigen to which the antibody of the present invention binds, and an antibody having any antigen-binding domain may be combined with any alteration as long as the effect of the present invention is exhibited.

Examples of the alteration for attenuating the CD16a-binding activity in the first CD16a-binding domain include substitution of at least one amino acid residue selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index, and preferred is substitution of at least one amino acid residue selected from amino acid residues at position 235, position 238, position 239, position 265, position 266, position 267, position 268, position 269, position 294, position 295, position 296, position 297, position 298, position 299, position 301, position 325, position 327, and position 332, and more preferred is substitution of at least one amino acid residue selected from amino acid residues at position 235, position 238, position 239, position 265, position 267, position 269, position 296, position 298, position 299, and position 327. As a combination of amino acid residue substitutions, for example, amino acid residue substitutions at position 238 and position 265 in combination (hereinafter such a combination is represented by position 238/position 265, or the like), at position 238/position 267, at position 265/position 267, or at position 238/position 265/position 267 is exemplified.

When the immunoglobulin subclass of the CH2 domain is IgG1, examples of the alteration for attenuating the CD16a-binding activity in the first CD16a-binding domain include substitution of at least one amino acid residue selected from Leu at position 235, Gly at position 236, Gly at position 237, Pro at position 238, Ser at position 239, Asp at position 265, Val at position 266, Ser at position 267, His at position 268, Glu at position 269, Glu at position 294, Gln at position 295, Tyr at position 296, Asn at position 297, Ser at position 298, Thr at position 299, Arg at position 301, Asn at position 325, Ala at position 327, and Ile at position 332 numbered according to the EU index, and preferred is substitution of at least one amino acid residue selected from Leu at position 235, Pro at position 238, Ser at position 239, Asp at position 265, Val at position 266, Ser at position 267, His at position 268, Glu at position 269, Glu at position 294, Gln at position 295, Tyr at position 296, Asn at position 297, Ser at position 298, Thr at position 299, Arg at position 301, Asn at position 325, Ala at position 327, and Ile at position 332, and more preferred is substitution of at least one amino acid residue selected from Leu at position 235, Pro at position 238, Ser at position 239, Asp at position 265, Ser at position 267, Glu at position 269, Tyr at position 296, Ser at position 298, Thr at position 299, and Ala at position 327. Specifically, for example, at least one amino acid residue substitution selected from L235R, P238A, S239R, D265A, D265N, D265E, S267L, S267K, E269P, Y296P, S298E, T299A, and A327I is exemplified.

Examples of the alteration for attenuating the CD16a-binding activity in the second CD16a-binding domain include substitution of at least one amino acid residue selected from amino acid residues at position 235, position 236, position 237, position 326, position 327, position 328, position 329, and position 330 numbered according to the EU index, and preferred is substitution of at least one amino acid residue selected from amino acid residues at position 326, position 328, position 329, and position 330, and more preferred is substitution of at least one amino acid residue selected from amino acid residues at position 326, position 328, and position 329. As a combination of amino acid residue substitutions, for example, amino acid residue substitutions at position 326/position 328, at position 326/position 329, at position 328/position 329, and at position 326/position 328/position 329 are exemplified.

When the immunoglobulin subclass of the CH2 domain is IgG1, examples of the alteration for attenuating the CD16a-binding activity in the second CD16a-binding domain include substitution of at least one amino acid residue selected from Leu at position 235, Gly at position 236, Gly at position 237, Lys at position 326, Ala at position 327, Leu at position 328, Pro at position 329, and Ala at position 330 numbered according to the EU index, and preferred is substitution of at least one amino acid residue selected from Lys at position 326, Leu at position 328, Pro at position 329, and Ala at position 330, and more preferred is substitution of at least one amino acid residue selected from Lys at position 326, Leu at position 328, and Pro at position 329. Specifically, at least one amino acid residue substitution selected from K326W, K326G, L328V, L328R, P329Y, P329K, P329W, and A330P is exemplified.

A combination of the alteration for attenuating the CD16a-binding activity in the CD16a-binding domain of the first IgG half-molecule and the alteration for attenuating the CD16a-binding activity in the CD16a-binding domain of the second IgG half-molecule is not particularly limited as long as asymmetric alterations are brought about, and the above-mentioned alterations can be appropriately combined. Specific examples include the following combinations of amino acid residue substitutions.

- amino acid residue substitutions at position 265 in the first IgG half-molecule and at position 329 in the second IgG half-molecule
- amino acid residue substitutions at position 329 in the first IgG half-molecule and at position 265 in the second IgG half-molecule
- amino acid residue substitutions at position 238/position 267 in the first IgG half-molecule and at position 329 in the second IgG half-molecule
- amino acid residue substitutions at position 329 in the first IgG half-molecule and at position 238/position 267 in the

second IgG half-molecule

The combination of the alteration for attenuating the CD16a-binding activity in the CD16a-binding domain of the first IgG half-molecule (first alteration) and the alteration for attenuating the CD16a-binding activity in the CD16a-binding domain of the second IgG half-molecule (second alteration) is preferably combinations shown in the following Table 1, and more preferably a combination of the first alteration and the second alteration as follows: S267K and P329Y, Y296P and P329Y, S298E and P329Y, D265A and P329Y, or S239R and P329Y.

[Table 1]

| First alteration | Second alteration |
|---|---|
| D265A | P329Y |
| P238A/S267L | P329Y |
| S239R | K326G |
| S239R | L328R |
| D265A | L328R |
| D265E | L328R |
| S267K | L328R |
| E269P | L328R |
| Y296P | L328R |
| S298E | L328R |
| T299A | L328R |
| S239R | P329Y |
| D265E | P329Y |
| S267K | P329Y |
| Y296P | P329Y |
| S298E | P329Y |
| T299A | P329Y |
| L235R | P329Y |
| A327I | P329Y |
| S239R | P329K |
| D265A | P329K |
| D265E | P329K |
| S267K | P329K |
| Y296P | P329K |
| S298E | P329K |
| T299A | P329K |
| S239R | P329W |
| D265A | P329W |
| D265N | P329W |
| D265E | P329W |
| S267K | P329W |
| E269P | P329W |

(continued)

| First alteration | Second alteration |
|---|---|
| Y296P | P329W |
| S298E | P329W |
| T299A | P329W |
| L235R | P329W |
| A3271 | P329W |
| S239R | A330P |
| D265A | A330P |
| D265E | A330P |
| S267K | A330P |
| E269P | A330P |
| Y296P | A330P |
| S298E | A330P |
| T299A | A330P |
| L235R | A330P |
| A327I | A330P |

An amino acid residue after substitution described above may be a mutually substitutable amino acid. Hereinafter, examples of the mutually substitutable amino acid are shown. Amino acids included in the same group can be mutually substituted.

group A: leucine, isoleucine, norleucine, valine, norvaline, alanine, 2-aminobutanoic acid, methionine, O-methylserine, t-butyl glycine, t-butyl alanine, and cyclohexylalanine
group B: aspartic acid, glutamic acid, isoaspartic acid, isoglutamic acid, 2-aminoadipic acid, and 2-aminosuberic acid
group C: asparagine and glutamine
group D: lysine, arginine, ornithine, 2,4-diaminobutanoic acid, and 2,3-diaminopropionic acid
group E: proline, 3-hydroxyproline, and 4-hydroxyproline
group F: serine, threonine, and homoserine
group G: phenylalanine and tyrosine

The above-mentioned amino acids to be substituted may be either a natural type or an unnatural type. Examples of the natural amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine, and the like. Examples of the unnatural amino acid include various amino acids having an amino group and a carboxyl group, however, preferably, derivatives of various types of natural amino acids are desired. Many unnatural amino acids are available from respective reagent companies (Sigma-Aldrich Co. LLC, TCI Co., Ltd., and the like). With respect to the unnatural amino acids, there are many disclosures in literature (Chem. Today 2003, 65; Curr Opin Chem Biol. 2000, 6, 645).

In the first IgG half-molecule, the CD16a-binding activity in the first CD16a-binding domain is attenuated by alteration, and also in the second IgG half-molecule, the CD16a-binding activity in the second CD16a-binding domain is attenuated by alteration. Accordingly, even if an antibody structure of a homo assembly is constituted by the first IgG half-molecules or the second IgG half-molecules, it cannot bind to CD16a, and therefore cannot exhibit the activity of an antibody.

On the other hand, when an antibody structure of a hetero assembly is constituted by the first and second IgG half-molecules, it can bind to CD16a through the second CD16a-binding domain in the first IgG half-molecule and the first CD16a-binding domain in the second IgG half-molecule (see Fig. 5).

The first and second IgG half-molecules have antigen-binding domains that are different from each other. Therefore, by constituting an antibody structure of a hetero assembly composed of the first and second IgG half-molecules, the assembly becomes an antibody composition which CD16a binds to and thus can specifically exhibit an effector

function such as an ADCC activity only when it binds to a target cell expressing two types of antigens that are different from each other on the same cell.

A hinge domain in each of the first and second IgG half-molecules is altered so as not to form a disulfide bond by substitution or deletion of a part or the whole thereof or modification. Accordingly, an inter-H chain disulfide bond is not formed between the first IgG half-molecule and the second IgG half-molecule.

Therefore, by the coexistence of the first and second IgG half-molecules, it becomes possible to make the first and second IgG half-molecules exist in an equilibrium state of an assembly or half-molecules, so that high specificity for a double-positive cell can be exhibited.

Accordingly, the antibody composition of the present invention can exhibit an effector function specifically for a target cell coexpressing a first antigen and a second antigen and damage the cell as compared with an effector function for a target cell expressing only the first antigen and a target cell expressing only the second antigen.

In the present invention, the phrase "exhibit an effector function specifically for a target cell coexpressing a first antigen and a second antigen as compared with an effector function for a target cell expressing only the first antigen and a target cell expressing only the second antigen" means that the effector function for a target cell coexpressing a first antigen and a second antigen (double-positive cell) is strong as compared with the effector function for a target cell expressing only the first antigen and a target cell expressing only the second antigen (single-positive cell).

Whether an effector function specific for a double-positive cell is exhibited as compared with a single-positive cell can be evaluated by a method described in the sections [1] High Specificity for Double-Positive Cell and [2] Enhanced Effector Function.

As the substitution of a part of a hinge domain so as not to form an inter-H chain disulfide bond between the first IgG half-molecule and the second IgG half-molecule, for example, amino acid residue substitutions at position 226 and position 229 numbered according to the EU index are exemplified. Alternatively, deletion of a part or the whole of a hinge domain containing the cysteine site is exemplified. As the substitution of a part of a hinge domain so as not to form an inter-H chain disulfide bond between the first IgG half-molecule and the second IgG half-molecule, specifically, for example, amino acid residue substitutions of C226A and C229A numbered according to the EU index are preferred.

In order to make the first and second IgG half-molecules exist in the above-mentioned equilibrium state, it is effective that the non-covalent bonding interaction between the H chains in the first and second IgG half-molecules is attenuated, and in particular, it is preferred that the inter-CH3 domain interaction is attenuated.

Specifically, for example, it is preferred that the inter-CH3 domain interaction of the H chains in the first and second IgG half-molecules is weaker than the inter-CH3 domain interaction of the IgG1 subclass.

As a method for attenuating the inter-CH3 domain interaction as compared with the inter-CH3 domain interaction of the IgG1 subclass, it is preferred to introduce an amino acid alteration (an amino acid residue substitution, deletion, or addition, or modification or the like) in the CH3 domain, and it is particularly preferred to introduce an amino acid residue substitution. Examples of the CH3 domain into which an amino acid alteration is introduced to attenuate the inter-CH3 domain interaction as compared with the inter-CH3 domain interaction of the IgG1 subclass include a CH3 domain of IgG1 and a CH3 domain of IgG4.

It has been reported that the interaction between CH3 domains is enhanced by substituting Arg at position 409 numbered according to the EU index in the CH3 domain of human IgG4 with Lys derived from human IgG1 (Structure 2011 19: 9: 1274-1282). Further, there has also been a report that an amino acid site important for the interaction between the CH3 domains of human IgG1 was identified based on changes in free energy by substituting a specific amino acid residue in the CH3 domain of human IgG1 with Ala (Biochemistry 1998: 37: 9266-9273).

The amino acid residue substitution for attenuating the inter-CH3 domain interaction may be any as long as it attenuates the inter-CH3 domain interaction based on the amino acid sequence of the CH3 domain to be used, and it may be introduced into at least one of the first IgG half-molecule and the second IgG half-molecule, but is preferably introduced into each of them. The amino acid residue substitution for attenuating the inter-CH3 domain interaction can be identified by evaluating a molecule including a desired amino acid residue substitution using a binding assay such as binding ELISA or an SPR method, a molecular weight analysis such as SDS-PAGE or native mass spectrometry, or the like.

As the amino acid residue substitution for attenuating the inter-CH3 domain interaction, specifically, substitution of at least one amino acid residue at position selected from position 349, position 351, position 366, position 368, position 399, position 405, position 407, and position 409 numbered according to the EU index in CH3 domain with another amino acid residue is exemplified.

More specifically, Y349A, L351A, T366A, L368A, D399A, F405A, Y407A, K409A, and K409R are preferred, L368A, Y407A, and K409R are more preferred, and K409R is most preferred. Among these amino acid residue substitutions, any one substitution may be introduced, or two or more substitutions may be introduced in combination.

2. Control of Effector Function of Antibody Composition

The antibody composition of the present invention can also be imparted with an effector function dependent on Fc in the first and second IgG half-molecules. The effector function of the antibody composition can be controlled by various methods.

For example, it is preferred to further enhance the CD16a-binding activity by further including at least one amino acid residue substitution for enhancing the CD16a-binding activity in the CH2 domain in the first and second IgG half-molecules. Accordingly, the effector function of the antibody composition can be enhanced.

The amino acid residue substitution for enhancing the CD16a-binding activity may be introduced into each of the first and second IgG half-molecules or may be introduced into only either one of them. When the amino acid residue substitution for enhancing the CD16a-binding activity is introduced into both the first and second IgG half-molecules, the amino acid residue substitution in the first IgG half-molecule and the amino acid residue substitution in the second IgG half-molecule may be the same or different, but are preferably the same.

When the amino acid residue substitution for enhancing the CD16a-binding activity of the antibody composition is introduced into both the first and second IgG half-molecules, the amino acid residue substitutions are in regions different from the regions altered for attenuating the CD16a-binding activity in the first and second IgG half-molecules.

Examples of a method for controlling the effector function of the antibody composition of the present invention include a method as described below.

For example, the effector function of the antibody composition can be controlled by a method for controlling the amount of fucose (also referred to as core fucose) that is α1,6-linked to N-acetylglucosamine (GlcNAc) present at the reducing end side of an N-linked complex sugar chain (hereinafter sometimes simply abbreviated as complex sugar chain) that is bound to Asn at position 297 according to the EU index using the amino acid sequence of Fc of the IgG1 subclass in the first and second IgG half-molecules (WO 2005/035586, WO 2002/31140, or WO 00/61739), or by substituting an amino acid residue of Fc of the antibody.

1) Control of Effector Function by Sugar Chain Alteration

The effector function of the antibody composition can be enhanced or decreased by controlling the content of fucose to be added to N-acetylglucosamine at the reducing end of the complex sugar chain bound to Fc of the first and second IgG half-molecules.

As for a method for decreasing the content of fucose to be added to the N-linked complex sugar chain bound to Fc of the IgG half-molecule, the IgG half-molecule in which fucose is not bound can be obtained by expressing the IgG half-molecule using an α1,6-fucosyltransferase (FUT8) gene-deficient CHO cell. The antibody composition composed of the IgG half-molecule in which fucose is not bound has a high ADCC activity.

On the other hand, as for a method for increasing the content of fucose to be added to the N-linked complex sugar chain bound to Fc of the IgG half-molecule, the IgG half-molecule in which fucose is bound can be obtained by expressing the IgG half-molecule using a host cell into which an α1,6-fucosyltransferase gene has been introduced. The antibody composition composed of the IgG half-molecule in which fucose is bound has a lower ADCC activity than the antibody composition composed of the IgG half-molecule in which fucose is not bound.

In Fc of the IgG half-molecule, the N-linked sugar chain is bound to the Asn residue at position 297 according to the EU index, however, it is not known that the sugar chain is bound to other Asn residues of Fc. Therefore, generally, two N-glycoside-linked sugar chains are bound per molecule of the antibody.

As the N-linked sugar chain, a high mannose type, a complex type, and a hybrid type are known, and a higher ADCC activity as compared with a sugar chain to which fucose is bound can be obtained by any N-linked sugar chain as long as it is a sugar chain to which fucose is not bound.

As the complex sugar chain to be bound to Fc of the IgG half-molecule, a sugar chain in which one or more N-acetylglucosamine (GlcNAc) residues or galactose-N-acetylglucosamine (hereinafter referred to as Gal-GlcNAc) residues are α1,2-linked or α1,4-linked to mannose (Man) at the non-reducing end side of a core structure (trimannosyl core structure) is exemplified.

Further, a complex type sugar chain having sialic acid, bisecting N-acetylglucosamine (hereinafter referred to as bisecting GlcNAc), or the like at the non-reducing end side of Gal-GlcNAc can be exemplified.

In the present invention, the core fucose or the α1,6-fucose refers to a sugar chain structure in which the 6-position of N-acetylglucosamine (hereinafter sometimes referred to as GlcNAc) at the reducing end of the N-glycoside-linked complex sugar chain and the 1-position of fucose (hereinafter sometimes referred to as Fuc) are α-linked. Further, the sugar chain in which fucose is not bound to N-acetylglucosamine at the reducing end of the N-glycoside-linked complex sugar chain is simply referred to as a sugar chain having no fucose or no core fucose.

Further, in the present invention, the core structure or the trimannosyl core structure refers to a Manα1-6(Manα1-3)Manβ1-4GlcNAcβ1-4GlcNAc structure.

As the sugar chain bound to the IgG half-molecule, a double-stranded N-glycoside-linked complex sugar chain (also referred to as a biantennary complex sugar chain) is represented by the following chemical formula.

[Chem. 1]

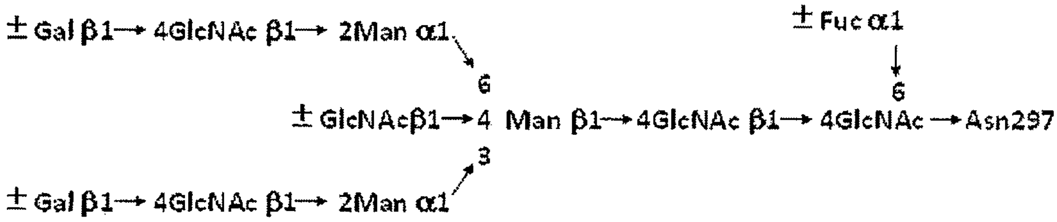

The first and second IgG half-molecules preferably have Fc in which the complex sugar chain is bound to Asn at position 297 according to the EU index. They may have a single sugar chain structure or a plurality of different sugar chain structures as long as they have the above-mentioned sugar chain structure. Specifically, an antibody composition in which the ratio of the sugar chain in which fucose is not bound to N-acetylglucosamine at the reducing end of the sugar chain (sugar chain having no core fucose) among all the N-glycoside-linked sugar chains bound to Fc in the first and second IgG half-molecules is 20% or more is exemplified.

The ratio of the sugar chain having no core fucose includes any ratio as long as the ADCC activity of the antibody composition is increased, but a ratio of preferably 20% or more, more preferably 51% to 100%, further more preferably 80% to 100%, particularly preferably 90%, 91%, 92%, 93%, 94%, 95%, 96%, 97%, 98%, or 99%, and most preferably, 100% can be exemplified.

The ratio of the sugar chain having no core fucose being 50% includes, for example, both an antibody composition containing a molecule in which fucose is not bound to one sugar chain of the N-glycoside-linked sugar chains bound to the first and second IgG half-molecules at 100%, and an antibody composition containing a molecule in which fucose is not bound to both sugar chains of the N-glycoside-linked sugar chains bound to the first and second IgG half-molecules at 50% and also containing a molecule in which fucose is bound to both sugar chains of the N-glycoside-linked sugar chains bound to the first and second IgG half-molecules at 50%.

In the present invention, as the sugar chain having no fucose, the structure of the sugar chain at the non-reducing end side may be any as long as fucose is not bound to N-acetylglucosamine at the reducing end side in the chemical formula shown above.

In the present invention, the state where fucose is not bound to N-acetylglucosamine at the reducing end of the sugar chain (having no core fucose) refers to a state where fucose is not substantially bound thereto. The IgG half-molecule in which fucose is not substantially bound specifically refers to a case where it is an IgG half-molecule to such an extent that fucose cannot be substantially detected in the below-mentioned sugar chain analysis. The extent that fucose cannot be substantially detected refers to that it is the detection limit or less in the measurement. The antibody composition including the first and second IgG half-molecules having no core fucose in all the sugar chains has the highest ADCC activity.

The ratio of the IgG half-molecule including a sugar chain having no fucose in the IgG half-molecule having Fc to which the N-glycoside-linked complex sugar chain is bound can be determined by releasing the sugar chains using a known method such as hydrazinolysis or enzyme digestion [Biochemical Experimentation Methods 23-Method for Studying Glycoprotein Sugar Chain (Japan Scientific Societies Press) edited by Reiko Takahashi (1989)] from the IgG half-molecule, subjecting the released sugar chains to fluorescence labeling or radioisotope labeling, and separating the labeled sugar chains by chromatography.

In addition, the ratio of the IgG half-molecule in which a sugar chain having no fucose is bound included in the IgG half-molecule containing Fc in which the complex sugar chain is bound can be determined by analyzing the released sugar chains using an HPAED-PAD method (J. Liq. Chromatogr., 6, 1577, 1983).

2) Control of Effector Function by Amino Acid Residue Substitution

The antibody composition of the present invention can enhance or attenuate an effector function such as an ADCC activity, an ADCP activity, and a CDC activity by changing the antibody subclass of Fc or by amino acid residue substitution in Fc in the first IgG half-molecule and the second IgG half-molecule.

The IgG1 subclass antibody is known to have the highest ADCC activity and CDC activity in the IgG subclasses, and the immunoglobulin subclass of the CH2 domain is preferably IgG1.

As the amino acid residue substitution in Fc, for example, by using the amino acid sequence of Fc described

in US Patent Application Publication No. 2007/0148165, the CDC activity of the antibody can be increased. Further, by performing an amino acid residue substitution described in US Patent No. 6,737,056, US Patent No. 7,297,775 and US Patent No. 7,317,091, the ADCC activity or the CDC activity of the antibody composition can be enhanced or attenuated.

Specific examples of the amino acid residue substitution for enhancing the ADCC activity include P247I, A339D, F243L, R292P, Y300L, P396L, T393A, H433P, S239D, S298A, A330L, I332E, E333A, K334A, and the like. On the other hand, specific examples of the amino acid residue substitution for decreasing the ADCC activity include L235E, P238A, N297A, K322A, P331S, and the like.

The ADCC activity can be enhanced by combining any two or more of the above-mentioned amino acid residue substitutions, and the number of amino acid residues to be substituted can be increased according to the purpose. As a combination of amino acid residue substitutions capable of enhancing the ADCC activity, S298A/E333A/K334A/P247L, S298A/E333A/K334A/H268E, S298A/E333A/K334A/P247L/N421K, S298A/E333A/K334A/E294W, S298A/E333A/K334A/K326T, S298A/E333A/R292L/K334E, S298A/E333A/K334A/S239D, S298A/E333A/K334A/K248M, S239D/I332E, S239D/A330F, S239D/K326T, S239D/K326E, S239D/K326I, S239D/I332D, S239E/I332Y, S239E/I332E, S239E/K326T, and S239E/K326I are preferred.

The amino acid residue substitution for enhancing the ADCC activity may affect the pharmacokinetics of the antibody, and therefore, it is preferred that the antibody in which the amino acid residue substitution is introduced has pharmacokinetics comparable to that of the wild-type IgG antibody in which the amino acid residue substitution is not introduced. As the specific amino acid substitution, S239D, S239E, S239D/K326T, S239D/A330F, S239D/K326E, S239E/I332E, S298A/E333A/K334A, S298A/E333A/K334A/H268E, and S239D/S298A/E333A/L242C/K334C are preferred, and S239D/K326T and S239D/S298A/E333A/L242C/K334C are more preferred. The comparable pharmacokinetics means 50% or more and 150% or less as compared with the wild-type IgG antibody in any index of maximum blood concentration (Cmax), blood half-life (t1/2), or area under the blood concentration-time curve (AUC).

As a specific amino acid residue substitution for increasing the CDC activity, at least one amino acid residue substitution selected from K326A, S267E, H268F, S324T, K274Q, N276K, Y296F, Y300F, K326W, K326Y, E333A, E333S, A339T, D356E, L358M, N384S, K392N, T394F, T394Y, V397M, and V422I is exemplified.

The CDC activity can also be increased by combining any two or more of the above-mentioned amino acid residue substitutions, and the number of amino acid residues to be substituted can be increased according to the purpose. As the amino acid residue substitution for increasing the CDC activity, preferably at least one amino acid residue substitution selected from N276K, A339T, T394F, and T394Y, amino acid residue substitutions of N276K and A339T, amino acid residue substitutions of K274Q, N276K, Y296F, Y300F, A339T, D356E, L358M, N384S, V397M, and V422I, and the like are exemplified. On the other hand, as a specific amino acid residue substitution for decreasing the CDC activity, L235E, N297A, K322A, P329A, P331S, and the like are exemplified.

In addition, the blood half-life can be extended by introducing an amino acid residue substitution such as T250Q, M428L, M252Y, S254T, or T256E into Fc of the human IgG1 subclass. Moreover, cellular cytotoxicity such as an ADCC activity, an ADCP activity, or a CDC activity can be attenuated or deleted using Fc from which an N-linked sugar chain has been removed, Fc of human IgG2 or IgG4 subclass, chimeric Fc of IgG2 and IgG4, or the like by introducing an amino acid residue substitution at position N297.

3. Method for Producing Antibody Composition

As the method for producing an antibody composition of the present invention, a production method including the following steps 1 to 3 is exemplified.

Step 1: a step of introducing a recombinant vector containing a DNA encoding the amino acid sequence of the IgG half-molecule (hereinafter also abbreviated as a recombinant vector for expression of the IgG half-molecule) into a cell, thereby obtaining a transformant
Step 2: a step of culturing the transformant obtained in the step 1 to accumulate the IgG half-molecule in a culture, and collecting the IgG half-molecule from the culture
Step 3: a step of obtaining an antibody composition composed of the IgG half-molecule collected in the step 2

Hereinafter, the respective steps will be described.

[Step 1]

The step 1 is a step of introducing a recombinant vector containing a DNA encoding the amino acid sequence of at least one of the first and second IgG half-molecules into a cell, thereby obtaining a transformant.

The step (1) specifically includes the following steps (1-1) to (1-3).

(1-1) a step of introducing an alteration for attenuating a CD16a-binding activity in the first CD16a-binding domain in the first IgG half-molecule

(1-2) a step of introducing an alteration for attenuating a CD16a-binding activity in the second CD16a-binding domain in the second IgG half-molecule

(1-3) a step of introducing an alteration for performing substitution or deletion of a part or the whole or modification of a hinge domain so as not to form an inter-H chain disulfide bond in the hinge domains of the first and second IgG half-molecules.

As for the step (1-1), for example, in the production of a recombinant vector for expression of the first IgG half-molecule, performing a step of substituting at least one amino acid residue selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index as appropriate according to the subclass of the CH2 domain is exemplified.

As for the step (1-2), for example, in the production of a recombinant vector for expression of the second IgG half-molecule, performing a step of substituting at least one amino acid residue selected from amino acid residues at position 235, position 236, position 237, position 326, position 327, position 328, position 329, and position 330 numbered according to the EU index as appropriate according to the subclass of the CH2 domain is exemplified.

As for the step (1-3), for example, in the production of a recombinant vector for expression of the IgG half-molecule, performing a step of adding substitutions of amino acid residues at position 226 and position 229 numbered according to the EU index as appropriate according to the subclass of the hinge domain is exemplified.

The IgG half-molecule can be obtained, for example, by expressing it in a transformant in the following manner using a method described in Molecular Cloning, Second Edition, Current Protocols in Molecular Biology, Antibodies, A Laboratory Manual, Cold Spring Harbor Laboratory, 1988, Monoclonal Antibodies: principles and practice, Third Edition, Acad. Press, 1993, Antibody Engineering, A Practical Approach, IRL Press at Oxford University Press, 1996, or the like.

(1) Construction of Recombinant Vector for Expression of IgG Half-Molecule

The recombinant vector for expression of the IgG half-molecule is an expression vector for animal cells into which a gene encoding the amino acid sequence of the IgG half-molecule constituting the antibody composition of the present invention is incorporated.

The recombinant vector can be constructed by cloning a DNA encoding the amino acid sequence of the IgG half-molecule into an expression vector for animal cells.

As for the DNA, the total DNA may be synthesized or synthesis by a polymerase chain reaction (PCR method) is also possible (Molecular Cloning, Second Edition). Further, it is also possible to produce the gene encoding the IgG half-molecule by combining a plurality of such methods.

When an animal cell is used as a host, as the expression vector, any vector can be used as long as it can exhibit its function in the animal cell. Examples thereof include pcDNAI, pCDM8 (manufactured by Funakoshi Co., Ltd.), pAGE107 [JP-A-H3-22979; and Cytotechnology, 3, 133 (1990)], pAS3-3 (JP-A-H2-227075), pCDM8 [Nature, 329, 840 (1987)], pcDNAI/Amp (manufactured by Invitrogen, Inc.), pcDNA3.1 (manufactured by Invitrogen, Inc.), pREP4 (manufactured by Invitrogen, Inc.), pAGE103 [J. Biochemistry, 101, 1307 (1987)], pAGE210, pME18SFL3, pKANTEX93 (WO 97/10354), N5KG1val (US Patent No. 6,001,358), a Tol2 transposon vector (WO 2010/143698), and the like.

As a promoter, any promoter can be used as long as it can exhibit its functions in an animal cell. Examples thereof include a cytomegalovirus (CMV) immediate early (IE) gene promoter, an SV40 early promoter, a retrovirus promoter, a metallothionein promoter, a heat-shock promoter, an SR$\alpha$ promoter, and a Moloney murine leukemia virus promoter or enhancer. In addition, a human CMV IE gene enhancer may be used together with the promoter.

As the expression vector, either of a type in which the H chain and the L chain of the antibody exist on separate vectors or a type in which both the H chain and the L chain of the antibody exist on the same vector (hereinafter referred to as tandem type) can be used.

(2) Acquisition of cDNA Encoding Variable Region

cDNAs encoding VH and VL of an arbitrary antibody can be obtained in the following manner. The cDNAs are synthesized using mRNA extracted from a hybridoma cell that produces the arbitrary antibody as a template. The synthesized cDNAs are inserted into a vector such as a phage or a plasmid, thereby producing a cDNA library.

A recombinant phage or a recombinant plasmid having a cDNA encoding VH and a recombinant phage or a recombinant plasmid having a cDNA encoding an L chain variable region are each isolated from the library using a DNA encoding a constant region or a variable region of an existing antibody as a probe. The entire base sequences of VH

and VL of the target antibody on the recombinant phage or the recombinant plasmid are determined, and then, the entire amino acid sequences of VH or VL are deduced from the base sequences.

The hybridoma cell that produces an arbitrary non-human animal antibody can be obtained as follows. A non-human animal is immunized with an antigen to which the antibody binds, and a hybridoma is produced from an antibody-producing cell of the immunized animal and a myeloma cell according to a well-known method [Molecular Cloning, Second Edition, Current Protocols in Molecular Biology, Antibodies, A Laboratory Manual, Cold Spring Harbor Laboratory, 1988, Monoclonal Antibodies: principles and practice, Third Edition, Acad. Press, 1993, Antibody Engineering, A Practical Approach, IRL Press at Oxford University Press, 1996]. Subsequently, a single-cell cloned hybridoma is selected and cultured, followed by purification from the culture supernatant.

As the non-human animal, any animal such as a mouse, a rat, a hamster, or a rabbit can be used as long as it can produce a hybridoma cell.

Examples of the method for preparing total RNA from the hybridoma cell include a guanidine thiocyanate-cesium trifluoroacetate method [Methods in Enzymol., 154, 3 (1987)], and an RNeasy Kit (manufactured by QIAGEN, Inc.), and also examples of the method for preparing mRNA from total RNA include an oligo (dT) immobilized cellulose column method [Molecular Cloning: A Laboratory Manual, Cold Spring Harbor Lab. Press New York, 1989] and the like.

Further, examples of a kit for preparing mRNA from the hybridoma cell include Fast Track mRNA Isolation Kit (manufactured by Invitrogen, Inc.), Quick Prep mRNA Purification Kit (manufactured by Pharmacia Company), and the like.

Examples of the methods for synthesizing a cDNA and preparing a cDNA library include conventional methods (Molecular Cloning: A Laboratory Manual, Cold Spring Harbor Lab. Press New York, 1989; and Current Protocols in Molecular Biology, Supplement 1-34), and a method using a commercially available kit. Examples of the commercially available kit include Super Script (registered trademark) Plasmid System for cDNA Synthesis and Plasmid Cloning (manufactured by GIBCO BRL, Inc.) and ZAP-cDNA Synthesis Kit (manufactured by Stratagene, Inc.).

When the cDNA library is produced, as the vector into which a cDNA synthesized using mRNA extracted from a hybridoma cell as a template is incorporated, any vector can be used as long as it can incorporate the cDNA.

For example, ZAP Express (Strategies, 5, 58, 1992), pBluescript II SK(+) (Nucleic Acids Research, 17, 9494, 1989), λZAP II (manufactured by Stratagene, Inc.), λgt 10 and λgt 11 (DNA Cloning: A Practical Approach, I, 49, 1985), Lambda BlueMid (manufactured by Clontech Laboratories, Inc.), λExCell, pT7T3 18U (manufactured by Pharmacia, Inc.), pcD2 (Mol. Cell. Biol., 3, 280, 1983), pUC18 (Gene, 33, 103, 1985), or the like can be used.

As Escherichia coli into which a cDNA library constructed by a phage or plasmid vector is introduced, any Escherichia coli can be used as long as it can introduce, express, and maintain the cDNA library.

For example, XL1-Blue MRF (Strategies, 5, 81, 1992), C600 (Genetics, 39, 440, 1954), Y1088, Y1090 (Science, 222, 778, 1983), NM522 Journal of Molecular Biology (J. Mol. Biol., 166, 1, 1983), K802 (J. Mol. Biol., 16, 118, 1966), JM105 (Gene, 38, 275, 1985), or the like is used.

As for the method for selecting cDNA clones encoding VH and VL of the non-human animal antibody from the cDNA library, the selection can be carried out by a colony hybridization method or a plaque hybridization method using a probe labeled with an isotope, fluoresce, or the like (Molecular Cloning: A Laboratory Manual, Cold Spring Harbor Lab. Press New York, 1989).

Further, it is also possible to prepare cDNAs encoding VH and VL by preparing primers and performing PCR (Molecular Cloning: A Laboratory Manual, Cold Spring Harbor Lab. Press New York, 1989; and Current Protocols in Molecular Biology, Supplement 1-34) using the cDNAs or the cDNA library as a template.

The cDNA selected by the above-mentioned method is cleaved with an appropriate restriction enzyme or the like, and then cloned into a plasmid such as pBluescript II SK(-) (manufactured by Stratagene, Inc.), and the base sequence of the cDNA can be determined by a commonly used base sequence analysis method, for example, by performing a reaction of a dideoxy method by Sanger et al. (Proc. Natl. Acad. Sci. U. S. A., 74, 5463, 1977) or the like, and then performing an analysis using an automatic base sequence analyzer, for example, a base sequence analyzer such as an ABI PRISM 377 DNA sequencer (manufactured by Applied Biosystems, Inc.).

By deducing the entire amino acid sequence of each of VH and VL from the determined base sequence and comparing it with the entire amino acid sequence of each of VH and VL of a known antibody (Sequences of Proteins of Immunological Interest, US Dept. Health and Human Services, 1991), it can be confirmed whether the obtained cDNA encodes the amino acid sequence completely including each of VH and VL of the antibody containing a secretion signal sequence.

Further, when the amino acid sequence of an antibody variable region or the base sequence of a DNA encoding the variable region is already known, it can be produced using the following method.

When the amino acid sequence is known, the DNA can be obtained by designing the base sequence of a DNA encoding the variable region in consideration of the codon usage frequency (Sequences of Proteins of Immunological Interest, US Dept. Health and Human Services, 1991), synthesizing several synthetic DNAs composed of approximately 100 to 150 bases based on the base sequence of the designed DNA, and carrying out the PCR method using them or

synthesizing a full-length DNA. When the base sequence is known, the DNA can be obtained in the same manner as described above based on the information.

(3) Analysis of Amino Acid Sequence of Variable Region of Antibody

With respect to the complete amino acid sequences of VH and VL of the antibody containing a secretion signal sequence, by comparing them with the amino acid sequences of VH and VL of a known antibody (Sequences of Proteins of Immunological Interest, US Dept. Health and Human Services, 1991), the length of the secretion signal sequence and the N-terminal amino acid sequence can be deduced, and further the subgroup to which the antibody belongs can be known. In addition, also the amino acid sequences of the respective CDRs of VH and VL can be found out by a similar method.

(4) Construction of cDNA Encoding Variable Region of Humanized Antibody

The cDNAs encoding VH and VL of a humanized antibody can be constructed in the following manner. First, the amino acid sequences of framework regions (hereinafter referred to as FRs) of VH and VL of a human antibody for grafting CDRs of VH and VL of a target non-human animal antibody are selected. As the amino acid sequences of FRs of VH and VL of the human antibody, any can be used as long as they are derived from a human antibody.
Examples thereof include amino acid sequences of FRs of VH and VL of human antibodies registered in a database such as Protein Data Bank, common amino acid sequences in each subgroup of FRs of VH and VL of human antibodies (Sequences of Proteins of Immunological Interest, US Dept. Health and Human Services, 1991), and the like.
Among them, in order to produce the humanized antibody having a sufficient activity, it is preferred to select amino acid sequences having a homology as high as possible (at least 60% or more) with the amino acid sequences of FRs of VH and VL of the target non-human animal antibody.
Subsequently, the amino acid sequences of CDRs of VH and VL of the target non-human animal antibody are grafted to the selected amino acid sequences of FRs of VH and VL of the human antibody, and the amino acid sequences of VH and VL of the humanized antibody are designed. The designed amino acid sequences are converted into base sequences of DNAs in consideration of the usage frequency of codons found in the base sequences of the antibody genes (Sequences of Proteins of Immunological Interest, US Dept. Health and Human Services, 1991), and the base sequences of DNAs encoding the amino acid sequences of VH and VL of the humanized antibody are designed. The designed base sequences of the DNAs are fully synthesized.
Further, cloning into the recombinant vector for expression of the IgG half-molecule constructed in the above 3(1) can be easily carried out by introducing an appropriate restriction enzyme recognition sequence at the 5' ends of the synthetic DNA located at both ends. After PCR, each of the amplification products is cloned into a plasmid such as pBluescript II SK(-) (manufactured by Stratagene, Inc.) and the base sequences are determined by the method described in the above 3(2), thereby obtaining a plasmid having the base sequences of the DNAs encoding the amino acid sequences of VH and VL of the desired humanized antibody.

(5) Alteration of Amino Acid Sequence of Variable Region of Humanized Antibody

It is known that the antigen-binding activity of a humanized antibody prepared merely by grafting only CDRs of VH and VL of a non-human animal antibody to FRs of VH and VL of a human antibody is decreased as compared with that of the original non-human animal antibody (BIO/TECHNOLOGY, 9, 266, 1991).
The reason for this is probably because in VH and VL of the original non-human animal antibody, not only CDRs but also some of the amino acid residues of FRs are directly or indirectly involved in the antigen-binding activity, and such amino acid residues are changed to different amino acid residues of FRs of VH and VL of the human antibody by grafting the CDRs.
In order to solve this problem, an attempt has been made for a humanized antibody to raise the lowered antigen-binding activity by identifying the amino acid residues directly involved in the binding to an antigen or the amino acid residues indirectly involved in the binding to an antigen through an interaction with the amino acid residues of CDRs to maintain the conformation of the antibody in the amino acid sequences of FRs of VH and VL of the human antibody, and altering them to amino acid residues derived from the original non-human animal antibody (BIO/TECHNOLOGY, 9, 266, 1991).
In the production of the humanized antibody, it is most important how efficiently the amino acid residues of FRs involved in the antigen-binding activity are identified, and therefore, the construction and analysis of the conformation of the antibody have been carried out by X-ray crystallography (J. Mol. Biol., 112, 535, 1977), computer modeling (Protein Engineering, 7, 1501, 1994), or the like.
Such information of the conformation of the antibody has provided much useful information for the production

of humanized antibodies. However, there has not yet been established any method for producing a humanized antibody adaptable to all types of antibodies. At present, it is still necessary to make various trial-and-error approaches such as production of several types of variants for each antibody and examination of the correlation with the antigen-binding activity among the variants.

Alteration of the amino acid residues of FRs of VH and VL of a human antibody can be achieved by performing the PCR method described in the above 3(4) using synthetic DNAs for alteration. With respect to the amplification product after the PCR, the base sequence is determined by the method described in 3(2) to confirm that the desired alteration has been carried out.

(6) Expression of IgG Half-Molecule

A transformant that transiently or stably produces at least one of the first and second IgG half-molecules can be obtained by introducing the recombinant vector for expression of the IgG half-molecule of the above 3(1) into an appropriate animal cell.

(6-a) Transient Expression of Antibody Composition

By performing transient expression of antibody compositions using the recombinant vector for expression of the IgG half-molecule obtained in (3) and (6) or a recombinant vector obtained by alteration thereof, the antigen-binding activity of many types of antibody compositions produced can be efficiently evaluated.

As a host cell into which the recombinant vector for expression of the IgG half-molecule is introduced, any cell can be used as long as it is a host cell capable of expressing at least one of the first and second IgG half-molecules. For example, COS-7 cells [American Type Culture Collection (ATCC) number: CRL1651] are exemplified (Methods in Nucleic Acids Res., CRC press, 283, 1991).

In the introduction of the recombinant vector for expression of the IgG half-molecule into COS-7 cells, a DEAE-dextran method (Methods in Nucleic Acids Res., CRC press, 1991), a lipofection method (Proc. Natl. Acad. Sci. USA, 84, 7413, 1987), or the like is used.

After the introduction of the recombinant vector for expression of the IgG half-molecule, the expression level and the antigen-binding activity of the IgG half-molecule in a culture supernatant are measured using an enzyme immunoassay method [Monoclonal Antibodies-Principles and practice, Third edition, Academic Press (1996), Antibodies-A Laboratory Manual, Cold Spring Harbor Laboratory (1988) and A manual for monoclonal antibody experiments, Kodansha scientific books (1987)] or the like.

(6-b) Stable Expression of IgG Half-Molecule

A transformant that stably expresses the IgG half-molecule can be obtained by introducing the recombinant vector for expression of the IgG half-molecule obtained in (1) into an appropriate host cell.

In the introduction of the recombinant vector into a host cell, any method can be used as long as it is a method for introducing a DNA into the host cell, and examples thereof include an electroporation method (Cytotechnology, 3, 133, 1990), a calcium phosphate method (JP-A-H2-227075), a lipofection method (Proc. Natl. Acad. Sci. U. S. A., 84, 7413, 1987), an injection method [Manipulating the Mouse Embryo A Laboratory Manual], a method using a particle gun (gene gun) (Japanese Patent No. 2606856 and Japanese Patent No. 2517813), a DEAE-dextran method [Biomanual Series 4-Methods of Gene Transfer, Expression and Analysis (Yodosha), edited by Takashi Yokota and Kenichi Arai (1994)], a virus vector method (Manipulating Mouse Embryo, Second Edition), and the like.

As the host cell into which the recombinant vector is introduced, any cell can be used as long as it is a host cell capable of expressing at least one of the first and second IgG half-molecules. Examples thereof include human leukemia cell Namalwa cells, monkey COS cells, CHO cells that are Chinese hamster cells, HBT5637 (JP-A-S63-299), rat myeloma cells, mouse myeloma cells, cells derived from Syrian hamster kidney, embryonic stem cells, fertilized egg cells, and the like.

Specific examples thereof include PER.C6, CHO-K1 (ATCC CCL-61), DUKXB11 (ATCC CCL-9096), Pro-5 (ATCC CCL-1781), CHO-S (Life Technologies, Cat #11619), Lec13 cells, rat myeloma cells YB2/3HL.P2.G11.16Ag.20 (ATCC NO: CRL1662, or also called YB2/0), mouse myeloma cells NS0, mouse myeloma cells SP2/0-Ag14 (ATCC NO: CRL1581), mouse P3X63-Ag8.653 cells (ATCC NO: CRL1580), dihydrofolate reductase gene (dihydroforate reductase, hereinafter referred to as dhfr)-deficient CHO cells (CHO/DG44) [Proc. Natl. Acad. Sci. USA, 77, 4216 (1980)], Syrian Hamster cells BHK, HBT563 cells, cells of substrains of the above-mentioned cell lines and cells prepared by acclimating the above-mentioned cell lines under serum-free culture, cells prepared by acclimating the above-mentioned cell lines under non-adhesion culture conditions, and the like.

As a cell used for the production of the IgG half-molecule, a cell for decreasing or deleting the amount of core

fucose of the sugar chain bound to Asn at position 297 according to the EU index in Fc can also be used. Specifically, a cell in which an enzyme involved in the synthesis of GDP-L-fucose or transport thereof to the Golgi body, or an enzyme involved in the binding of core fucose has been decreased or deleted is selected, or a cell obtained by any of various artificial methods can also be used as the host cell.

Specifically, a cell in which core fucose is controlled can be produced by a method for decreasing or deleting an enzyme activity involved in the sugar chain modification of core fucose, a method for increasing an activity of a core fucose cleavage enzyme, or the like.

Examples of the enzyme involved in the sugar chain modification of core fucose include an enzyme involved in the synthesis or transport of GDP-L-fucose, and an enzyme involved in the binding of core fucose to an N-glycoside-linked complex sugar chain.

Specific examples of the enzyme involved in the synthesis of GDP-L-fucose or the transport thereof to the Golgi body include GDP-mannose 4,6-dehydratase (hereinafter referred to as GMD), GDP-4-keto-6-deoxy-D-mannose-3,5-epimerase (hereinafter referred to as Fx), GDP-beta-L-fucose pyrophosphorylase (GFPP), fucokinase, GDP-L-fucose transporter, and the like.

Examples of the enzyme involved in the binding of core fucose include $\alpha$1,6-fucosyltransferase (hereinafter referred to as FUT8), and the like.

In the cell that produces the IgG half-molecule, one of the above-mentioned enzyme activities may be decreased or deleted or a plurality of enzyme activities may be combined and decreased or deleted.

Examples of the method for decreasing or deleting the above-mentioned enzyme activities include (a) a method for gene disruption targeting the gene of the enzyme; (b) a method for introducing a dominant-negative mutant of the gene of the enzyme; (c) a method for introducing a mutation into the enzyme; (d) a method for suppressing transcription or translation of the gene of the enzyme; (e) a method for selecting a cell line that is resistant to a lectin and recognizes a sugar chain structure in which the 6-position of N-acetylglucosamine at the reducing end of the N-glycoside-linked sugar chain and the 1-position of fucose are $\alpha$-linked; and the like.

Examples of the lectin include a lectin that is bound to $\alpha$1,6-fucose such as lentil lectin LCA (lentil agglutinin derived from Lens culinaris), pea lectin PSA (pea lectin derived from Pisum sativum), broad bean lectin VFA (agglutinin derived from Vicia faba), and Aleuria aurantia lectin AAL (lectin derived from Aleuria aurantia).

Specific examples of the cell include FUT8 gene-deficient CHO cells (WO 2005/035586, WO 2002/31140, and WO 2000/061739), Lec13 that has acquired lectin resistance (Somatic Cell and Molecular genetics, 12, 55, 1986), GDP-fucose transporter gene-deficient cells (WO 2003/085102), GDP-mannose 4,6-dehydratase (GMD) gene-deficient cells (WO 2002/31140), WGA lectin resistant cells, LCA lectin resistant cells (WO 2002/31140), and the like.

In addition to the above-mentioned methods, the IgG half-molecule in which high mannose-type N-linked sugar chain is bound and the amount of core fucose is decreased can also be expressed by inhibiting an enzyme such as mannosidase I or mannosidase II that is an enzyme involved in an N-linked sugar chain synthesis system.

Further, by using a host cell made to overexpress N-acetylglucosamine transferase III (GnTIII), the IgG half-molecule in which a bisecting GlcNAc-bound complex and a hybrid sugar chain are bound, and the amount of core fucose is decreased can also be produced.

After introduction of the recombinant vector, a transformant that stably expresses the IgG half-molecule is selected by culturing it in a culture medium for animal cell culture containing an agent such as G418 sulfate (hereinafter referred to as G418), cycloheximide (hereinafter abbreviated as CHX), or methotrexate (hereinafter abbreviated as MTX) (JP-AH2-257891).

Examples of the culture medium for animal cell culture include RPMI 1640 medium (manufactured by Invitrogen, Inc.), GIT medium (manufactured by Nihon Pharmaceutical Co., Ltd.), EX-CELL301 medium, EX-CELL302, EX-CELL325 medium (manufactured by JRH), IMDM medium (manufactured by Invitrogen, Inc.), Hybridoma-SFM medium (manufactured by Invitrogen, Inc.), culture media obtained by adding various additives such as fetal bovine serum (hereinafter abbreviated as FBS) to these culture media, and the like.

The IgG half-molecule is expressed and accumulated in a culture supernatant by culturing the obtained transformant in a culture medium. The expression level and the antigen-binding activity of the IgG half-molecule in the culture supernatant can be measured by an ELISA method or the like. In addition, the expression level of the IgG half-molecule produced by the transformant can be improved using a dhfr gene amplification system (JP-AH2-257891) or the like.

The method for expressing the IgG half-molecule using an animal cell as the host has been described above, however, the IgG half-molecule can be expressed also in yeast, an insect cell, a plant cell, or an animal individual or a plant individual in the same manner as in the animal cell based on a known technique.

When yeast is used as the host cell, a microorganism belonging to the genus Saccharomyces, the genus Schizosaccharomyces, the genus Kluyveromyces, the genus Trichosporon, the genus Schwanniomyces, or the like, for example, Saccharomyces cerevisiae, Schizosaccharomyces pombe, Kluyveromyces lactis, Trichosporon pullulans, Schwanniomyces alluvius, or the like can be exemplified.

As a method for introducing the recombinant vector, any method can be used as long as it is a method for

introducing a DNA into yeast, and for example, methods described in an electroporation method (Methods Enzymol., 194, 182, 1990), a spheroplast method (Proc. Natl. Acad. Sci. U. S. A., 84, 1929, 1978), a lithium acetate method (J. Bacteriology, 153, 163, 1983, Proc. Natl. Acad. Sci. U. S. A., 75, 1929, 1978), and the like are exemplified.

When an insect cell is used as the host, the IgG half-molecule can be expressed by, for example, a method described in current Protocols in Molecular Biology (Baculovirus Expression Vectors, A Laboratory Manual, W. H. Freeman and Company, New York, 1992), Bio/Technology, 6, 47, 1988, or the like.

[Step 2]

The step 2 is a step of culturing the transformant obtained in the step 1 to produce and accumulate the IgG half-molecule in a culture, and collecting the IgG half-molecule from the culture and purifying the IgG half-molecule.

The first and second IgG half-molecules may be collected from the same transformant, or may be collected from transformants individually expressing each of the first and second IgG half-molecules. Generally, the first and second IgG half-molecules are collected from transformants individually expressing each of the first and second IgG half-molecules, and mixed after purification, whereby the antibody composition is prepared.

When the host cell prepared in the step 1 has an ability to express the IgG half-molecule, after the IgG half-molecule is introduced into the host cell described below, the cell is cultured, and the target IgG half-molecule can be collected from the culture.

Further, an animal individual into which a gene is introduced (transgenic non-human animal) or a plant individual into which a gene is introduced (transgenic plant) is prepared by redifferentiation of an animal or plant cell into which a gene is introduced, and the IgG half-molecule may be collected using such an individual.

When the transformant is an animal individual or a plant individual, the individual is raised or cultivated according to a conventional method to produce and accumulate the IgG half-molecule, and the IgG half-molecule can be collected from the animal individual or the plant individual.

Examples of the method for producing the IgG half-molecule using an animal individual include a method for producing the target IgG half-molecule in an animal prepared by introducing a gene according to a known method (American Journal of Clinical Nutrition, 63, 639S, 1996; American Journal of Clinical Nutrition, 63, 627S, 1996; Bio/Technology, 9, 830, 1991).

In the case of an animal individual, for example, a transgenic non-human animal into which a DNA encoding the IgG half-molecule is introduced is raised to produce and accumulate the IgG half-molecule in the animal, and the IgG half-molecule can be collected from the inside of the animal.

As a place of production and accumulation in the animal, for example, milk (JP-AS63-309192), egg, or the like of the animal can be exemplified. As the promoter used at that time, any promoter can be used as long as it enables expression in an animal. For example, an $\alpha$-casein promoter, a $\beta$-casein promoter, a $\beta$-lactoglobulin promoter, and a whey acidic protein promoter that are mammary gland cell-specific promoters, and the like are preferably used.

Examples of the method for producing the IgG half-molecule using a plant individual include a method for cultivating a transgenic plant into which a DNA encoding the IgG half-molecule is introduced according to a known method [Tissue Culture, 20 (1994); Tissue Culture, 21 (1995); and Trends in Biotechnology, 15, 45 (1997)] to produce and accumulate the IgG half-molecule in the plant, and collecting the IgG half-molecule from the inside of the plant.

The IgG half-molecule can be purified in the following manner. When the IgG half-molecule produced by the transformant into which the gene encoding the IgG half-molecule is introduced is, for example, expressed as a soluble protein in the cells, after culture is completed, the cells are collected by centrifugation and suspended in an aqueous buffer solution, and thereafter, the cells are homogenized using an ultrasonic homogenizer, a French press, a Manton Gaulin homogenizer, a dynomill, or the like, whereby a cell-free extract is obtained.

From the supernatant obtained by centrifugation of the cell-free extract, the IgG half-molecule can be purified by a general enzyme isolation and purification method, that is, by using methods such as a solvent extraction method, a salting-out method using ammonium sulfate or the like, a desalting method, a precipitation method using an organic solvent, anion exchange chromatography using a resin such as diethylaminoethyl (DEAE)-sepharose or DIAION HPA-75 (manufactured by Mitsubishi Chemical Corporation), cation exchange chromatography using a resin such as S-Sepharose FF (Pharmacia, Inc.), hydrophobic chromatography using a resin such as butyl sepharose or phenyl sepharose, a gel filtration method using a molecular sieve, affinity chromatography, a chromatofocusing method, and electrophoresis such as isoelectric focusing electrophoresis alone or in combination.

In the present invention, as the affinity chromatography, affinity chromatography using a CH-binding body or an Fc-binding body is used (Monoclonal Antibodies-Principles and practice, Third edition, Academic Press, 1996, Antibodies-A Laboratory Manual, Cold Spring Harbor Laboratory, 1988).

Further, when the IgG half-molecule is expressed within the cells by forming an insoluble body, the cells are collected and homogenized in the same manner, followed by centrifugation, whereby the insoluble body of the IgG half-molecule is collected as a precipitated fraction. The collected insoluble body of the IgG half-molecule is solubilized with

a protein denaturing agent. The IgG half-molecule is returned to a normal conformation by diluting or dialyzing the solubilized solution, and thereafter, the IgG half-molecule can be purified by the same isolation and purification method as described above.

When the IgG half-molecule is secreted extracellularly, the IgG half-molecule or a derivative thereof can be recovered in the culture supernatant. That is, the culture supernatant is obtained by treating the culture by a method such as centrifugation in the same manner as described above, and the IgG half-molecule can be purified from the culture supernatant using the same isolation and purification method as described above.

Specifically, the CH-binding body or the Fc-binding body may be any material such as a protein or a resin as long as it binds to CH or Fc, and examples thereof include an Fc-binding protein, an antibody that binds to an H chain constant region (CH) of an antibody, and the like.

Example of the Fc-binding protein include Protein A derived from Staphylococcus Aureus, Protein G derived from hemolytic Streptococcus, an Fc receptor and the subclasses thereof (FcγRI, IIA, IIB, IIIA, and IIIB) and binding portion fragments thereof, and the like.

Examples of the antibody that binds to CH include antibodies that bind to a CH1 domain, a hinge domain, a CH2 domain, or a CH3 domain.

In the present invention, more preferred examples of the CH-binding body include Protein A, Protein G, an anti-CH1 antibody, and binding portion fragments thereof.

As the method for purifying the IgG half-molecule, for example, the culture supernatant obtained by culturing using the transformant described above in [Step 1] (6) is loaded onto a Protein A column or a Protein G column, and thereafter, the column is washed with a phosphate buffer (phosphate buffer saline, hereinafter abbreviated as PBS).

Thereafter, the IgG half-molecule is eluted from the column using a citric acid buffer at low pH (pH 2.0 to 6.0) or the like, and the eluate is neutralized with an alkaline Tris buffer or the like. The neutralized eluate is subjected to dialysis using a sufficient amount of PBS or the like, whereby the purified IgG half-molecule can be obtained.

The molecular weight of the purified IgG half-molecule can be measured using polyacrylamide gel electrophoresis [Nature, 227, 680 (1970)], Western blotting [Monoclonal Antibodies-Principles and practice, Third edition, Academic Press (1996), Antibodies-A Laboratory Manual, Cold Spring Harbor Laboratory (1988)], or the like.

[Step 3]

The step 3 is a step of mixing the first and second IgG half-molecules collected and purified in the step 2, thereby obtaining the antibody composition. It is preferred that the mixing ratio of the first and second IgG half-molecules is appropriately set according to a binding activity to an antigen, a binding activity to an antigen-positive cultured cell line, the strength of the CD16a-binding activity in each CD16a-binding domain, an interaction between the CH3 of the first IgG half-molecule and the CH3 of the second IgG half-molecule, or the like.

Further, the antibody composition of the present invention can also be produced as an antibody composition in which the first and second IgG half-molecules are mixed by simultaneously expressing the recombinant vectors for expression of the first and second IgG half-molecules in the host cell and performing purification. In this case, as for the mixing ratio of the first and second IgG half-molecules, an antibody composition having a desired mixing ratio can be produced by controlling the expression level of the gene encoding each half-molecule. In addition, the genes encoding the first and second IgG half-molecules may be expressed by the same recombinant expression vector or may be expressed by separate recombinant expression vectors.

4. Evaluation of Activity of Antibody Composition

As a method for measuring the protein amount of the purified IgG half-molecule, an FcR-binding activity, a C1q-binding activity, an antigen-binding activity, or cellular cytotoxicity such as an ADCC activity or a CDC activity of the antibody composition constituted by the IgG half-molecule, known methods described in, for example, Molecular Cloning 2nd Edition, Current Protocols in Molecular Biology; Antibodies, A Laboratory manual, Cold Spring Harbor Laboratory, 1988; Monoclonal Antibodies: principles and practice, Third Edition, Acad. Press, 1993; Antibody Engineering, A Practical Approach, IRL Press at Oxford University Press, 1996, and the like are exemplified.

As a specific example, the binding activity of the antibody composition to an antigen or the binding activity thereof to an antigen-positive cultured cell line can be measured by an ELISA method, a fluorescent antibody method (Cancer Immunol. Immunother, 36, 373, 1993), or the like. The cellular cytotoxicity against an antigen-positive cultured cell line can be evaluated by measuring a CDC activity, an ADCC activity, or the like (Cancer Immunol. Immunother, 36, 373, 1993; US Patent Application Publication No. 2004/0259150).

Whether or not the antibody composition has a binding activity to CD16a can be confirmed by producing a recombinant CD16a protein and then measuring a binding activity (US Patent Application Publication No. 2004/0259150).

Example of the method for measuring an ADCC activity include a method in which a target cell labeled with a

radioisotope, a fluorescent substance, a dye, or the like, the antibody composition, and an effector cell are brought into contact with one another, and then, the activity of the labeling substance released from the damaged target cell or the biological activity or the like of an enzyme released therefrom is measured, and the like.

Example of the method for measuring a CDC activity include a method in which a target cell labeled with a radioisotope, a fluorescent substance, a dye, or the like, the antibody composition, and a biological sample such as serum containing a complement component are brought into contact with one another, and then, the activity of the labeling substance released from the damaged target cell or the biological activity of an enzyme released therefrom is measured, and the like.

5. Analysis of Sugar Chain Structure

The sugar chain structure of the IgG half-molecule expressed in various types of cells can be analyzed according to a general analysis of a sugar chain structure of a glycoprotein.

For example, a sugar chain bound to the IgG half-molecule is constituted by a neutral sugar such as galactose (Gal), mannose (Man), or fucose (Fuc), an amino sugar such as N-acetylglucosamine (GlcNAc), or an acidic sugar such as sialic acid (Sial), and can be analyzed using a technique such as a sugar chain structure analysis using a sugar composition analysis and a two-dimensional sugar chain mapping method.

(1) Neutral Sugar and Amino Sugar Composition Analysis

A composition analysis of a sugar chain of the IgG half-molecule can be carried out by performing acid hydrolysis of a sugar chain with trifluoroacetic acid or the like to release a neutral sugar or an amino sugar and analyzing the composition ratio thereof.

Specific examples of the method include a method using a sugar composition analyzer manufactured by Dionex Corporation. BioLC is a device for analyzing a sugar composition by an HPAEC-PAD (high performance anion-exchange chromatography-pulsed amperometric detection) method (J. Liq. Chromatogr., 6, 1577, 1983).

Further, a composition ratio can also be analyzed by a fluorescence labeling method using 2-aminopyridine. Specifically, a sample that is acid-hydrolyzed according to a known method [Agric. Biol. Chem., 55(1), 283-284, 1991] is fluorescently labeled by 2-aminopyridylation, and a composition ratio can be calculated by performing an HPLC analysis.

(2) Sugar Chain Structure Analysis

The structure analysis of a sugar chain in the IgG half-molecule can be carried out by a two-dimensional sugar chain mapping method (Anal. Biochem., 171, 73, 1988; Biochemical Experimentation Methods 23-Methods of Studies on Glycoprotein Sugar Chains, Japan Scientific Societies Press, edited by Reiko Takahashi, 1989). The two-dimensional sugar chain mapping method is, for example, a method for deducing a sugar chain structure by plotting the retention time or elution position of a sugar chain by reverse-phase chromatography on the X axis and the retention time or elution position of the sugar chain by normal-phase chromatography on the Y axis, respectively, and comparing them with results of those of known sugar chains.

Specifically, a sugar chain is released from the IgG half-molecule by hydrazinolysis of the IgG half-molecule and fluorescence labeling of the sugar chain with 2-aminopyridine (hereinafter abbreviated as PA) (J. Biochem., 95, 197, 1984) is performed, and thereafter, the sugar chain is separated from an excess amount of a PA-treating reagent by gel filtration, followed by reverse-phase chromatography. Then, each peak of the fractionated sugar chain is subjected to normal-phase chromatography. The sugar chain structure can be deduced by plotting on a two-dimensional sugar chain map based on these results and comparing them with the spots of a sugar chain standard (manufactured by TaKaRa, Inc.) or those in literature (Anal. Biochem., 171, 73, 1988).

Further, mass spectrometry such as MALDI-TOF-MS of each sugar chain is performed, and the structure deduced by the two-dimensional sugar chain mapping method can be confirmed.

Which portion of Fc of the IgG half-molecule a sugar chain is bound to can be confirmed by treating the IgG half-molecule subjected to reductive alkylation with an endoprotease such as trypsin, pepsin, Lys-C, or Asp-N, and separating the resultant by reverse-phase chromatography (LC), followed by an analysis using a mass spectrometer (MS) or the like.

In other words, whether or not a sugar chain is actually bound can be confirmed by whether or not the molecular weight of a peptide that can be produced by a protease treatment and the molecular weight of a peptide to which the sugar chain is bound match the MS analytical values based on the amino acid sequence of the Fc of the target IgG half-molecule.

6. Method for Identifying Sugar Chain Structure

The ratio of sugar chains having no core fucose among all the N-glycoside-linked complex sugar chains bound to Fc in the IgG half-molecule can be identified using the method for analyzing a sugar chain structure described in the above 5. In addition, it can also be identified by an immunological quantitative method using a lectin.

The identification of a sugar chain structure in the IgG half-molecule by an immunological quantitative method using a lectin can be carried out according to an immunological quantitative method such as Western staining, RIA (radioimmunoassay), VIA (viroimmunoassay), EIA (enzymoimmunoassay), FIA (fluoroimmunoassay), or MIA (metalloimmunoassay) described in literature [Monoclonal Antibodies: Principles and Applications, Wiley-Liss, Inc. (1995); Enzyme Immunoassay, 3rd Ed., IGAKU-SHOIN Ltd. (1987); Enzyme Antibody Technique, Revised Edition, Gakusai Kikaku (1985)], or the like, for example, in the following manner.

A lectin that recognizes the sugar chain structure of the IgG half-molecule is labeled, and the labeled lectin and the antibody composition that is a sample are allowed to react with each other. Subsequently, the amount of a complex of the labeled lectin with the antibody composition is measured.

Examples of the lectin used for identification of the sugar chain structure of the IgG half-molecule include WGA (wheat-germ agglutinin derived from T. vulgaris), ConA (concanavalin A derived from C. ensiformis), RIC (a toxin derived from R. communis), L-PHA (leucoagglutinin derived from P. vulgaris), LCA (lentil agglutinin derived from L. culinaris), PSA (pea lectin derived from P. sativum), AAL (Aleuria aurantia Lectin), ACL (Amaranthus caudatus Lectin), BPL (Bauhinia purpurea Lectin), DSL (Datura stramonium Lectin), DBA (Dolichos biflorus Agglutinin), EBL (Elderberry Balk Lectin), ECL (Erythrina cristagalli Lectin), EEL (Euonymus europaeus Lectin), GNL (Galanthus nivalis Lectin), GSL (Griffonia simplicifolia Lectin), HPA (Helix pomatia Agglutinin), HHL (Hippeastrum Hybrid Lectin), Jacalin, LTL (Lotus tetragonolobus Lectin), LEL (Lycopersicon esculentum Lectin), MAL (Maackia amurensis Lectin), MPL (Maclura pomifera Lectin), NPL (Narcissus pseudonarcissus Lectin), PNA (Peanut Agglutinin), E-PHA (Phaseolus vulgaris Erythroagglutinin), PTL (Psophocarpus tetragonolobus Lectin), RCA (Ricinus communis Agglutinin), STL (Solanum tuberosum Lectin), SJA (Sophora japonica Agglutinin), SBA (Soybean Agglutinin), UEA (Ulex europaeus Agglutinin), VVL (Vicia villosa Lectin), and WFA (Wisteria floribunda Agglutinin).

A lectin that specifically recognizes core fucose is preferably used, and specific examples thereof include lentil lectin LCA (lentil agglutinin derived from Lens culinaris), pea lectin PSA (pea lectin derived from Pisum sativum), broad bean lectin VFA (agglutinin derived from Vicia faba), and Aleuria aurantia lectin AAL (lectin derived from Aleuria aurantia).

7. Use of Antibody Composition of the Present Invention

The antibody composition of the present invention can recognize two types of antigens that are different from each other by including the first and second IgG half-molecules having antigen-binding domains for antigens that are different from each other. Therefore, a pharmaceutical composition containing the antibody composition of the present invention can have a molecular form in accordance with two different types of target antigens, and thus can exhibit high specificity for a double-positive cell that expresses the two types of antigens.

When the antibody composition of the present invention is used for a pharmaceutical composition, it is preferred that the antibody composition has the following properties [1] to [3]. The following properties [1] to [3] can serve as indices for selecting an excellent antibody composition in the present invention.

[1] High Specificity for Double-Positive Cell
[2] Enhanced Effector Function
[3] Blood Half-Life Comparable to that of Wild-Type IgG Antibody

Hereinafter, the respective properties will be described.

[1] High Specificity for Double-Positive Cell

The antibody composition of the present invention having high specificity for a double-positive cell as compared with a single-positive cell can be selected, for example, by a method including the following steps (1a) to (1d).

(1a) The first IgG half-molecule in which each of an amino acid alteration for deleting an inter-H chain disulfide bond in a hinge domain, an amino acid alteration for attenuating a CD16a-binding activity in a first CD 16a-binding domain, and an amino acid alteration for attenuating an inter-CH3 domain interaction is introduced is produced.

(1b) The second IgG half-molecule in which each of an amino acid alteration for deleting an inter-H chain disulfide bond in a hinge domain, an amino acid alteration for attenuating a CD16a-binding activity in a second CD16a-binding domain, and an amino acid alteration for attenuating an inter-CH3 domain interaction is introduced is produced in the same manner as in (1a).

(1c) A double-positive cell that expresses first and second antigens, a single-positive cell that expresses the first antigen, and a single-positive cell that expresses the second antigen are prepared.

In order to use cells in which the expression level of the antigen in each cell is adjusted, each antigen transfectant may be prepared. The effector function of the antibody composition in which the first IgG half-molecule and the second IgG half-molecule are mixed is evaluated for each cell.

(1d) An antibody composition having high specificity for the double-positive cell as compared with the single-positive cells is obtained by selecting an antibody composition having a strong effector function for the double-positive cell as compared with the effector function for each of the single-positive cells based on the results of evaluation in (1c).

As a specific method, for example, a method described below in [Example 3] is exemplified. Specifically, for example, when the effector function is an ADCC activity, an antibody composition having high specificity for the double-positive cell as compared with the single-positive cells can be obtained by selecting an antibody composition having a strong effector function for the double-positive cell as compared with the effector function for each of the single-positive cells as follows.

1-i) As the target cell, the single-positive cell that expresses the first antigen, the single-positive cell that expresses the second antigen, and the double-positive cell that expresses the first antigen and the second antigen are used. The expression levels of the first antigen and the second antigen in each cell are measured using a flow cytometer, and it is confirmed that the target antigen is expressed in the cell.

1-ii) As the effector cell, a human peripheral blood mononuclear cell (PBMC) or a human CD16a-expressing cell line which is transfected with a gene encoding human CD16a to stably express the gene, and the target cell are prepared using a culture medium (for example, RPMI medium).

1-iii) The cells are left to stand for about 2 to 6 hours in a $CO_2$ incubator. At the same time, a solubilizing solution (for example, an aqueous solution containing an acid, an alkali, a surfactant, or the like) is added to a 100% reaction well, and the target cells are completely lysed. After the reaction container is centrifuged, the supernatant is collected and dispensed into an ELISA plate. A coloring solution is applied thereto to cause a reaction, and thereafter, a stopping solution is added thereto, and an absorbance (A450) is measured using a plate reader.

1-iv) The ADCC activity (%) is calculated using the following formula.

$$\text{ADCC activity (\%)} = 100 \times (S - E - T) / (Max - T)$$

$$S = \text{absorbance of sample reaction well - absorbance of culture medium well}$$

$$E = \text{absorbance of effector well - absorbance of culture medium well}$$

$$T = \text{absorbance of target well - absorbance of culture medium well}$$

$$Max = \text{100\% reaction well - 100\% reaction control well}$$

1-vi) As the positive control antibody, a normal IgG1-type antibody against the first antigen and a normal IgG1-type antibody against the second antigen are used. It is confirmed that the normal IgG1-type antibody against the first antigen exhibits an ADCC activity against the single-positive cell that expresses the first antigen, and the normal IgG1-type antibody against the second antigen exhibits an ADCC activity against the single-positive cell that expresses the second antigen.

On the other hand, when an antibody solution obtained by mixing a half-molecule of the antibody against the first antigen and a half-molecule of the antibody against the second antigen does not exhibit an ADCC activity against the single-positive cell that expresses the first antigen and the single-positive cell that expresses the second antigen, and specifically exhibits an ADCC activity only against the double-positive cell that expresses the first antigen and the second antigen, it is evaluated to be an antibody composition having a strong effector function for the double-positive cell as compared with the effector function for each of the single-positive cells.

As one aspect of the case where "an antibody solution obtained by mixing a half-molecule of the antibody against the first antigen and a half-molecule of the antibody against the second antigen does not exhibit an ADCC activity against the single-positive cell that expresses the first antigen and the single-positive cell that expresses the second

antigen, and specifically exhibits an ADCC activity only against the double-positive cell that expresses the first antigen and the second antigen", for example, a case where the ADCC activity against the single-positive cell that expresses the first antigen and the single-positive cell that expresses the second antigen is comparable to the ADCC activity of the negative control, and also the ADCC activity against the double-positive cell that expresses the first antigen and the second antigen is comparable to the ADCC activity of the positive control is exemplified. As another aspect, for example, a case where the ADCC activity against the single-positive cell that expresses the first antigen and the single-positive cell that expresses the second antigen is attenuated as compared with the ADCC activity of the positive control, and also the ADCC activity against the double-positive cell that expresses the first antigen and the second antigen is comparable to the ADCC activity of the positive control is exemplified.

Here, the description "the ADCC activity is comparable" means that the ADCC activity is within a range of preferably ±0 to 50%, and more preferably ±0 to 30% with respect to the ADCC activity of the control, and the description "the ADCC activity is attenuated" means that the ADCC activity is within a range of preferably 0 to 60%, and more preferably 0 to 30% with respect to the ADCC activity of the control.

As the negative control at this evaluation, it is preferred to use the antibody against the first antigen and the antibody against the second antigen. The antibody against the first antigen exhibits an effector function for the double-positive cell and the single-positive cell that expresses the first antigen, and the antibody against the second antigen exhibits an effector function for the double-positive cell and the single-positive cell that expresses the second antigen.

Further, in place of each antigen-expressing cell, a bead or the like with each antigen fixed thereto can be used. For example, a double-positive bead with the first antigen and the second antigen fixed thereto, a single-positive bead with only the first antigen fixed thereto, and a single-positive bead with only the second antigen fixed thereto are prepared. To each bead, the antibody composition of the present invention is added, and thereafter, further a recombinant CD16a protein is added thereto. Thereafter, by examining the binding activity of the recombinant CD16a protein to the antibody composition bound onto the double-positive bead and two single-positive beads, an antibody composition having high selectivity for both target antigens can be selected. As a method for measuring an interaction with the recombinant CD16a protein, a binding ELISA method, a surface plasmon resonance method, or the like can be used.

[2] Enhanced Effector Function

The antibody composition of the present invention having an enhanced effector function can be selected, for example, by a method including the following steps (2a) to 2(c).

(2a) The effector function of the antibody composition having high specificity for the double-positive cell as compared with the single-positive cells selected by the method described in the above [1] is enhanced.

As a means for enhancing the effector function of the antibody composition of the present invention having high specificity for the double-positive cell selected by the method described in the above [1], for example, performing an amino acid alteration for enhancing the CD16a-binding activity, and/or decreasing the content of the core fucose bound to Fc or deleting the core fucose is exemplified.

When an amino acid residue substitution for enhancing the CD 16a-binding activity is introduced, the amino acid residue may be any as long as it is an amino acid residue contained in the CD16a-binding domain formed in the hetero assembly of the IgG half-molecules.

Further, the amino acid residue substitution may be introduced into both the first and second IgG half-molecules or may be introduced into either one of them. However, in order to achieve the effect of the present invention, in the same IgG half-molecule, it is preferred that the position of the amino acid residue substitution for enhancing the CD16a-binding activity and the position of the amino acid residue substitution for attenuating the CD 16a-binding activity are different.

In other words, if the IgG half-molecules are mutually different, the position of the amino acid residue substitution for enhancing the CD16a-binding activity and the position of the amino acid residue substitution for attenuating the CD 16a-binding activity may be the same.

Which position the amino acid residue substitution for enhancing the CD16a-binding activity is introduced into can be determined by producing hetero assemblies composed of various IgG half-molecules in combination with the position of the amino acid residue substitution for attenuating the CD 16a-binding activity, measuring the ADCC activity or the like, and evaluating that the activity is an activity specific for the double-positive cell.

The effector function can be enhanced by decreasing the content of the core fucose bound to Fc or deleting the core fucose in addition to the amino acid residue substitution for enhancing the CD16a-binding activity.

(2b) With respect to the antibody composition with an effector function enhanced in (2a), the effector function (for example, the ADCC activity or the like) for the single-positive cell that expresses the first antigen, the single-positive cell that expresses the second antigen, and the double-positive cell that expresses the first and second antigens is evaluated.

(2c) An antibody composition having an enhanced effector function specific for the double-positive cell is obtained by selecting an antibody composition having a strong effector function for the double-positive cell as compared with the effector function for each of the single-positive cells based on the results of evaluation in (2b).

By the above-mentioned method, an antibody composition having an enhanced effector function and exhibiting high specificity for the double-positive cell can be selected.

The antibody composition having an enhanced effector function specific for the double-positive cell can be evaluated for an effector function closer to a clinical effect by an evaluation system mimicking human blood. As the evaluation system mimicking human blood, for example, an ADCC evaluation system in which a protein component of blood such as human albumin, an immunoglobulin, human plasma, or human serum, and human NK cells, human peripheral blood mononuclear cells (PBMC), human granulocytes, or the like are added, a cellular cytotoxicity evaluation system using human whole blood, and the like are exemplified.

Examples of a specific method for the evaluation system mimicking human blood include a method using an ADCC evaluation system described below in [Example 8] and a method using a cellular cytotoxicity evaluation system described below in [Example 9].

As the method using an ADCC evaluation system, specifically, for example, the following method is exemplified. The ADCC activity is measured and evaluated in the same manner as in 1-i) to 1-vi) except that in the above-mentioned 1-ii), an evaluation system mimicking the internal human body is constructed by adding an immunoglobulin at a final concentration of preferably 0.01 to 10 mass%, more preferably 0.1 to 10 mass%, and further more preferably 1 to 4 mass%.

As the method using a cellular cytotoxicity evaluation system by a human blood reconstitution system, specifically, for example, the following method is exemplified.

2-i) As the target cell, the single-positive cell that expresses the first antigen, the single-positive cell that expresses the second antigen, and the double-positive cell that expresses the first antigen and the second antigen are used. The expression levels of the first antigen and the second antigen in each cell are measured using a flow cytometer, and it is confirmed that the target antigen is expressed in the cell.

2-ii) Plasma is obtained by centrifuging human blood and recovering the supernatant. Further, a fraction of peripheral blood mononuclear cells and granulocytes is obtained from the human blood using a red blood cell depletion reagent (for example, Hetasep: Stemcell technology, #07806).

2-iii) The plasma and the fraction of peripheral blood mononuclear cells and granulocytes recovered in 2-ii) are mixed, and the mixture is seeded in a cell culture plate, and thereafter, an antibody composition obtained by mixing a half-molecule of the antibody against the first antigen and a half-molecule of the antibody against the second antigen is added thereto, and the cells are cultured at 37°C for about 12 to 48 hours.

2-iv) The ratio of the double-positive cell that expresses the first antigen and the second antigen is measured by flow cytometry. As the negative control, a wild-type IgG antibody is used.

As a result, when the antibody composition obtained by mixing a half-molecule of the antibody against the first antigen and a half-molecule of the antibody against the second antigen removes the double-positive cell that expresses the first antigen and the second antigen in an antibody concentration-dependent manner, but does not remove the single-positive cell that expresses the first antigen and the single-positive cell that expresses the second antigen, it is evaluated that the effector function specific for the double-positive cell is enhanced.

The description "removes the double-positive cell" means that the ratio of the double-positive cell that expresses the first antigen and the second antigen when the antibody composition of the present invention is added is lower than the ratio in the case of the negative control. The description "the ratio of the double-positive cell is lower" means that it is within a range of preferably 0 to 70%, and more preferably 0 to 50% with respect to the ratio of the double-positive cell in the case of the negative control.

The description "does not remove the single-positive cell" means that the ratio of the single-positive cell that expresses the first antigen and the single-positive cell that expresses the second antigen when the antibody composition of the present invention is added is comparable to the ratio in the case of the negative control. The description "the ratio of the single-positive cell is comparable" means that it is within a range of preferably ±0 to 40%, and more preferably ±0 to 20% with respect to the ratio of the single-positive cell in the case of the negative control.

By this evaluation, an antibody composition that exhibits an enhanced effector function even in human blood can be selected.

(3a) The antibody composition having both high specificity for the double-positive cell and an enhanced effector function selected using the above-mentioned method in [1] and [2] is subjected to PK studies (pharmacokinetics and pharmacodynamics studies) using an animal such as a mouse or a monkey.

As the negative control, a wild-type IgG antibody is used.

(3b) As a result of the PK studies in (3a), an antibody composition that exhibits kinetics close to that of the wild-type IgG antibody is selected as an antibody composition having a blood half-life comparable to that of the wild-type IgG antibody. As a specific method, for example, a method described below in [Example 7] is exemplified.

As the method for the PK studies, specifically, for example, the following method is exemplified.

3-i) After the antibody composition (preferably 0.1 to 10 mg/kg) having both high specificity for the double-positive cell and an enhanced effector function is administered to a mouse (for example, BALB/c mouse) through the tail vein, the blood is collected preferably after 1 hour, 4 hours, 24 hours, and 72 hours. After piercing the cheek with a lancet under isoflurane anesthesia and collecting the blood, serum is recovered by centrifuging the blood at preferably 4,000 to 16,000 rpm at room temperature preferably for 5 to 20 minutes with a micro blood collection tube [for example, Microtainer (registered trademark) manufactured by BD Company]. As the positive control antibody, a wild-type IgG antibody is used.
3-ii) The concentration of the antibody present in the serum is measured. The concentration of the antibody in the serum can be measured by a known method, and for example, the measurement can be carried out using AlphaLISA Human Kappa light chain immunoassay kit (AL3023, PerkinElmer), and a calibration curve can be created with Kappa light chain in the kit.
3-iii) An antibody composition in which the blood half-life (t1/2) that can be calculated from the concentration of the antibody measured in 3-ii) is within a range of preferably 50% to 150% as compared with that of the wild-type IgG antibody as the positive control is evaluated to be an antibody composition having a blood half-life comparable to that of the wild-type IgG antibody.

The antibody composition having a long blood half-life can be selected also by measuring the denaturation midpoint (Tm) value by differential scanning calorimetry (DSC) as a physicochemical characteristic of an antibody in place of the PK studies using an animal.
Among the antibody compositions of the present invention, those having a Tm value (denaturation midpoint temperature) comparable to that of the wild-type IgG antibody exhibit favorable PK. As a specific method for measuring the Tm value, for example, a method described below in [Example 7] is exemplified. Specifically, the measurement can be carried out by differential scanning fluorimetry (DSF), and as a measuring apparatus, for example, Prometheus NT.48 of NanoTemper Technologies GmbH is exemplified. The measurement of the Tm value of the antibody can be carried out preferably by elevating the temperature to 80°C to 100°C starting from 10°C to 30°C at a temperature elevation rate of preferably 0.5°C to 2°C/min.
Further, it is known that the higher the binding activity at pH 6.0 of the antibody to FcRn (neonatal Fc receptor) is, the longer the blood half-life is. Therefore, by measuring the binding activity to FcRn, an antibody composition having a long blood half-life can be selected. The binding activity to FcRn can be measured, for example, by a method disclosed in J. Immunol. 2002; 169: 5171-80.
By the evaluation in [1] to [3] described above, an antibody composition having preferred properties when it is used in a pharmaceutical product can be selected. A combination of amino acid residue substitutions included in the preferred antibody composition of the present invention having such properties is preferably a combination shown in Table 2, more preferably a combination shown in Table 3A or 3B, and most preferably a combination shown in Table 4.
[Table 2]

| Domain name | Purpose of amino acid residue substitution | Combination of amino acid residue substitutions | |
|---|---|---|---|
| | | First IgG half-molecule | Second IgG half-molecule |
| Hinge | To form hetero assembly | C226A/C229A | |
| CH2 | To bind to CD16a only when hetero assembly is formed | D265A, S267K, Y296P, S298E, or S239R | P329Y |
| CH2 | To enhance CD16a binding activity | S239D, S239E, S239D/K326T, S239D/A330F, S239D/K326E, S239E/I332E, S298A/E333A/K334A/H268E, or S239D/S298A/E333A/L242C/K334C | |
| CH3 | To form hetero assembly | Y349A, L351A, T366A, L368A, D399A, F405A, Y407A, K409A, or K409R | |

[Table 3A]

| Domain name | Purpose of amino acid residue substitution | Combination of amino acid residue substitutions | |
|---|---|---|---|
| | | First IgG half-molecule | Second IgG half-molecule |
| Hinge | To form hetero assembly | C226A/C229A | |
| CH2 | To bind to CD16a only when hetero assembly is formed | D265A | P329Y |
| CH2 | To enhance CD16a binding activity | S239D/K326T or S239D/S298A/E333A/L242C/K334C | |
| CH3 | To form hetero assembly | L368A, Y407A, or K409R | |

[Table 3B]

| Domain name | Purpose of amino acid residue substitution | Combination of amino acid residue substitutions | |
|---|---|---|---|
| | | First IgG half-molecule | Second IgG half-molecule |
| Hinge | To form hetero assembly | C226A/C229A | |
| CH2 | To bind to CD16a only when hetero assembly is formed | S298E | P329Y |
| CH2 | To enhance CD16a binding activity | S239D/K326T or S239D/S298A/E333A/L242C/K334C | |
| CH3 | To form hetero assembly | L368A, Y407A, or K409R | |

That is, the combinations of amino acid residue substitutions shown in Tables 3A and 3B are as follows.

<First IgG Half-Molecule>

1) C226A, C229A, D265A, S239D, K326T, L368A
2) C226A, C229A, D265A, S239D, K326T, Y407A
3) C226A, C229A, D265A, S239D, K326T, K409R
4) C226A, C229A, D265A, S239D, S298A, E333A, L242C, K334C, L368A
5) C226A, C229A, D265A, S239D, S298A, E333A, L242C, K334C, Y407A
6) C226A, C229A, D265A, S239D, S298A, E333A, L242C, K334C, K409R
7) C226A, C229A, S298E, S239D, K326T, L368A
8) C226A, C229A, S298E, S239D, K326T, Y407A
9) C226A, C229A, S298E, S239D, K326T, K409R
10) C226A, C229A, S298E, S239D, E333A, L242C, K334C, L368A
11) C226A, C229A, S298E, S239D, E333A, L242C, K334C, Y407A
12) C226A, C229A, S298E, S239D, E333A, L242C, K334C, K409R

<Second IgG Half-Molecule>

1) C226A, C229A, P329Y, S239D, K326T, L368A
2) C226A, C229A, P329Y, S239D, K326T, Y407A
3) C226A, C229A, P329Y, S239D, K326T, K409R
4) C226A, C229A, P329Y, S239D, S298A, E333A, L242C, K334C, L368A
5) C226A, C229A, P329Y, S239D, S298A, E333A, L242C, K334C, Y407A
6) C226A, C229A, P329Y, S239D, S298A, E333A, L242C, K334C, K409R

The antibody composition of the present invention preferably contains the second IgG half-molecule [1) to 6)]

with the same number with respect to the first IgG half-molecule [1) to 6)]. Further, the antibody composition of the present invention preferably contains the second IgG half-molecule so that the combination thereof with the first IgG half-molecule [7) to 12)] becomes as follows.

- the first IgG half-molecule 7) and the second IgG half-molecule 1)
- the first IgG half-molecule 8) and the second IgG half-molecule 2)
- the first IgG half-molecule 9) and the second IgG half-molecule 3)
- the first IgG half-molecule 10) and the second IgG half-molecule 4)
- the first IgG half-molecule 11) and the second IgG half-molecule 5)
- the first IgG half-molecule 12) and the second IgG half-molecule 6)

More specifically, as preferred embodiments of the combination of amino acid residue substitutions included in the antibody composition of the present invention, for example, the following <1> to <12> are exemplified.

<1>

(2A) Each of the first IgG half-molecule and the second IgG half-molecule includes amino acid residue substitutions of C226A and C229A numbered according to the EU index.
(3A) The first IgG half-molecule includes an antigen-binding domain that binds to the first antigen and includes an amino acid residue substitution of D265A numbered according to the EU index in the first CD16a-binding domain.
(4A) The second IgG half-molecule includes an antigen-binding domain that binds to the second antigen and includes an amino acid residue substitution of P329Y numbered according to the EU index in the second CD16a-binding domain.
(5A) Each of the first IgG half-molecule and the second IgG half-molecule includes amino acid residue substitutions of (a) S239D and K326T numbered according to the EU index.
(6A) Each of the first IgG half-molecule and the second IgG half-molecule includes L368A numbered according to the EU index.

<2>

(2A) Each of the first IgG half-molecule and the second IgG half-molecule includes amino acid residue substitutions of C226A and C229A numbered according to the EU index.
(3A) The first IgG half-molecule each includes an antigen-binding domain that binds to the first antigen and includes an amino acid residue substitution of D265A numbered according to the EU index in the first CD16a-binding domain.
(4A) The second IgG half-molecule includes an antigen-binding domain that binds to the second antigen and includes an amino acid residue substitution of P329Y numbered according to the EU index in the second CD16a-binding domain.
(5A) Each of the first IgG half-molecule and the second IgG half-molecule includes amino acid residue substitutions of (a) S239D and K326T numbered according to the EU index.
(6A) Each of the first IgG half-molecule and the second IgG half-molecule includes Y407A numbered according to the EU index.

<3>

(2A) Each of the first IgG half-molecule and the second IgG half-molecule includes amino acid residue substitutions of C226A and C229A numbered according to the EU index.
(3A) The first IgG half-molecule each includes an antigen-binding domain that binds to the first antigen and includes an amino acid residue substitution of D265A numbered according to the EU index in the first CD16a-binding domain.
(4A) The second IgG half-molecule includes an antigen-binding domain that binds to the second antigen and includes an amino acid residue substitution of P329Y numbered according to the EU index in the second CD16a-binding domain.
(5A) Each of the first IgG half-molecule and the second IgG half-molecule includes amino acid residue substitutions of (a) S239D and K326T numbered according to the EU index.
(6A) Each of the first IgG half-molecule and the second IgG half-molecule includes K409R numbered according

to the EU index.

<4>

(2A) Each of the first IgG half-molecule and the second IgG half-molecule includes amino acid residue substitutions of C226A and C229A numbered according to the EU index.
(3A) The first IgG half-molecule each includes an antigen-binding domain that binds to the first antigen and includes an amino acid residue substitution of D265A numbered according to the EU index in the first CD16a-binding domain.
(4A) The second IgG half-molecule includes an antigen-binding domain that binds to the second antigen and includes an amino acid residue substitution of P329Y numbered according to the EU index in the second CD16a-binding domain.
(5A) Each of the first IgG half-molecule and the second IgG half-molecule includes amino acid residue substitutions of (b) S239D, S298A, E333A, L242C, and K334C numbered according to the EU index.
(6A) Each of the first IgG half-molecule and the second IgG half-molecule includes L368A numbered according to the EU index.

<5>

(2A) Each of the first IgG half-molecule and the second IgG half-molecule includes amino acid residue substitutions of C226A and C229A numbered according to the EU index.
(3A) The first IgG half-molecule includes an antigen-binding domain that binds to the first antigen and includes an amino acid residue substitution of D265A numbered according to the EU index in the first CD16a-binding domain.
(4A) The second IgG half-molecule includes an antigen-binding domain that binds to the second antigen and includes an amino acid residue substitution of P329Y numbered according to the EU index in the second CD16a-binding domain.
(5A) Each of the first IgG half-molecule and the second IgG half-molecule includes amino acid residue substitutions of (b) S239D, S298A, E333A, L242C, and K334C numbered according to the EU index.
(6A) Each of the first IgG half-molecule and the second IgG half-molecule includes K407A numbered according to the EU index.

<6>

(2A) Each of the first IgG half-molecule and the second IgG half-molecule includes amino acid residue substitutions of C226A and C229A numbered according to the EU index.
(3A) The first IgG half-molecule includes an antigen-binding domain that binds to the first antigen and includes an amino acid residue substitution of D265A numbered according to the EU index in the first CD16a-binding domain.
(4A) The second IgG half-molecule includes an antigen-binding domain that binds to the second antigen and includes an amino acid residue substitution of P329Y numbered according to the EU index in the second CD16a-binding domain.
(5A) Each of the first IgG half-molecule and the second IgG half-molecule includes amino acid residue substitutions of (b) S239D, S298A, E333A, L242C, and K334C numbered according to the EU index.
(6A) Each of the first IgG half-molecule and the second IgG half-molecule includes K409R numbered according to the EU index.

<7>

(2A) Each of the first IgG half-molecule and the second IgG half-molecule includes amino acid residue substitutions of C226A and C229A numbered according to the EU index.
(3A) The first IgG half-molecule includes an antigen-binding domain that binds to the first antigen and includes an amino acid residue substitution of S298E numbered according to the EU index in the first CD16a-binding domain.
(4A) The second IgG half-molecule includes an antigen-binding domain that binds to the second antigen and includes an amino acid residue substitution of P329Y numbered according to the EU index in the second CD16a-binding domain.
(5A) Each of the first IgG half-molecule and the second IgG half-molecule includes amino acid residue substitutions of (a) S239D and K326T numbered according to the EU index.

(6A) Each of the first IgG half-molecule and the second IgG half-molecule includes K409R numbered according to the EU index.

<8>

(2A) Each of the first IgG half-molecule and the second IgG half-molecule includes amino acid residue substitutions of C226A and C229A numbered according to the EU index.

(3A) The first IgG half-molecule includes an antigen-binding domain that binds to the first antigen and includes an amino acid residue substitution of S298E numbered according to the EU index in the first CD16a-binding domain.

(4A) The second IgG half-molecule includes an antigen-binding domain that binds to the second antigen and includes an amino acid residue substitution of P329Y numbered according to the EU index in the second CD16a-binding domain.

(5A) Each of the first IgG half-molecule and the second IgG half-molecule includes amino acid residue substitutions of (a) S239D and K326T numbered according to the EU index.

(6A) Each of the first IgG half-molecule and the second IgG half-molecule includes L368A numbered according to the EU index.

<9>

(2A) Each of the first IgG half-molecule and the second IgG half-molecule includes amino acid residue substitutions of C226A and C229A numbered according to the EU index.

(3A) The first IgG half-molecule each includes an antigen-binding domain that binds to the first antigen and includes an amino acid residue substitution of S298E numbered according to the EU index in the first CD16a-binding domain.

(4A) The second IgG half-molecule includes an antigen-binding domain that binds to the second antigen and includes an amino acid residue substitution of P329Y numbered according to the EU index in the second CD16a-binding domain.

(5A) Each of the first IgG half-molecule and the second IgG half-molecule includes amino acid residue substitutions of (a) S239D and K326T numbered according to the EU index.

(6A) Each of the first IgG half-molecule and the second IgG half-molecule includes Y407A numbered according to the EU index.

<10>

(2A) Each of the first IgG half-molecule and the second IgG half-molecule includes amino acid residue substitutions of C226A and C229A numbered according to the EU index.

(3A) The first IgG half-molecule each includes an antigen-binding domain that binds to the first antigen and includes an amino acid residue substitution of S298E numbered according to the EU index in the first CD16a-binding domain.

(4A) The second IgG half-molecule includes an antigen-binding domain that binds to the second antigen and includes an amino acid residue substitution of P329Y numbered according to the EU index in the second CD16a-binding domain.

(5A) The first IgG half-molecule includes amino acid residue substitutions of S239D, E333A, L242C, and K334C numbered according to the EU index, and the second IgG half-molecule includes amino acid residue substitutions of S239D, S298A, E333A, L242C, and K334C numbered according to the EU index.

(6A) Each of the first IgG half-molecule and the second IgG half-molecule includes L368A numbered according to the EU index.

<11>

(2A) Each of the first IgG half-molecule and the second IgG half-molecule includes amino acid residue substitutions of C226A and C229A numbered according to the EU index.

(3A) The first IgG half-molecule includes an antigen-binding domain that binds to the first antigen and includes an amino acid residue substitution of S298E numbered according to the EU index in the first CD16a-binding domain.

(4A) The second IgG half-molecule includes an antigen-binding domain that binds to the second antigen and

includes an amino acid residue substitution of P329Y numbered according to the EU index in the second CD16a-binding domain.
(5A) The first IgG half-molecule includes amino acid residue substitutions of S239D, E333A, L242C, and K334C numbered according to the EU index, and the second IgG half-molecule includes amino acid residue substitutions of S239D, S298A, E333A, L242C, and K334C numbered according to the EU index.
(6A) Each of the first IgG half-molecule and the second IgG half-molecule includes Y407A numbered according to the EU index.

<12>

(2A) Each of the first IgG half-molecule and the second IgG half-molecule includes amino acid residue substitutions of C226A and C229A numbered according to the EU index.
(3A) The first IgG half-molecule includes an antigen-binding domain that binds to the first antigen and includes an amino acid residue substitution of S298E numbered according to the EU index in the first CD 16a-binding domain.
(4A) The second IgG half-molecule includes an antigen-binding domain that binds to the second antigen and includes an amino acid residue substitution of P329Y numbered according to the EU index in the second CD 16a-binding domain.
(5A) The first IgG half-molecule includes amino acid residue substitutions of S239D, E333A, L242C, and K334C numbered according to the EU index, and the second IgG half-molecule includes amino acid residue substitutions of S239D, S298A, E333A, L242C, and K334C numbered according to the EU index.
(6A) Each of the first IgG half-molecule and the second IgG half-molecule includes K409R numbered according to the EU index.

[Table 4]

| Domain name | Purpose of amino acid residue substitution | Combination of amino acid residue substitutions | |
|---|---|---|---|
| | | First IgG half-molecule | Second IgG half-molecule |
| Hinge | To form hetero assembly | C226A/C229A | |
| CH2 | To bind to CD16a only when hetero assembly is formed | D265A | P329Y |
| CH2 | To enhance CD16a binding activity | S239D/K326T or S239D/S298A/E333A/L242C/K334C | |
| CH3 | To form hetero assembly | K409R | |

Further, the antibody composition of the present invention is preferably an antibody composition containing the first IgG half-molecule and the second IgG half-molecule shown in Table 5. In Table 5, each SEQ ID NO denotes the sequence number of the amino acid sequence of the CH domain.

[Table 5]

| Antibody composition No. | SEQ ID NO of CH of First IgG half-molecule (amino acid sequence) | SEQ ID NO of CH of second IgG half-molecule (amino acid sequence) |
|---|---|---|
| 1 | 248 | 252 |
| 2 | 272 | 292 |

As one aspect of the present invention, the first IgG half-molecule or the second IgG half-molecule constituting the above-mentioned antibody composition is exemplified.

Examples of the first IgG half-molecule of the present invention include a first IgG half-molecule characterized by being associated with the second IgG half-molecule because an effector function can be exhibited specifically for a double-positive cell that expresses a desired first antigen and a desired second antigen by being associated with the second IgG half-molecule in the double-positive cell. The first IgG half-molecule of the present invention can be used in combination with the second IgG half-molecule, in the production of an antibody composition including the first IgG half-molecule and the second IgG half-molecule, and in the production of a pharmaceutical composition containing an antibody composition including the first IgG half-molecule and the second IgG half-molecule.

Further, examples of the second IgG half-molecule of the present invention include a second IgG half-molecule characterized by being associated with the first IgG half-molecule because an effector function can be exhibited specifically for a double-positive cell that expresses a desired first antigen and a desired second antigen by being associated with the first IgG half-molecule in the double-positive cell. The second IgG half-molecule of the present invention can be used in combination with the first IgG half-molecule, in the production of an antibody composition including the first IgG half-molecule and the second IgG half-molecule, and in the production of a pharmaceutical composition containing an antibody composition including the first IgG half-molecule and the second IgG half-molecule.

The first IgG half-molecule or the second IgG half-molecule contained in the antibody composition exemplified above is each an IgG half-molecule having a characteristic of being associated with the corresponding IgG half-molecule, and therefore is included in the first or second IgG half-molecule of the present invention.

Further, the first or second IgG half-molecule of the present invention may be an IgG half-molecule that specifically binds to any antigen, and the antigen to which the first IgG half-molecule binds and the antigen to which the second IgG half-molecule binds only need to be different from each other.

The antigen to which the antibody composition of the present invention binds may be any antigen, and preferably, an antigen molecule associated with a cancer, an immune disease, an allergic disease, a cardiovascular disease, or the like is exemplified. Examples thereof include a cytokine, a chemokine, a growth factor, and a receptor therefor, a CD antigen, and the like.

Examples of the receptor for a cytokine or a growth factor include receptors for interferon (hereinafter referred to as IFN)-α, IFN-β, IFN-y, interleukin (hereinafter referred to as IL)-2, IL-3, IL-4, IL- 5, IL-6, IL-7, IL-8, IL-9, IL-10, IL-12, IL-13, IL-15, IL-17, IL-18, IL-21, IL-23, IL-27, a granulocyte colony-stimulating factor (G-CSF), a granulocyte/macrophage colony-stimulating factor (GM-CSF), or a macrophage colony-stimulating factor (M-CSF), and the like.

Examples of the receptor for a chemokine include receptors for SLC, ELC, I-309, TARC, MDC, MIP-3α, or CTACK.

Examples of the receptor for a growth factor include receptors for epidermal growth factor (EGF), vascular endothelial growth factor (VEGF), angiopoietin, fibroblast growth factor (FGF), hepatocyte growth factor (HGF), platelet-derived growth factor (PDGF), insulin-like growth factor (IGF), erythropoietin (EPO), Trombopoietin (TPO), TGFβ, Iephrin, angiopoietin, Frizzled ligand, or SDF-1, and the like.

Examples of the cluster of differentiation (hereinafter referred to as CD) antigens include CD1a, CD1c (BDCA1), CD1d, CD2, CD3, CD4, CD5, CD6, CD7, CD8, CD10, CD11a, CD11b, CD11c, CD14, CD15, CD16, CD18, CD19, CD20, CD21, CD22, CD23, CD24, CD25, CD26 (DPP-4), CD27, CD28, CD30, CD32, CD32a, CD33, CD34, CD37, CD38, CD39, CD40, CD43, CD44, CD45, CD47, CD48, CD49, CD49a, CD49b, CD51, CD52, CD53, CD54, CD55, CD56, CD57, CD59, CD62E, CD62L, CD62P, CD63, CD64, CD66a (CEACAM1), CD66b (NCA-95), CD66c (NCA-50/90), CD66d (CGM1), CD66e (CEA), CD66f (PSG), CD68, CD69, CD70, CD71, CD72, CD73, CD74, CD75, CD76, CD77, CD78, CD79a, CD79b, CD80 (B7.1), CD81, CD82, CD83, CD84 (SLAMF5), CD85a (ILT-5), CD85b (ILT8), CD85c (LIR8), CD85d (ILT4), CD85f (ILT11), CD85g (ILT7), CD85h (ILT1), CD85i (LIR6a), CD85j (ILT2), CD85k (ILT3), CD85m (ILT10), CD86 (B7.2), CD87, CD89, CD94 (NKG2), CD95 (Fas), CD97, CD98, CD103, CD106, CD107a (LAMP1), CD114 (G-CSFR), CD115 (M-CSFR, CSF1R), CD116 (GM-CSFR), CD117 (SCF-R, C-KIT), CD119 (IFNGR1), CD121a (IL-1R1), CD122 (IL-2Rb), CD123 (IL-3Ra), CD124 (IL-4Ra), CD125 (IL-5Ra), CD126 (IL-6Ra), CD127 (IL-7Ra), CD134 (OX40), CD135 (FLT3), CD137 (4-1BB), CD138 (Syndecan-1), CD140 (PDGFR), CD146 (MUC18), CD147 (EMMRRIN), CD152 (CTLA-4), CD153 (CD30 ligand), CD158a (KIR2DL1), CD158b1 (KIR2DL2), CD158b2 (KIR2DL3), CD158c (KIR2DS6), CD158d (KIR2DL4), CD158e1 (KIR3DL1), CD158e2 (KIR3DS1), CD158f (KIR2DL5), CD158g (KIR2DS5), CD158h (KIR2DS1), CD158i (KIR2DS4), CD158j (KIR2DS2), CD158k (KIR3DL2), CD159a (NKG2A), CD159c (NKG2C), CD161 (NKRP1A), CD162 (PSGL-1), CD163, CD169 (SIGLEC1), CD177, CD178 (FasL), CD183 (CXCR3), CD184 (CXCR4), CD185 (CXCR5), CD191 (CCR1), CD193 (CCR3), CD194 (CCR4), CD195 (CCR5), CD196 (CCR6), CD197 (CCR7), CD198 (CCR8), CD199 (CCR9), CD200 (OX2), CD206 (MMR), CD207 (Langerin), CD209 (DC-SIGN), CD212 (IL-12Rβ1), CD213a1 (IL-13Ra1), CD213a2 (IL-13Ra2), CD215 (IL-15RA), CD217 (IL-17R), CD218a (IL-18Ra), CD218b (IL-18Rβ), CD223 (LAG3), CD226 (DNAM-1), CD229 (SLAMF3), CD252 (OX40L), CD269 (BCMA), CD272 (BTLA), CD274 (PD-L1), CD275 (ICOS ligand), CD276 (B7H3), CD278 (ICOS), CD279 (PD-1), CD281 (TLR1), CD282 (TLR2), CD283 (TLR3), CD284 (TLR4), CD286 (TLR6), CD288 (TLR8), CD289 (TLR9), CD294 (CRTH2), CD301 (CLEC10A, MGL), CD302 (DCL1), CD303 (BDCA2), CD304 (BDCA4), CD305, CD314 (NKG2D), CD317 (BST2), CD319 (CS1), CD324 (E-cadherin), CD326 (EpCAM), CD357 (GITR), CD358 (DR6), CD360 (IL-21R), CD365 (TIM-1), CD366 (TIM-3), CD369 (DECTIN-1), CD370 (CLEC9A), CD371 (CLEC12A), human leukocyte antigen (HLA)-Class II (for example, HLA-DR), HLA-I, and the like.

Further, examples of an antigen involved in pathogenesis of tumors or an antigen for an antibody, which regulates an immunological function, include ganglioside GM1, GM2, GD2, GD3, Lewis X (CD15s), Lewis Y, CD3, CD4, CD40, CD40 ligand, a B7 family molecule (for example, CD80, CD86, CD274, B7-DC, B7-H2, B7-H3, B7-H4, B7-H5, B7-H6, or B7-H7), a ligand for a B7 family molecule (for example, CD28, CTLA-4, ICOS, PD-1, or BTLA), OX-40, OX-40 ligand, CD137, a tumor necrosis factor (TNF) receptor family molecule (for example, DR3, DR4, DR5, BAFFR, LIGHT,

TNFR1, or TNFR2), a TNF-related apoptosis-inducing ligand receptor (TRAIL) family molecule, a receptor family of a TRAIL family molecule (for example, TRAIL-R1, TRAIL-R2, TRAIL-R3, or TRAIL-R4), a receptor activator of nuclear factor kappa B ligand (RANK), RANK ligand, CD25, a folate receptor, Mesothelin, Siglec-8, a cytokine/chemokine receptor [for example, IL-1RII, IL-12Rβ2, IL-17RB, IL-23R, IL-27Rα, IL-31R, IL-33Rα, IL-36R, transforming growth factor (TGF)βRII, CCR2, CCR10, CXCR1, CXCR2, a leukotriene B4 receptor (BLT1)], an NK cell receptor (for example, NKG2D, E4BP4, NKp30, NKp44, NKp46, or AhR), a T cell receptor (for example, TCRα/β, TCR Vβ11, TCRγ/δ, TSLPR, SLAM, SLAMF6, LAP,GARP, SR-A1, CD200R, DCR3, or TIGIT), a B cell receptor (for example, BLYS, APRIL, or TSLPR), a dendritic cell receptor (for example, FCER1A, TLR7, CADM1, XCR1, BTLA, SIRPA, DCIR, TROP2, AXL, SIGLEC6, SIGLEC15, CX3CR1, S100A8, S100A9, or ASGR1), an amino acid transporter (for example, ASCT2), a serine protease (for example, proteinase-3 or PR3), and the like.

Examples of a receptor tyrosine kinase include an EGF receptor, an insulin receptor, an IGF-1 receptor, an NGF receptor, a PDGF receptor, an M-CSF receptor, an FGF receptor, a VEGF receptor, an Eph receptor, and the like. Examples of a tyrosine kinase-associated receptor include a cytokine receptor, an Fc receptor, and the like. Further, examples of a cell adhesion molecule include cadherin, integrin, and the like. Examples of a G protein-coupled receptor include an adenosine receptor, a cannabinoid receptor, a glucagon receptor, and the like.

Specific examples of the receptor tyrosine kinase include Epidermal Growth Factor Receptor (EGFR), V-ERB-B2 Avian Erythroblastic Leukemia Viral Oncogene Homolog 2 (HER2), V-ERB-B2 Avian Erythroblastic Leukemia Viral Oncogene Homolog 3 (HER3), V-ERB-B2 Avian Erythroblastic Leukemia Viral Oncogene Homolog 4 (HER4), Insulin Receptor (INSR), Insulin-like Growth Factor I Receptor (IGF1R), Nerve Growth Factor Receptor (NGFR), Platelet-derived Growth Factor Receptor, Alpha (PDGFRA), Platelet-derived Growth Factor Receptor, Beta (PDGFRB), Colony-stimulationg Factor Receptor (CSF1R), Colony-stimulationg Factor 2 Receptor, Alpha (CSF2RA), Colony-stimulationg Factor 3 Receptor, Granulocyte (CSF3R), Fibroblast Growth Factor Receptor 1 (FGFR1), Fibroblast Growth Factor Receptor 2 (FGFR2), Fibroblast Growth Factor Receptor 3 (FGFR3), Fibroblast Growth Factor Receptor 4 (FGFR4), Kinase Insert Domain Receptor (KDR), Ephrin Receptor EphA1 (EPHA1), Ephrin Receptor EphA2 (EPHA2), Ephrin Receptor EphA3 (EPHA3), and the like.

Further, examples of the tyrosine kinase-associated receptor include Interleukin-1 Receptor 1 (IL-1R1), Interleukin-1 Receptor Accessory Protein (IL-1RAP), Interleukin-1 Receptor Like 1 (IL-1RL1, ST2), Hepatocyte Growth factor Receptor (c-Met), Macrophage Stimulating 1 Receptor (RON), Platelet-Derived Growth Factor Receptor (PDGFR), Junctional Adhesion Molecule-Like (JAML), Nectin-like protein 5 (Necl-5), Tumor Necrosis Factor Receptor 1 (TNF-R1), Tumor Necrosis Factor Receptor 2 (TNF-R2), TNF-Related Apoptosis-Inducing Ligand Receptor 1 (TRAIL-R1), TNF-Related Apoptosis-Inducing Ligand Receptor 2 (TRAIL-R2), Death Receptor 3 (DR3), Death Receptor 6 (DR6), Receptor Activator of NF-kB (RANK), Nerve Growth Factor Receptor (NGFR), Lymphotoxin-beta Receptor (LTβR), OX40 (TNFRSF4), Fas (TNFRSF6), 4-1BBL (TNFRSF9), Fn14 (TNFRSF12A), TACI (TNFRSF13B), BAFF-R (TNFRSF13C), HVEM (TNFRSF14), BCMA (TNFRSF17), GITR (TNFRSF18), TROY (TNFRSF19), Ectodysplasin A1 Receptor (EDAR), Ectodysplasin A2 Receptor (XEDAR), Receptor Expressed in Lymphoid Tissues (RELT), CD3, CD27, CD30, CD40, FcaRI, FcyRIII and FcεRI, Fc Fragment of IgG, Receptor Transpoter, Alpha (FCGRT), and the like.

Further, examples of the cell adhesion molecule include Integrin, Alpha 9 (ITGA9), P-selectin Glycoprotein Ligand-1 (PSGL-1), Cadherin-11 (CDH11), Mucosal Vascular Addression Cell Adhesion Molecule 1 (MADCAM1), Integrin, Alpha 4 (ITGA4), Integrin, Beta 4 (ITGB4), Integrin, Alpha 4 Beta 7, Integrin, Alpha 4 Beta 1, and collagen (Type I Collagen), and examples of the G protein-coupled receptor include Adenosine A2A Receptor (ADORA2A), Adenosine A2B Receptor (ADORA2B), Repulsive Guidance Molecule A (RGMA), Glucagon Receptor (GCGR), Prolactin Receptor (PRLR), Glucagon-like Peptide-1 Receptor (GLP1R), Cannabinoid Receptor 1 (CB1), Mas-related G protein Coupled Receptor-X2 (MRGPRX2), and the like.

The pharmaceutical composition containing the antibody composition of the present invention is preferably used in a treatment of a disease associated with a cell that expresses the antigen molecule on the surface, and more preferably used in a treatment of a cancer, an autoimmune disease, or an allergic disease.

Examples of the cancer include leukemia, lymphoma, multiple myeloma, brain tumor, breast cancer, uterine body cancer, cervical cancer, ovarian cancer, esophageal cancer, gastric cancer, colon cancer, liver cancer, gallbladder cancer, bile duct cancer, pancreatic cancer, adrenal cancer, gastrointestinal stromal tumor, mesothelial tumor, head and neck cancer, kidney cancer, lung cancer, sarcoma, prostate cancer, testicular tumor, bladder cancer, skin cancer, pediatric cancer, and the like. Further, various cancer treatments by removing suppressive immune cells are also included.

Examples of the autoimmune disease or allergy include Guillain-Barre syndrome, myasthenia gravis, multiple sclerosis, chronic gastritis, chronic atrophic gastritis, autoimmune hepatitis, primary biliary cholangitis, ulcerative colitis, Crohn's disease, autoimmune pancreatitis, Takayasu's arteritis, Goodpasture's syndrome, rapidly progressive glomerulonephritis, IgA nephropathy, megaloblastic anemia, autoimmune hemolytic anemia, autoimmune neutropenia, idiopathic thrombocytopenic purpura, Basedow's disease, Hashimoto's disease, primary hypothyroidism, idiopathic Addison's disease, type 1 diabetes mellitus, chronic discoid lupus erythematosus, localized scleroderma, pemphigus, pustular psoriasis, psoriasis arthropathica, plaque psoriasis, bullous pemphigoid, herpes gestationis, linear IgA bullous dermatosis, epidermolysis bullosa acquisita, alopecia areata, vitiligo, Harada disease, autoimmune optic neuropathy, autoimmune inner ear disorder, idiopathic azoospermia, habitual abortion, rheumatoid arthritis, systemic lupus erythematosus, antiphospholipid antibody syndrome, polymyositis, dermatomyositis, scleroderma, Sjogren's syndrome, an IgG4-related disease, vasculitic syndrome, mixed connective tissue disease, autoimmune hemolytic anemia, incompatible transfusion, idiopathic thrombocytopenic purpura, atopic dermatitis, polyarteritis, allergic rhinitis (pollinosis), allergic conjunctivitis, allergic gastroenteritis, bronchial asthma, childhood asthma, a food allergy, a drug allergy, urticaria, anaphylactic shock, transplantation rejection, contact dermatitis, Behcet's disease, and the like.

Examples of a specific combination of the first antigen and the second antigen to which the antibody composition of the present invention binds, the target cell, and the disease to be treated with a pharmaceutical composition containing the antibody composition are shown below. Further, with respect to such examples, correspondence relationships are shown in Tables 6A to 6E.

c1) An antibody composition for which the first antigen is CADM1, the second antigen is CCR4, and the target cell is an ATL (adult T-cell leukemia-lymphoma) cell.

As the disease to be treated with a pharmaceutical composition containing the antibody composition, ATL is exemplified.

c2) An antibody composition for which the first antigen is EGFR, the second antigen is HER2, and the target cell is a cancer cell.

As the pharmaceutical composition containing the antibody composition, a solid cancer such as gastric cancer or breast cancer is exemplified.

c3) An antibody composition for which the first antigen is CD52, the second antigen is CD70, and the target cell is an activated T cell, an activated B cell, or activated APC.

c4) An antibody composition for which the first antigen is CD2, the second antigen is CD70, and the target cell is an activated T cell.

c5) An antibody composition for which the first antigen is CD19, the second antigen is CD70, and the target cell is an activated B cell.

c6) An antibody composition for which the first antigen is CD2, the second antigen is CD40 ligand, and the target cell is an activated effector T cell (activated Teff).

As the disease to be treated with a pharmaceutical composition containing the antibody composition, Sjogren's syndrome is exemplified.

c7) An antibody composition for which the first antigen is PD-L1, the second antigen is one selected from CD19, CD30, CCR4, CD20, CD22, and CD79b, and the target cell is lymphoma.

As the disease to be treated with a pharmaceutical composition containing the antibody composition, lymphoma is exemplified.

c8) An antibody composition for which the first antigen is PD-L1, the second antigen is one selected from CD19, CD30, CCR4, CD20, CD22, and CD79b, and the target cell is leukemia.

As the disease to be treated with a pharmaceutical composition containing the antibody composition, leukemia is exemplified.

c9) An antibody composition for which the first antigen is CD8, the second antigen is CCR4, and the target cell is a regulatory T cell (Treg).

As the disease to be treated with a pharmaceutical composition containing the antibody composition, a cancer is exemplified. The pharmaceutical composition is preferably used for cancer immunotherapy.

c10) An antibody composition for which the first antigen is CTLA-4, the second antigen is one selected from CD4, CCR4, and GITR, and the target cell is a regulatory T cell (Treg).

As the disease to be treated with a pharmaceutical composition containing the antibody composition, a cancer is exemplified. The pharmaceutical composition is preferably used for cancer immunotherapy.

c11) An antibody composition for which the first antigen is TIGIT, the second antigen is one selected from CD4, CCR4, and GITR, and the target cell is a regulatory T cell (Treg).

As the disease to be treated with a pharmaceutical composition containing the antibody composition, a cancer is exemplified. The pharmaceutical composition is preferably used for cancer immunotherapy.

c12) An antibody composition for which the first antigen is PD-1, the second antigen is one selected from CD4, CCR4, and GITR, and the target cell is a regulatory T cell (Treg).

As the disease to be treated with a pharmaceutical composition containing the antibody composition, a cancer is exemplified. The pharmaceutical composition is preferably used for cancer immunotherapy.

c13) An antibody composition for which the first antigen is OX40, the second antigen is one selected from CD127, CD26, CD70, and CD15s, and the target cell is an effector T cell (Teff).

As the disease to be treated with a pharmaceutical composition containing the antibody composition, an autoimmune disease is exemplified.

c14) An antibody composition for which the first antigen is 4-1BB, the second antigen is one selected from

CD127, CD26, CD70, and CD15s, and the target cell is an effector T cell (Teff).

As the disease to be treated with a pharmaceutical composition containing the antibody composition, an autoimmune disease is exemplified.

c15) An antibody composition for which the first antigen is GITR, the second antigen is one selected from CD127, CD26, CD70, and CD15s, and the target cell is an effector T cell (Teff).

As the disease to be treated with a pharmaceutical composition containing the antibody composition, an autoimmune disease is exemplified.

c16) An antibody composition for which the first antigen is CD40 ligand, the second antigen is one selected from CD127, CD26, CD70, and CD15s, and the target cell is an effector T cell (Teff).

As the disease to be treated with a pharmaceutical composition containing the antibody composition, an autoimmune disease is exemplified.

c17) An antibody composition for which the first antigen is CD4, the second antigen is CD69, and the target cell is a tissue-resident memory T (TRM) cell.

As the disease to be treated with a pharmaceutical composition containing the antibody composition, ANCA (anti-neutrophil cytoplasmic antibody)-associated glomerulonephritis is exemplified.

c18) An antibody composition for which the first antigen is PD-1, the second antigen is CD3, and the target cell is at least one of a peripheral helper T (TPH) cell and a PD-$1^{high}$ CD8-positive T cell.

As the disease to be treated with a pharmaceutical composition containing the antibody composition, rheumatoid arthritis is exemplified.

c19) An antibody composition for which the first antigen is PD-1, the second antigen is CD4, and the target cell is a PD-1-positive T cell.

As the disease to be treated with a pharmaceutical composition containing the antibody composition, celiac disease is exemplified.

c20) An antibody composition for which the first antigen is integrin $\alpha4\beta7$ and the second antigen is one selected from CCR6, CXCR3, and CD161, and the target cell is at least one selected from an effector T cell (Teff) and an innate lymphoid cell (ILC).

As the disease to be treated with a pharmaceutical composition containing the antibody composition, an inflammatory bowel disease is exemplified.

c21) An antibody composition for which the first antigen is integrin $\alpha4\beta7$ and the second antigen is CD127, and the target cell is a T cell infiltrating the intestinal tract other than all innate lymphoid cells (ILC) and Treg.

As the disease to be treated with a pharmaceutical composition containing the antibody composition, an inflammatory bowel disease is exemplified.

c22) An antibody composition for which the first antigen is integrin $\alpha4\beta7$ and the second antigen is CD40 ligand, and the target cell is a local migratory activated T cell.

As the disease to be treated with a pharmaceutical composition containing the antibody composition, at least one of multiple sclerosis and an inflammatory bowel disease is exemplified.

c23) An antibody composition for which the first antigen is integrin $\alpha4\beta1$ and the second antigen is CD40 ligand, and the target cell is a local migratory activated T cell.

As the disease to be treated with a pharmaceutical composition containing the antibody composition, at least one of multiple sclerosis and an inflammatory bowel disease is exemplified.

c24) An antibody composition for which the first antigen is CXCR5 and the second antigen is CD 127, and the target cell is an innate lymphoid cell (ILC).

As the disease to be treated with a pharmaceutical composition containing the antibody composition, an allergic disease is exemplified.

c25) An antibody composition for which the first antigen is a TPO receptor (c-mpl) and the second antigen is CD34, and the target cell is an abnormally proliferating hematopoietic stem cell.

As the disease to be treated with a pharmaceutical composition containing the antibody composition, primary myelofibrosis is exemplified.

c26) An antibody composition for which the first antigen is a TPO receptor (c-mpl) and the second antigen is CD 123, and the target cell is a CD 123-positive leukemic stem cell.

As the disease to be treated with a pharmaceutical composition containing the antibody composition, primary myelofibrosis is exemplified.

c27) An antibody composition for which the first antigen is CXCR3 and the second antigen is CD3, and the target cell is a CX3CR1-positive cell (a cytotoxic effector T-cell or the like, excluding NK cells).

As the disease to be treated with a pharmaceutical composition containing the antibody composition, at least one selected from an autoimmune disease (for example, Crohn's disease or the like), arteriosclerosis, and an ischemic heart disease is exemplified.

c28) An antibody composition for which the first antigen is CXCR3 and the second antigen is CD127 or CD40

ligand, and the target cell is a Th1 cell.

As the disease to be treated with a pharmaceutical composition containing the antibody composition, systemic lupus erythematosus is exemplified.

c29) An antibody composition for which the first antigen is CCR4 and the second antigen is CD127 or CD40 ligand, and the target cell is a CCR4-positive T cell other than Treg.

As the disease to be treated with a pharmaceutical composition containing the antibody composition, an allergic disease is exemplified.

c30) An antibody composition for which the first antigen is CCR6 and the second antigen is CD127 or CD40 ligand, and the target cell is a Th17 cell.

As the disease to be treated with a pharmaceutical composition containing the antibody composition, psoriasis is exemplified.

c31) An antibody composition for which the first antigen is CRTH2 and the second antigen is CD127 or CD40 ligand, and the target cell is a Th2 cell.

As the disease to be treated with a pharmaceutical composition containing the antibody composition, an allergic disease is exemplified.

c32) An antibody composition for which the first antigen is CRTH2 and the second antigen is CCR4, and the target cell is a Th2 cell.

As the disease to be treated with a pharmaceutical composition containing the antibody composition, at least one selected from asthma, eosinophilic sinusitis, and atopic dermatitis is exemplified.

c33) An antibody composition for which the first antigen is CRTH2 and the second antigen is ST2, and the target cell is a Tpath2 cell.

As the disease to be treated with a pharmaceutical composition containing the antibody composition, at least one of asthma and eosinophilic sinusitis is exemplified.

c34) An antibody composition for which the first antigen is CRTH2 and the second antigen is CCR6, and the target cell is a Th2/Th17 cell (a heterozygous cell that appears only in severe asthma).

As the disease to be treated with a pharmaceutical composition containing the antibody composition, an allergic disease is exemplified.

c35) An antibody composition for which the first antigen is CRTH2 and the second antigen is CCR3, and the target cell is an eosinophil, a basophil, or a Th2 cell.

As the disease to be treated with a pharmaceutical composition containing the antibody composition, an allergic disease is exemplified.

c36) An antibody composition for which the first antigen is CD207 and the second antigen is CD11b or CD1a, and the target cell is a Langerhans cell precursor cell.

As the disease to be treated with a pharmaceutical composition containing the antibody composition, Langerhans cell histiocytosis (LCH) is exemplified.

c37) An antibody composition for which the first antigen is CD123 and the second antigen is HLA-DR, and the target cell is a plasmacytoid dendritic cell.

As the disease to be treated with a pharmaceutical composition containing the antibody composition, systemic lupus erythematosus is exemplified.

c38) An antibody composition for which the first antigen is CD123 and the second antigen is ASCT2, and the target cell is at least one selected from a plasmacytoid dendritic cell, a Th1 cell, and a Th17 cell.

As the disease to be treated with a pharmaceutical composition containing the antibody composition, systemic lupus erythematosus is exemplified.

c39) An antibody composition for which the first antigen is CSF1R and the second antigen is CD14, and the target cell is a monocytic myeloid-derived suppressor cell.

As the disease to be treated with a pharmaceutical composition containing the antibody composition, idiopathic pulmonary fibrosis is exemplified.

c40) An antibody composition for which the first antigen is CSF1R and the second antigen is CD33, and the target cell is a myeloid-derived suppressor cell.

As the disease to be treated with a pharmaceutical composition containing the antibody composition, a cancer is exemplified.

c41) An antibody composition for which the first antigen is CD19 and the second antigen is CD38, and the target cell is a plasma cell.

As the disease to be treated with a pharmaceutical composition containing the antibody composition, systemic lupus erythematosus is exemplified.

c42) An antibody composition for which the first antigen is CD3 and the second antigen is IL-23R, and the target cell is at least one selected from a Th1 cell, a Th17 cell, and a Tfh cell.

As the disease to be treated with a pharmaceutical composition containing the antibody composition, systemic

lupus erythematosus is exemplified.

c43) An antibody composition for which the first antigen is CD3 and the second antigen is CX3CR1, and the target cell is a CX3CR1-positive cell (for example, a cytotoxic effector T-cell or the like, excluding NK cells).

As the disease to be treated with a pharmaceutical composition containing the antibody composition, at least one selected from an autoimmune disease (for example, Crane disease or the like), arteriosclerosis, and an ischemic heart disease is exemplified.

c44) An antibody composition for which the first antigen is CXCR4 and the second antigen is type 1 collagen, and the target cell is a fiber cell.

As the disease to be treated with a pharmaceutical composition containing the antibody composition, at least one selected from various fibrotic diseases such as idiopathic pulmonary fibrosis, systemic sclerosis, and hepatic cirrhosis is exemplified.

c45) An antibody composition for which the first antigen is CXCR4 and the second antigen is CD14, and the target cell is at least one of a monocyte and a fiber cell.

As the disease to be treated with a pharmaceutical composition containing the antibody composition, idiopathic pulmonary fibrosis is exemplified.

c46) An antibody composition for which the first antigen is CXCR4 and the second antigen is CD 16, and the target cell is an activated neutrophil.

As the disease to be treated with a pharmaceutical composition containing the antibody composition, at least one selected from neutrophilic asthma, chronic obstructive pulmonary disease, and Alzheimer's disease is exemplified.

c47) An antibody composition for which the first antigen is CLEC10A and the second antigen is CD 14 or CD 16, and the target cell is an intermediate monocyte.

As the disease to be treated with a pharmaceutical composition containing the antibody composition, at least one selected from Crohn's disease, rheumatoid arthritis, and asthma is exemplified.

c48) An antibody composition for which the first antigen is CD70 and the second antigen is CD38, and the target cell is at least one of an activated T cell and an activated B cell.

As the disease to be treated with a pharmaceutical composition containing the antibody composition, at least one of multiple sclerosis and neuromyelitis optica is exemplified.

c49) An antibody composition for which the first antigen is CD70 and the second antigen is one selected from CD4, CD127, and TIM-1, and the target cell is at least one selected from a Th1 cell, a Th17 cell, and a plasmacytoid dendritic cell.

As the disease to be treated with a pharmaceutical composition containing the antibody composition, systemic lupus erythematosus is exemplified.

c50) An antibody composition for which the first antigen is CD4 and the second antigen is TIM-1, and the target cell is at least one selected from a Th1 cell, a Th17 cell, and a plasmacytoid dendritic cell.

As the disease to be treated with a pharmaceutical composition containing the antibody composition, systemic lupus erythematosus is exemplified.

c51) An antibody composition for which the first antigen is CD70 and the second antigen is CD52, and the target cell is at least one of an activated T cell and an activated B cell.

As the disease to be treated with a pharmaceutical composition containing the antibody composition, multiple sclerosis is exemplified.

c52) An antibody composition for which the first antigen is CB1 and the second antigen is AT1R, and the target cell is a GPCR heteromer expressing bad astrocyte.

As the disease to be treated with a pharmaceutical composition containing the antibody composition, hepatic cirrhosis is exemplified.

c53) An antibody composition for which the first antigen is CD4 or PD-1 and the second antigen is CD153, and the target cell is a senescent T cell.

As the disease to be treated with a pharmaceutical composition containing the antibody composition, systemic lupus erythematosus is exemplified.

c54) An antibody composition for which the first antigen is FCεRI and the second antigen is CD34 or C-KIT, and the target cell is at least one of a mast cell and a mast cell precursor cell.

As the disease to be treated with a pharmaceutical composition containing the antibody composition, a mast cell activation syndrome (MCAS) including at least one selected from urticaria, a food allergy, and mastocytosis is exemplified.

c55) An antibody composition for which the first antigen is CD34 and the second antigen is CD203 or MRGPRX2, and the target cell is a mast cell precursor cell.

As the disease to be treated with a pharmaceutical composition containing the antibody composition, a mast cell activation syndrome (MCAS) including at least one selected from urticaria, a food allergy, and mastocytosis is exemplified.

c56) An antibody composition for which the first antigen is CD52 and the second antigen is CD 127, and the target cell is at least one of a T cell other than Treg and a B cell.

As the disease to be treated with a pharmaceutical composition containing the antibody composition, multiple sclerosis is exemplified.

c57) An antibody composition for which the first antigen is CD69 and the second antigen is CD21, and the target cell is an activated mature naive B cell.

As the disease to be treated with a pharmaceutical composition containing the antibody composition, at least one selected from multiple sclerosis, type 1 diabetes mellitus, and rheumatoid arthritis is exemplified.

c58) An antibody composition for which the first antigen is CD 106 and the second antigen is one selected from CD11c, CD 19, CD21, and CD72, and the target cell is a follicular dendritic cell.

As the disease to be treated with a pharmaceutical composition containing the antibody composition, at least one selected from secondary progressive multiple sclerosis (SPMS), rheumatoid arthritis for which anti-TNF therapy is not effective, transplantation, and systemic lupus erythematosus is exemplified.

c59) An antibody composition for which the first antigen is 4-1BB and the second antigen is one selected from CD11c, CD 19, CD21, and CD72, and the target cell is a follicular dendritic cell.

As the disease to be treated with a pharmaceutical composition containing the antibody composition, at least one selected from secondary progressive multiple sclerosis (SPMS), rheumatoid arthritis for which anti-TNF therapy is not effective, transplantation, and systemic lupus erythematosus is exemplified.

c60) An antibody composition for which the first antigen is BLT1 and the second antigen is CD49b, and the target cell is a BLT1-positive CD8-positive cell.

As the disease to be treated with a pharmaceutical composition containing the antibody composition, asthma is exemplified.

c61) An antibody composition for which the first antigen is CD226 and the second antigen is CD8, and the target cell is a CD226-positive CD8-positive effector memory cell.

As the disease to be treated with a pharmaceutical composition containing the antibody composition, systemic scleroderma is exemplified.

c62) An antibody composition for which the first antigen is CXCR3 and the second antigen is one selected from CD8, CD49a, IL-15R, and NKG2D, and the target cell is an effector memory CD8-positive cell.

As the disease to be treated with a pharmaceutical composition containing the antibody composition, at least one of vitiligo and psoriasis is exemplified.

c63) An antibody composition for which the first antigen is CD49a and the second antigen is one selected from CD8, IL-15R, and NKG2D, and the target cell is an effector memory CD8-positive cell.

As the disease to be treated with a pharmaceutical composition containing the antibody composition, at least one of vitiligo and psoriasis is exemplified.

c64) An antibody composition for which the first antigen is IL-15R and the second antigen is CD8 or NKG2D, and the target cell is an effector memory CD8-positive cell.

As the disease to be treated with a pharmaceutical composition containing the antibody composition, at least one of vitiligo and psoriasis is exemplified.

c65) An antibody composition for which the first antigen is NKG2D and the second antigen is CD8, and the target cell is an effector memory CD8-positive cell.

As the disease to be treated with a pharmaceutical composition containing the antibody composition, at least one of vitiligo and psoriasis is exemplified.

c66) An antibody composition for which the first antigen is CD177 and the second antigen is PR3, and the target cell is at least one of a neutrophil infiltrating outside the blood vessel and a PR3-presenting neutrophil.

As the disease to be treated with a pharmaceutical composition containing the antibody composition, at least one of ANCA (antineutrophil cytoplasmic antibody)-associated vasculitis and systemic lupus erythematosus is exemplified.

c67) An antibody composition for which the first antigen is CD4 and the second antigen is CD127, and the target cell is a CD4-positive T cell other than Treg.

As the disease to be treated with a pharmaceutical composition containing the antibody composition, a T cell-dependent immune-related disease is exemplified.

c68) An antibody composition for which the first antigen is CD40 ligand and the second antigen is IL-6, and the target cell is an activated T cell.

As the disease to be treated with a pharmaceutical composition containing the antibody composition, an immune-related disease is exemplified.

c69) An antibody composition for which the first antigen is IL-17R and the second antigen is membrane-associated TNF, and the target cell is an IL-17R and membrane-associated TNF-expressing cell.

As the disease to be treated with a pharmaceutical composition containing the antibody composition, psoriatic

arthritis is exemplified.

c70) An antibody composition for which the first antigen is IL-23R and the second antigen is CCR6, and the target cell is a Th17 cell.

As the disease to be treated with a pharmaceutical composition containing the antibody composition, an autoimmune disease including at least one of Sjogren's syndrome and psoriasis is exemplified.

c71) An antibody composition for which the first antigen is CD64 and the second antigen is one selected from CD206, CD163, and CD68, and the target cell is an M2 macrophage.

As the disease to be treated with a pharmaceutical composition containing the antibody composition, a cancer is exemplified.

c72) An antibody composition for which the first antigen is CD163 and the second antigen is CD206 or CD68, and the target cell is an M2 macrophage.

As the disease to be treated with a pharmaceutical composition containing the antibody composition, a cancer is exemplified.

c73) An antibody composition for which the first antigen is CD206 and the second antigen is CD68, and the target cell is an M2 macrophage.

As the disease to be treated with a pharmaceutical composition containing the antibody composition, a cancer is exemplified.

c74) An antibody composition for which the first antigen is CD14 and the second antigen is one selected from CD48, CD84, CD97, and CD305, and the target cell is a myeloid-derived suppressor cell.

As the disease to be treated with a pharmaceutical composition containing the antibody composition, a cancer is exemplified.

c75) An antibody composition for which the first antigen is CD15 and the second antigen is one selected from CD48, CD84, CD97, and CD305, and the target cell is a myeloid-derived suppressor cell.

As the disease to be treated with a pharmaceutical composition containing the antibody composition, a cancer is exemplified.

c76) An antibody composition for which the first antigen is CD33 and the second antigen is one selected from CD48, CD84, CD97, and CD305, and the target cell is a myeloid-derived suppressor cell.

As the disease to be treated with a pharmaceutical composition containing the antibody composition, a cancer is exemplified.

c77) An antibody composition for which the first antigen is CD11b and the second antigen is one selected from CD48, CD84, CD97, and CD305, and the target cell is a myeloid-derived suppressor cell.

As the disease to be treated with a pharmaceutical composition containing the antibody composition, a cancer is exemplified.

c78) An antibody composition for which the first antigen is CCR4 and the second antigen is one selected from CADM1, CD30, and CD70, and the target cell is a tumor cell.

As the disease to be treated with a pharmaceutical composition containing the antibody composition, at least one of leukemia and lymphoma is exemplified.

c79) An antibody composition for which the first antigen is CD38 and the second antigen is CD138 or BCMA, and the target cell is a tumor cell.

As the disease to be treated with a pharmaceutical composition containing the antibody composition, at least one of leukemia and lymphoma is exemplified.

c80) An antibody composition for which the first antigen is BCMA and the second antigen is CD56 or CS 1, and the target cell is a tumor cell.

As the disease to be treated with a pharmaceutical composition containing the antibody composition, at least one of leukemia and lymphoma is exemplified.

c81) An antibody composition for which the first antigen is CD40 ligand and the second antigen is one selected from CD36, CD62P, and CD63, and the target cell is an abnormal megakaryocyte.

As the disease to be treated with a pharmaceutical composition containing the antibody composition, at least one of leukemia and lymphoma is exemplified.

c82) An antibody composition for which the first antigen is TIM-3 and the second antigen is one selected from CD123, CD33, CD47, CD70, and CLEC12A, and the target cell is a tumor cell.

As the disease to be treated with a pharmaceutical composition containing the antibody composition, at least one of leukemia and lymphoma is exemplified.

c83) An antibody composition for which the first antigen is CD123 and the second antigen is one selected from CD33, CD47, CD70, and CLEC12A, and the target cell is a tumor cell.

As the disease to be treated with a pharmaceutical composition containing the antibody composition, at least one of leukemia and lymphoma is exemplified.

c84) An antibody composition for which the first antigen is CD5 and the second antigen is CD23, and the target

cell is a tumor cell.

As the disease to be treated with a pharmaceutical composition containing the antibody composition, at least one of leukemia and lymphoma is exemplified.

c85) An antibody composition for which the first antigen is CD10 or CD5 and the second antigen is CD20, and the target cell is a tumor cell.

As the disease to be treated with a pharmaceutical composition containing the antibody composition, at least one of leukemia and lymphoma is exemplified.

c86) An antibody composition for which the first antigen is CD40 and the second antigen is one selected from CD80, CD86, ICOS ligand, 4-1BB ligand, OX40 ligand, CD70, GITR, PD-L1, PD-L2, B7-DC, B7H3, B7H4, B7H5, B7H6, and B7H7, and the target cell is an activated antigen-presenting cell.

As the disease to be treated with a pharmaceutical composition containing the antibody composition, an inflammatory disease is exemplified.

c87) An antibody composition for which the first antigen is PTPRS and the second antigen is IL-21R, and the target cell is at least one selected from a plasmacytoid dendritic cell (pDC), a T cell, and a B cell.

As the disease to be treated with a pharmaceutical composition containing the antibody composition, an autoimmune disease is exemplified.

c88) An antibody composition for which the first antigen is PTPRS and the second antigen is CD38, and the target cell is at least one selected from a plasmacytoid dendritic cell (pDC), an activated T cell, a B cell, and a plasma cell.

As the disease to be treated with a pharmaceutical composition containing the antibody composition, an autoimmune disease is exemplified.

c89) An antibody composition for which the first antigen is PTPRS and the second antigen is CD32a, and the target cell is at least one of a plasmacytoid dendritic cell (pDC) and a myeloid cell.

As the disease to be treated with a pharmaceutical composition containing the antibody composition, an autoimmune disease is exemplified.

c90) An antibody composition for which the first antigen is OX40 and the second antigen is CD127 or CD40 ligand, and the target cell is a helper T cell other than Treg.

As the disease to be treated with a pharmaceutical composition containing the antibody composition, an allergic disease is exemplified.

c91) An antibody composition for which the first antigen is OX40 and the second antigen is CD8 or NKG2D, and the target cell is a CD8 memory T cell.

As the disease to be treated with a pharmaceutical composition containing the antibody composition, at least one of vitiligo and psoriasis is exemplified.

c92) An antibody composition for which the first antigen is OX40 and the second antigen is CD226, and the target cell is a CD8 memory T cell.

As the disease to be treated with a pharmaceutical composition containing the antibody composition, scleroderma is exemplified.

c93) An antibody composition for which the first antigen is OX40 and the second antigen is CRTH2, and the target cell is an activated Th2 cell.

As the disease to be treated with a pharmaceutical composition containing the antibody composition, an allergic disease is exemplified.

c94) An antibody composition for which the first antigen is CRTH2 and the second antigen is CD2, and the target cell is at least one of a Th2 cell and an innate lymphoid cell (ILC2 cell).

As the disease to be treated with a pharmaceutical composition containing the antibody composition, an allergic disease is exemplified.

c95) An antibody composition for which the first antigen is CRTH2 and the second antigen is CD7, and the target cell is at least one of a Th2 cell and an innate lymphoid cell (ILC2 cell).

As the disease to be treated with a pharmaceutical composition containing the antibody composition, an allergic disease is exemplified.

c96) An antibody composition for which the first antigen is CRTH2 and the second antigen is CD45, and the target cell is a CRTH2-positive blood cell.

As the disease to be treated with a pharmaceutical composition containing the antibody composition, an allergic disease is exemplified.

[Table 6A]

| First antigen | Second antigen | Disease to be treated | Target cell |
|---|---|---|---|
| CADM1 | CCR4 | adult T-cell leukemia | adult T-cell leukemia (ATL) cell |

(continued)

| First antigen | Second antigen | Disease to be treated | Target cell |
|---|---|---|---|
| EGFR | HER2 | solid cancer such as gastric cancer or breast cancer | cancer cell |
| CD52 | CD70 | - | activated T cell, activated B cell, or activated APC |
| CD2 | CD70 | - | activated T cell |
| CD19 | CD70 | - | activated B cell |
| CD2 | CD40 ligand | Sjogren's syndrome | activated effector T cell (activated Teff) |
| PD-L1 | CD19 | lymphoma or leukemia | lymphoma or leukemia cell |
| | CD30 | | |
| | CCR4 | | |
| | CD20 | | |
| | CD22 | | |
| | CD79b | | |
| CD8 | CCR4 | cancer (cancer immunotherapy) | regulatory T cell (Treg) |
| CTLA-4 | CD4 | | |
| | CCR4 | | |
| | GITR | | |
| TIGIT | CD4 | | |
| | CCR4 | | |
| | GITR | | |
| PD-1 | CD4 | | |
| | CCR4 | | |
| | GITR | | |

(continued)

| First antigen | Second antigen | Disease to be treated | Target cell |
|---|---|---|---|
| OX40 | CD127 | autoimmune disease | effector T cell (Teff) |
| | CD26 | | |
| | CD70 | | |
| | CD15s | | |
| 4-1BB | CD127 | | |
| | CD26 | | |
| | CD70 | | |
| | CD15s | | |
| GITR | CD127 | | |
| | CD26 | | |
| | CD70 | | |
| | CD15s | | |
| CD40 ligand | CD127 | | |
| | CD26 | | |
| | CD70 | | |
| | CD15s | | |
| CD4 | CD69 | ANCA-associated glomerulonephritis | tissue-resident memory T (TRM) cell |

[Table 6B]

| First antigen | Second antigen | Disease to be treated | Target cell |
|---|---|---|---|
| PD-1 | CD3 | rheumatoid arthritis | peripheral helper T (TPH) cell and PD-1high CD8-positive T cell |
| | CD4 | celiac disease | PD-1-positive T cell |
| integrin $\alpha 4\beta 7$ | CCR6 | inflammatory bowel disease | effector T cell (Teff) and innate lymphoid cell (ILC) |
| | CXCR3 | | |
| | CD161 | | |
| | CD127 | inflammatory bowel disease | T cell infiltrating intestinal tract other than all innate lymphoid cells (ILC) and Treg |
| | CD40 ligand | multiple sclerosis or inflammatory bowel disease | local migratory activated T cell |
| integrin $\alpha 4\beta 1$ | CD40 ligand | multiple sclerosis or inflammatory bowel disease | local migratory activated T cell |
| CXCR5 | CD127 | allergic disease | innate lymphoid cell (ILC) |
| TPO receptor (c-mpl) | CD34 | primary myelofibrosis | abnormally proliferating hematopoietic stem cell |
| | CD123 | | CD123-positive leukemic stem cell |

(continued)

| First antigen | Second antigen | Disease to be treated | Target cell |
|---|---|---|---|
| CXCR3 | CD3 | autoimmune disease (Crohn's disease or the like), arteriosclerosis, or ischemic heart disease | CX3CR1-positive cell (cytotoxic effector T-cell or the like, excluding NK cells) |
| | CD127 | systemic lupus erythematosus | Th1 cell |
| | CD40 ligand | | |
| CCR4 | CD127 | allergic disease | CCR4-positive T cell other than Treg |
| | CD40 ligand | | |
| CCR6 | CD127 | psoriasis | Th17 cell |
| | CD40 ligand | | |
| CRTH2 | CD127 | allergic disease | Th2 cell |
| | CD40 ligand | | |
| | CCR4 | asthma, eosinophilic sinusitis, or atopic dermatitis | |
| | ST2 | asthma and eosinophilic sinusitis | Tpath2 cell |
| | CCR6 | allergic disease | Th2/Th17 cell (heterozygous cell that appears only in severe asthma) |
| | CCR3 | | eosinophil, basophil, or Th2 cell |
| CD207 | CD11b | Langerhans cell histiocytosis (LCH) | Langerhans cell precursor cell |
| | CD1a | | |
| CD123 | HLA-DR | systemic lupus erythematosus | plasmacytoid dendritic cell |
| | ASCT2 | | plasmacytoid dendritic cell, Th1 cell, and Th17 cell |
| CSF1R | CD14 | idiopathic pulmonary fibrosis | monocytic myeloid-derived suppressor cell |
| | CD33 | cancer | myeloid-derived suppressor cell |
| CD19 | CD38 | systemic lupus erythematosus | plasma cell |
| CD3 | IL-23R | systemic lupus erythematosus | Th1 cell, Th17 cell, or Tfh cell |
| | CX3CR1 | autoimmune disease (Crohn's disease or the like), arteriosclerosis, or ischemic heart disease | CX3CR1-positive cell (cytotoxic effector T-cell or the like, excluding NK cells) |
| CXCR4 | type 1 collagen | various fibrotic diseases such as idiopathic pulmonary fibrosis, systemic sclerosis, and hepatic cirrhosis | fiber cell |
| | CD14 | idiopathic pulmonary fibrosis | monocyte and fiber cell |
| | CD16 | neutrophilic asthma, chronic obstructive pulmonary disease, or Alzheimer's disease | activated neutrophil |
| CLEC10A | CD14 | Crohn's disease, rheumatoid arthritis, or asthma | intermediate monocyte |
| | CD16 | | |

(continued)

| First antigen | Second antigen | Disease to be treated | Target cell |
|---|---|---|---|
| CD70 | CD38 | multiple sclerosis or neuromyelitis optica | activated T cell and activated B cell |
| | CD4 | systemic lupus erythematosus | Th1 cell, Th17 cell, and plasmacytoid dendritic cell |
| | CD127 | | |
| | TIM-1 | | |
| CD4 | TIM-1 | | |
| CD70 | CD52 | multiple sclerosis | activated T cell and activated B cell |

[Table 6C]

| First antigen | Second antigen | Disease to be treated | Target cell |
|---|---|---|---|
| CB1 | AT1R | hepatic cirrhosis | GPCR heteromer expressing bad astrocyte |
| CD4 | CD153 | systemic lupus erythematosus | senescent T cell |
| PD-1 | | | |
| FCεRI | CD34 | mast cell activation syndromes (MCAS) such as urticaria, food allergy, and mast-cell disease | mast cell and mast cell precursor cell |
| | C-KIT | | |
| CD34 | CD203 | mast cell activation syndromes (MCAS) such as urticaria, food allergy, and mast-cell disease | mast cell precursor cell |
| | MRGPRX2 | | |
| CD52 | CD127 | multiple sclerosis | T cell other than Treg and B cell |
| CD69 | CD21 | multiple sclerosis, type 1 diabetes mellitus, and rheumatoid arthritis | activated mature naive B cell |
| CD106 | CD11c | secondary progressive multiple sclerosis (SPMS), rheumatoid arthritis for which anti-TNF therapy is not effective, transplantation, or systemic lupus erythematosus | follicular dendritic cell |
| | CD19 | | |
| | CD21 | | |
| | CD72 | | |
| 4-1 BB | CD11c | | |
| | CD19 | | |
| | CD21 | | |
| | CD72 | | |
| BLTI | CD49b | asthma | BLT1-positive CD8-positive cell |
| CD226 | CD8 | systemic scleroderma | CD226-positive CD8-positive effector memory cell |

(continued)

| First antigen | Second antigen | Disease to be treated | Target cell |
|---|---|---|---|
| CXCR3 | CD8 | vitiligo or psoriasis | effector memory CD8-positive cell |
| | CD49a | | |
| | IL-15R | | |
| | NKG2D | | |
| CD49a | CD8 | | |
| | IL-15R | | |
| | NKG2D | | |
| IL-15R | CD8 | | |
| | NKG2D | | |
| NKG2D | CD8 | | |
| CD177 | PR3 | ANCA-associated vasculitis or systemic lupus erythematosus | neutrophil infiltrating outside blood vessel and PR3-presenting neutrophil |
| CD4 | CD127 | T cell-dependent immune-related disease | CD4-positive T cell other than Treg |
| CD40 ligand | IL-6 | immune-related disease | activated T cell |
| IL-17R | membrane-associated TNF | psoriatic arthritis | IL-17R and membrane-associated TNF-expressing cell |
| IL-23R | CCR6 | autoimmune disease (Sjogren's syndrome or psoriasis) | Th17 cell |

[Table 6D]

| First antigen | Second antigen | Disease to be treated | Target cell |
|---|---|---|---|
| CD64 | CD206 | cancer | M2 macrophage |
| CD64 | CD163 | | |
| CD64 | CD68 | | |
| CD163 | CD206 | | |
| CD163 | CD68 | | |
| CD206 | CD68 | | |

(continued)

| First antigen | Second antigen | Disease to be treated | Target cell |
|---|---|---|---|
| CD14 | CD48 | cancer | myeloid-derived suppressor cell |
| CD14 | CD84 | | |
| CD14 | CD97 | | |
| CD14 | CD305 | | |
| CD15 | CD48 | | |
| CD15 | CD84 | | |
| CD15 | CD97 | | |
| CD15 | CD305 | | |
| CD33 | CD48 | | |
| CD33 | CD84 | | |
| CD33 | CD97 | | |
| CD33 | CD305 | | |
| CD11b | CD48 | | |
| CD11b | CD84 | | |
| CD11b | CD97 | | |
| CD11b | CD305 | | |
| CCR4 | CADM1 | leukemia/lymphoma | tumor cell |
| CCR4 | CD30 | | |
| CCR4 | CD70 | | |
| CD38 | CD138 | leukemia/lymphoma | tumor cell |
| CD38 | BCMA | | |
| BCMA | CD56 | | |
| BCMA | CS1 | | |
| CD40 ligand | CD36 | leukemia/lymphoma | abnormal megakaryocyte |
| CD40 ligand | CD62P | | |
| CD40 ligand | CD63 | | |
| TIM-3 | CD123 | leukemia/lymphoma | tumor cell |
| TIM-3 | CD33 | | |
| TIM-3 | CD47 | | |
| TIM-3 | CD70 | | |
| TIM-3 | CLEC12A | | |
| CD123 | CD33 | | |
| CD123 | CD47 | | |
| CD123 | CD70 | | |
| CD123 | CLEC12A | | |
| CD5 | CD23 | leukemia/lymphoma | tumor cell |
| CD10 | CD20 | leukemia/lymphoma | tumor cell |
| CD5 | CD20 | leukemia/lymphoma | tumor cell |

[Table 6E]

| First antigen | Second antigen | Disease to be treated | Target cell |
|---|---|---|---|
| CD40 | CD80 | inflammatory disease | activated antigen-presenting cell |
| | CD86 | | |
| | ICOS ligand | | |
| | 4-1BB ligand | | |
| | OX40 ligand | | |
| | CD70 | | |
| | GITR | | |
| | PD-L1 | | |
| | PD-L2 | | |
| | B7-DC | | |
| | 87H3 | | |
| | B7H4 | | |
| | B7H5 | | |
| | B7H6 | | |
| | B7H7 | | |
| PTPRS | IL-21 R | autoimmune disease | plasmacytoid dendritic cell (pDC), T cell, and B cell |
| | CD38 | | plasmacytoid dendritic cell (pDC), activated T cell, B cell, and plasma cell |
| | CD32a | | plasmacytoid dendritic cell (pDC) and myeloid cell |
| OX40 | CD127 | allergic disease | helper T cell other than Treg |
| | CD40 ligand | allergic disease | helper T cell other than Treg |
| | CD8 | vitiligo or psoriasis | CD8 memory T cell |
| | NKG2D | vitiligo or psoriasis | CD8 memory T cell |
| | CD226 | scleroderma | CD8 memory T cell |
| | CRTH2 | allergic disease | activated Th2 cell |
| CRTH2 | CD2 | allergic disease | Th2 cell and innate lymphoid cell (ILC2 cell) |
| | CD7 | allergic disease | Th2 cell and innate lymphoid cell (ILC2 cell) |
| | CD45 | allergic disease | CRTH2-positive blood cell |

A pharmaceutical composition containing the antibody composition of the present invention can be administered alone as a therapeutic agent, however, it is preferably provided as a pharmaceutical preparation produced by generally mixing it together with one or more pharmaceutically acceptable carriers using an arbitrary method well known in the technical field of pharmaceutics.

As the route of administration, it is preferred to use the most effective route in the treatment. For example, oral administration or parenteral administration such as intraoral, intra-airway, intrarectal, subcutaneous, intramuscular, and intravenous administration can be exemplified, and in the case of an antibody composition preparation, preferably, intravenous administration can be exemplified.

Examples of a dosage form include a spray, a capsule, a tablet, a granule, a syrup, an emulsion, a suppository, an injection, an ointment, a tape, and the like.

Examples of the pharmaceutical preparation suitable for oral administration include an emulsion, a syrup, a capsule, a tablet, a powder, a granule, and the like.

A liquid preparation such as an emulsion or a syrup can be produced using, as an additive, water, a saccharide such as sucrose, sorbitol, or fructose, a glycol such as polyethylene glycol or propylene glycol, an oil such as sesame oil, olive oil, or soybean oil, a preservative such as a p-hydroxybenzoate ester, a flavor such as strawberry flavor or peppermint, or the like.

A capsule, a tablet, a powder, a granule, or the like can be produced using, as an additive, an excipient such as lactose, glucose, sucrose, or mannitol, a disintegrating agent such as starch or sodium alginate, a lubricant such as magnesium stearate or talc, a binder such as polyvinyl alcohol, hydroxypropyl cellulose, or gelatin, a surfactant such as a fatty acid ester, a plasticizer such as glycerin, or the like.

Examples of the pharmaceutical preparation suitable for parenteral administration include an injection, a suppository, a spray, or the like.

An injection is prepared using a carrier composed of a salt solution or a glucose solution, or a mixture thereof, or the like. Alternatively, it is also possible to prepare a powder injection by freeze-drying the antibody composition in accordance with a conventional method and adding sodium chloride thereto.

A suppository is prepared using a carrier such as cacao butter, a hydrogenated fat, or carboxylic acid.

Further, a spray is prepared using the antibody composition itself, or using a carrier which does not stimulate the buccal or airway mucous membrane of a recipient and facilitates absorption of the antibody composition by dispersing it as fine particles, or the like.

Specific examples of the carrier include lactose, glycerin, and the like. A pharmaceutical preparation such as an aerosol or a dry powder is possible according to the properties of the antibody composition and the carrier to be used. Further, also for these parenteral preparations, components exemplified as additives for the oral preparations can be added.

A dose or administration frequency varies depending on a desired therapeutic effect, an administration method, a treatment duration, an age, a body weight, or the like, however, a dose of the active ingredient is generally 10 μg/kg to 20 mg/kg per day for an adult.

Further, as a method for studying the anti-tumor effect of the antibody composition on various tumor cells, a CDC activity measurement method, an ADCC activity measurement method, and the like are exemplified as an in vitro experiment, and an anti-tumor experiment, and the like using a tumor system in an experimental animal such as a mouse are exemplified as an in vivo experiment.

As one aspect of the present invention, a kit including the first and second IgG half-molecules is exemplified. In the kit, other materials including an appropriate container (for example, a bottle, a vial, or a test tube), a label showing explanation or the like, a filter, a needle, a syringe, and an instruction manual may be optionally included.

In the above kit, in addition to the IgG half-molecule as the active ingredient, for example, sterile water, physiological saline, a vegetable oil, a surfactant, a lipid, a solubilizing agent, a buffer, a protein stabilizer (for example, BSA, gelatin, or the like), a preservative, a blocking solution, a reaction solution, a reaction stopping solution, a reagent for treating a sample, or the like may be mixed as needed.

Examples of the mode of usage of the kit include (1) a method in which the first IgG half-molecule and the second IgG half-molecule are mixed in advance and then administered, and (2) a method in which the first IgG half-molecule and the second IgG half-molecule are separately administered. Examples of the method (2) in which the first IgG half-molecule and the second IgG half-molecule are separately administered include a method in which the first IgG half-molecule and the second IgG half-molecule are administered simultaneously or sequentially. The amounts and ratios of the first IgG half-molecule and the second IgG half-molecule can be appropriately adjusted.

Further, as one aspect of the present invention, a first IgG half-molecule to be used for producing an antibody composition against a first antigen and a second antigen that are different from each other, the antibody composition including the first IgG half-molecule and a second IgG half-molecule is exemplified.

As another aspect of the present invention, a second IgG half-molecule to be used for producing an antibody composition against a first antigen and a second antigen that are different from each other, the antibody composition including a first IgG half-molecule and the second IgG half-molecule is exemplified.

As one aspect of the present invention, a first IgG half-molecule to be used in combination with a second IgG half-molecule is exemplified. Further, as one aspect of the present invention, a second IgG half-molecule to be used in combination with a first IgG half-molecule is exemplified. Here, the use of the first IgG half-molecule and the second IgG half-molecule "in combination" means that the first IgG half-molecule and the second IgG half-molecule are administered simultaneously or sequentially so as to form an antibody composition including the first IgG half-molecule and the second IgG half-molecule and includes the half-molecules binding to a first antigen and a second antigen that are different from each other. When the first half-molecule and the second half-molecule are administered simultaneously or sequentially,

a case where they are mixed in an injection vial, a case where they are mixed in an infusion bag, a case where they are directly administered to a patient, and a case where they are administered in any form are included in the use of the antibody composition of the present invention.

Hereinafter, the present invention will be more specifically described by way of Examples, but the present invention is not limited to the following Examples.

EXAMPLES

The present inventors conceived that in a constant region of an antibody molecule in the present invention, the following elements that are different from a normal human IgG1 antibody are needed.

1) As shown in Fig. 4, it is a mixture of antibody half-molecules against different antigen molecules X and Y, that is, it is an "HL molecule" in which a covalent bond formed by an inter-H chain disulfide bond is not present in a hinge domain.
2) As shown in Fig. 3, the HL molecules against each of the antigen molecules X and Y bind to the surface of a target cell expressing both X and Y and thereafter are associated with each other to form an H2L2 molecule in the same manner as normal IgG and constitute a CD16a-binding domain to cause an effector function (for example, an ADCC activity) of an antibody.
3) As shown in Fig. 3, a CD16a-binding domain is not formed even if the HL molecules against X or Y are associated with each other on a cell surface so that the antibody activity is not caused against a cell expressing only a single antigen molecule.

In this Example, with respect to the above 1), an attempt was made by substituting cysteine of a hinge domain with alanine.

It is known by X-ray crystallography that Fc of human IgG1 and CD16a show an asymmetric binding mode (Nature 2000; 406: 267-73, J Biol Chem 2001; 276: 16469-77, Mizushima T, Genes Cells 2011; 16: 1071-80). Therefore, the present inventors conceived that the below-mentioned amino acid alteration utilizing such an asymmetric binding mode is introduced into the CH2 domain of the above-mentioned HL molecules for achieving the above 2) and 3).

As shown in Fig. 5, CD16a comes into contact with two CH2 domains in Fc at different sites, respectively. The two CH2 domains are tentatively named CH2-A and CH2-B, and an interacting site with CD16a on CH2-A is defined as a region 1, and an interacting site with CD16a on CH2-B is defined as a region 2.

As the region 1, L235, G236, G237, P238, S239, D265, V266, S267, H268, E269, E294, Q295, Y296, N297, S298, T299, R301, N325, A327, I332, and the like are known. Further, as the region 2, L235, G236, G237, K326, A327, L328, P329, A330, and the like are known.

As shown in Fig. 5, due to the symmetry of the structure of Fc, on the opposite side to the actual binding domain where Fc and CD16a are bound, another binding domain that is composed of the region 2 of CH2-A and the region 1 of CH2-B, and is not actually used for binding to CD16a is present.

As shown in Fig. 6, the region 2 of CH2-A and the region 1 of CH2-B that are not used for binding to the CD16a are "disrupted" by introducing an amino acid alteration into each region. In this case, when HL molecules (X) having such altered CH2-A or HL molecules (Y) having such altered CH2-B are homo-associated to form an H2L2 molecule (XX or YY), only the region 1 or only the region 2 is present. Therefore, it is considered that to such a homo assembly, CD16a may not be able to bind with sufficient affinity. On the other hand, it is considered that an H2L2 molecule (XY) in which the HL molecules having such altered CH2-A and altered CH2-B, respectively, as a constituent element are hetero-associated may satisfy the above conditions 2) and 3).

[Example 1]

Production of human IgG1 anti-CCR6 antibody in which CD 16a binding was "disrupted"

In order to specify a site on CH2 capable of attenuating the binding of CD16a, an antibody in which the above-mentioned region 1 or region 2 was "disrupted" by an amino acid alteration was produced. That is, as shown in Fig. 7, an amino acid residue at a candidate site was altered on a format of normal human IgG1.

In this case, in order to express human IgG1 as a recombinant antibody, two H chains are encoded by the same gene on an expression vector, and an amino acid residue is simultaneously altered in both without distinction between CH2-A and CH2-B, and the CD16a-binding site is simultaneously disrupted at two sites on both sides, resulting in searching for an altered site for attenuating an ADCC activity.

Incidentally, in this Example, unless otherwise stated, as all the antibody molecules, antibodies in which $\alpha$1,6-fucose of the N-linked sugar chain to be bound to asparagine at position 297 of the H chain is not bound so that an

ADCC activity is enhanced were produced. Therefore, as the host cell for expressing the antibody, a fucosyltransferase (FUT8) knockout CHO cell (WO 2005/035586 and WO 02/31140) was used.

(1) Production of expression vector for human IgG1 anti-CCR6 antibody in which CD16a binding was "disrupted"

In Figs. 5 and 6, a schematic diagram of the "region 1" and the "region 2" on the CH2 domain is shown. An expression vector for a human IgG1 anti-human CCR6 antibody (WO 2013/005649) in which an amino acid alteration was introduced with respect to amino acid sites P238, P265, and S267 binding to CD16a in the "region 1" on the CH2 domain and amino acid sites K326, L328, and P329 binding to CD16a in the "region 2" on the CH2 domain for "disrupting" the binding to CD16a as shown in Fig. 6 was constructed. The gene sequences and the amino acid sequences of the antibodies used for the construction are shown in Table 7.

[Table 7]

| Sequence name | Gene sequence | Amino acid sequence |
|---|---|---|
| CCR6 (KG1684) VL | SEQ ID NO: 1 | SEQ ID NO: 2 |
| CCR6 (KG1684) VH | SEQ ID NO: 3 | SEQ ID NO: 4 |
| IgG1 CK | SEQ ID NO: 5 | SEQ ID NO: 6 |
| IgG1 CH | SEQ ID NO: 7 | SEQ ID NO: 8 |
| IgG1 P238A CH | SEQ ID NO: 9 | SEQ ID NO: 10 |
| IgG1 D265A CH | SEQ ID NO: 11 | SEQ ID NO: 12 |
| IgG1 S267L CH | SEQ ID NO: 13 | SEQ ID NO: 14 |
| IgG1 P238A/D265A CH | SEQ ID NO: 15 | SEQ ID NO: 16 |
| IgG1 P238A/S267L CH | SEQ ID NO: 17 | SEQ ID NO: 18 |
| IgG1 D265A/S267L CH | SEQ ID NO: 19 | SEQ ID NO: 20 |
| IgG1 P238A/D265A/S267L CH | SEQ ID NO: 21 | SEQ ID NO: 22 |
| IgG1 K326W CH | SEQ ID NO: 23 | SEQ ID NO: 24 |
| IgG1 L328V CH | SEQ ID NO: 25 | SEQ ID NO: 26 |
| IG1 P329Y CH | SEQ ID NO: 27 | SEQ ID NO: 28 |
| IgG1 K326W/L328V CH | SEQ ID NO: 29 | SEQ ID NO: 30 |
| IgG1 K326W/P329Y CH | SEQ ID NO: 31 | SEQ ID NO: 32 |
| IgG1 L328V/P329Y CH | SEQ ID NO: 33 | SEQ ID NO: 34 |
| IgG1 K326W/L328V/P329Y CH | SEQ ID NO: 35 | SEQ ID NO: 36 |

A PCR reaction was carried out in accordance with a package insert of PrimeSTAR Max DNA Polymerase (Takara Bio) using a human IgG1 anti-human CCR6 antibody expression vector pCI-IgG1_KG1684 composed of the base sequences represented by SEQ ID NOS: 1, 3, 5, and 7 as a template, and also using PrimeSTAR Max DNA Polymerase and primers (Sigma-oligo) into which an altered site was introduced.

In the PCR reaction, GeneAmp PCR System 9700 (Applied Biosystems) was used, and after thermal denaturation at 98°C for 1 minute, a reaction at 98°C for 10 seconds, at 58°C for 5 seconds, and at 72°C for 5 seconds was carried out 30 cycles. The reaction solution was subjected to electrophoresis using 0.8% agarose gel, and an amplified fragment was recovered using QIAquick Gel Extraction Kit (Qiagen). A ligation reaction with a plasmid pCI vector (Promega) was carried out using In-Fusion HD Cloning Kit (Clontech), and Escherichia coli DH5α competent cells (Takara Bio) were transformed using the reaction solution.

Each plasmid DNA was prepared from the thus obtained transformant clones and allowed to react using Big Dye Terminator Cycle Sequencing Kit v3.1 (Applied Biosystems) in accordance with the instruction attached thereto, and then, the base sequence of the DNA inserted into the plasmid was analyzed by a DNA sequencer ABI PRISM 3700 DNA Analyzer of the same company.

(2) Expression of human IgG1 anti-CCR6 antibody in which CD16a binding was "disrupted"

The expression vector produced in (1) was introduced into a host cell by the following method. As the host cell, a FUT8 knockout CHO cell (WO 2005/035586 and WO 02/31140) was used. A method for introducing a plasmid was carried out in accordance with the instruction attached thereto.

The amount of the culture solution was set to 200 mL, and the culture was carried out for 5 days under the set conditions of 37°C, 5% $CO_2$, and 125 rpm. After culture, the cell suspension was centrifuged, and passed through a 0.2 μm filter (Thermo Scientific), whereby the culture supernatant containing an altered antibody was recovered.

The name of a purified antibody sample of each antibody is shown in Table 8.

[Table 8]

| Purified antibody (name) | Region 1 of CH2 | Region 2 of CH2 |
|---|---|---|
| IgG1 | - | - |
| IgG1 P238A | P238A | P238A |
| IgG1 D265A | D265A | D265A |
| IgG1 S267L | S267L | S267L |
| IgG1 P238A/D265A | P238A/D265A | |
| IgG1 P238A/S267L | P238A/S267L | |
| IgG1 D265A/S267L | D265A/S267L | |
| IgG1 P238A/D265A/S267L | P238A/D265A/S267L | - |
| IgG1 K326W | - | K326W |
| IgG1 L328V | - | L328V |
| IG 1 P329Y | - | P329Y |
| IgG1 K326W/L328V | - | K326W/L328V |
| IgG1 K326W/P329Y | - | K326W/P329Y |
| IgG1 L328V/P329Y | - | L328V/P329Y |
| IgG1 K326W/L328V/P329Y | - | K326W/L328V/P329Y |

(3) Purification of human IgG1 anti-CCR6 antibody in which CD16a binding was "disrupted"

The altered antibody was purified by affinity purification using MabSelect SuRe (GE Healthcare) shown below. After a resin was equilibrated with PBS, the culture supernatant obtained in (2) was loaded, followed by washing twice with PBS. After washing, the antibody was eluted using an elution buffer (100 mM citric acid, pH 3.5), followed by neutralization by adding 1/10 amount of a neutralization buffer (2 M Tris-HCl, pH 8.0).

Subsequently, concentration and replacement with buffer (10 mM citric acid, 150 mM NaCl, pH 6.0) by ultrafiltration were carried out using Amicon Ultra-4 Centerifugal Filter Units (Millipore), and an absorbance (A280) at 280 nM was measured using NanoDrop 8000 (Thermo Scientific), whereby the concentration measurement and preparation of an antibody solution were carried out.

(4) Evaluation of purification degree by SDS-PAGE of human IgG1 anti-CCR6 antibody in which CD16a binding was "disrupted"

In order to evaluate the purification degree of the purified samples of various types of CD16-binding-deficient human IgG1 anti-CCR6 antibodies, by using about 1 μg of each antibody purified sample, SDS denaturing polyacrylamide gel electrophoresis (hereinafter referred to as SDS-PAGE) was carried out according to a known method [Nature, 227, 680 (1970)].

As a result, under reducing conditions, in the various types of CD16-binding-deficient human IgG1 anti-CCR6 antibodies, in the same manner as in the normal IgG1 type, a band was observed in the vicinity of about 50 kilodaltons (hereinafter referred to as kDa) for the H chain and in the vicinity of about 25 kDa for the L chain. Further, under non-reducing conditions, a band was observed in the vicinity of about 150 kDa, and therefore, it was confirmed that the

produced anti-CCR6 domain-exchanged antibodies are constituted by the target H chain and L chain.

From the above results, it was confirmed that in the purified samples of various types of CD16-binding-deficient human IgG1 anti-CCR6 antibodies obtained in the section 3 of this Example, target IgG molecules each constituted by the H chain and the L chain are contained at a sufficient ratio.

(5) ADCC activity of human IgG1 anti-CCR6 antibody in which CD16a-binding domain was "disrupted"

As the effector cell, an NK-92/CD16 transfectant obtained by transfecting NK-92 (ATCC) that is an NK cell line with a DNA encoding human CD16a (Val type) to stably express it was used, and as the target cell, a human CCR6/CHO transfectant was used. The respective cultured cells were recovered and counted, and then prepared using RPMI medium (phenol red-free RPMI 1640 medium supplemented with 5% FB S/ 1 % PS) at a cell density of $8 \times 10^5$ cells/mL and $2 \times 10^5$ cells/mL, respectively.

After the antibody solution was dispensed into a 96-well plate at 50 μL/well with a continuous dispenser, and thereafter, the target cells were dispensed at 50 μL/well. The effector cells were dispensed at 50 μL/well, followed by centrifugation at 1800 rpm for 2 minutes, and it was confirmed that the cells were evenly seeded.

After the plate was left to stand at 37°C for 3 hours and 15 minutes in a $CO_2$ incubator, 10/1 amount of a solubilizing solution was dispensed into a Total control, and the plate was left to stand at 37°C for 45 minutes. After centrifugation at 1800 rpm for 2 minutes, 50 μL of the supernatant was dispensed into an ELISA plate. A substrate (powder) was dissolved in 12 mL of a suspension buffer to prepare a coloring solution, and the coloring solution was applied at 50 μL/well to cause a reaction. A stopping solution was added at 50 μL/well, and an absorbance (A450) was measured using a plate reader.

Note that as a kit for ADCC measurement, CytoTox 96(R) Non-Radioactive Cytotoxicity Assay (Promega) was used. An ADCC activity (%) was calculated using the following formula.

$$\text{ADCC activity (\%)} = 100 \times (S - E - T) / (Max - T)$$

$$S = \text{absorbance of sample reaction well - absorbance of culture medium well}$$

$$E = \text{absorbance of effector well - absorbance of culture medium well}$$

$$T = \text{absorbance of target well - absorbance of culture medium well}$$

$$Max = \text{100\% reaction well - 100\% reaction control well}$$

The results are shown in Fig. 8. As shown in Fig. 8, it was confirmed that in the region 1 of CH2, in variants composed of IgG1_D265A, IgG1_P238A/S267L, or IgG1_D265A/S267L, the ADCC activity is significantly attenuated against the target cells.

Further, it was confirmed that in the region 2 of CH2, in variants composed of IgG1_P329Y, IgG1_K326W/P329Y, IgG1_L328V/P329Y, or IgG1_K326W/L328V/P329Y, the ADCC activity is significantly attenuated, and it was found that a P329Y alteration is included in any of the variants.

From the above results, it was revealed that the CD16a-binding site can be disrupted by introducing an amino acid alteration of D265A or P238A/S267L in the "region 1" on the CH2 domain, and by introducing an amino acid alteration of at least P329Y in the "region 2" on the CH2 domain.

[Example 2]

Production of human IgG1 anti-CCR6 monovalent antibody into which CD16a-binding asymmetric alteration was introduced

As an example of an amino acid sequence capable of disrupting an asymmetric binding site of CD16a to Fc in Example 1, D265A or P238A/S267L derived from the "region 1" was selected in CH2-A, and P329Y derived from the "region 2" was selected in CH2-B.

In this section, in order to evaluate an ADCC activity by asymmetrically introducing the above-mentioned

alterations into only CH2-A and CH2-B, respectively, "a monovalent antibody" in which individual genes encoding two molecules of H chain are expressed, and the molecules can be hetero-associated (WO 2011/108502) was used as a basic skeleton. In Fig. 9, a schematic diagram of one embodiment of such a monovalent antibody is shown. In Fig. 9, the above-mentioned hetero assembly is "asymmetric variant #1".

(1) Production of expression vector for CD16a-binding asymmetrically altered monovalent antibody

An expression vector for a human IgG1 anti-CCR6 monovalent antibody into which CH2-A (D265A or P238A/S267L) and CH2-B (P329Y) were asymmetrically or symmetrically introduced (WO 2011/108502) was constructed.

A PCR reaction was carried out in accordance with a package insert of PrimeSTAR Max DNA Polymerase (Takara Bio) using the human IgG1 anti-CCR6 monovalent antibody expression vector pCI-mvG1_KG1684 as a template, and also using PrimeSTAR Max DNA Polymerase and primers (Sigma-oligo) into which an altered site was introduced.

In the PCR reaction, GeneAmp PCR System 9700 (Applied Biosystems) was used, and after thermal denaturation at 98°C for 1 minute, a reaction at 98°C for 10 seconds, at 58°C for 5 seconds, and at 72°C for 5 seconds was carried out 30 cycles.

The reaction solution was subjected to electrophoresis using 0.8% agarose gel, and an amplified fragment was recovered using QIAquick Gel Extraction Kit (Qiagen). A ligation reaction with a plasmid pCI vector (Promega) was carried out using In-Fusion HD Cloning Kit (Clontech), and Escherichia coli DH5α competent cells (Takara Bio) were transformed using the reaction solution.

Each plasmid DNA was prepared from the thus obtained transformant clones and allowed to react using Big Dye Terminator Cycle Sequencing Kit v3.1 (Applied Biosystems) in accordance with the instruction attached thereto, and then, the base sequence of the DNA inserted into the plasmid was analyzed by a DNA sequencer ABI PRISM 3700 DNAAnalyzer of the same company.

(2) Expression of CD16a-binding asymmetrically altered monovalent antibody

The expression of the antibody was carried out in the same manner as in the section 2 of Example 1, and the culture supernatant containing the antibody was recovered. Each purified antibody sample and an altered site thereof are shown in Table 9. Note that an amino acid residue substitution at the altered site was carried out in the same manner as in Example 1.

[Table 9]

| Purified antibody (name) | CH2-A (Region 1, Region 2) | CH2-B (Region 1, Region 2) |
|---|---|---|
| wild type | - | - |
| asymmetric variant #1 | D265A | P329Y |
| asymmetric variant #2 | P329Y | D265A |
| asymmetric variant #3 | P238A/S267L | P329Y |
| asymmetric variant #4 | P329Y | P238A/S267L |
| symmetric variant #1 | D265A | D265A |
| symmetric variant #2 | P329Y | P329Y |
| symmetric variant #3 | P238A/S267L | P238A/S267L |
| symmetric variant #4 | D265A, P329Y | D265A, P329Y |
| symmetric variant #5 | P238A/S267L, P329Y | P238A/S267L, P329Y |

(3) Purification of CD16a-binding asymmetrically altered monovalent antibody

The purification of the antibody was carried out in the same manner as in the section 3 of Example 1. As the elution buffer, 100 mM citric acid, pH 3.9 was used. Thereafter, a monomer fraction was fractionated from the antibody solution using AKTA FPLC (GE Healthcare) and Superdex High-performance Column (GE Healthcare). By performing filter sterilization with a membrane filter (Millex-GV, Millipore) with a pore diameter of 0.22 μm of an AKTA system, a purified antibody was obtained. An absorbance (A280) at 280 nm was measured using NanoDrop 8000 (Thermo Scientific).

(4) Evaluation of purification degree by SDS-PAGE of human IgG1 anti-CCR6 monovalent antibody into which CD16a-binding asymmetric alteration was introduced

In order to evaluate the purification degree of the purified samples of human IgG1 anti-CCR6 monovalent antibodies into which various types of CD16a-binding asymmetric alterations were introduced, SDS-PAGE was carried out using about 1 μg of each antibody purified sample.

As a result, under reducing conditions, in all the variants, a band was observed in the vicinity of about 50 kD for the H chain and the Fc-fused L-chain in the same manner as in the wild-type monovalent antibody. Further, under non-reducing conditions, a band was observed in the vicinity of about 100 kDa, and therefore, it was confirmed that the produced asymmetric variants and symmetric variants are constituted by the target H chain and L chain.

From the above results, it was confirmed that in the purified samples of human IgG1 anti-CCR6 monovalent antibodies into which various types of CD16a-binding asymmetric alterations were introduced obtained in the section 3 of this Example, target monovalent antibody molecules each constituted by the H chain and the Fc-fused L chain are contained at a sufficient ratio.

(5) ADCC activity of CD16a-binding asymmetrically altered monovalent antibody

An ADCC activity was measured in the same manner as in the section 5 of Example

1. The results are shown in Fig. 10. As shown in Fig. 10, it was confirmed that the monovalent antibodies (asymmetric variants #1 to 4) into which a CD16a-binding asymmetric alteration was introduced damage the target cells in an antibody concentration-dependent manner. On the other hand, it was confirmed that the monovalent antibodies (symmetric variants #1 to #5) into which a CD16a-binding symmetric alteration was introduced do not show an ADCC activity against the target cells.

From the above results, it was confirmed that a monovalent antibody into which a CD16a-binding asymmetric alteration was introduced maintains the binding activity to human CD16a and can exhibit an ADCC activity against the target cells.

On the other hand, when the same alteration is introduced into both CH2 domains, an ADCC activity is lost, and therefore, it was confirmed that an ADCC activity is not induced when antibodies having the same altered CH2 are homophilically associated on a cell surface, and altered CH2 domains for constituting an HL antibody that exhibits an ADCC activity only when it is hetero-associated with two antigens on a cell surface aimed at by the present invention were found.

[Example 3]

In this Example, it was verified whether or not an ADCC activity can be induced specifically for a target cell coexpressing two types of antigens by mounting altered CH2 molecules that were obtained in Examples 1 and 2 and can induce an ADCC activity only when they are hetero-associated on two types of antibody half-molecules (HL molecules) against different antigens.

An interaction between CH3 domains has been reported in J. Immunol. 2011; 187: 3238-3246. The interaction ($K_D$ value) of the CH3 domains of human IgG1 is $3.0 \times 10^{-9}$ M, the $K_D$ value of the CH3 domains of human IgG4 is $4.8 \times 10^{-8}$ M, so that the interaction between the CH3 domains of human IgG4 is about 6 to 7 times weaker than that of IgG1.

The production of an antibody half-molecule was attempted by utilizing this property and an amino acid alteration (C226A/C229A: AA) for cleaving an inter-H chain disulfide bond in a hinge domain. A domain-exchanged antibody (hereinafter referred to as IgG1114_AA type) in which for the H chain constant region of a half-molecule, human IgG1 was used as a basic skeleton, and only the CH3 domain was exchanged with a human IgG4 sequence, and further, an amino acid alteration (AA) was added to the hinge domain was used.

In addition, in order to enhance an ADCC activity, a known ADCC activity enhancing amino acid alteration was made in the CH2 domain, and further, α1,6-fucose of the N-linked sugar chain that is bound to Asn297 was removed, and finally, a CD16a-binding asymmetric amino acid alteration was introduced. In Fig. 11, a schematic diagram of an IgG1114_AA_AAA_D265A (/P329Y)-type IgG half-molecule is shown. As two types of model antigens, CD4 and CD70 were used.

An anti-CD4 antibody (J. Immunol. 1992; 149: 1779-1787) and an anti-CD70 antibody (WO 2007/03637) in which the above-mentioned alterations were combined were produced according to the following procedure. The anti-CD4 and anti-CD70 antibodies having an H chain constant region constituted by an amino acid sequence in which a

known ADCC activity enhancing amino acid alteration (S298A/E333A/K334A: AAA) alteration and a CD16a-binding asymmetric amino acid alteration (D265A or P329Y) were introduced into the IgG1114_AA type are referred to as an IgG1114_AA_AAA_D265A (/P329Y)-type anti-CD4 antibody half-molecule, and an IgG1114_AA_AAA_D265A (/P329Y)-type anti-CD70 antibody half-molecule, respectively.

A subclass from which each domain of various types of designed anti-CD4 antibody half-molecules and anti-CD70 antibody half-molecules is derived, and the correspondence of the amino acid sequence of the H chain constant region are shown in Table 10. Further, the base sequences and the amino acid sequences of antibodies against CD4 and CD70 used are shown in Table 11.

[Table 10]

| Structure name | CH1 | Hinge | CH2 | CH3 | Gene and amino acid sequences |
|---|---|---|---|---|---|
| IgG1114_AA_AAA_D265A | G1 | GI(AA) | G1(AAA_D265A) | G4 | SEQ ID NOS: 37 and 38 |
| IgG1114_AA_AAA_P329Y | G1 | GI(AA) | G1(AAA_P329Y) | G4 | SEQ ID NOS: 39 and 40 |

[Table 11]

| Sequence name | Gene sequence | Amino acid sequence |
|---|---|---|
| CD4 (ibalizumab) VL | SEQ ID NO: 41 | SEQ ID NO: 42 |
| CD4 (ibalizumab) VH | SEQ ID NO: 43 | SEQ ID NO: 44 |
| CD70 (2H5) VL | SEQ ID NO: 45 | SEQ ID NO: 46 |
| CD70 (2H5) VH | SEQ ID NO: 47 | SEQ ID NO: 48 |

(1) Production of expression vector for anti-CD4 antibody half-molecule and anti-CD70 antibody half-molecule

A DNA fragment of about 9 kbp was cut out using restriction enzymes NheI and NotI from a human IgG1 anti-CD4 antibody expression vector pCI-IgG1_CD4 (ibalizumab) (SEQ ID NOS: 5, 7, 41, and 43) or a human IgG1 anti-CD70 antibody expression vector pCI-IgG1_CD70 (2H5) (SEQ ID NOS: 5, 7, 45, and 47), and purified.

A ligation reaction of the purified DNA fragment with an artificial synthetic gene composed of the CH1 domain, the hinge domain (to which a C226A/C229A alteration was added) and the CH2 domain (to which a S298A/K333A/E334A alteration and a D265A or P329Y alteration were added) of a human IgG1 antibody, and the CH3 domain of a human IgG4 antibody was carried out using In-Fusion HD Cloning Kit (Clontech), and Escherichia coli DH5α competent cells (Takara Bio) were transformed using the reaction solution. Each plasmid DNA was prepared from the obtained transformant clones, and the base sequence of the DNA was analyzed by Fasmac.

(2) Expression of various types of anti-CD4 IgG1 antibodies and anti-CD70 IgG1 antibodies and half-molecules of these antibodies

The expression of the antibody was carried out in the same manner as in the section 2 of Example 1, and the culture supernatant containing the antibody was recovered.

The names of the purified antibody samples are shown in Table 12.

[Table 12]

| Purified antibody (name) | Structure of antibodv |
|---|---|
| CD4 antibody | IgG1 |
| CD70 antibody | IgG1 |
| CD4 antibodv half- molecule 1 | IgG1114_AA_AAA_D265A |
| CD4 antibodv half- molecule 2 | IgG1114_AA_AAA_P329Y |
| CD70 antibody half- molecule 1 | IgG1114_AA_AAA_D265A |
| CD70 antibody half- molecule 2 | IgG1114_AA_AAA_P329Y |

(3) Purification of anti-CD4 antibody half-molecule and anti-CD70 antibody half-molecule

The purification of the antibody was carried out in the same manner as in the section 3 of Example 1, and the concentration measurement and preparation of an antibody solution were carried out.

(4) Evaluation of purification degree by SDS-PAGE of anti-CD4 antibody half-molecule and anti-CD70 antibody half-molecule

In order to evaluate the purification degree of the prepared various types of antibody samples, SDS-PAGE was carried out using about 1 μg of each antibody purified sample. The results are shown in Fig. 12.

As shown in Fig. 12, in all the purified antibodies, under reducing conditions, a band was observed in the vicinity of about 50 kDa for the H chain and in the vicinity of about 25 kDa for the L chain. Further, under non-reducing conditions, a band was observed in the vicinity of about 150 kDa for the anti-CD4 IgG1 antibody and the anti-CD70 IgG1 antibody, and in the vicinity of about 75 kDa for the anti-CD4 half-molecule and the anti-CD70 half-molecule, and therefore, it was confirmed that the produced anti-CD4 IgG1 antibody and anti-CD70 IgG1 antibody are each an antibody structure constituted by the target H chain and L chain, and the anti-CD4 antibody half-molecule and the anti-CD70 antibody half-molecule are each constituted by the target H chain and L chain, and have an antibody structure that easily forms a half-molecule.

From the above results, it was confirmed that in the purified samples of the anti-CD4 antibody half-molecule and the anti-CD70 antibody half-molecule obtained in the section 3 of this Example, the target antibody molecule is contained at a sufficient ratio.

(5) ADCC activity of anti-CD4 IgG1 antibody and anti-CD70 IgG1 antibody, and half-molecules of these antibodies against CD4/CD70 double-positive cell, and CD4 and CD70 single-positive cells

An ADCC activity was measured in the same manner as in the section 5 of Example 1. As for the target cell, a CD4/EL-4 transfectant was used as a CD4 single-positive cell, MT-1 was used as a CD70 single-positive cell, and TL-Om1 was used as a CD4/CD70 double-positive cell. The results of measuring the expression levels of CD4 and CD70 in these cells using a flow cytometer are shown in Fig. 13.

As shown in Fig. 13, it was confirmed that the target antigen was expressed in the cells. Further, the results of evaluating the ADCC activity are shown in Fig. 14. As the positive control antibody, the anti-CD4 IgG1 antibody and the anti-CD70 IgG1 antibody were used.

As shown in Fig. 14, it was demonstrated as expected that the anti-CD4 antibody exhibits an ADCC activity against the CD4/EL-4 transfectant, the anti-CD70 antibody exhibits an ADCC activity against MT-1, and the anti-CD4 antibody and the anti-CD70 antibody exhibit an ADCC activity against TL-Om1.

On the other hand, an antibody solution obtained by mixing the anti-CD4 antibody half-molecule 1 and the anti-CD70 antibody half-molecule 2 or an antibody solution obtained by mixing the anti-CD4 antibody half-molecule 2 and the anti-CD70 antibody half-molecule 1 does not exhibit an ADCC activity against the CD4 and CD70 single-positive cells, but exhibits an ADCC activity specifically only against the CD4/CD70 double-positive cell.

[Example 4]

It was verified whether or not an ADCC activity can be induced specifically against a target cell coexpressing two types of antigens by mounting altered CH2 in which CD 16a binding was disrupted by amino acid residue alteration at position 235, position 239, position 265, position 267, position 269, position 296, position 298, position 299, or position 327 numbered according to the EU index in the first CD16a-binding domain, and at position 326, position 328, position 329, or position 330 numbered according to the EU index in the second CD 16a-binding domain on the half-molecules (HL molecules) of two types of antibodies against different antigens.

Designed various types of anti-CD4 antibody half-molecules and anti-CD70 antibody half-molecules were prepared in the same manner as in Example 3, and an ADCC activity was evaluated. An antibody solution obtained by mixing the anti-CD4 half-molecule and the anti-CD70 half-molecule was added to give 1 μg/mL. Similarly, the anti-CD4 IgG1 antibody and the anti-CD70 IgG1 antibody used as the positive control antibody were also added to give 1 μg/mL. A subclass from which each domain of various types of designed anti-CD4 antibody half-molecules and anti-CD70 antibody half-molecules is derived, and the correspondence of the amino acid sequence of the H chain constant region are shown in Table 13.

[Table 13]

| Purified antibody (name) | Structure of antibody | SEQ ID NO | |
|---|---|---|---|
| CD4 antibody half- molecule 3 | IgG1114_AA_AAA S239R CH | 49 | gene sequence |
| CD4 antibody half- molecule 3 | IgG1114_AA_AAA_S239R CH | 50 | amino acid sequence |
| CD4 antibody half- molecule 4 | IgG1114_AA_AAA_D265N CH | 51 | gene sequence |
| CD4 antibody half- molecule 4 | IgG1114_AA_AAA D265N CH | 52 | amino acid sequence |
| CD4 antibody half- molecule 5 | IgG1114_AA_AAA_D265E CH | 53 | gene sequence |
| CD4 antibody half- molecule 5 | IgG1114_AA_AAA_D265E CH | 54 | amino acid sequence |
| CD4 antibody half- molecule 6 | IgG1114_AA_AAA S267K CH | 55 | gene sequence |
| CD4 antibody half- molecule 6 | IgG1114_AA_AAA S267K CH | 56 | amino acid sequence |
| CD4 antibody half- molecule 7 | IgG 1114_AA_AAA E269P CH | 57 | gene sequence |
| CD4 antibody half- molecule 7 | IgG1114_AA_AAA E269P CH | 58 | amino acid sequence |
| CD4 antibody half- molecule 8 | IgG1114_AA_AAA Y296P CH | 59 | gene sequence |
| CD4 antibody half- molecule 8 | IgG1114_AA_AAA_Y296P CH | 60 | amino acid sequence |
| CD4 antibody half- molecule 9 | IgG1114_AA_AAA_S298E CH | 61 | gene sequence |
| CD4 antibody half- molecule 9 | IgG1114_AA_AAA_S298E CH | 62 | amino acid sequence |
| CD4 antibody half- molecule 10 | IgG1114_AA_AAA_T299A CH | 63 | gene sequence |
| CD4 antibody half- molecule 10 | IgG1114 AA AAA T299A CH | 64 | amino acid sequence |
| CD4 antibody half- molecule 11 | IgG1114_AA_AAA_L235R CH | 65 | gene sequence |
| CD4 antibody half- molecule 11 | IgG1114_AA_AAA_L235RCH | 66 | amino acid sequence |
| CD4 antibody half- molecule 12 | IgG1114_AA_AAA A327I CH | 67 | gene sequence |
| CD4 antibody half- molecule 12 | IgG1114_AA_AAA_A327I CH | 68 | amino acid sequence |
| CD70 antibody half- molecule 3 | IeG1114_AA_AAA_K326G CH | 69 | gene sequence |
| CD70 antibody half- molecule 3 | IgG1114_AA_AAA_K326G CH | 70 | amino acid sequence |
| CD70 antibody half- molecule 4 | IgG1114_AA_AAA_L328RCH | 71 | gene sequence |
| CD70 antibody half- molecule 4 | IgG1114_AA_AAA L328R CH | 72 | amino acid sequence |
| CD70 antibody half- molecule 5 | IgG1114_AA_AAA_P329K CH | 73 | gene sequence |
| CD70 antibody half- molecule 5 | IgG1114_AA_AAA_P329K CH | 74 | amino acid sequence |
| CD70 antibody half- molecule 6 | IgG1114_AA_AAA_P329W CH | 75 | gene sequence |
| CD70 antibody half- molecule 6 | IgG1114_AA_AAA_P329WCH | 76 | amino acid sequence |
| CD70 antibody half- molecule 7 | IgG1114_AA_AAA_A330P CH | 77 | gene sequence |
| CD70 antibody half- molecule 7 | IgG1114_AA_AAA_ A330P CH | 78 | amino acid sequence |

As shown in Figs. 15A to 15H, it was demonstrated that the antibody solution obtained by mixing the anti-CD4 antibody half-molecule mounted with CH2 with an amino acid alteration in the first CD16a-binding domain and the anti-CD70 antibody half-molecule mounted with CH2 with an amino acid alteration in the second CD16a-binding domain does not exhibit an ADCC activity against the CD4 and CD70 single-positive cells, but exhibits an ADCC activity specifically only against the CD4/CD70 double-positive cell.

[Example 5]

Search for CD16a-Binding Enhancing Amino Acid Alteration

Many known amino acid alterations for enhancing binding to CD16a are known in addition to the S298A/E333A/K334A alteration (WO 99/51642, WO 2006/020114, WO 2004/099249, WO 2004/063351, and WO 2013/118858). In this Example, CD16a-binding enhancing amino acid alterations other than the S298A/E333A/K334A alteration were confirmed.

(1) Production of expression vector for human IgG1 anti-CCR4 antibody in which CD16a-binding enhancing amino acid alteration was introduced

An expression vector for an antibody in which a CD16a-binding enhancing amino acid alteration was introduced into a human IgG1 anti-human CCR4 antibody (WO 2005/053741) was constructed. A DNA fragment of about 9 kbp was cut out using restriction enzymes NheI and NotI from a human IgG1 anti-CCR4 antibody expression vector pCI-IgG1_CCR4 (KM2160) (SEQ ID NOS: 5, 7, 79, and 81), and purified.

A ligation reaction of the purified DNA fragment with an artificial synthetic gene composed of the Fc of the antibody in which the CD16a-binding enhancing amino acid alteration was introduced was carried out using In-Fusion HD Cloning Kit (Clontech), and Escherichia coli DH5α competent cells (Takara Bio) were transformed using the reaction solution. Each plasmid DNA was prepared from the obtained transformant clones, and the base sequence of the DNA was analyzed by Fasmac.

(2) Expression of human IgG1 anti-CCR4 antibody in which CD16a-binding enhancing amino acid alteration was introduced

The expression vector produced in (1) was introduced into a host cell by the following method. As the host cell, a FUT8 knockout CHO cell (WO 2005/035586 and WO 02/31140) was used. A method for introducing a plasmid was carried out in accordance with the instruction attached thereto.

The amount of the culture solution was set to 200 mL, and the culture was carried out for 5 days under the set conditions of 37°C, 5% $CO_2$, and 125 rpm. After culture, the cell suspension was centrifuged, and passed through a 0.2 μm filter (Thermo Scientific), whereby the culture supernatant containing an altered antibody was recovered.

(3) Purification of human IgG1 anti-CCR4 antibody in which CD16a-binding enhancing amino acid alteration was introduced

The altered antibody was purified by affinity purification using MabSelect SuRe (GE Healthcare) shown below. After a resin was equilibrated with PBS, the culture supernatant obtained in (2) was loaded, followed by washing twice with PBS. After washing, the antibody was eluted using an elution buffer (100 mM citric acid, pH 3.5), followed by neutralization by adding 1/10 amount of a neutralization buffer (2 M Tris-HCl, pH 8.0).

Subsequently, concentration and replacement with buffer (10 mM citric acid, 150 mM NaCl, pH 6.0) by ultrafiltration were carried out using Amicon Ultra-4 Centerifugal Filter Units (Millipore), and an absorbance (A280) at 280 nM was measured using NanoDrop 8000 (Thermo Scientific), whereby the concentration measurement and preparation of an antibody solution were carried out.

(4) Evaluation of purification degree of human IgG1 anti-CCR4 antibody in which CD16a-binding enhancing amino acid alteration was introduced

In order to evaluate the purification degree of the prepared various types of antibody samples, by using about 1 μg of each antibody purified sample, gel electrophoresis (LabChip GX, PerkinElmer) was carried out, and as a result, in all the purified antibodies, under reducing conditions, a band was observed in the vicinity of about 50 kDa for the H chain and in the vicinity of about 25 kDa for the L chain. Further, under non-reducing conditions, a band was observed in the vicinity of about 150 kDa, and therefore, it was confirmed that the produced anti-CCR4 IgG1 antibody is an antibody structure constituted by the target H chain and L chain.

(5) ADCC activity of human IgG1 anti-CCR4 antibody in which CD16a-binding enhancing amino acid alteration was introduced

An ADCC activity was measured in the same manner as in Example 1 (5). As the target cell, TL-Om1 (CCR4-positive cell) was used. SEQ ID NOS of the base sequences and the amino acid sequences of VH and VL of the used anti-CCR4 antibody are shown in Table 14, and the base sequence and the amino acid sequence of CH of each anti-CCR4 antibody in which a CD16a-binding enhancing amino acid alteration was introduced are shown in Tables 15 and 16.

[Table 14]

| Sequence name | SEQ ID NO of base sequence | SEQ ID NO of amino acid sequence |
|---|---|---|
| CCR4 (KM2160) VH | 79 | 80 |
| CCR4 (KM2160) VL | 81 | 82 |

The results of evaluating the ADCC activity are shown in the following Tables 15 and 16. The ADCC activity of the antibody in which any of various types of amino acid alterations was introduced was calculated while taking the ADCC activity of the IgG1 antibody including the S298A/E333A/K334A (AAA) alteration at 0.1 μg/mL as 100.

[Table 15]

| CD16a-binding enhancing amino acid alteration introduced into IgG1 anti-CCR4 antibody | relative ADCC activity (AAA = 100) | SEQ ID NO of CH (base sequence) | SEQ ID NO of CH (amino acid sequence) |
|---|---|---|---|
| S298A/E333A/K334A (AAA) | 100 | 83 | 84 |
| S239D/I332E (DE) | 201 | 85 | 86 |
| S239D/A330F | 161 | 87 | 88 |
| S239D/K326T | 179 | 89 | 90 |
| S298A/E333A/K334A (AAA)/P247L | 160 | 91 | 92 |
| S298A/E333A/K334A (AAA)/R292P | 125 | 93 | 94 |
| S298A/E333A/K334A (AAA)/H268E | 164 | 95 | 96 |
| S298A/E333A/K334A (AAA)/K326T | 136 | 97 | 98 |
| S298A/E333A/K334A (AAA)/P247L/N421K | 175 | 99 | 100 |
| S298A/E333A/R292L/K334E | 163 | 101 | 102 |
| S298A/E333A/K334A (AAA)/P329R | 5 | 103 | 104 |
| S298A/E333A/K334A (AAA)/A330F | 96 | 105 | 106 |
| S298A/E333A/K334E | 114 | 107 | 108 |
| S298A/E333A/K334A (AAA)/A339T | 36 | 109 | 110 |
| S298A/E333A/K334A (AAA)/F372Y | 70 | 111 | 112 |
| S298AIE333AIK334A (AAA)/V379M | 51 | 113 | 114 |
| S298A/E333A/K334A (AAA)/S239D/I332E | 119 | 115 | 116 |
| S298A/E333A/K334A (AAA)/E283L | 82 | 117 | 118 |
| S298T/E333A/K334A | -97 | 119 | 120 |
| S298A/E333A/K334A (AAA)/E294W | 171 | 121 | 122 |
| S298A/E333A/K334A (AAA)/Q295T | 4 | 123 | 124 |
| S298A/E333A/K334A (AAA)/A327D | -28 | 125 | 126 |
| S298A/E333A/K334A (AAA)/L235T | -69 | 127 | 128 |
| S298A/E333A/K334A (AAA)/G236S | -46 | 129 | 130 |
| S298A/E333A/K334A (AAA)/S239D | 291 | 131 | 132 |
| S298A/E333A/K334A (AAA)/F243L | 79 | 133 | 134 |

(continued)

| CD16a-binding enhancing amino acid alteration introduced into IgG1 anti-CCR4 antibody | relative ADCC activity (AAA = 100) | SEQ ID NO of CH (base sequence) | SEQ ID NO of CH (amino acid sequence) |
|---|---|---|---|
| S298A/E333A/K334A (AAA)/K248M | 165 | 135 | 136 |
| S298A/E333A/K334A (AAA)/E258H | 94 | 137 | 138 |
| S298A/E333A/K334A (AAA)/D265G | -89 | 139 | 140 |
| S239D | 116 | 141 | 142 |
| S239E | 88 | 143 | 144 |

[Table 16]

| CD16a-binding enhancing amino acid alteration introduced into IgG1 anti-CCR4 antibody | relative ADCC activity (AAA = 100) | SEQ ID NO of CH (base sequence) | SEQ ID NO of CH (amino acid sequence) |
|---|---|---|---|
| S239D/I332D | 150 | 145 | 146 |
| S239D/I332T | 72 | 147 | 148 |
| S239D/I332L | 82 | 149 | 150 |
| S239D/I332A | 94 | 151 | 152 |
| S239D/I332Y | 123 | 153 | 154 |
| S239D/I332F | 93 | 155 | 156 |
| S239D/I332W | 94 | 157 | 158 |
| S239D/I332H | 84 | 159 | 160 |
| S239D/K326L | 113 | 161 | 162 |
| S239D/K326E | 147 | 163 | 164 |
| S239D/K3261 | 146 | 165 | 166 |
| S239E/I332Y | 130 | 167 | 168 |
| S239E/I332F | 34 | 169 | 170 |
| S239E/I332W | 44 | 171 | 172 |
| S239E/I332H | 71 | 173 | 174 |
| S239E/I332E | 153 | 175 | 176 |
| S239E/I332D | 90 | 177 | 178 |
| S239EII332T | 52 | 179 | 180 |
| S239E/I332L | 57 | 181 | 182 |
| S239E/I332A | 62 | 183 | 184 |
| S239E/K326T | 132 | 185 | 186 |
| S239E/K326L | 109 | 187 | 188 |
| S239E/K326E | 129 | 189 | 190 |
| S239E/K3261 | 135 | 191 | 192 |
| F243L/R292P/Y300L | 3 | 193 | 194 |
| F243L/R292P/Y300L/V305I/P396L | -9 | 195 | 196 |
| P230A/E233D/I332E | 53 | 197 | 198 |

(continued)

| CD16a-binding enhancing amino acid alteration introduced into IgG1 anti-CCR4 antibody | relative ADCC activity (AAA = 100) | SEQ ID NO of CH (base sequence) | SEQ ID NO of CH (amino acid sequence) |
|---|---|---|---|
| K288N/A330S/P396L | -131 | 199 | 200 |
| K334E/T359N/T366S | -110 | 201 | 202 |
| G316D/A378V/D399E | -38 | 203 | 204 |

As shown in Tables 15 and 16, CD16a-binding enhancing amino acid alterations applicable to the antibody composition of the present invention were confirmed other than S298A/E333A/K334A (AAA).

[Example 6]

Amino Acid Substitution in CH3 Domain of IgG1

In Example 3, it was shown that the ADCC activity is exhibited specifically for the CD4/CD70 double-positive cell using the IgG half-molecule of IgG1 114_AA_ AAA_D265A (/P329Y). As the portion corresponding to the CH3 domain of the Fc region of the antibody used here, the sequence of human IgG4 is used.

An interaction between CH3 domains has been reported in J. Immunol. 2011; 187: 3238-3246, and the interaction ($K_D$ value) of the CH3 domains of human IgG1 is $3.0 \times 10^{-9}$ M, the $K_D$ value of the CH3 domains of human IgG4 is $4.8 \times 10^{-8}$ M, so that the interaction between the CH3 domains of human IgG4 is about 6 to 7 times weaker than that of human IgG1.

Further, it has been reported that by substituting R409 in the CH3 domain of human IgG4 with K of human IgG1, the interaction between the CH3 domains is enhanced (Structure 2011 19: 9: 1274-1282).

In addition, there has also been a report that an amino acid site important for the interaction between the CH3 domains of human IgG1 was identified based on changes in free energy by substituting an amino acid in the CH3 domain of human IgG1 with Ala (Biochemistry 1998: 37: 9266-9273). From this, the interaction between the CH3 domains of human IgG1 can be attenuated by adding a known amino acid alteration to the CH3 domain of human IgG1.

Therefore, it was verified whether the ADCC activity is exhibited specifically for the double-positive cell by adding an amino acid alteration to the CH3 domain of human IgG1 so as to attenuate the interaction between the CH3 domains of human IgG1.

An anti-CD4 antibody (J. Immunol. 1992; 149; 1779-1787) and an anti-CD70 antibody (WO 2007/03637), in which the interaction between the CH3 domains of IgG1 was attenuated were produced according to the following procedure.

A subclass from which each domain of various types of designed anti-CD4 and anti-CD70 half-molecules is derived, and the correspondence of the amino acid sequence of the H chain constant region are shown in Table 17. When a human IgG1 type has an H chain constant region constituted by an amino acid sequence in which C266A/C229A: AA was introduced as an amino acid alteration for cutting the disulfide bond between the H chains in the hinge domain, S239D/I332E: DE was introduced as an amino acid alteration for enhancing the ADCC activity, D265A was introduced as a CD16a-binding asymmetric amino acid alteration, and a K409R alteration was introduced as an amino acid alteration for attenuating the inter-CH3 domain interaction, it is referred to as "IgG1_AA_DE_D265A_K409R".

[Table 17]

| Purified antibody name (inter-CH3 domain interaction attenuating alteration) | Structure of CH of antibody | SEQ ID NO of base sequence | SEQ ID NO of amino acid sequence |
|---|---|---|---|
| anti-CD4 antibody half-molecule (K409R) | IgG1_AA_ DE_ P329Y_K409R CH | 205 | 206 |
| anti-CD4 antibody half-molecule (Y349A) | IgG1_AA_DE_ D265A_Y349A CH | 207 | 208 |
| anti-CD4 antibody half-molecule (L351A) | IgG1_AA_DE_ D265A_L351A CH | 209 | 210 |
| anti-CD4 antibody half-molecule (T366A) | IgG1_AA_ DE_ D265A_T366A CH | 211 | 212 |

(continued)

| Purified antibody name (inter-CH3 domain interaction attenuating alteration) | Structure of CH of antibody | SEQ ID NO of base sequence | SEQ ID NO of amino acid sequence |
|---|---|---|---|
| anti-CD4 antibody half-molecule (L368A) | IgG1_AA_DE_ D265A_L368A CH | 213 | 214 |
| anti-CD4 antibody half-molecule (D399A) | IgG1_AA_ DE_ D265A_D399A CH | 215 | 216 |
| anti-CD4 antibody half-molecule (F405A) | IgG1_AA_DE_ D265A_F405A CH | 217 | 218 |
| anti-CD4 antibody half-molecule (Y407A) | IgG1_M_DE_ D265A_Y407A CH | 219 | 220 |
| anti-CD4 antibody half-molecule (K409A) | IgG1_AA_DE_ D265A_K409A CH | 221 | 222 |
| anti-CD70 antibody half-molecule (K409R) | IgG1_AA_DE_ P329Y_K409R CH | 223 | 224 |
| anti-CD70 antibody half-molecule (Y349A) | IgG1_AA_ DE_ P329Y_Y349A CH | 225 | 226 |
| anti-CD70 antibody half-molecule (L351A) | IgG1_AA_DE_ P329Y_L351A CH | 227 | 228 |
| anti-CD70 antibody half-molecule (T366A) | IgG1_AA_DE_ P329Y_T366A CH | 229 | 230 |
| anti-CD70 antibody half-molecule (L368A) | IgG1_M_DE_ P329Y_L368A CH | 231 | 232 |
| anti-CD70 antibody half-molecule (D399A) | IgG1_AA_DE_ P329Y_D399A CH | 233 | 234 |
| anti-CD70 antibody half-molecule (F405A) | IgG1_AA_DE_ P329Y_F405A CH | 235 | 236 |
| anti-CD70 antibody half-molecule (Y407A) | IgG1_AA_DE_ P329Y_Y407A CH | 237 | 238 |
| anti-CD70 antibody half-molecule (K409A) | IgG1_AA_DE_ P329Y_K409A CH | 239 | 240 |

(1) Production of expression vector for anti-CD4 antibody half-molecule and anti-CD70 antibody half-molecule

A DNA fragment of about 9 kbp was cut out using restriction enzymes NheI and NotI from a human IgG1 anti-CD4 antibody expression vector pCI-IgG1_CD4 (ibalizumab) (SEQ ID NOS: 5, 7, 41, and 43) or a human IgG1 anti-CD70 antibody expression vector pCI-IgG1_CD70 (2H5) (SEQ ID NOS: 5, 7, 45, and 47), and purified.

A ligation reaction of the purified DNA fragment with an artificial synthetic gene composed of the CH1 domain, the hinge domain (to which a C226A/C229A alteration was added), the CH2 domain (to which a S239D/I332E alteration and a D265A or P329Y alteration were added), and the CH3 domain (to which an amino acid alteration for attenuating the CH3 domain interaction was added) of a human IgG1 antibody was carried out using In-Fusion HD Cloning Kit (Clontech), and Escherichia coli DH5α competent cells (Takara Bio) were transformed using the reaction solution. Each plasmid DNA was prepared from the obtained transformant clones, and the base sequence of the DNA was analyzed by Fasmac.

(2) Expression of anti-CD4 antibody half-molecule and anti-CD70 antibody half-molecule

The expression vector produced in (1) was introduced into a host cell in the same manner as in Example 5.

(3) Purification of anti-CD4 antibody half-molecule and anti-CD70 antibody half-molecule

The altered antibody was purified in the same manner as in Example 5.

(4) Evaluation of purification degree by SDS-PAGE of anti-CD4 antibody half-molecule and anti-CD70 antibody half-molecule

As a result of evaluating the purification degree of the prepared various types of antibody samples in the same manner as in Example 5, in all the purified antibodies, under reducing conditions, a band was observed in the vicinity of about 50 kDa for the H chain and in the vicinity of about 25 kDa for the L chain. Further, under non-reducing conditions, a band was observed in the vicinity of about 150 kDa for the anti-CD4 IgG1 antibody and the anti-CD70 IgG1 antibody, and in the vicinity of about 75 kDa for the anti-CD4 half-molecule and the anti-CD70 half-molecule, and therefore, it was confirmed that the produced anti-CD4 and anti-CD70 IgG1 antibodies are each an antibody structure constituted by the target H chain and L chain, and the anti-CD4 antibody half-molecule and the anti-CD70 antibody half-molecule are each constituted by the target H chain and L chain, and have an antibody structure that easily forms a half-molecule.

(5) ADCC activity of anti-CD4 IgG1 antibody and anti-CD70 IgG1 antibody, and half-molecules of these antibodies against CD4/CD70 double-positive cell and CD4 and CD70 single-positive cells

The ADCC activity of an antibody composition obtained by mixing the anti-CD4 antibody half-molecule and the anti-CD70 antibody half-molecule to which an amino acid alteration for attenuating the interaction between CH3 domains was added was evaluated in the same manner as in Example 5. As the control antibody, the anti-CD4 IgG1 antibody and the anti-CD70 IgG1 antibody were used.

As shown in Figs. 16A and 16B, in the antibody solution obtained by mixing the anti-CD4 antibody half-molecule and the anti-CD70 antibody half-molecule to which an amino acid alteration for attenuating the interaction between the CH3 domains of human IgG1 was added, the ADCC activity against the CD4/CD70 double-positive cell is stronger as compared with the ADCC activity against the CD4 and CD70 single-positive cells, and it was shown that it is specific for the CD4/CD70 double-positive cell.

[Example 7]

Kinetic Test in Mouse and Measurement of Thermal Stability of CH2 Domain

(Kinetic Test in Mouse)

A kinetic test for an antibody composition obtained by combining the CD16a-binding asymmetric alteration (D265A or P329Y) obtained in Examples 1 to 3, any of various types of CD16a-binding enhancing alterations obtained in Example 5, and the amino acid alteration (K409R) for attenuating the interaction between the CH3 domains of human IgG1 obtained in Example 6 was carried out. As the variable region, a DNA fragment encoding an altered region of an anti-2,4-dinitrophenol (DNP) IgG1 antibody (clone name: DNP2) described in Clin Cancer Res. 11(8): 3126-3135, 2005 which does not react with a specific protein was used. The base sequences and the amino acid sequences of the VH and VL of the anti-DNP antibody are shown in Table 18. Further, the base sequences and the amino acid sequences of anti-DNP antibody half-molecules 1 to 6 used for evaluation are shown in Table 19.

[Table 181

| Sequence name | SEQ ID NO of base sequence | SEQ ID NO of amino acid sequence |
|---|---|---|
| Anti-DNP antibody VH | 241 | 242 |
| Anti-DNP antibody VL | 243 | 244 |

[Table 19]

| Purified antibody | Structure of CH of antibody | SEQ ID NO of base sequence | SEQ ID NO of amino acid sequence |
|---|---|---|---|
| anti-DNP antibody half-molecule 1 | IgG1_AA_AAA/S239D_D265A_ K409R CH | 245 | 246 |

(continued)

| Purified antibody | Structure of CH of antibody | SEQ ID NO of base sequence | SEQ ID NO of amino acid sequence |
|---|---|---|---|
| anti-DNP antibody half-molecule 2 | IgG1_AA_S239D/I332E_D265A_ K409R CH | 205 | 206 |
| anti-DNP antibody half-molecule 3 | IgG1_AA_S239D/K326T_ D265A_ K409R CH | 247 | 248 |
| anti-DNP antibody half-molecule 4 | IgG1_AA_AAA/S239D_P329Y_ K409R CH | 249 | 250 |
| anti-DNP antibody half-molecule 5 | IgG1_AA_S239D/I332E_P329Y_ K409R CH | 223 | 224 |
| anti-DNP antibody half-molecule 6 | IgG1_AA_S239D/K326T_P329Y_ K409R CH | 251 | 252 |

The anti-DNP IgG1 antibody and the anti-DNP antibody half-molecules were produced by the same method as described in Example 6. In all the purified antibodies, under reducing conditions, a band was observed in the vicinity of about 50 kDa for the H chain and in the vicinity of about 25 kDa for the L chain. Further, under non-reducing conditions, a band was observed in the vicinity of about 150 kDa for the anti-DNP antibody, and in the vicinity of about 75 kDa for the anti-DNP antibody half-molecule, and therefore, it was confirmed that the produced anti-DNP antibody is an antibody structure constituted by the target H chain and L chain, and the anti-DNP antibody half-molecule is constituted by the target H chain and L chain, and has an antibody structure that easily forms a half-molecule.

The antibody compositions of the present invention were prepared by mixing the half-molecules 1 and 4, or the half-molecules 2 and 5, or the half-molecules 3 and 6 of the anti-DNP antibody, respectively. After the antibody composition (1 mg/kg) was administered to a BALB/c mouse through the tail vein, the blood was collected after 1 hour, 4 hours, 24 hours, and 72 hours. After piercing the cheek with a lancet under isoflurane anesthesia and collecting the blood, serum was recovered by centrifuging the blood at 8,000 rpm at room temperature for 10 minutes with Microtainer (registered trademark) (BD).

As the amount of the antibody present in the serum, the concentration of the antibody in the serum was measured using AlphaLISA Human Kappa light chain immunoassay kit (AL3023, PerkinElmer). A calibration curve was drawn with Kappa light chain in the kit. The results are shown in Fig. 17.

As shown in Fig. 17, it was found that the antibody composition of the present invention exhibits various kinetics depending on the difference in the CD16a-binding enhancing amino acid alteration. Above all, it was found that the antibody composition of the present invention having S239D/K326T exhibits kinetics close to that of the anti-DNP IgG1 antibody.

(Measurement of Tm value of CH2 domain)

In order to study the effect of various CD16a-binding enhancing amino acid alterations introduced into the half-molecule on the thermal stability of the CH2 domain, the Tm value was measured by differential scanning fluorimetry (DSF). The anti-CD4 antibody half-molecules and the anti-CD70 antibody half-molecules shown in Table 20 into which a CD16a-binding enhancing amino acid alteration was introduced were prepared in the same manner as in [Example 6]. The Tm values of the CH2 domains of the prepared anti-CD4 antibody half-molecules and anti-CD70 antibody half-molecules were measured under the condition of temperature elevation rate of 1°C/min from 20°C to 95°C using Prometheus NT.48 manufactured by NanoTemper Technologies GmbH.

[Table 20]

| Purified antibody name (CD16a-binding enhancing amino acid alteration) | Structure of CH of antibody | Tm value (°) | SEQ ID NO of base sequence | SEQ ID NO of amino acid sequence |
|---|---|---|---|---|
| anti-CD4 antibody half-molecule (AAA/S239D) | IgG1_AA_AAA/S239D_D265A_K409R CH | 47.4 | 245 | 246 |
| anti-CD4 antibody half-molecule (S239D/I332E) | IgG1_AA_S239D/I332E_D265A_K409R CH | 42.5 | 205 | 206 |
| anti-CD4 antibody half-molecule (S239D/K326T) | IgG1_AA_S239D/K326T_D265A_K409R CH | 56.8 | 247 | 248 |
| anti-CD4 antibody half-molecule (S239D/S298A/E333A/L242C/K334C) | IgG1_AA_S239D/S298A/E333A/L242C/K334C_ D265A_K409R CH | 66.2 | 271 | 272 |
| anti-CD70 antibody half-molecule (AAA/S239D) | IgG1_AA_AAA/S239D_P329Y_K409R CH | 48.5 | 249 | 250 |
| anti-CD70 antibody half-molecule (S239D/I332E) | IgG1_AA_S239D/I332E_P329Y_K409R CH | 44.4 | 223 | 224 |
| anti-CD70 antibody half-molecule (S239D/K326T) | IgG1_AA_S239D/K326T_P329Y_K409R CH | 57.0 | 251 | 252 |
| anti-CD70 antibody half-molecule (S239D/S298A/E333A/L242C/K334C) | IgG1_AA_S239D/S298A/E333A/L242C/K334C_ P329Y_K409R CH | 65.5 | 291 | 292 |

When considering the results of the PK test in mouse shown in Fig. 17 together with the Tm values in Table 20, the higher the Tm value of the CH2 domain with the CD16a-binding enhancing amino acid alteration is, the longer the blood half-life tends to be. From this, it was found that by measuring the Tm value, the antibody composition of the present invention having a favorable blood half-life can be selected.

[Example 8]

ADCC Activity Test When Adding Immunoglobulin

An evaluation system that mimics the internal human body was constructed by adding Venoglobulin (Japan Blood Products Organization, #867279694) at a final concentration of 8 mass% to the ADCC evaluation system described in Example 1 (5). The antibody compositions evaluated are shown in Table 21.

[Table 21]

| Purified antibody name (CD16a-binding enhancing amino acid alteration) | Structure of CH of antibody | SEQ ID NO of base sequence | SEQ ID NO of amino acid sequence |
|---|---|---|---|
| anti-CD4 antibody half-molecule (S239D/I332E) | IgG1_AA_S239D/I332E_D265A_ K409R CH | 205 | 206 |
| anti-CD4 antibody half-molecule (S239D/K326E) | IgG1_AA_S239D/K326E_D265A_ K409R CH | 253 | 254 |
| anti-CD4 antibody half-molecule (S239D/K326I) | IgG1_AA_S239D/K326I_D265A_ K409R CH | 255 | 256 |
| anti-CD4 antibody half-molecule (AAA/H268E) | IgG1_AA_AAA/H268E_D265A_K409R CH | 257 | 258 |
| anti-CD4 antibody half-molecule (S298A/E333A/K334E) | IgG1_AA_S298A/E333A/K334E_ D265A_K409R CH | 259 | 260 |
| anti-CD4 antibody half-molecule (AAA/S239D) | IgG1_AA_AAA/S239D_D265A_K409R CH | 245 | 246 |
| anti-CD4 antibody half-molecule (S239D/K326T) | IgG1_AA_S239D/K326T_D265A_ K409R CH | 247 | 248 |
| anti-CD4 antibody half-molecule (S239E/I332E) | IgG1_AA_S239E/I332E_D265A_ K409R CH | 261 | 262 |
| anti-CD4 antibody half-molecule (S239D/I332T) | IgG1_AA_S239D/I332T_D265A_ K409R CH | 263 | 264 |
| anti-CD4 antibody half-molecule (S239D/A330F) | IgG1_AA_S239D/A330F_D265A_ K409R CH | 265 | 266 |
| anti-CD4 antibody half-molecule (S239D) | IgG1_AA_S239D_D265A_K409R CH | 267 | 268 |
| anti-CD4 antibody half-molecule (AAA/S239D/L309K) | IgG1_AA_AAA/S239D/L309K_D265A_ K409R CH | 269 | 270 |
| anti-CD4 antibody half-molecule (S239D/S298A/E333A/L242C/K334C) | IgG1_AA_ S239D/S298A/E333A/L242C/K334C_ D2 65A_K409R CH | 271 | 272 |
| anti-CD70 antibody half-molecule (S239D/I332E) | IgG1_AA_S239D/I332E_P329Y_ K409R CH | 223 | 224 |
| anti-CD70 antibody half-molecule (S239D/K326E) | IgG1_AA_S239D/K326E_P329Y_ K409R CH | 273 | 274 |
| anti-CD70 antibody half-molecule (S239D/K326I) | IgG1_AA_S239D/K326I_P329Y_ K409R CH | 275 | 276 |

(continued)

| Purified antibody name (CD16a-binding enhancing amino acid alteration) | Structure of CH of antibody | SEQ ID NO of base sequence | SEQ ID NO of amino acid sequence |
|---|---|---|---|
| anti-CD70 antibody half-molecule (AAA/H268E) | IgG1_AA_AAA/H268E_P329Y_K409R CH | 277 | 278 |
| anti-CD70 antibody half-molecule (S298A/E333A/K334E) | IgG1_AA_S298A/E333A/K334E_ P329Y_K409R CH | 279 | 280 |
| anti-CD70 antibody half-molecule (AAA/S239D) | IgG1_AA_AAA/S239D_P329Y_K409R CH | 249 | 250 |
| anti-CD70 antibody half-molecule (S239D/K326T) | IgG1_AA_S239D/K326T_P329Y_ K409R CH | 251 | 252 |
| anti-CD70 antibody half-molecule (S239E/I332E) | IgG1_AA_S239E/I332E_ P329Y_ K409R CH | 281 | 282 |
| anti-CD70 antibody half-molecule (S239D/ 1332T) | IgG1_AA_S239D/I332T_P329Y_ K409R CH | 283 | 284 |
| anti-CD70 antibody half-molecule (S239D/D330F) | IgG1_AA_ S239D/D330F_P329Y_ K409R CH | 285 | 286 |
| anti-CD70 antibody half-molecule (S239D) | IgG1_AA_S239D_P329Y_K409R CH | 287 | 288 |
| anti-CD70 antibody half-molecule (AAA/S239D/L309K) | IgG1_AA _AAA/S239D/L309K_ P329Y_K409R CH | 289 | 290 |
| anti-CD70 antibody half-molecule (S239D/S298A/E333A/L242C/K334C) | IgG1_AA_ S239D/S298A/E333A/L242C/K334C_ P3 29Y_K409R CH | 291 | 292 |

The anti-CD4 antibody half-molecule and the anti-CD70 antibody half-molecule were prepared in the same manner as in [Example 6]. As the VH and VL of the anti-CD4 antibody and the VH and VL of the anti-CD70 antibody, those shown in Table 11 were used.

The ADCC activity of the antibody composition obtained by mixing the anti-CD4 antibody half-molecule and the anti-CD70 antibody half-molecule was evaluated in the same manner as in Example 5. As the positive control antibody, the anti-CD4 IgG1 antibody and the anti-CD70 IgG1 antibody were used.

As shown in Figs. 18A to 18C, it was confirmed that the antibody composition of the present invention exhibits the ADCC activity specific for the CD4/CD70 double-positive cell even in the presence of Venoglobulin.

[Example 9]

ADCC Activity in Human Blood Reconstitution System

Whether a double-positive cell can be specifically removed was studied using an evaluation system containing a human blood component brought closer to the state of the internal human body. The evaluation target antibodies are shown in Table 22.

[Table 22]

| Purified antibody name (CD16a-binding enhancing amino acid alteration) | Structure of CH of antibody | SEQ ID NO of base sequence | SEQ ID NO of amino acid sequence |
|---|---|---|---|
| anti-CD4 antibody half-molecule (AAA/S239D) | IgG1_AA_AAA/S239D_ D265A_K409R CH | 245 | 246 |
| anti-CD4 antibody half-molecule (S239D/I332E) | IgG1_AA_ S239D/I332E_D265A_ K409R CH | 205 | 306 |

(continued)

| Purified antibody name (CD16a-binding enhancing amino acid alteration) | Structure of CH of antibody | SEQ ID NO of base sequence | SEQ ID NO of amino acid sequence |
|---|---|---|---|
| anti-CD4 antibody half-molecule (S239D/K326T) | IgG1_AA_ S239D/K326T_D265A_ K409R CH | 247 | 248 |
| anti-CCR6 antibody half-molecule (AAA/S239D) | IgG1_AA_AAA/S239D_ P329Y_K409R CH | 249 | 250 |
| anti-CCR6 antibody half-molecule (S239D/I332E) | IgG1_AA_S239D/ 1332E_P329Y_K409R CH | 223 | 224 |
| anti-CCR6 antibody half-molecule (S239D/K326T) | IgG1_AA_ S239D/K326T_P329Y_ K409R CH | 251 | 252 |

(1) Production of expression vector for anti-CD4 antibody half-molecule and anti-CCR6 antibody half-molecule

A vector was produced in the same manner as in Example 5 (1).

(2) Expression of anti-CD4 IgG1 antibody and anti-CCR6 IgG1 antibody (KG1684) and half-molecules of these antibodies

The antibodies were expressed in the same manner as in Example 5 (2).

(3) Purification of anti-CD4 antibody half-molecule and anti-CCR6 antibody half-molecule

The antibodies were purified in the same manner as in Example 5 (3).

(4) Evaluation of purification degree of anti-CD4 antibody half-molecule and anti-CCR6 antibody half-molecule

As a result of evaluating the purification degree of each antibody in the same manner as in Example 5 (4), in all the purified antibodies, under reducing conditions, a band was observed in the vicinity of about 50 kDa for the H chain and in the vicinity of about 25 kDa for the L chain. Further, under non-reducing conditions, a band was observed in the vicinity of about 150 kDa for the anti-CD4 IgG1 antibody and the anti-CCR6 IgG1 antibody, and in the vicinity of about 75 kDa for the anti-CD4 antibody half-molecule and the anti-CCR6 antibody half-molecule, and therefore, it was confirmed that the produced anti-CD4 IgG1 antibody and anti-CCR6 IgG1 antibody are each an antibody structure constituted by the target H chain and L chain, and the anti-CD4 antibody half-molecule and the anti-CCR6 antibody half-molecule are each constituted by the target H chain and L chain, and have an antibody structure that easily forms a half-molecule.

(5) ADCC activity of antibody composition obtained by mixing anti-CD4 antibody half-molecule and anti-CCR6 antibody half-molecule against CD4/CCR6 double-positive cell

In 50 mL of human blood, a 20 mL portion was used for plasma and a 30 mL portion was used for collecting a fraction of peripheral blood mononuclear cells and granulocytes. In order to obtain plasma, the blood was centrifuged at 2000 rpm for 20 minutes at 4°C in a 15 mL centrifuge tube, and the supernatant was recovered.

In order to collect the fraction of peripheral blood mononuclear cells and granulocytes, with respect to 10 mL of the blood, 2 mL of Hetasep (Stemcell technology, #07806) was added and mixed well, and then, the mixture was centrifuged at 50 × g for 1 minute at room temperature. The mixture was left to stand at room temperature until it is separated, and a layer other than red blood cells was recovered and the volume was made up to 50 mL with PBS and the resultant was centrifuged at 540 × g for 5 minutes.

The recovered plasma and the fraction of peripheral blood mononuclear cells and granulocytes were mixed and prepared at $5 \times 10^6$ cells/mL and seeded at $5 \times 10^5$ cells/100 μL/well, and then, 20 μL of the antibody composition obtained by mixing the anti-CD4 antibody half-molecule and the anti-CCR6 antibody half-molecule, and 80 μL of the plasma were added thereto, and the cells were cultured at 37°C for 24 hours. The ratio of the CD4/CCR6 double-positive cell per CD3-positive T cell was measured by flow cytometry. As the negative control, the anti-DNP antibody used in Example 7 was used.

As shown in Fig. 19A, it was found that all the antibody compositions of the present invention remove the CD4/CCR6 double-positive cell in an antibody concentration-dependent manner, but do not remove the CD4 or CCR6 single-positive cell.

[Example 9-2]

Whether a double-positive cell can be specifically removed was studied using the antibody composition of the present invention having an amino acid alteration different from those in [Example 9]. The evaluation target antibodies are shown in Table 23.

[Table 23]

| Antibody composition name | Purified antibody name (CD16a-binding enhancing amino acid alteration) | Structure of CH of antibody | SEQ ID NO of base sequence | SEQ ID NO of amino acid sequence |
|---|---|---|---|---|
| A | anti-CD4 antibody half-molecule (S239D/S298A/E333A/L242C/K334C) | IgG1 _AA_S239D/S298A/E333A/L242C/K334C _D265A_K409R CH | 271 | 272 |
| | anti-CCR6 antibody half-molecule (S239D/S298A/E333A/L242C/K334C) | IgG1_AA_S239D/S298A/E333A/L242C/K334C _P329Y_K409R CH | 291 | 292 |
| B | anti-CD4 antibody half-molecule (S239D/K326T) | IgG1_AA_S239D/K326T_S298E_K409R CH | 293 | 294 |
| | anti-CCR6 antibody half-molecule (S239D/K326T) | IgG1_AA_S239D/K326T_P329Y_K409RCH | 251 | 252 |
| C | anti-CD4 antibody half-molecule (AAA/S239D) | IgG1_AA_AAA/S239D_S298E_K409R CH | 295 | 296 |
| | anti-CCR6 antibody half-molecule (AAA/S239D) | IgG1_AA _AAA/S239D_P329Y_K409R CH | 249 | 250 |
| D | anti-CD4 antibody half-molecule (S239D/E333A/L242C/K334C) | IgG1_AA_S239D/E333A/L242C/K334C_S298 E_ K409R CH | 297 | 298 |
| | anti-CCR6 antibody half-molecule (S239D/S298A/E333A/L242C/K334C) | IgG1 _AA_S239D/S298A/E333A/L242C/K334C _P329Y_K409R CH | 291 | 292 |

Preparation of the evaluation target antibodies and evaluation were carried out in the same manner as in [Example 9].

As shown in Fig. 19B, it was found that all the antibody compositions of the present invention remove the CD4/CCR6 double-positive cell in an antibody concentration-dependent manner, but do not remove the CD4 or CCR6 single-positive cell.

[Example 10]

ADCC Activity When Antigen-Binding Domains of First IgG Half-Molecule and Second IgG Half-Molecule were Exchanged

The effect on the ADCC activity when the antigen-binding domains of the first IgG half-molecule and the second IgG half-molecule were exchanged was studied. The evaluation target antibodies are shown in Table 24.

[Table 24]

| Purified antibody name (CD16a-binding enhancing amino acid alteration) | Structure of CH of antibody | SEQ ID NO of base sequence | SEQ ID NO of amino acid sequence |
|---|---|---|---|
| anti-CD4 antibody half-molecule A (S239D/K326T) | IgG1_AA_S239D/K326T_D265A_ K409R CH | 247 | 248 |
| anti-CCR4 antibody half-molecule A (S239D/K326T) | IgG1_AA_S239D/K326T_P329Y_ K409R CH | 251 | 252 |
| anti-CD4 antibody half-molecule B (S239D/K326T) | IgG1_AA_S239D/K326T_P329Y_ K409R CH | 251 | 252 |
| anti-CCR4 antibody half-molecule B (S239D/K326T) | IgG1_AA_S239D/K326T_D265A_ K409R CH | 247 | 248 |
| anti-CD4 antibody half-molecule C (S239D/S298A/E333A/L242C/K334C) | IgG1_AA_ S239D/S298A/E333A/L242C/K334C_D 265A _ K409R CH | 271 | 272 |
| anti-CCR4 antibody half-molecule C (S239D/S298A/E333A/L242C/K334C) | IgG1_AA_ S239D/S298A/E333A/L242C/K334C_ P3 29Y_K409R CH | 291 | 292 |
| anti-CD4 antibody half-molecule D (S239D/S298A/E333A/L242C/K334C) | IgG1_AA_ S239D/S298A/E333A/L242C/K334C_ P3 29Y_K409R CH | 291 | 292 |
| anti-CCR4 antibody half-molecule D (S239D/S298A/E333A/L242C/K334C) | IgG1_AA_ S239D/S298A/E333A/L242C/K334C_D 265A_K409R CH | 271 | 272 |

The anti-CD4 antibody half-molecule and the anti-CCR4 antibody half-molecule, and as the positive control, an anti-CD4 IgG1 antibody and an anti-CCR4 IgG1 antibody were prepared in the same manner as in [Example 5] and [Example 6]. As the VH and VL of the anti-CD4 antibody, those shown in Table 11 were used, and as the VH and VL of the anti-CCR4 antibody, those shown in Table 14 were used.

Antibody solutions were prepared by mixing equal amounts of each of the half-molecules A to D of the anti-CD4 antibody and each of the half-molecules A to D of the anti-CCR4 antibody to give a concentration of 1.0 μg/mL. The results of measuring the ADCC activity of each antibody solution against a CD4/CCR4 double-positive cell (TL-Om1), a CCR4 single-positive cell (MT-1), and a CD4 single-positive cell (CD4/EL4) are shown in Fig. 20.

As shown in Fig. 20, it was confirmed that all the antibody compositions obtained by mixing the half-molecules A to D exhibit the ADCC activity specific for the CD4/CCR4 double-positive cell. That is, it was shown that the specific ADCC activity is exhibited even when the antigen-binding domains of the first IgG half-molecule and the second IgG half-molecule were exchanged.

[Example 10-2]

The effect on the ADCC activity when the antigen-binding domains of the first IgG half-molecule and the second IgG half-molecule were exchanged was studied using the antibody composition of the present invention having an amino acid alteration different from those in [Example 10]. The evaluation target antibodies are shown in Table 25.

[Table 25]

| Purified antibody name (CD16a-binding enhancing amino acid alteration) | Structure of CH of antibody | SEQ ID NO of base sequence | SEQ ID NO of amino acid sequence |
|---|---|---|---|
| anti-CD4 antibody half-molecule E (S239D/K326T) | IgG1_AA_S239D/K326T_S298E_ K409R CH | 293 | 294 |
| anti-CCR4 antibody half-molecule E (S239D/K326T) | IgG1_AA_S239D/K326T_P329Y_ K409R CH | 251 | 252 |
| anti-CD4 antibody half-molecule F (S239D/K326T) | IgG1_AA_S239D/K326T_P329Y_ K409R CH | 251 | 252 |
| anti-CCR4 antibody half-molecule F (S239D/K326T) | IgG1_AA_S239D/K326T_S298E_ K409R CH | 293 | 294 |
| anti-CD4 antibody half-molecule G (S239D/E333A/L242C/K334C) | IgG1_AA_ S239D/E333A/L242C/K334C_S298E_ K 409R CH | 297 | 298 |
| anti-CCR4 antibody half-molecule G (S239D/S298A/E333A/L242C/K334C) | IgG1_AA_ S239D/S298A/E333A/L242C/K334C_ P3 29Y_K409R CH | 291 | 292 |
| anti-CD4 antibody half-molecule H (S239D/S298A/E333A/L242C/K334C) | IgG1_AA_ S239D/S298A/E333A/L242C/K334C_ P3 29Y_K409R CH | 291 | 292 |
| anti-CCR4 antibody half-molecule H (S239D/E333A/L242C/K334C) | IgG1_AA_ S239D/E333A/L242C/K334O_S298E_ K 409R CH | 297 | 298 |

The anti-CD4 antibody half-molecule and the anti-CCR4 antibody half-molecule, and as the positive control, an anti-CD4 IgG1 antibody and an anti-CCR4 IgG1 antibody were prepared in the same manner as in [Example 5] and [Example 6]. As the VH and VL of the anti-CD4 antibody, those shown in Table 11 were used, and as the VH and VL of the anti-CCR4 antibody, those shown in Table 14 were used.

Antibody solutions were prepared by mixing equal amounts of each of the half-molecules E to F of the anti-CD4 antibody and each of the half-molecules E to F of the anti-CCR4 antibody to give a concentration of 0.1 μg/mL. The results of measuring the ADCC activity of each antibody solution against a CD4/CCR4 double-positive cell (HH), a CCR4 single-positive cell (L428), and a CD4 single-positive cell (TALL1) are shown in Fig. 21.

As shown in Fig. 21, it was confirmed that all the antibody compositions obtained by mixing the half-molecules A to D exhibit the ADCC activity specific for the CD4/CCR4 double-positive cell. That is, it was shown that the specific ADCC activity is exhibited even when the antigen-binding domains of the first IgG half-molecule and the second IgG half-molecule were exchanged.

[Example 11]

Whether the antibody composition of the present invention exhibits an ADCC activity specific only for a double-positive cell also in a combination of different antigens (CCR4 and CD70) was studied. The evaluation target antibodies are shown in Table 26.

[Table 26]

| Antibody composition name | Purified antibody name (CD16a-binding enhancing amino acid alteration) | Structure of CH of antibody | SEQ ID NO of base sequence | SEQ ID NO of amino acid sequence |
|---|---|---|---|---|
| E | anti-CCR4 antibody half-molecule (S239D/K326T) | IgG1_AA_S239D/K326T_D265A_K409R CH | 247 | 248 |
| | anti-CD70 antibody half-molecule (S239D/K326T) | IgG1_AA_S239D/K326T_P329Y_K409R CH | 251 | 252 |
| F | anti-CCR4 antibody half-molecule (S239D/E333A/L242C/K334C) | IgG1_AA_S239D/S298A/E333A/L242C/K334C _D265A _ K409R CH | 271 | 272 |
| | anti-CD70 antibody half-molecule (S239D/S298A/E333A/L242C/K334C) | IgG1_AA_S239D/S298A/E333A/L242C/K334C _P329Y_K409R CH | 291 | 292 |
| G | anti-CCR4 antibody (S239D/K326T) | IgG1_AA_S239D/K326T_S298E_K409R CH | 293 | 294 |
| | anti-CD70 antibody half-molecule (S239D/K326T) | IgG1_AA_S239D/K326T_P329Y_K409R CH | 251 | 252 |
| H | anti-CCR4 antibody half-molecule (S239D/E333A/L242C/K334C) | IgG1_AA_S239D/E333A/L242C/K334C_ S298 E_ K409R CH | 297 | 298 |
| | anti-CD70 antibody half-molecule (S239D/S298A/E333A/L242C/K334C) | IgG1_AA_S239D/S298A/E333A/L242C/K334C _P329Y_ K409R CH | 291 | 292 |

The anti-CCR4 antibody half-molecule and the anti-CD70 antibody half-molecule, and as the positive control, an anti-CCR4 IgG1 antibody and an anti-CD70 IgG1 antibody were prepared in the same manner as in [Example 5] and [Example 6]. As the base sequences and the amino acid sequences of the VH and VL of the anti-CCR4 antibody, those shown in Table 14 were used, and as the base sequences and the amino acid sequences of the VH and VL of the anti-CD70 antibody, those shown in Table 11 were used.

Antibody solutions were prepared by mixing equal amounts of each of the half-molecules A to D of the anti-CCR4 antibody and each of the half-molecules A to D of the anti-CD70 antibody to give a concentration of 0.1 μg/mL. The results of measuring the ADCC activity of each antibody solution against a CCR4/CD70 double-positive cell (L428), a CCR4 single-positive cell (PEER), and a CD70 single-positive cell (SUP-M2) are shown in Fig. 22.

As shown in Fig. 22, it was confirmed that all the antibody compositions obtained by mixing the half-molecules A to D exhibit the ADCC activity specific for the CCR4/CD70 double-positive cell.

[Example 12]

CD 16a Binding Analysis

It was verified that an antibody composition binds to CD16a only when it is the antibody composition of the first IgG half-molecule and the second IgG half-molecule of the present invention.

As the evaluation target antibodies, the following antibodies were used.

- the anti-DNP antibody half-molecule 3 (265A) described in [Example 7]
- the anti-DNP antibody half-molecule 6 (329Y) described in [Example 7]
- the antibody composition (265A+329Y) obtained by mixing the anti-DNP antibody half-molecules 3 and 6 described in [Example 7]
- the anti-DNP antibody (positive control) described in [Example 7]

In the measurement, BIAcore (manufactured by Biacore, Inc.) was used, and the measurement method was performed by the following method according to the description in J. Immunol. Methods, 200, 121 (1997).

By using BIAcore T100 (manufactured by Biacore, Inc.), an interaction analysis between each evaluation target antibody and CD16a was performed. As a running buffer, HBS-EP+ 10x (GE Healthcare) was diluted to 1/10 and used, and the measurement temperature was set to 25°C. A chip in which immobilization was carried out on Series S Sensor Chip CM5 (GE Healthcare) with Human Fab Capture Kit (GE Healthcare) was used.

The intended evaluation target antibody was captured on this sensor chip, and CD16a diluted with the running buffer was allowed to interact therewith, and the measurement was performed. Further, the evaluation target antibody captured onto the sensor chip was washed by allowing the buffer attached to the Human Fab Capture Kit (GE Healthcare) to react therewith, so that the sensor chip was regenerated and used repeatedly. The measurement results are shown in Fig. 23.

As shown in Fig. 23, in the case of the anti-DNP antibody half-molecule 3 (265A) alone and the anti-DNP antibody half-molecule 6 (329Y) alone, the binding activity to CD16a was not observed, and only in the case of the antibody composition of the anti-DNP antibody half-molecule 3 (265A) and the anti-DNP antibody half-molecule 6 (329Y), the binding activity to CD16a was observed.

The present invention has been described in detail using the specific aspects, but it is obvious to those skilled in the art that various changes and modifications may be made without departing from the spirit and scope of the present invention. Note that the present application is based on the Japanese patent application (Japanese Patent Application No. 2020-066313) filed on April 1, 2020 and the Japanese patent application (Japanese Patent Application No. 2020-181493) filed on October 29, 2020, which are incorporated by reference in their entirety.

SEQUENCE LISTING FREE TEXT

SEQ ID NO: 1: base sequence of human CCR6 (KG1684) VL
SEQ ID NO: 2: amino acid sequence of human CCR6 (KG1684) VL
SEQ ID NO: 3: base sequence of human CCR6 (KG1684) VH
SEQ ID NO: 4: amino acid sequence of human CCR6 (KG1684) VH
SEQ ID NO: 5: base sequence of human IgG1 CK
SEQ ID NO: 6: amino acid sequence of human IgG1 CK
SEQ ID NO: 7: base sequence of human IgG1 CH

SEQ ID NO: 8: amino acid sequence of human IgG1 CH
SEQ ID NO: 9: base sequence of human IgG1_P238A CH
SEQ ID NO: 10: amino acid sequence of human IgG1_P238A CH
SEQ ID NO: 11: base sequence of human IgG1_D265A CH
SEQ ID NO: 12: amino acid sequence of human IgG1_D265A CH
SEQ ID NO: 13: base sequence of human IgG1_S267L CH
SEQ ID NO: 14: amino acid sequence of human IgG1_S267L CH
SEQ ID NO: 15: base sequence of human IgG1_P238A/D265A CH
SEQ ID NO: 16: amino acid sequence of human IgG1_P238A/D265A CH
SEQ ID NO: 17: base sequence of human IgG1_P238A/S267L CH
SEQ ID NO: 18: amino acid sequence of human IgG1 _P238A/S267L CH
SEQ ID NO: 19: base sequence of human IgG1_D265A/S267L CH
SEQ ID NO: 20: amino acid sequence of human IgG1_D265A/S267L CH
SEQ ID NO: 21: base sequence of human IgG1_P238A/D265A/S267L CH
SEQ ID NO: 22: amino acid sequence of human IgG1_P238A/D265A/S267L CH
SEQ ID NO: 23: base sequence of human IgG1_K326W CH
SEQ ID NO: 24: amino acid sequence of human IgG1_K326W CH
SEQ ID NO: 25: base sequence of human IgG1_L328V CH
SEQ ID NO: 26: amino acid sequence of human IgG1_L328V CH
SEQ ID NO: 27: base sequence of human IgG1_P329Y CH
SEQ ID NO: 28: amino acid sequence of human IgG1_P329Y CH
SEQ ID NO: 29: base sequence of human IgG1_K326W/L328V CH
SEQ ID NO: 30: amino acid sequence of human IgG1 _K326W/L328V CH
SEQ ID NO: 31: base sequence of human IgG1_K326W/P329Y CH
SEQ ID NO: 32: amino acid sequence of human IgG1_K326W/P329Y CH
SEQ ID NO: 33: base sequence of human IgG1_L328V/P329Y CH
SEQ ID NO: 34: amino acid sequence of human IgG1_L328V/P329Y CH
SEQ ID NO: 35: base sequence of human IgG1 _K326W/L328V/P329Y CH
SEQ ID NO: 36: amino acid sequence of human IgG1_K326W/L328V/P329Y CH
SEQ ID NO: 37: base sequence of human IgG1114_AA_AAA_D265A CH
SEQ ID NO: 38: amino acid sequence of human IgG1114_AA_AAA_D265A CH
SEQ ID NO: 39: base sequence of human IgG1114_AA_AAA_P329Y CH
SEQ ID NO: 40: amino acid sequence of human IgG1114_AA_AAA_P329Y CH
SEQ ID NO: 41: base sequence of human CD4 (ibalizumab) VL
SEQ ID NO: 42: amino acid sequence of human CD4 (ibalizumab) VL
SEQ ID NO: 43: base sequence of human CD4 (ibalizumab) VH
SEQ ID NO: 44: amino acid sequence of human CD4 (ibalizumab) VH
SEQ ID NO: 45: base sequence of human CD70 (2H5) VL
SEQ ID NO: 46: amino acid sequence of human CD70 (2H5) VL
SEQ ID NO: 47: base sequence of human CD70 (2H5) VH
SEQ ID NO: 48: amino acid sequence of human CD70 (2H5) VH
SEQ ID NO: 49: base sequence of human IgG1114_AA_AAA_S239R CH
SEQ ID NO: 50: amino acid sequence of human IgG1114_AA_AAA_S239R CH
SEQ ID NO: 51: base sequence of human IgG1114_AA_AAA_D265N CH
SEQ ID NO: 52: amino acid sequence of human IgG1114_AA_AAA_D265N CH
SEQ ID NO: 53: base sequence of human IgG1114_AA_AAA_D265E CH
SEQ ID NO: 54: amino acid sequence of human IgG1114_AA_AAA_D265E CH
SEQ ID NO: 55: base sequence of human IgG1114_AA_AAA_S267K CH
SEQ ID NO: 56: amino acid sequence of human IgG1114_AA_AAA_S267K CH
SEQ ID NO: 57: base sequence of human IgG1114_AA_AAA_E269P CH
SEQ ID NO: 58: amino acid sequence of human IgG1114_AA_AAA_E269P CH
SEQ ID NO: 59: base sequence of human IgG1114_AA_AAA_Y296P CH
SEQ ID NO: 60: amino acid sequence of human IgG1114_AA_AAA_Y296P CH
SEQ ID NO: 61: base sequence of human IgG1114_AA_AAA_S298E CH
SEQ ID NO: 62: amino acid sequence of human IgG1114_AA_AAA_S298E CH
SEQ ID NO: 63: base sequence of human IgG1114_AA_AAA_T299A CH
SEQ ID NO: 64: amino acid sequence of human IgG1114_AA_AAA_T299ACH
SEQ ID NO: 65: base sequence of human IgG1114_AA_AAA_L235R CH

SEQ ID NO: 66: amino acid sequence of human IgG1114_AA_AAA_L235R CH
SEQ ID NO: 67: base sequence of human IgG1114_AA_AAA_A327I CH
SEQ ID NO: 68: amino acid sequence of human IgG1114_AA_AAA_A327I CH
SEQ ID NO: 69: base sequence of human IgG1114_AA_AAA_K326G CH
SEQ ID NO: 70: amino acid sequence of human IgG1114_AA_AAA_K326G CH
SEQ ID NO: 71: base sequence of human IgG1114_AA_AAA_L328R CH
SEQ ID NO: 72: amino acid sequence of human IgG1114_AA_AAA_L328R CH
SEQ ID NO: 73: base sequence of human IgG1114_AA_AAA_P329K CH
SEQ ID NO: 74: amino acid sequence of human IgG1114_AA_AAA_P329K CH
SEQ ID NO: 75: base sequence of human IgG1114_AA_AAA_P329W CH
SEQ ID NO: 76: amino acid sequence of human IgG1114_AA_AAA_P329W CH
SEQ ID NO: 77: base sequence of human IgG1114_AA_AAA_A330P CH
SEQ ID NO: 78: amino acid sequence of human IgG1114_AA_AAA_A330P CH
SEQ ID NOS: 79 to 82: base sequence or amino acid sequence of anti-CCR4 antibody used in Example 5
SEQ ID NOS: 83 to 204: base sequence or amino acid sequence of anti-CCR4 antibody, in which CD16a-binding enhancing amino acid alteration was introduced, used in Example 5
SEQ ID NOS: 205 to 222: base sequence or amino acid sequence of anti-CD4 antibody half-molecule, in which amino acid alteration for attenuating interaction between CH3 domains of IgG1 was introduced, used in Example 6
SEQ ID NOS: 223 to 240: base sequence or amino acid sequence of anti-CD70 antibody half-molecule, in which amino acid alteration for attenuating interaction between CH3 domains of IgG1 was introduced, used in Example 6
SEQ ID NOS: 241 to 252: base sequences and amino acid sequences of VH and VL of anti-DNP antibody, and anti-DNP antibody half-molecules 1 to 6 used in Example 7, 9, 10, 11, or 12
SEQ ID NOS: 253 to 292: base sequence or amino acid sequence of anti-CD4 antibody half-molecule or anti-CD70 antibody half-molecule, in which CD16a-binding enhancing amino acid alteration was introduced, used in Example 8, 9-2, or 11.
SEQ ID NOS: 293 to 298: base sequence or amino acid sequence of anti-CD4 antibody half-molecule or half-molecule of anti-CCR4 antibody half-molecule, in which CD16a-binding enhancing amino acid alteration was introduced, used in Example 9-2, 10-2, or 11.

## Claims

1. An antibody composition, which is an antibody composition against a first antigen and a second antigen that are different from each other, comprising a first IgG half-molecule and a second IgG half-molecule, wherein the following (1A) to (6A) are satisfied:

    (1A) each of the first IgG half-molecule and the second IgG half-molecule is composed of one immunoglobulin light chain (hereinafter abbreviated as L chain) and one immunoglobulin heavy chain (hereinafter abbreviated as H chain), and the H chain includes an H chain variable region, a hinge domain, a CH1 domain, a CH2 domain, and a CH3 domain, and has a first Fcγ receptor IIIA (hereinafter CD16a)-binding domain and a second CD 16a-binding domain that are different from each other in the CH2 domain,
    (2A) each of the first IgG half-molecule and the second IgG half-molecule includes amino acid residue substitutions of C226A and C229A numbered according to the EU index,
    (3A) the first IgG half-molecule includes an antigen-binding domain that binds to the first antigen, and includes an amino acid residue substitution of D265A numbered according to the EU index in the first CD16a-binding domain,
    (4A) the second IgG half-molecule includes an antigen-binding domain that binds to the second antigen, and includes an amino acid residue substitution of P329Y numbered according to the EU index in the second CD16a-binding domain,
    (5A) each of the first IgG half-molecule and the second IgG half-molecule includes amino acid residue substitutions of (a) S239D and K326T numbered according to the EU index, or
    each of the first IgG half-molecule and the second IgG half-molecule includes amino acid residue substitutions of (b) S239D, S298A, E333A, L242C, and K334C numbered according to the EU index, and
    (6A) at least one of the first IgG half-molecule and the second IgG half-molecule includes an amino acid residue substitution in the CH3 domain as an alteration for attenuating an inter-CH3 domain interaction as compared with an inter-CH3 domain interaction of the IgG1 subclass.

2. The antibody composition according to claim 1, wherein the antibody composition exhibits an effector function

specifically for a target cell coexpressing the first antigen and the second antigen and damages the target cell as compared with an effector function for a target cell expressing only the first antigen and a target cell expressing only the second antigen.

**3.** The antibody composition according to claim 1 or 2, wherein each of the first IgG half-molecule and the second IgG half-molecule includes at least one amino acid residue substitution selected from Y349A, L351A, T366A, L368A, D399A, F405A, Y407A, K409A, and K409R numbered according to the EU index as the alteration for attenuating the inter-CH3 domain interaction as compared with the inter-CH3 domain interaction of the IgG1 subclass.

**4.** The antibody composition according to claim 3, wherein each of the first IgG half-molecule and the second IgG half-molecule includes an amino acid residue substitution of K409R numbered according to the EU index as the alteration for attenuating the inter-CH3 domain interaction as compared with the inter-CH3 domain interaction of the IgG1 subclass.

**5.** The antibody composition according to any one of claims 1 to 4, wherein each of the first IgG half-molecule and the second IgG half-molecule includes the amino acid residue substitutions of (a) S239D and K326T.

**6.** The antibody composition according to any one of claims 1 to 5, wherein

an H chain constant region (hereinafter abbreviated as CH) of the first IgG half-molecule contains an amino acid sequence represented by SEQ ID NO: 248, and
a CH of the second IgG half-molecule contains an amino acid sequence represented by SEQ ID NO: 252.

**7.** The antibody composition according to any one of claims 1 to 6, wherein a ratio of sugar chains in which fucose is not bound to N-acetylglucosamine at a reducing end of the sugar chain among the total N-glycoside-linked type sugar chains bound to an Fc region in the first IgG half-molecule and the second IgG half-molecule is 20% or more.

**8.** The antibody composition according to any one of claims 1 to 7, wherein the immunoglobulin subclass of the first IgG half-molecule and the second IgG half-molecule is IgG1.

**9.** A first IgG half-molecule, which is a first IgG half-molecule **characterized by** being associated with a second IgG half-molecule, wherein the first IgG half-molecule and the second IgG half-molecule satisfy the following (1B) to (7B):

(1B) the first IgG half-molecule and the second IgG half-molecule form an antibody composition against a first antigen and a second antigen that are different from each other,
(2B) each of the first IgG half-molecule and the second IgG half-molecule is composed of one L chain and one H chain, and the H chain includes an H chain variable region, a hinge domain, a CH1 domain, a CH2 domain, and a CH3 domain, and has a first CD 16a-binding domain and a second CD 1 6a-binding domain that are different from each other in the CH2 domain,
(3B) each of the first IgG half-molecule and the second IgG half-molecule includes amino acid residue substitutions of C226A and C229A numbered according to the EU index,
(4B) the first IgG half-molecule includes an antigen-binding domain that binds to the first antigen, and includes an amino acid residue substitution of D265A numbered according to the EU index in the first CD16a-binding domain,
(5B) the second IgG half-molecule includes an antigen-binding domain that binds to the second antigen, and includes an amino acid residue substitution of P329Y numbered according to the EU index in the second CD16a-binding domain,
(6B) each of the first IgG half-molecule and the second IgG half-molecule includes amino acid residue substitutions of (a) S239D and K326T numbered according to the EU index, or
each of the first IgG half-molecule and the second IgG half-molecule includes amino acid residue substitutions of (b) S239D, S298A, E333A, L242C, and K334C numbered according to the EU index, and
(7B) at least one of the first IgG half-molecule and the second IgG half-molecule includes an amino acid residue substitution in the CH3 domain as an alteration for attenuating an inter-CH3 domain interaction as compared with an inter-CH3 domain interaction of the IgG1 subclass.

**10.** A second IgG half-molecule, which is a second IgG half-molecule **characterized by** being associated with a first IgG half-molecule, wherein the first IgG half-molecule and the second IgG half-molecule satisfy the following (1C) to (7C):

(1C) the first IgG half-molecule and the second IgG half-molecule form an antibody composition against a first antigen and a second antigen that are different from each other,
(2C) each of the first IgG half-molecule and the second IgG half-molecule is composed of one L chain and one H chain, and the H chain includes an H chain variable region, a hinge domain, a CH1 domain, a CH2 domain, and a CH3 domain, and has a first CD16a-binding domain and a second CD16a-binding domain that are different from each other in the CH2 domain,
(3C) each of the first IgG half-molecule and the second IgG half-molecule includes amino acid residue substitutions of C226A and C229A numbered according to the EU index,
(4C) the first IgG half-molecule includes an antigen-binding domain that binds to the first antigen, and includes an amino acid residue substitution of D265A numbered according to the EU index in the first CD16a-binding domain,
(5C) the second IgG half-molecule includes an antigen-binding domain that binds to the second antigen, and includes an amino acid residue substitution of P329Y numbered according to the EU index in the second CD16a-binding domain,
(6C) each of the first IgG half-molecule and the second IgG half-molecule includes amino acid residue substitutions of (a) S239D and K326T numbered according to the EU index, or
each of the first IgG half-molecule and the second IgG half-molecule includes amino acid residue substitutions of (b) S239D, S298A, E333A, L242C, and K334C numbered according to the EU index, and
(7C) at least one of the first IgG half-molecule and the second IgG half-molecule includes an amino acid residue substitution in the CH3 domain as an alteration for attenuating an inter-CH3 domain interaction as compared with an inter-CH3 domain interaction of the IgG1 subclass.

**11.** A DNA encoding the following amino acid sequence a) or b):

a) an amino acid sequence of the first IgG half-molecule according to claim 9 or
b) an amino acid sequence of the second IgG half-molecule according to claim 10.

**12.** A recombinant vector, comprising at least one of the DNA encoding the amino acid sequence a) and the DNA encoding the amino acid sequence b) according to claim 11.

**13.** A transformant, in which the recombinant vector according to claim 12 is introduced.

**14.** A kit, comprising the first IgG half-molecule according to claim 9 and the second IgG half-molecule according to claim 10.

**15.** A method for inducing an effector function specifically for a target cell coexpressing the first antigen and the second antigen as compared with an effector function for a target cell expressing only the first antigen and a target cell expressing only the second antigen using the antibody composition according to any one of claims 1 to 8.

**16.** A pharmaceutical composition, comprising the antibody composition according to any one of claims 1 to 8.

**17.** The pharmaceutical composition according to claim 16, for use in a treatment of a cancer, an autoimmune disease, or an allergic disease.

*FIG. 1*

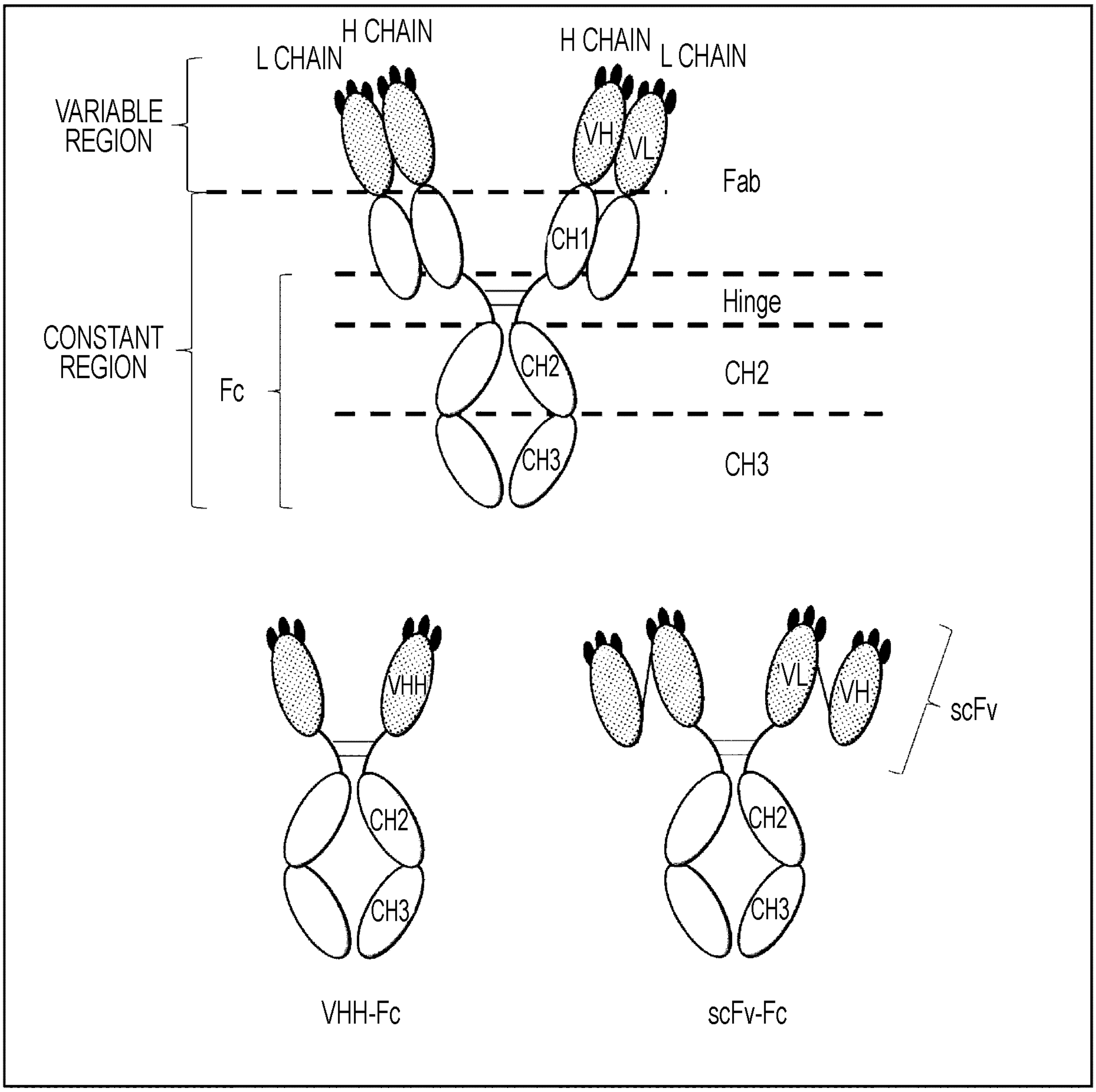
H CHAIN
L CHAIN
H CHAIN
L CHAIN
VARIABLE REGION
VH
VL
Fab
CH1
Hinge
CONSTANT REGION
Fc
CH2
CH2
CH3
CH3
VHH
VL
VH
scFv
CH2
CH3
CH2
CH3
VHH-Fc
scFv-Fc

FIG. 2A

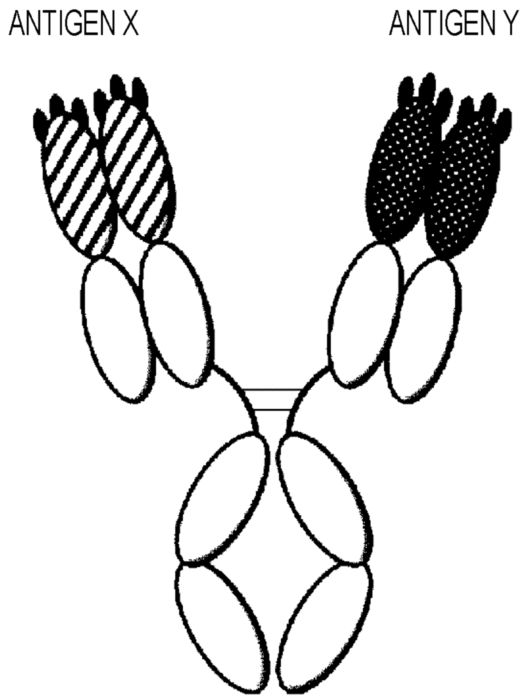
ANTIGEN X
ANTIGEN Y

FIG. 2B

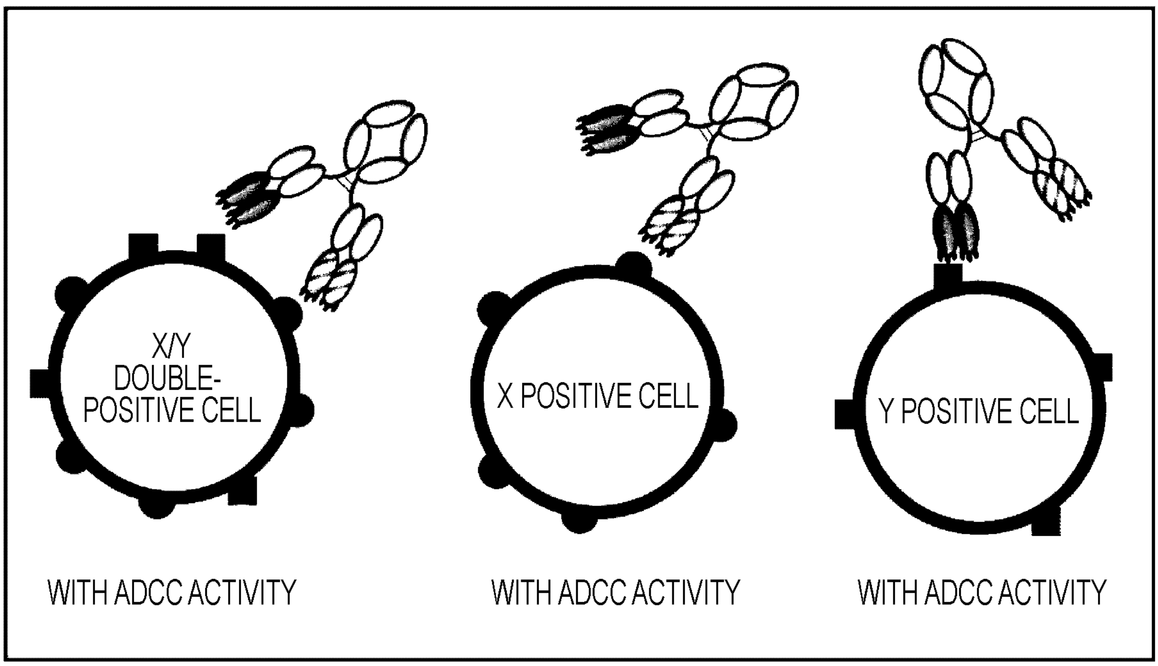
X/Y DOUBLE-POSITIVE CELL
X POSITIVE CELL
Y POSITIVE CELL
WITH ADCC ACTIVITY
WITH ADCC ACTIVITY
WITH ADCC ACTIVITY

FIG. 3

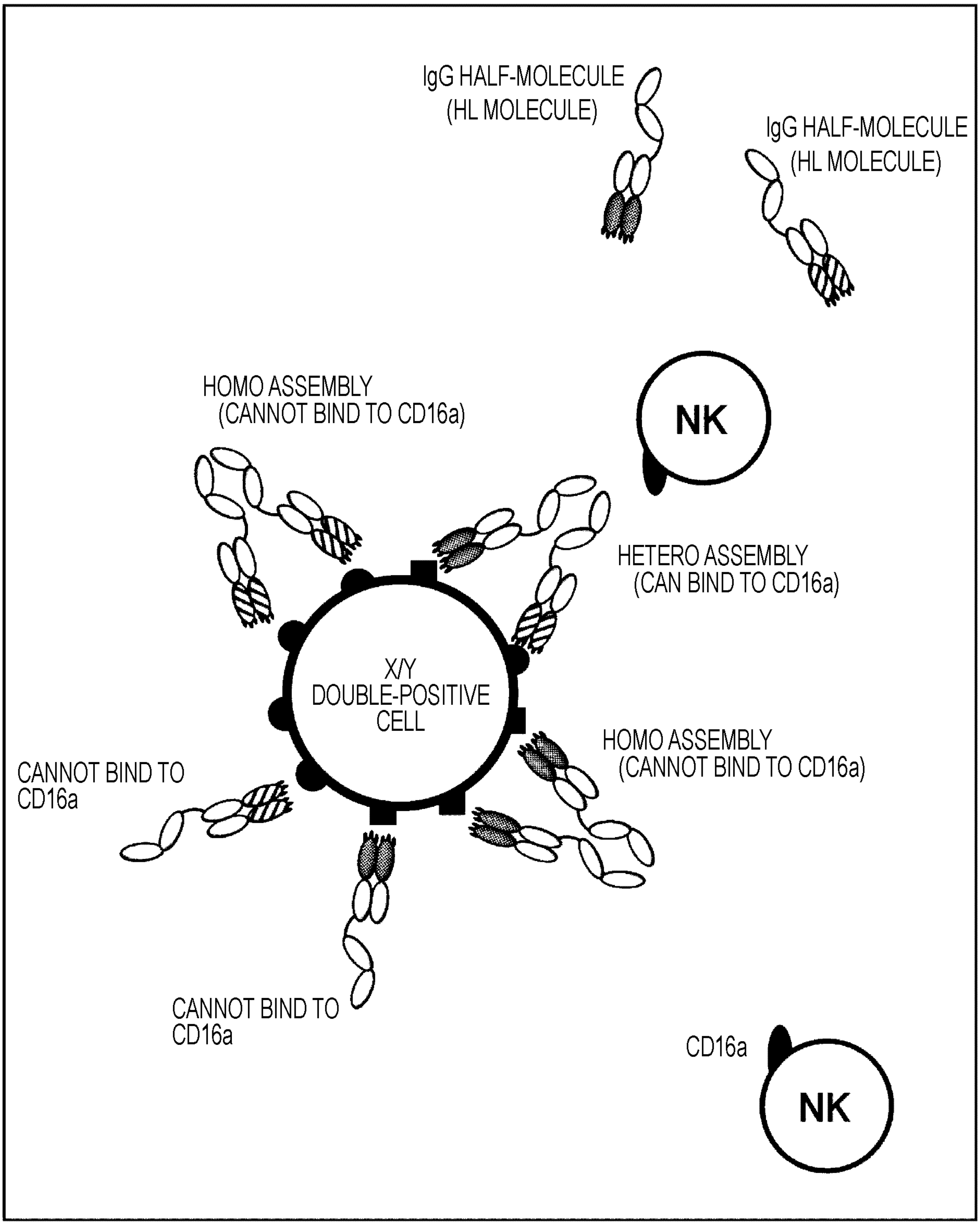
IgG HALF-MOLECULE
(HL MOLECULE)
IgG HALF-MOLECULE
(HL MOLECULE)
HOMO ASSEMBLY
(CANNOT BIND TO CD16a)
NK
HETERO ASSEMBLY
(CAN BIND TO CD16a)
X/Y
DOUBLE-POSITIVE
CELL
HOMO ASSEMBLY
(CANNOT BIND TO CD16a)
CANNOT BIND TO
CD16a
CANNOT BIND TO
CD16a
CD16a
NK

*FIG. 4*

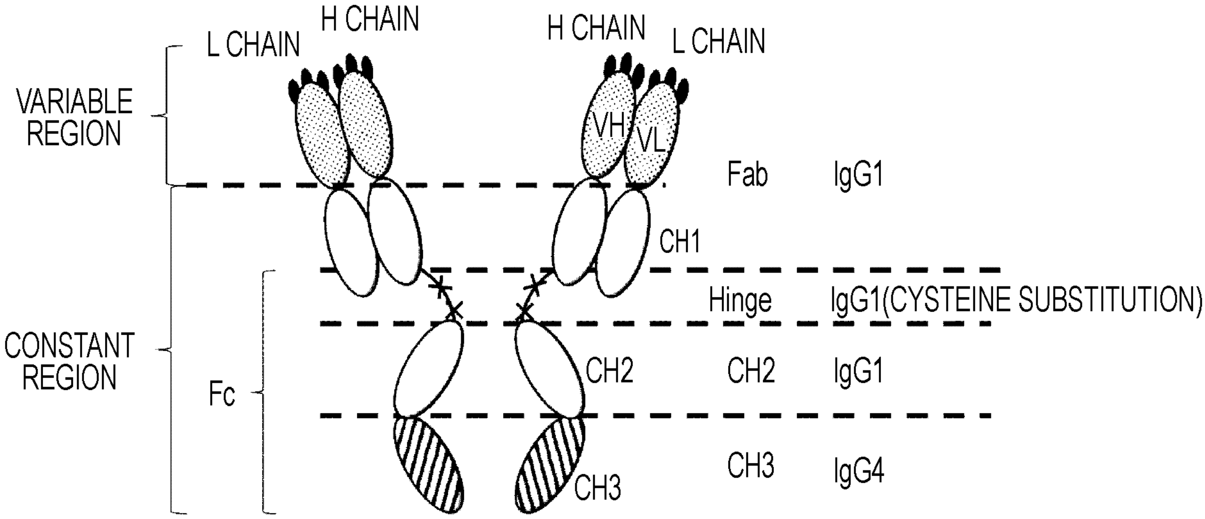
L CHAIN
H CHAIN
H CHAIN
L CHAIN
VARIABLE REGION
VH
VL
Fab
IgG1
CH1
Hinge
IgG1(CYSTEINE SUBSTITUTION)
CONSTANT REGION
Fc
CH2
CH2
IgG1
CH3
CH3
IgG4

FIG. 5

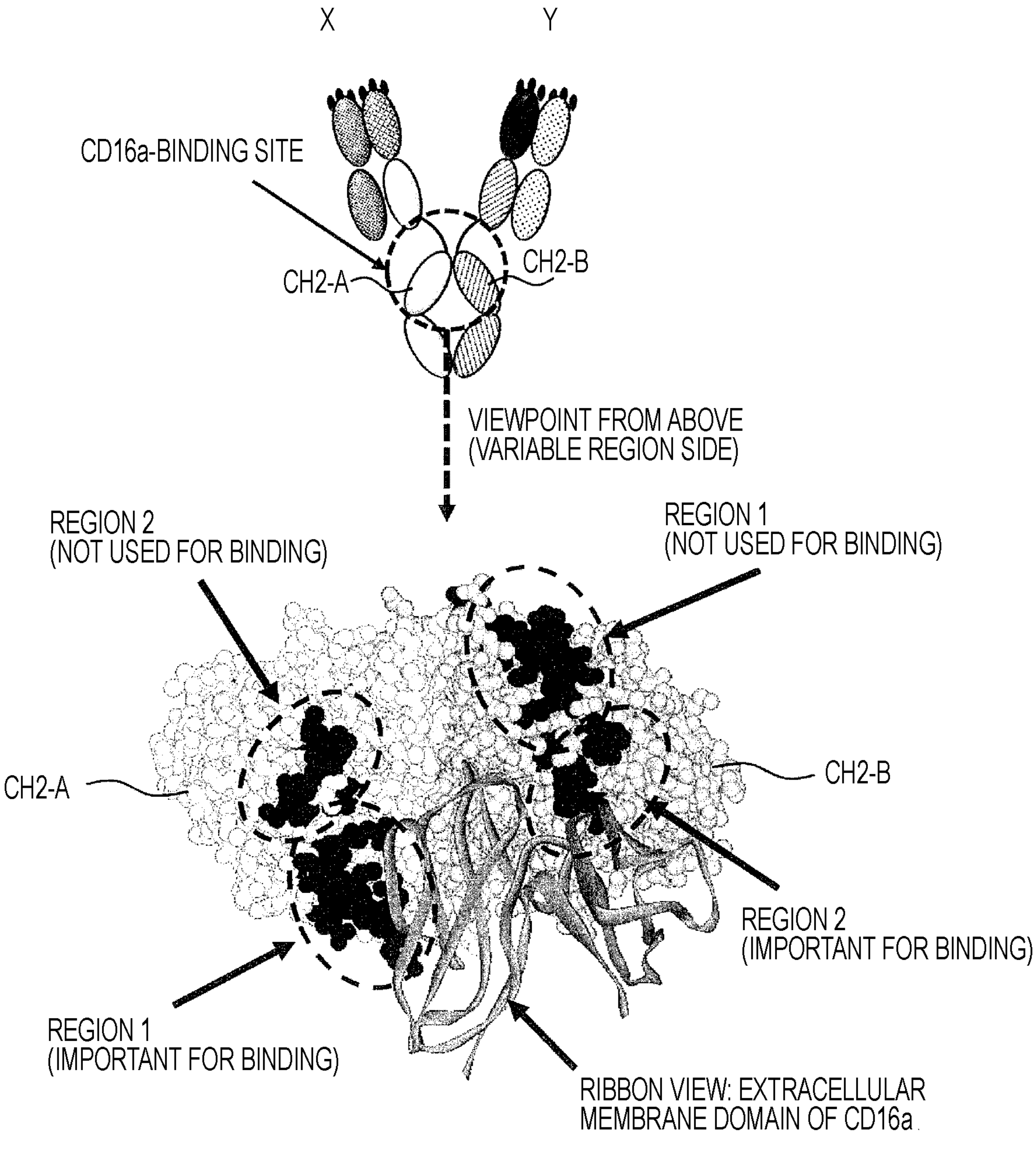
X
Y
CD16a-BINDING SITE
CH2-A
CH2-B
VIEWPOINT FROM ABOVE
(VARIABLE REGION SIDE)
REGION 2
(NOT USED FOR BINDING)
REGION 1
(NOT USED FOR BINDING)
CH2-A
CH2-B
REGION 2
(IMPORTANT FOR BINDING)
REGION 1
(IMPORTANT FOR BINDING)
RIBBON VIEW: EXTRACELLULAR
MEMBRANE DOMAIN OF CD16a

FIG. 6

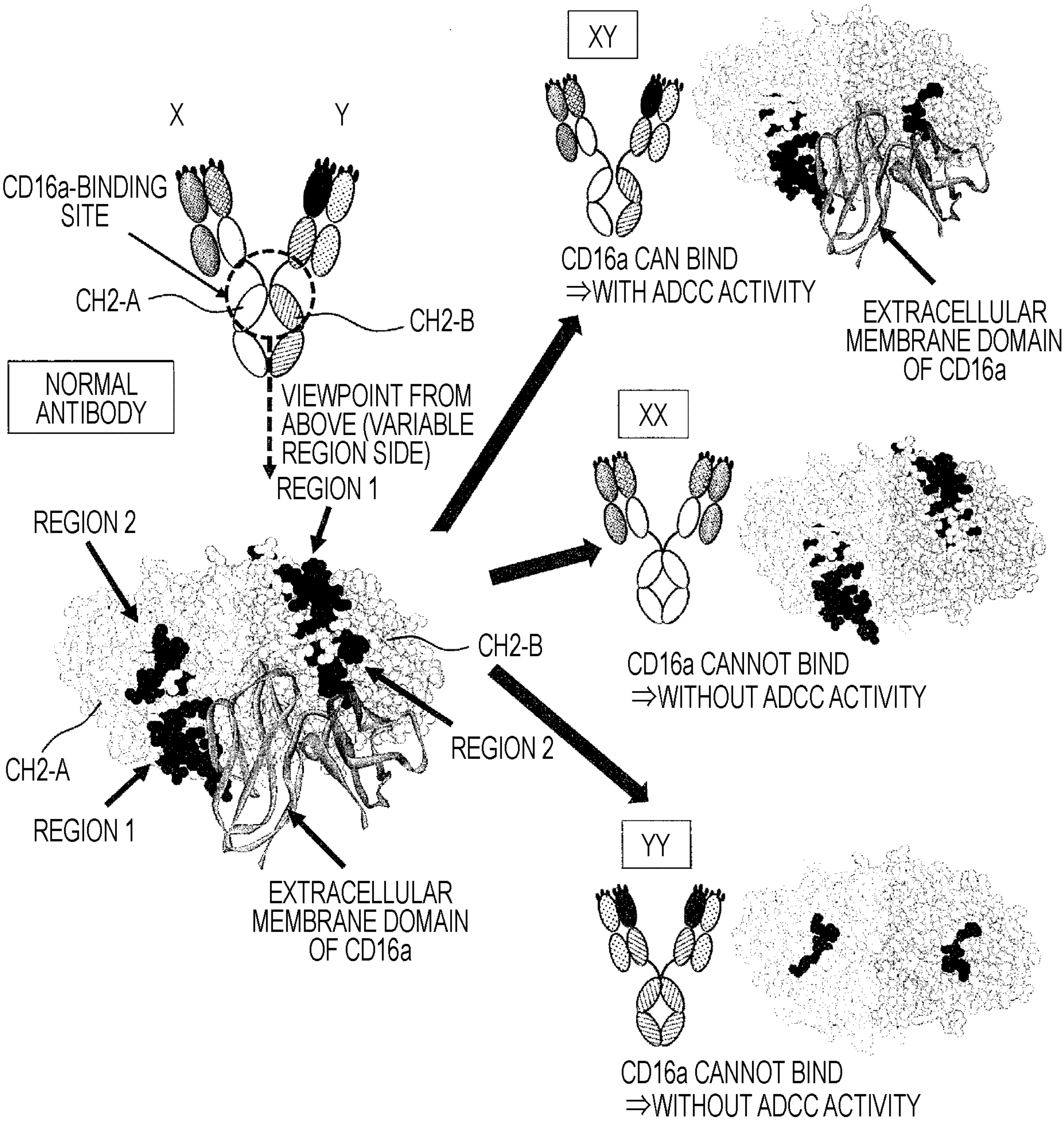
XY
X
Y
CD16a-BINDING SITE
CH2-A
CH2-B
NORMAL ANTIBODY
VIEWPOINT FROM ABOVE (VARIABLE REGION SIDE)
REGION 1
REGION 2
CH2-B
REGION 2
CH2-A
REGION 1
EXTRACELLULAR MEMBRANE DOMAIN OF CD16a
CD16a CAN BIND
⇒WITH ADCC ACTIVITY
EXTRACELLULAR MEMBRANE DOMAIN OF CD16a
XX
CD16a CANNOT BIND
⇒WITHOUT ADCC ACTIVITY
YY
CD16a CANNOT BIND
⇒WITHOUT ADCC ACTIVITY

FIG. 7

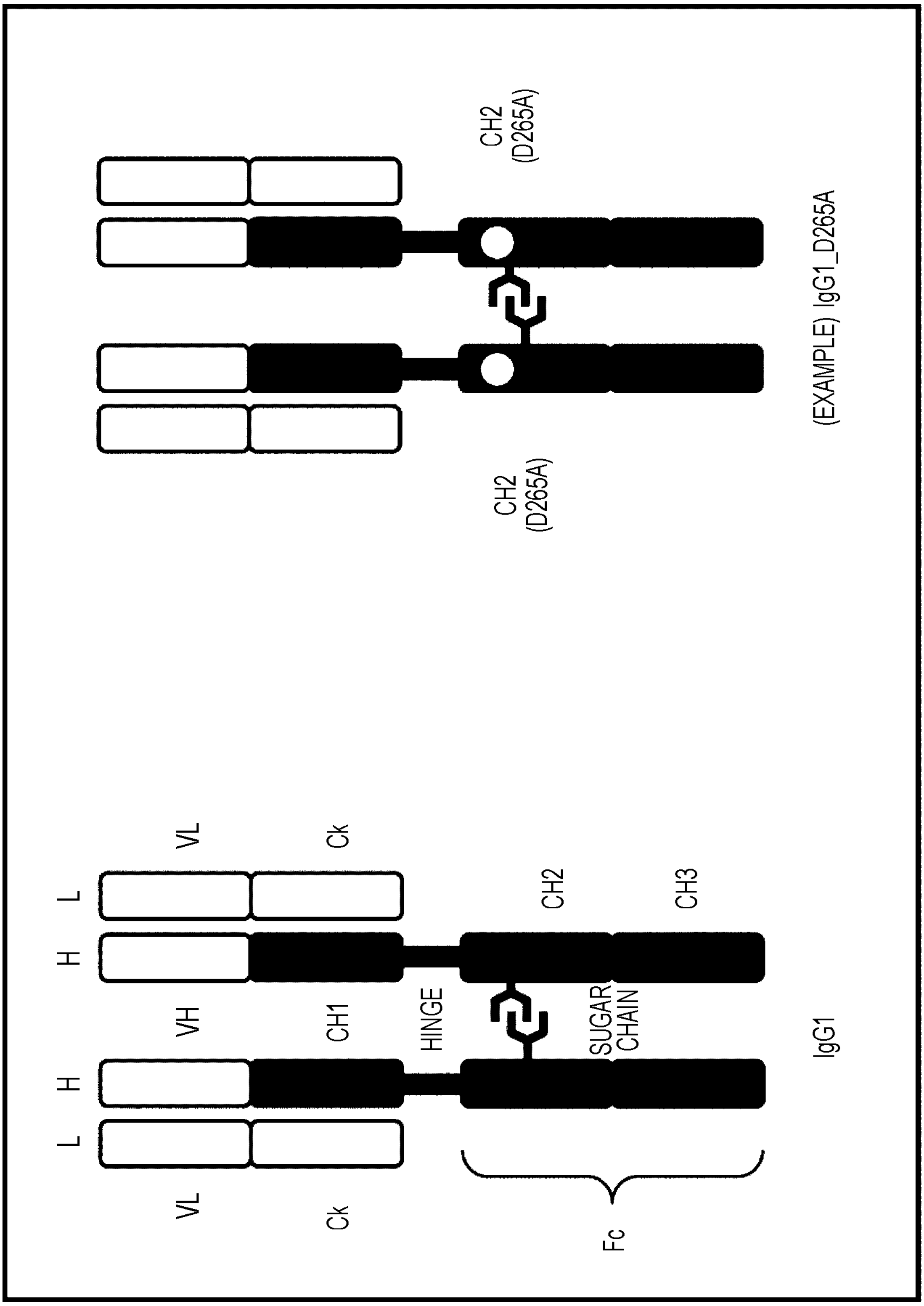

L
H
H
L
VL
Ck
VH
CH1
HINGE
CH2
CH3
SUGAR CHAIN
Fc
IgG1
CH2 (D265A)
CH2 (D265A)
(EXAMPLE) IgG1_D265A

FIG. 8

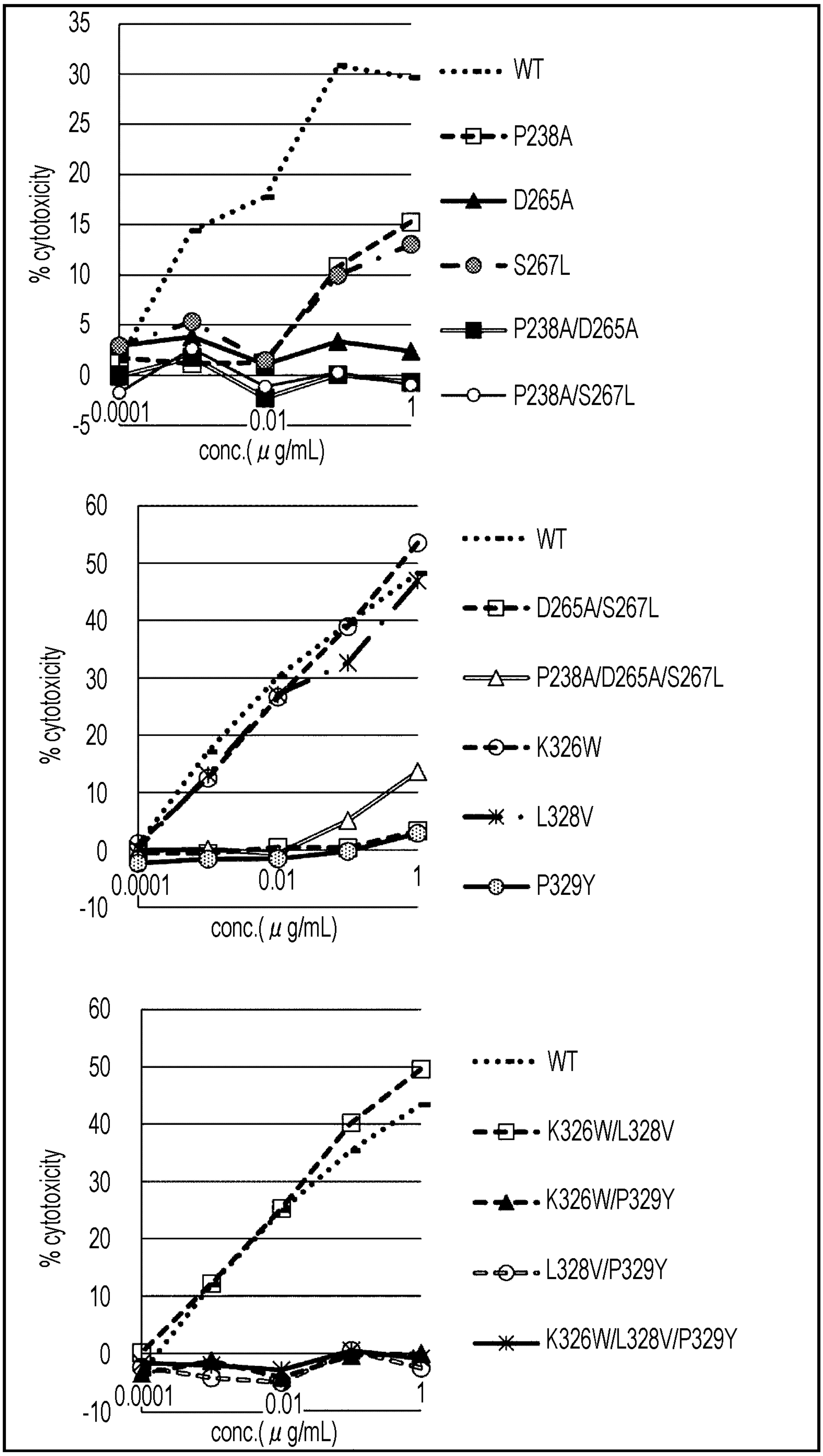

35
30
25
20
15
10
5
0
-5
% cytotoxicity
0.0001
0.01
1
conc.( μ g/mL)
WT
P238A
D265A
S267L
P238A/D265A
P238A/S267L
60
50
40
30
20
10
0
-10
% cytotoxicity
0.0001
0.01
1
conc.( μ g/mL)
WT
D265A/S267L
P238A/D265A/S267L
K326W
L328V
P329Y
60
50
40
30
20
10
0
-10
% cytotoxicity
0.0001
0.01
1
conc.( μ g/mL)
WT
K326W/L328V
K326W/P329Y
L328V/P329Y
K326W/L328V/P329Y

*FIG. 9*

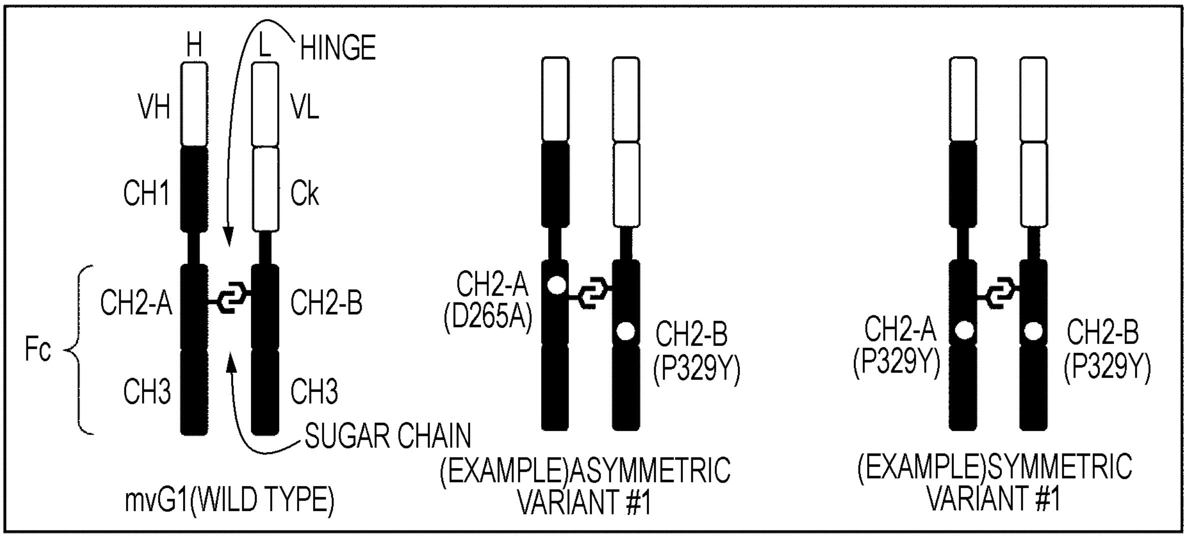
H
L
HINGE
VH
VL
CH1
Ck
CH2-A
CH2-B
Fc
CH3
CH3
SUGAR CHAIN
mvG1(WILD TYPE)
CH2-A
(D265A)
CH2-B
(P329Y)
(EXAMPLE)ASYMMETRIC
VARIANT #1
CH2-A
(P329Y)
CH2-B
(P329Y)
(EXAMPLE)SYMMETRIC
VARIANT #1

*FIG. 10*

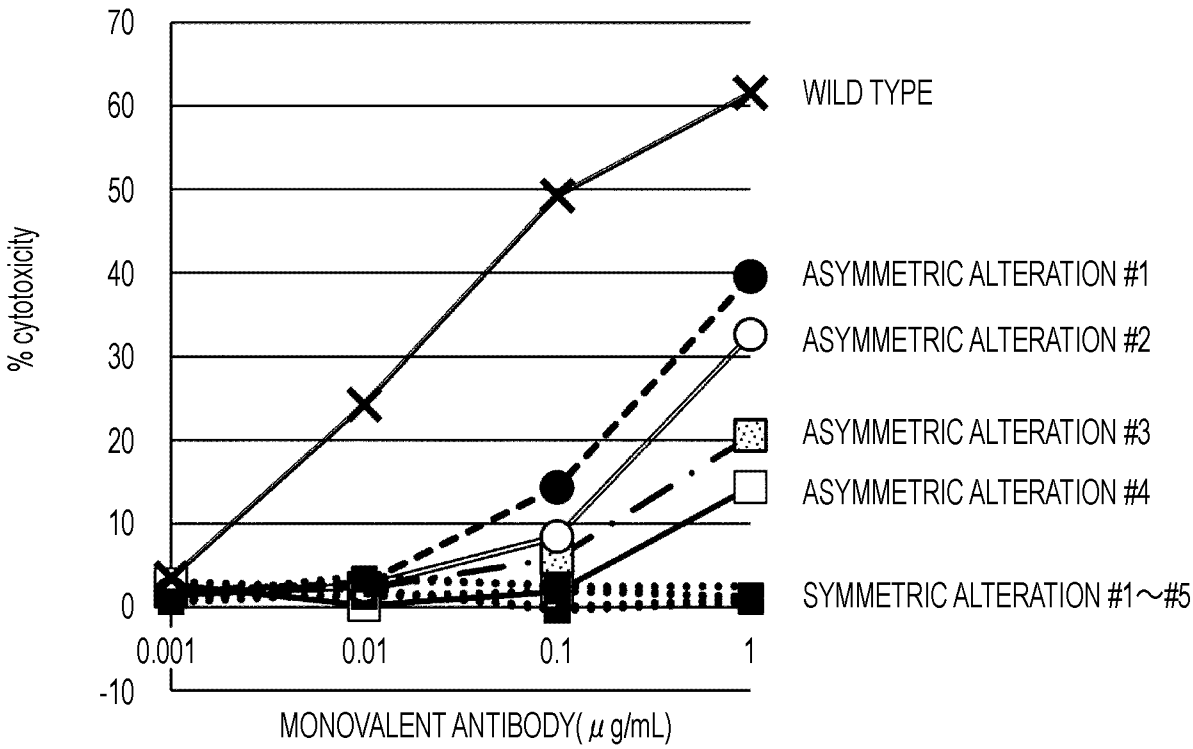
% cytotoxicity
70
60
50
40
30
20
10
0
-10
0.001
0.01
0.1
1
MONOVALENT ANTIBODY(μg/mL)
WILD TYPE
ASYMMETRIC ALTERATION #1
ASYMMETRIC ALTERATION #2
ASYMMETRIC ALTERATION #3
ASYMMETRIC ALTERATION #4
SYMMETRIC ALTERATION #1～#5

FIG. 11

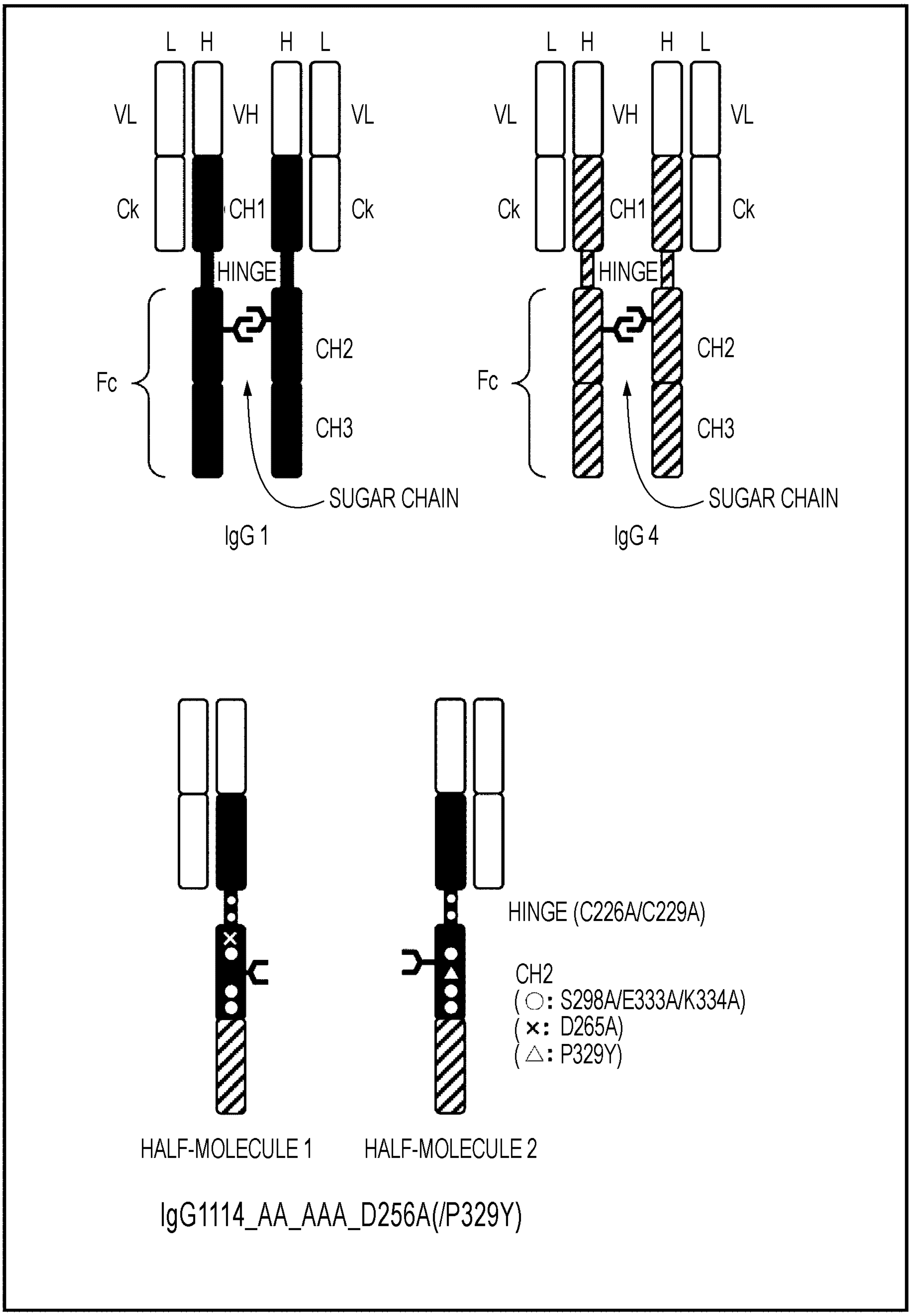
L
H
H
L
VL
VH
VL
Ck
CH1
Ck
HINGE
CH2
Fc
CH3
SUGAR CHAIN
IgG 1
L
H
H
L
VL
VH
VL
Ck
CH1
Ck
HINGE
CH2
Fc
CH3
SUGAR CHAIN
IgG 4
HINGE (C226A/C229A)
CH2
(○: S298A/E333A/K334A)
(×: D265A)
(△: P329Y)
HALF-MOLECULE 1
HALF-MOLECULE 2
IgG1114_AA_AAA_D256A(/P329Y)

FIG. 12

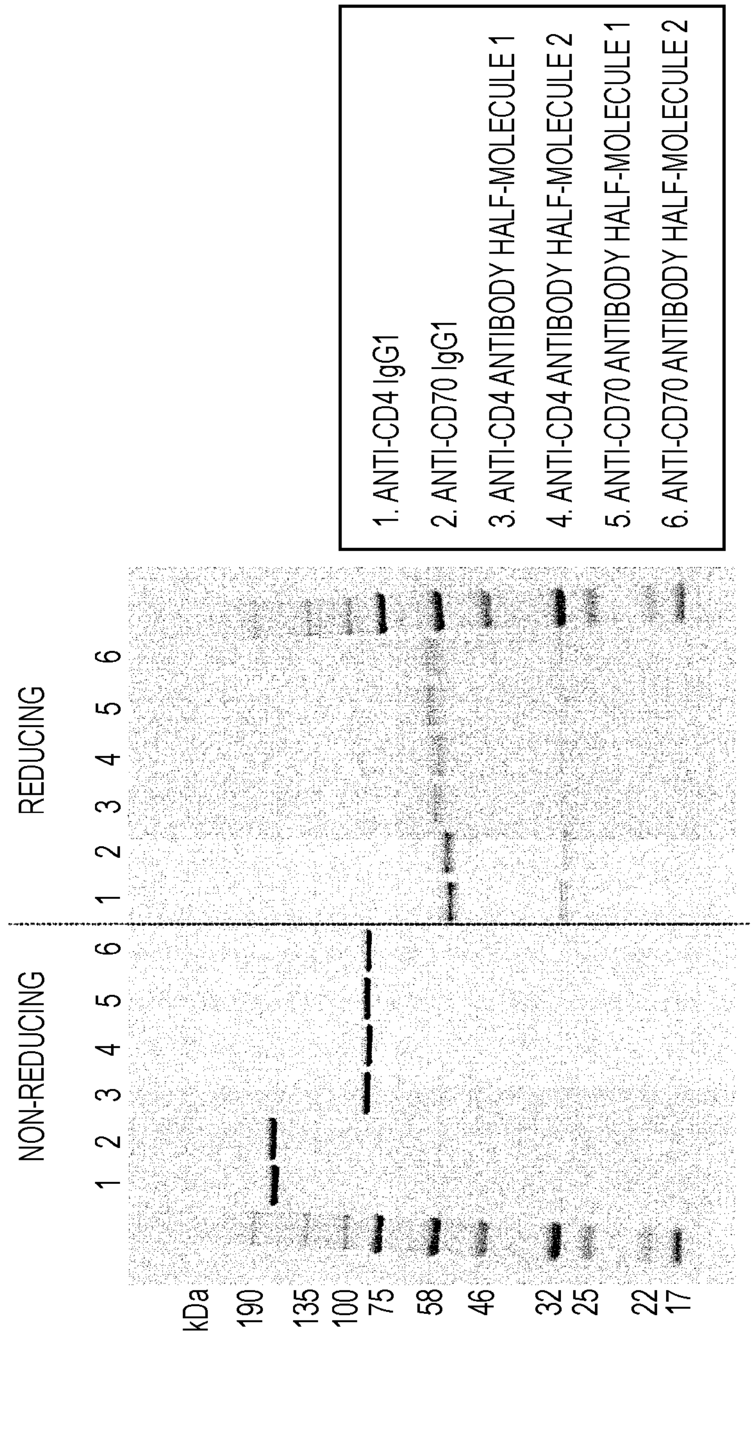
NON-REDUCING
REDUCING
1 2 3 4 5 6
1 2 3 4 5 6
kDa
190
135
100
75
58
46
32
25
22
17
1. ANTI-CD4 IgG1
2. ANTI-CD70 IgG1
3. ANTI-CD4 ANTIBODY HALF-MOLECULE 1
4. ANTI-CD4 ANTIBODY HALF-MOLECULE 2
5. ANTI-CD70 ANTIBODY HALF-MOLECULE 1
6. ANTI-CD70 ANTIBODY HALF-MOLECULE 2

FIG. 13

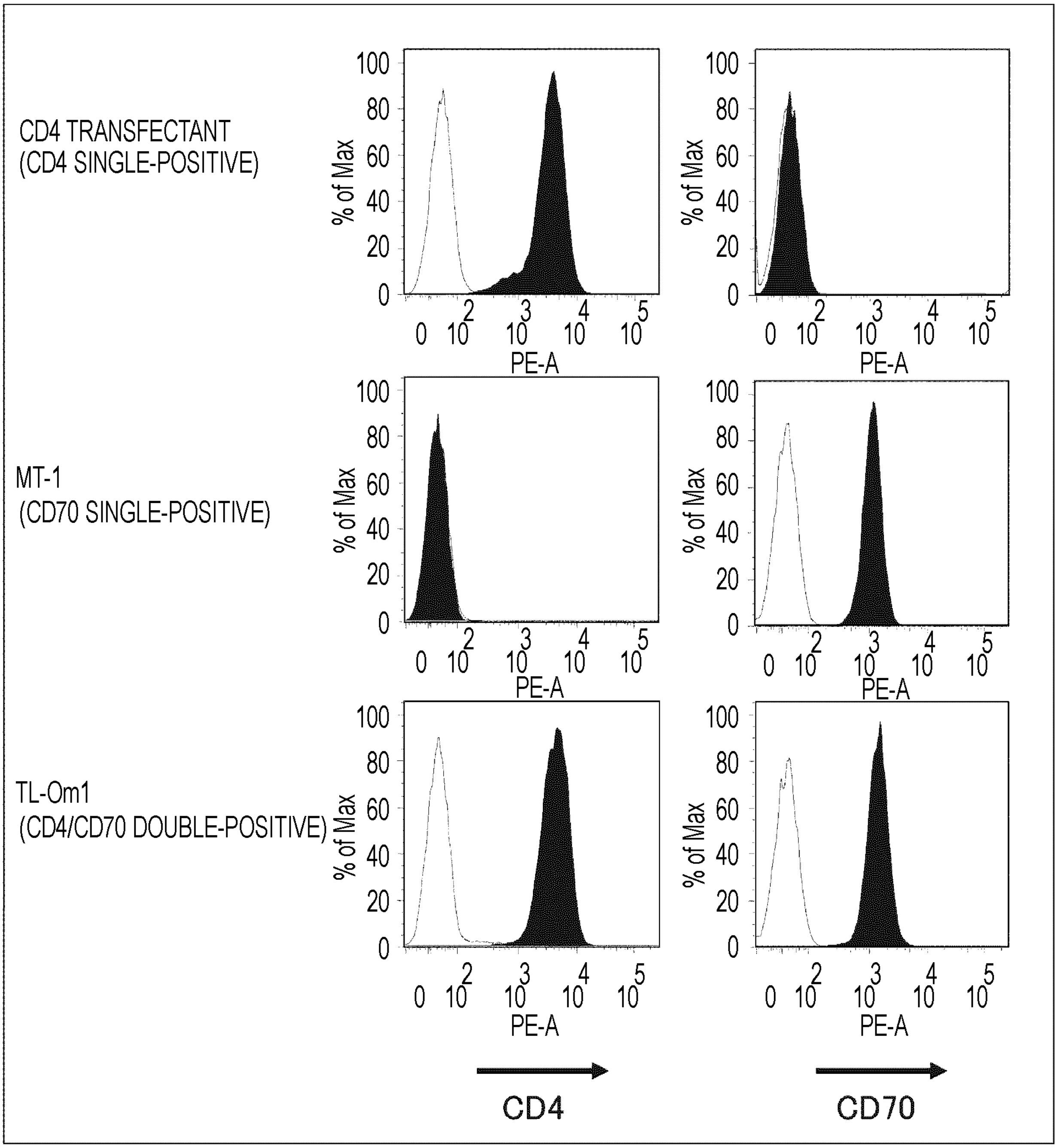
CD4 TRANSFECTANT
(CD4 SINGLE-POSITIVE)
MT-1
(CD70 SINGLE-POSITIVE)
TL-Om1
(CD4/CD70 DOUBLE-POSITIVE)
% of Max
PE-A
CD4
CD70

FIG. 14

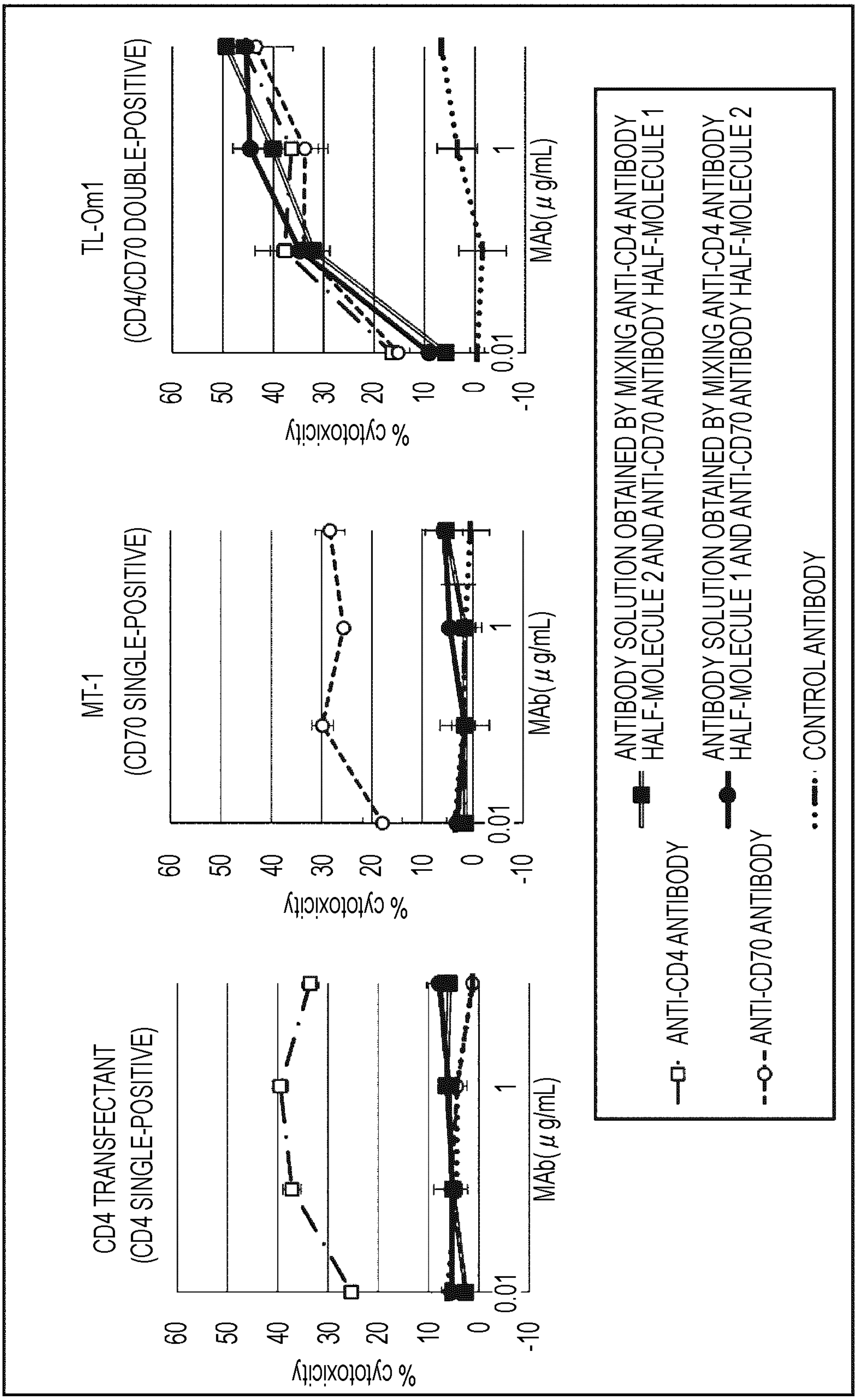

CD4 TRANSFECTANT
(CD4 SINGLE-POSITIVE)
MT-1
(CD70 SINGLE-POSITIVE)
TL-Om1
(CD4/CD70 DOUBLE-POSITIVE)
% cytotoxicity
60
50
40
30
20
10
0
-10
0.01
1
MAb(μ g/mL)
ANTI-CD4 ANTIBODY
ANTI-CD70 ANTIBODY
ANTIBODY SOLUTION OBTAINED BY MIXING ANTI-CD4 ANTIBODY HALF-MOLECULE 2 AND ANTI-CD70 ANTIBODY HALF-MOLECULE 1
ANTIBODY SOLUTION OBTAINED BY MIXING ANTI-CD4 ANTIBODY HALF-MOLECULE 1 AND ANTI-CD70 ANTIBODY HALF-MOLECULE 2
CONTROL ANTIBODY

FIG. 15A

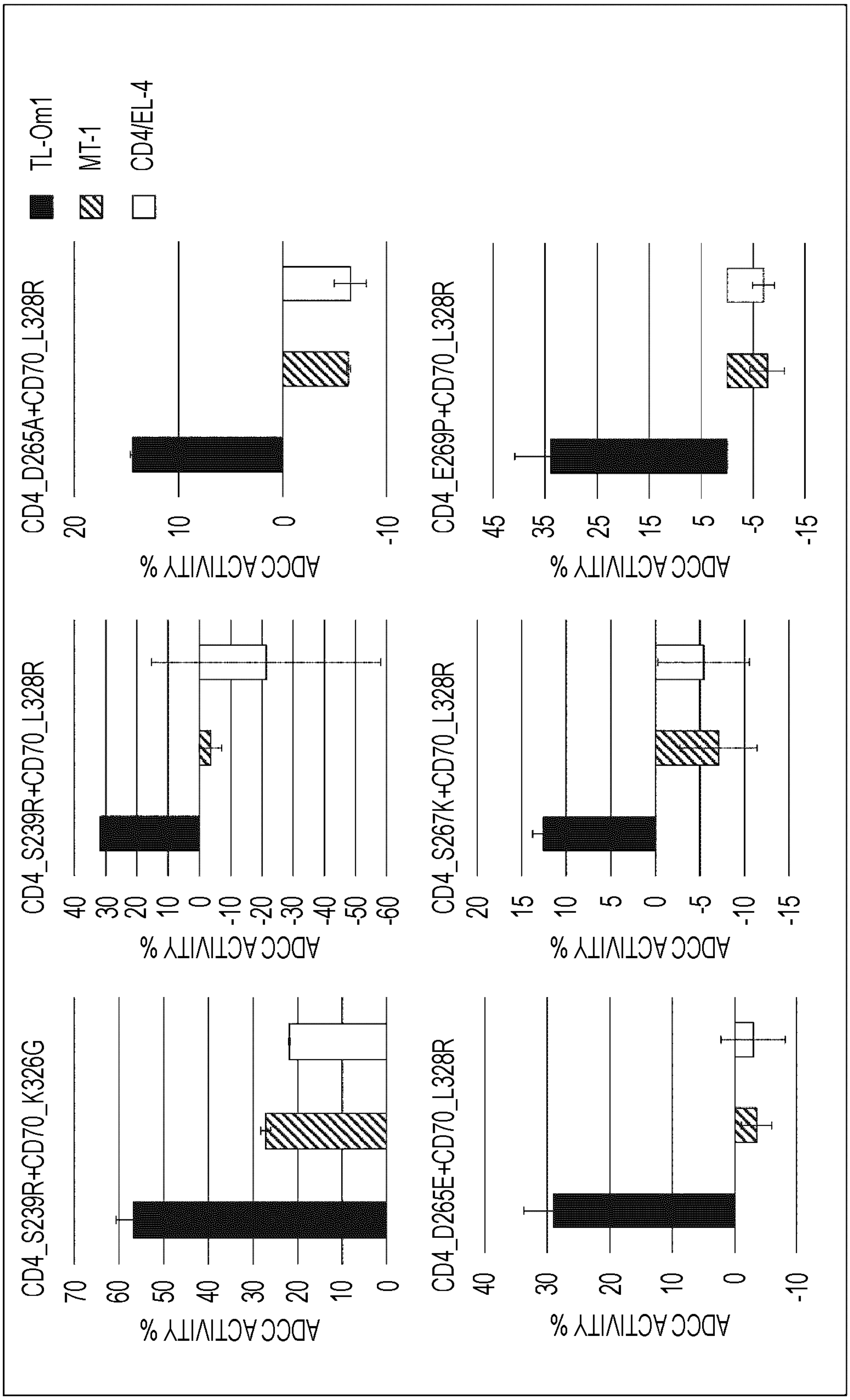
CD4_S239R+CD70_K326G
CD4_S239R+CD70_L328R
CD4_D265A+CD70_L328R
CD4_D265E+CD70_L328R
CD4_S267K+CD70_L328R
CD4_E269P+CD70_L328R
ADCC ACTIVITY %
TL-Om1
MT-1
CD4/EL-4

FIG. 15B

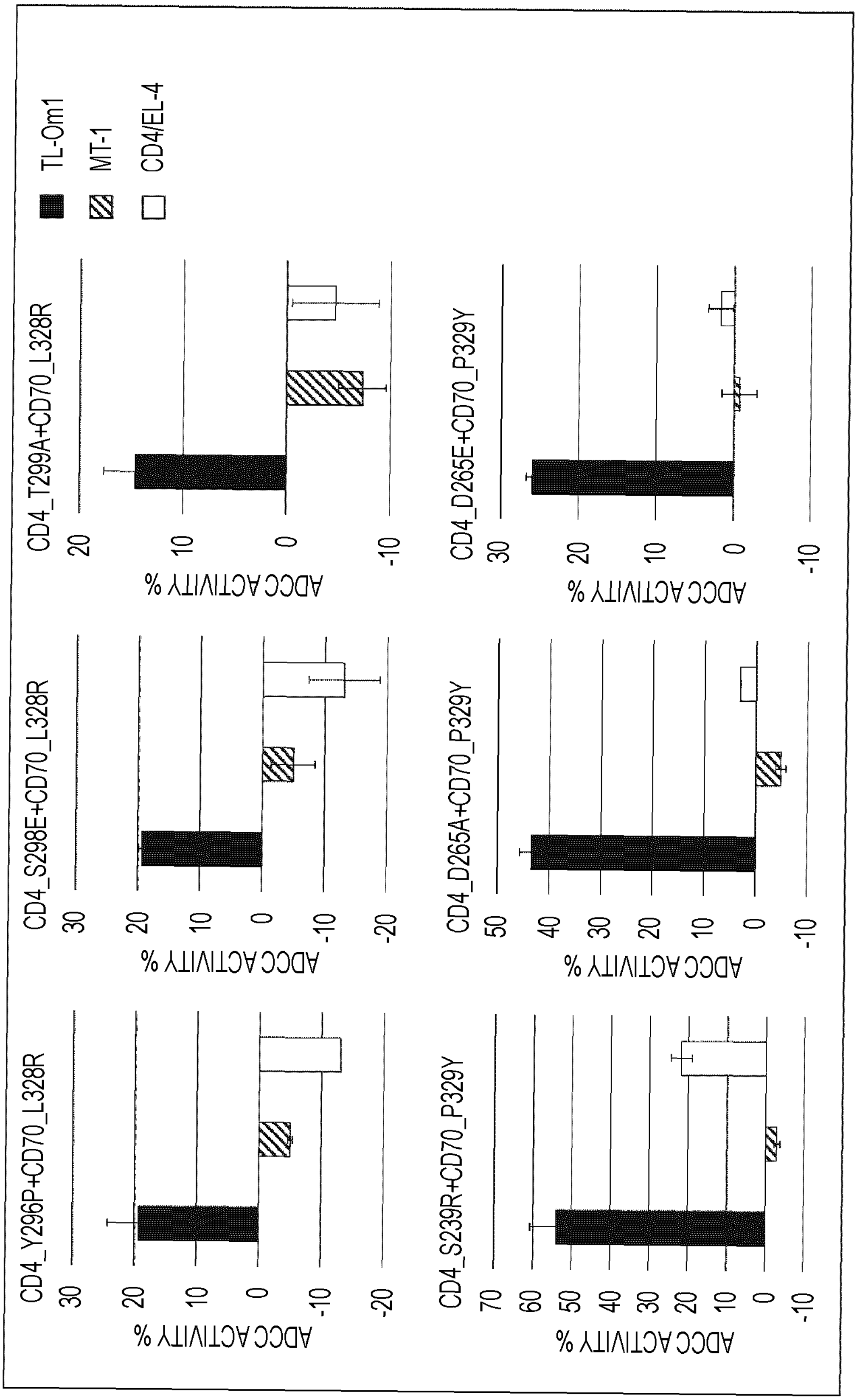
CD4_Y296P+CD70_L328R
CD4_S298E+CD70_L328R
CD4_T299A+CD70_L328R
CD4_S239R+CD70_P329Y
CD4_D265A+CD70_P329Y
CD4_D265E+CD70_P329Y
ADCC ACTIVITY %
TL-Om1
MT-1
CD4/EL-4

FIG. 15C

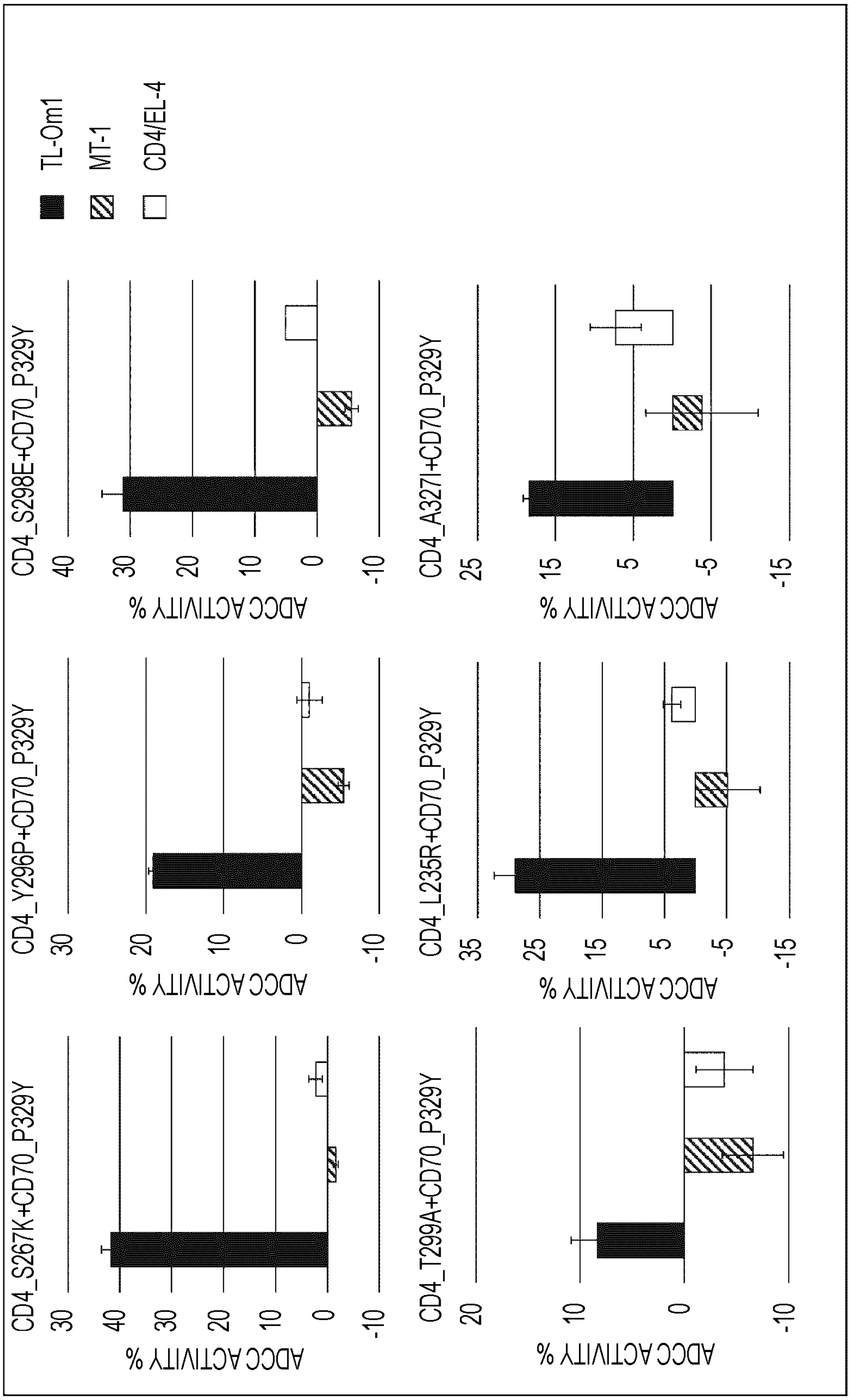

CD4_S267K+CD70_P329Y
CD4_Y296P+CD70_P329Y
CD4_S298E+CD70_P329Y
CD4_T299A+CD70_P329Y
CD4_L235R+CD70_P329Y
CD4_A327I+CD70_P329Y
ADCC ACTIVITY %
TL-Om1
MT-1
CD4/EL-4

FIG. 15D

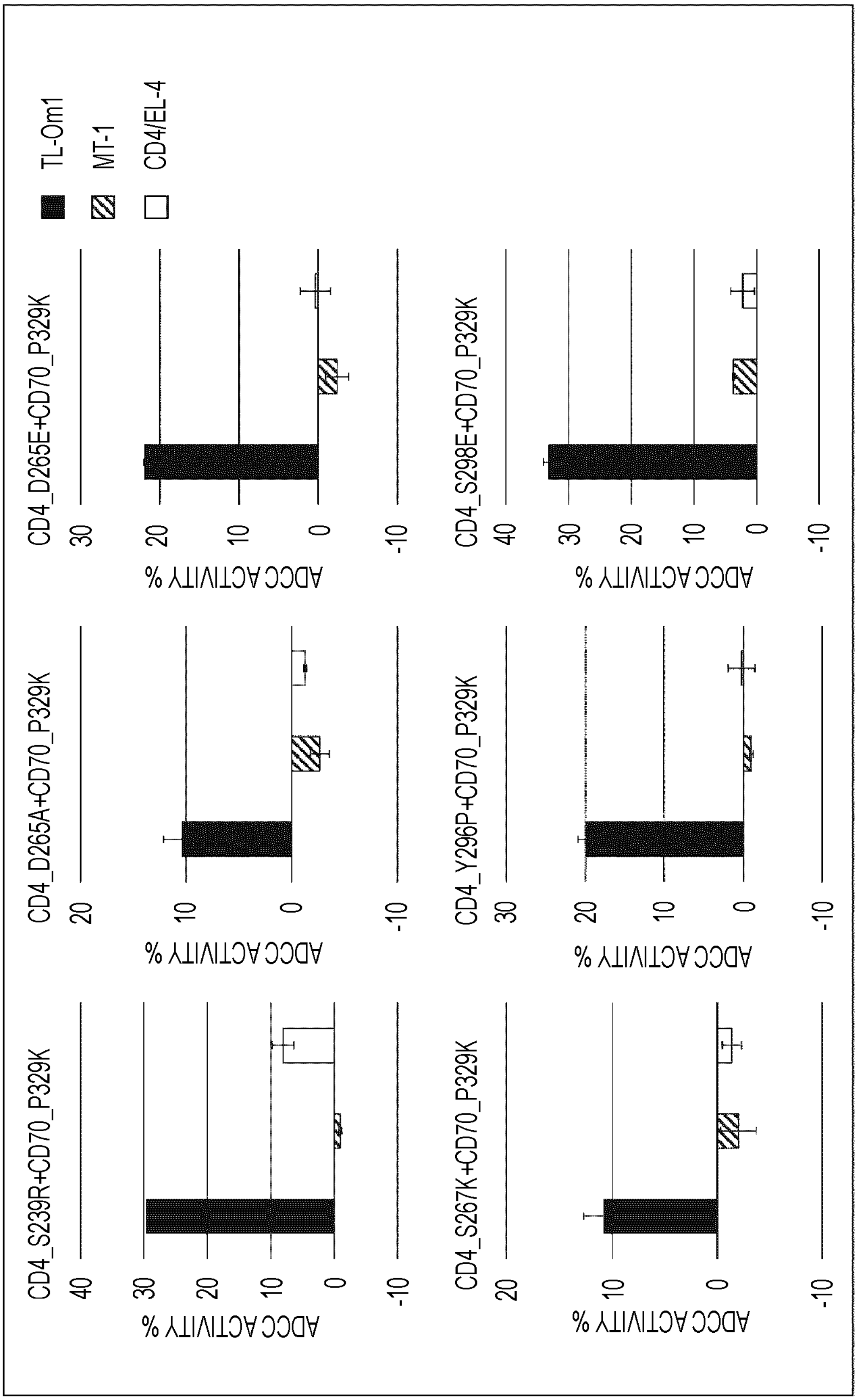
TL-Om1
MT-1
CD4/EL-4
CD4_S239R+CD70_P329K
CD4_D265A+CD70_P329K
CD4_D265E+CD70_P329K
CD4_S267K+CD70_P329K
CD4_Y296P+CD70_P329K
CD4_S298E+CD70_P329K
ADCC ACTIVITY %
40
30
20
10
0
-10

FIG. 15E

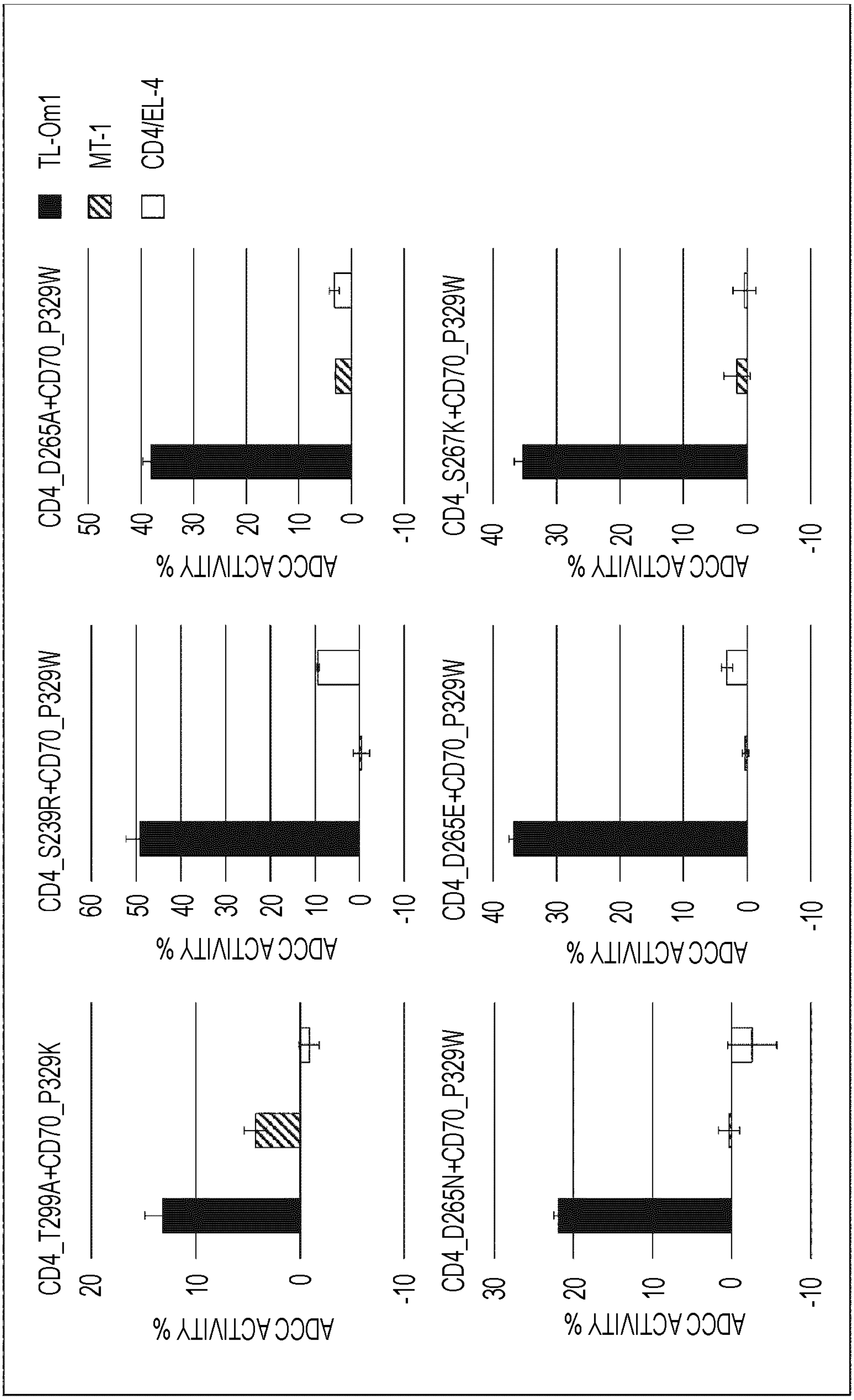
TL-Om1
MT-1
CD4/EL-4
CD4_T299A+CD70_P329K
CD4_S239R+CD70_P329W
CD4_D265A+CD70_P329W
CD4_D265N+CD70_P329W
CD4_D265E+CD70_P329W
CD4_S267K+CD70_P329W
ADCC ACTIVITY %
60
50
40
30
20
10
0
-10

FIG. 15F

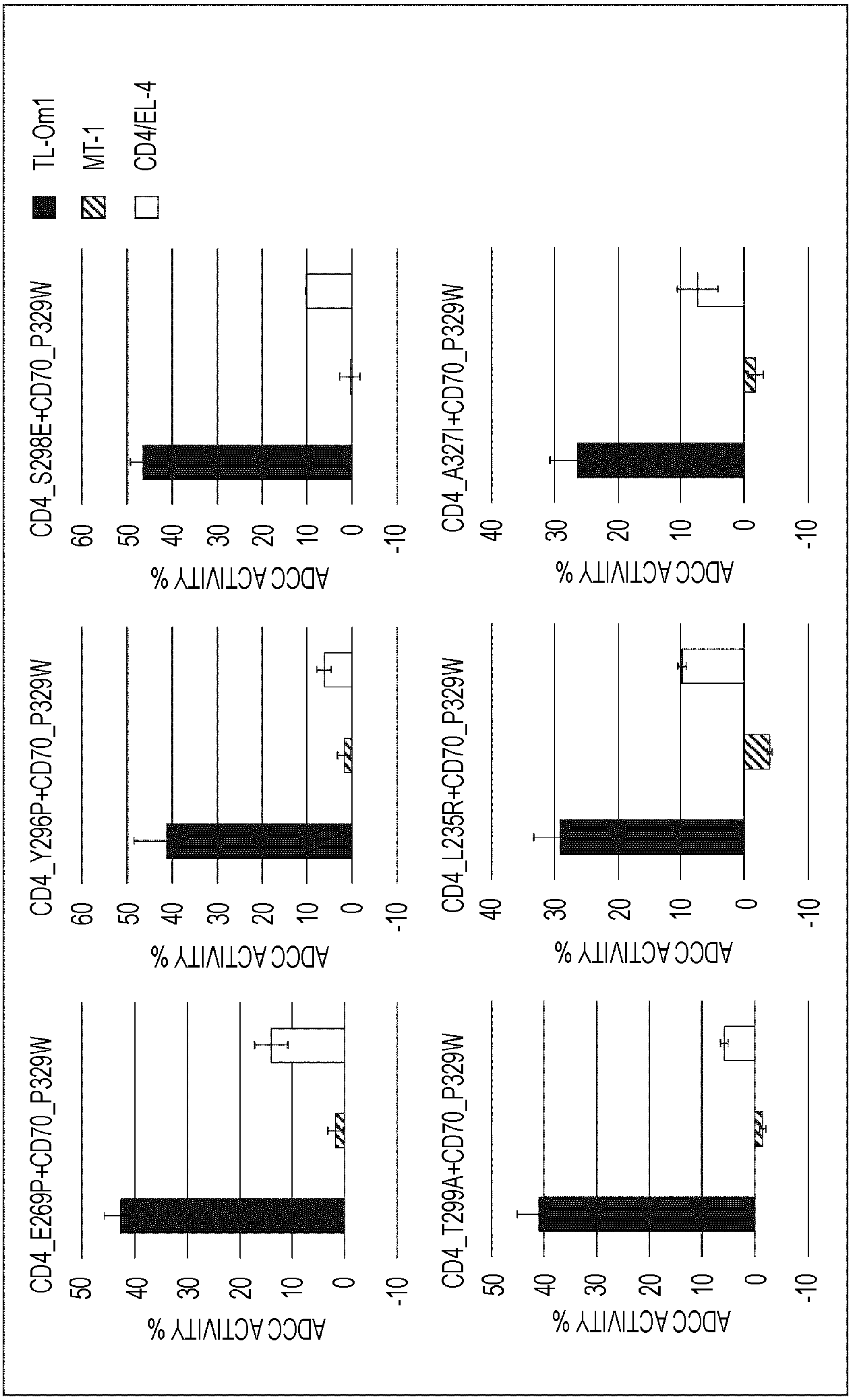
TL-Om1
MT-1
CD4/EL-4
CD4_E269P+CD70_P329W
CD4_Y296P+CD70_P329W
CD4_S298E+CD70_P329W
CD4_T299A+CD70_P329W
CD4_L235R+CD70_P329W
CD4_A327I+CD70_P329W
ADCC ACTIVITY %
60
50
40
30
20
10
0
-10

FIG. 15G

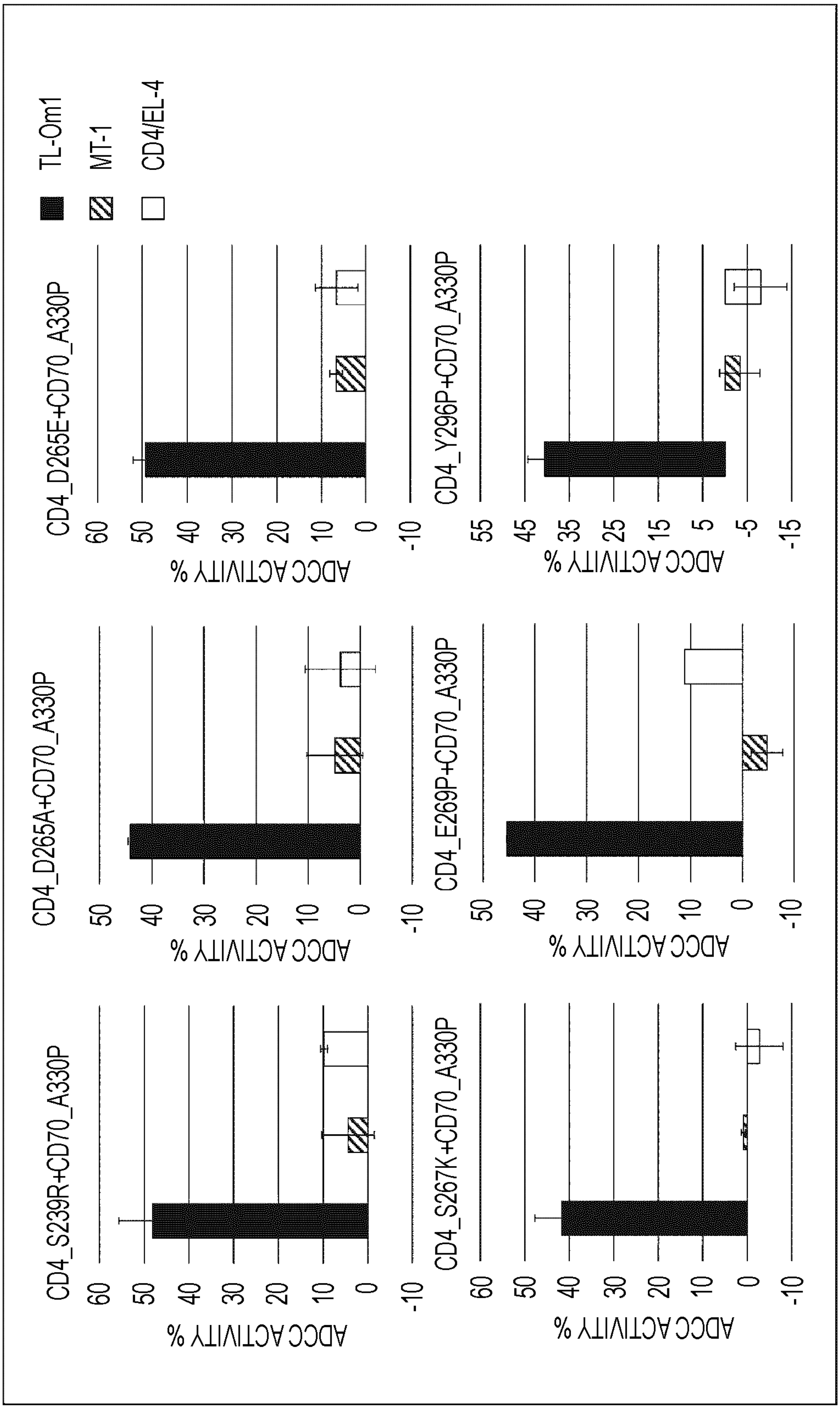
TL-Om1
MT-1
CD4/EL-4
CD4_S239R+CD70_A330P
CD4_D265A+CD70_A330P
CD4_D265E+CD70_A330P
CD4_S267K+CD70_A330P
CD4_E269P+CD70_A330P
CD4_Y296P+CD70_A330P
ADCC ACTIVITY %
60
50
40
30
20
10
0
-10
55
45
35
25
15
5
-5
-15

FIG. 15H

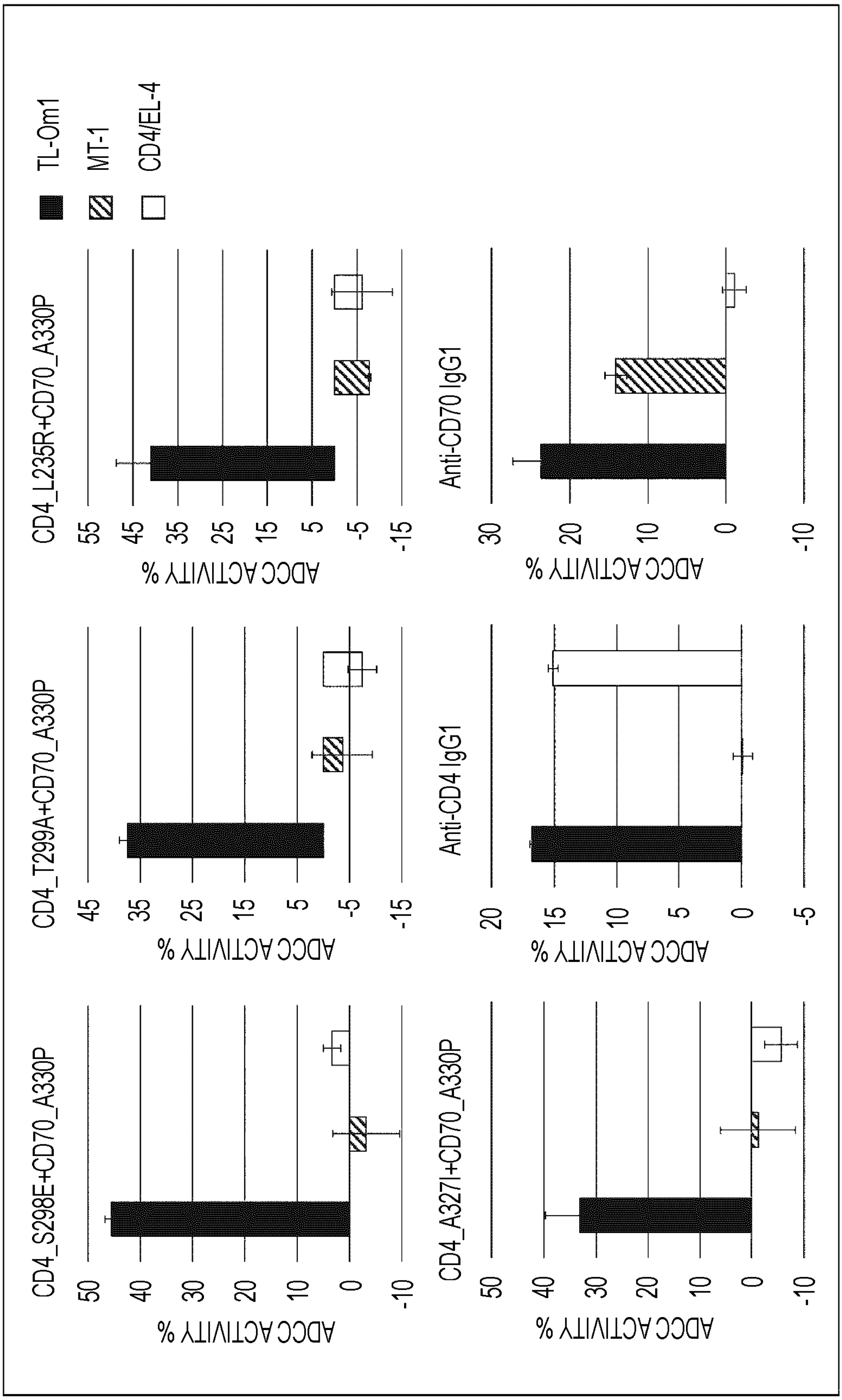
CD4_S298E+CD70_A330P
CD4_T299A+CD70_A330P
CD4_L235R+CD70_A330P
CD4_A327I+CD70_A330P
Anti-CD4 IgG1
Anti-CD70 IgG1
ADCC ACTIVITY %
TL-Om1
MT-1
CD4/EL-4

FIG. 16A

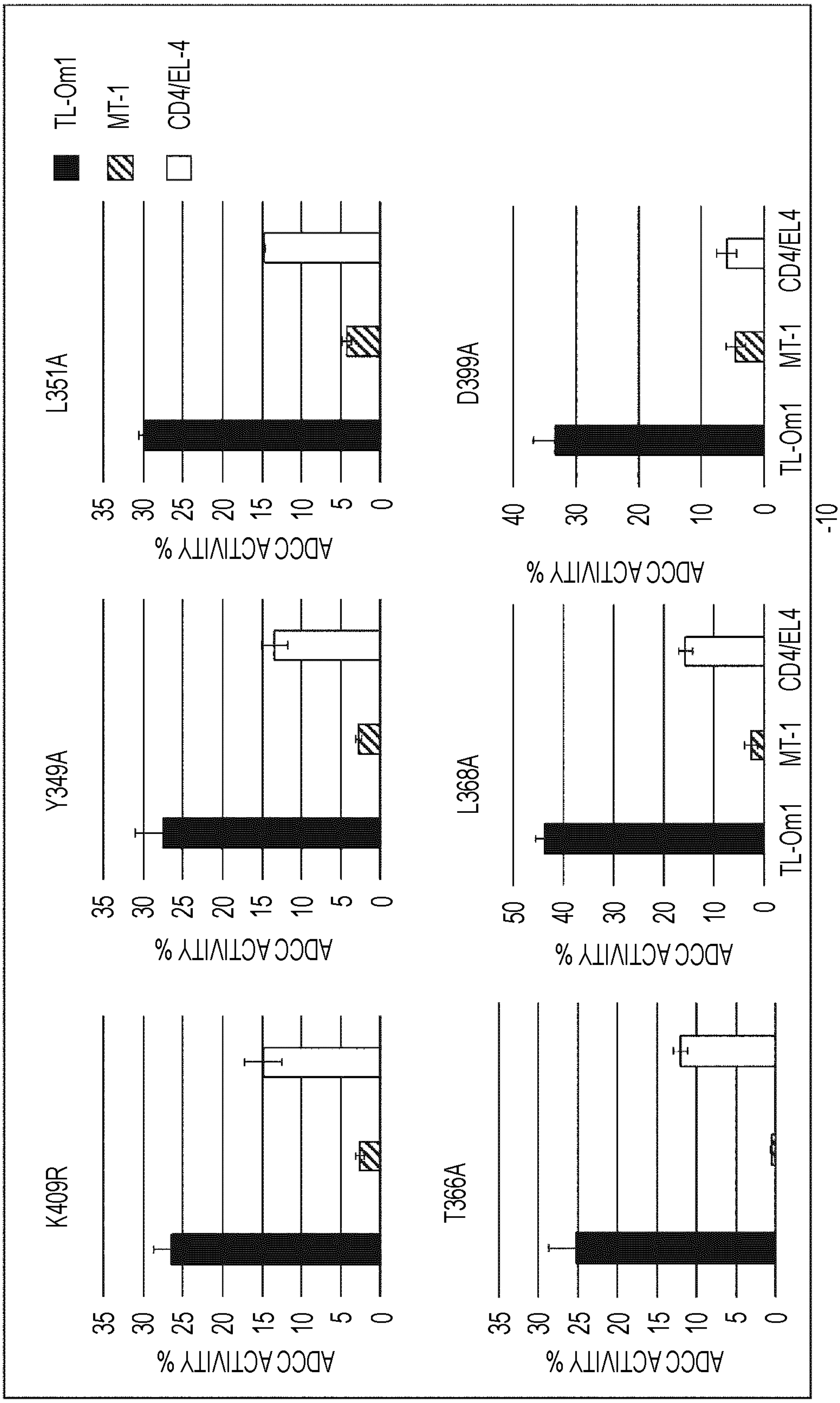

TL-Om1
MT-1
CD4/EL-4
K409R
Y349A
L351A
T366A
L368A
D399A
ADCC ACTIVITY %
TL-Om1
MT-1
CD4/EL4

FIG. 16B

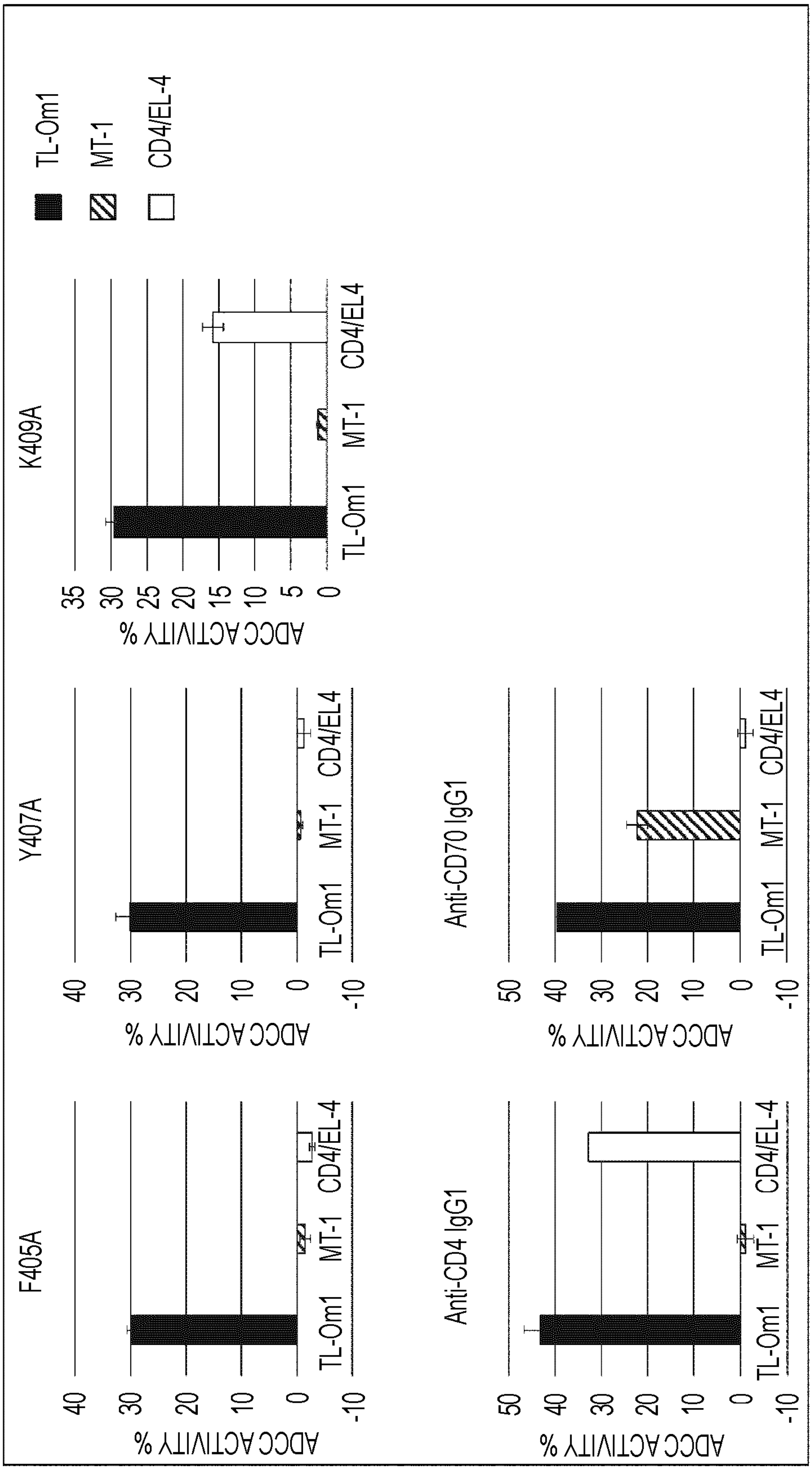
F405A
Y407A
K409A
Anti-CD4 IgG1
Anti-CD70 IgG1
ADCC ACTIVITY %
TL-Om1
MT-1
CD4/EL-4
CD4/EL4

FIG. 17

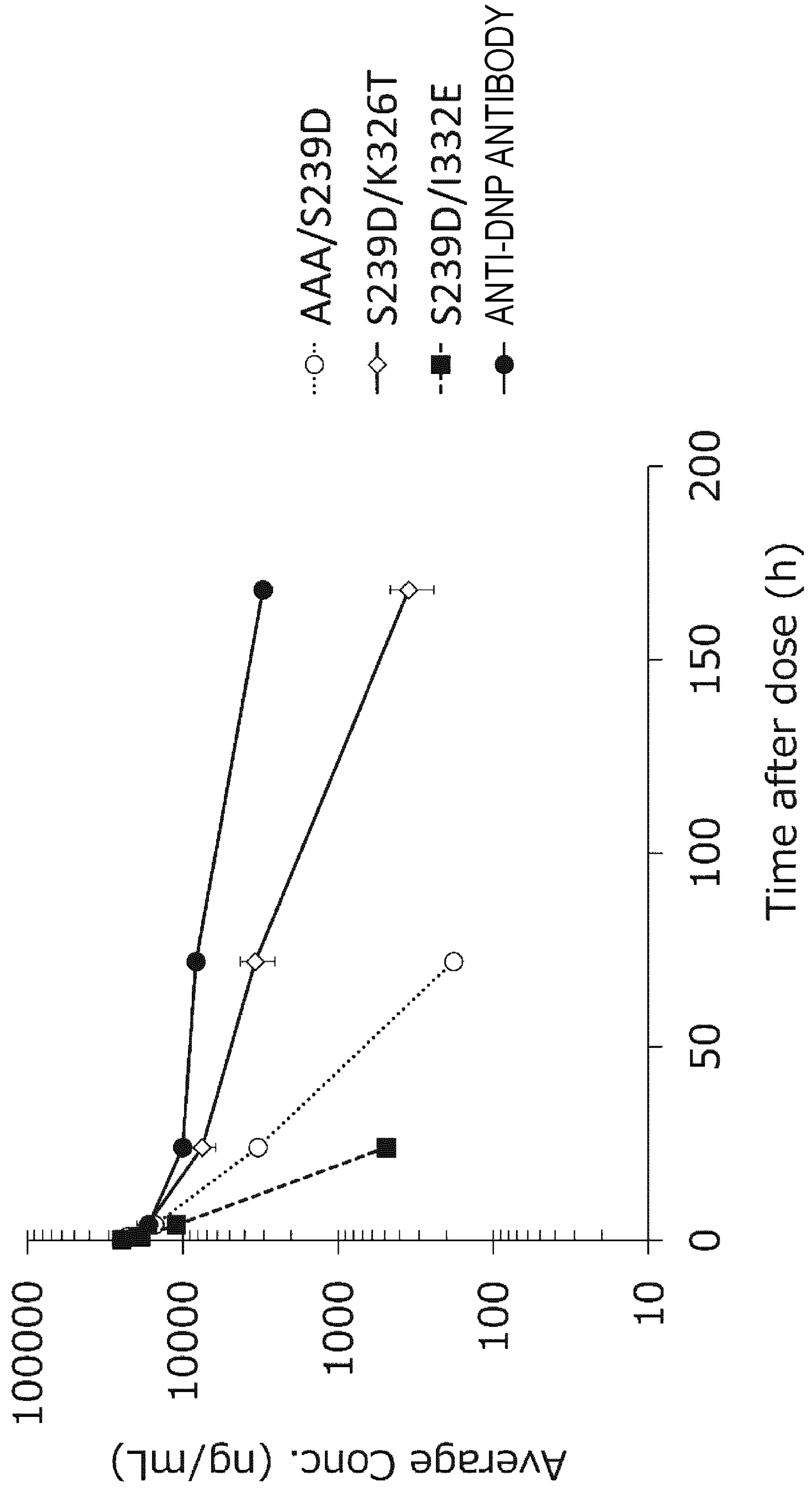
AAA/S239D
S239D/K326T
S239D/I332E
ANTI-DNP ANTIBODY
Average Conc. (ng/mL)
100000
10000
1000
100
10
Time after dose (h)
0
50
100
150
200

FIG. 18A

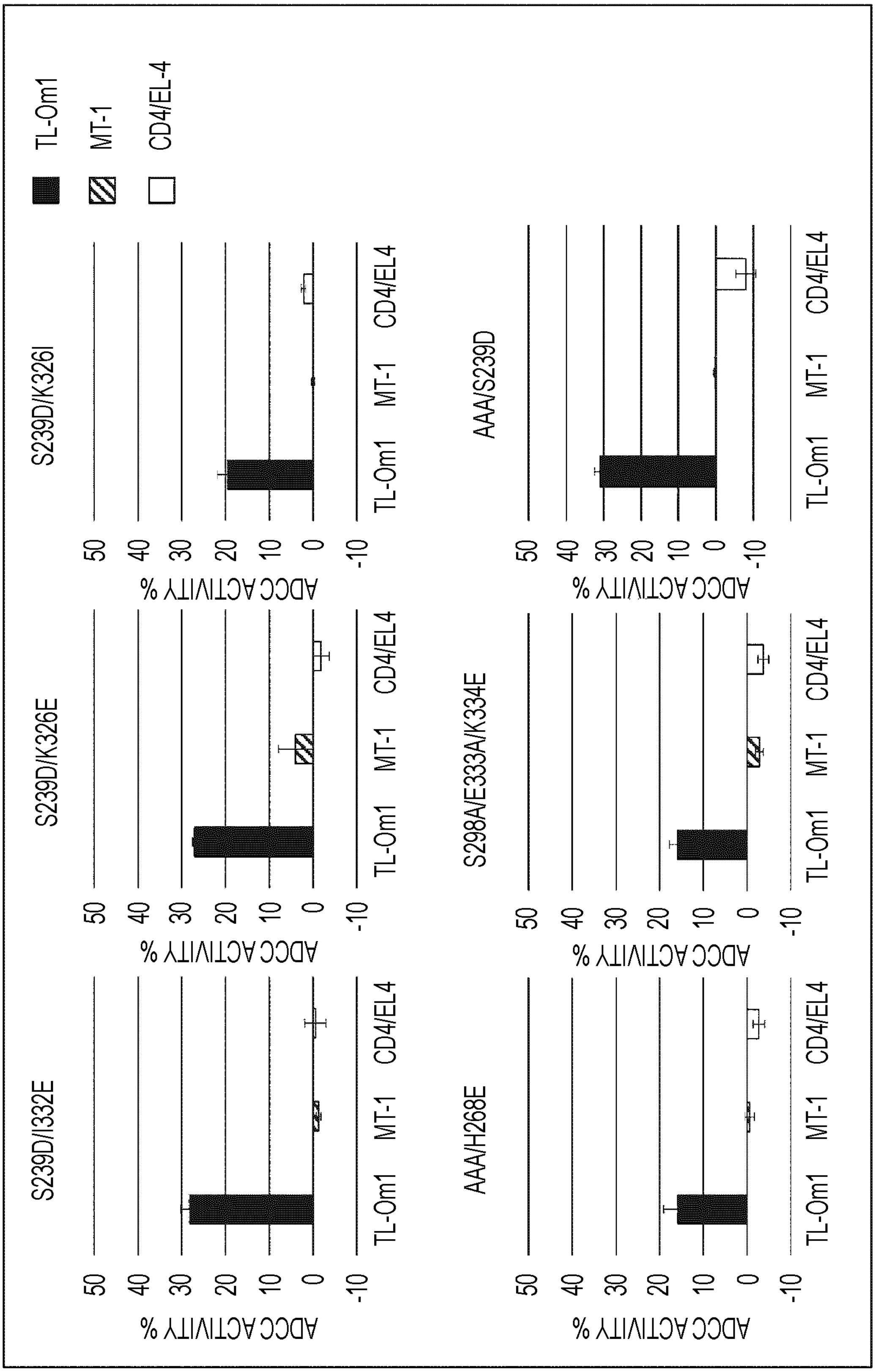

S239D/I332E
S239D/K326E
S239D/K326I
AAA/H268E
S298A/E333A/K334E
AAA/S239D
ADCC ACTIVITY %
50
40
30
20
10
0
-10
TL-Om1
MT-1
CD4/EL4
TL-Om1
MT-1
CD4/EL-4

FIG. 18B

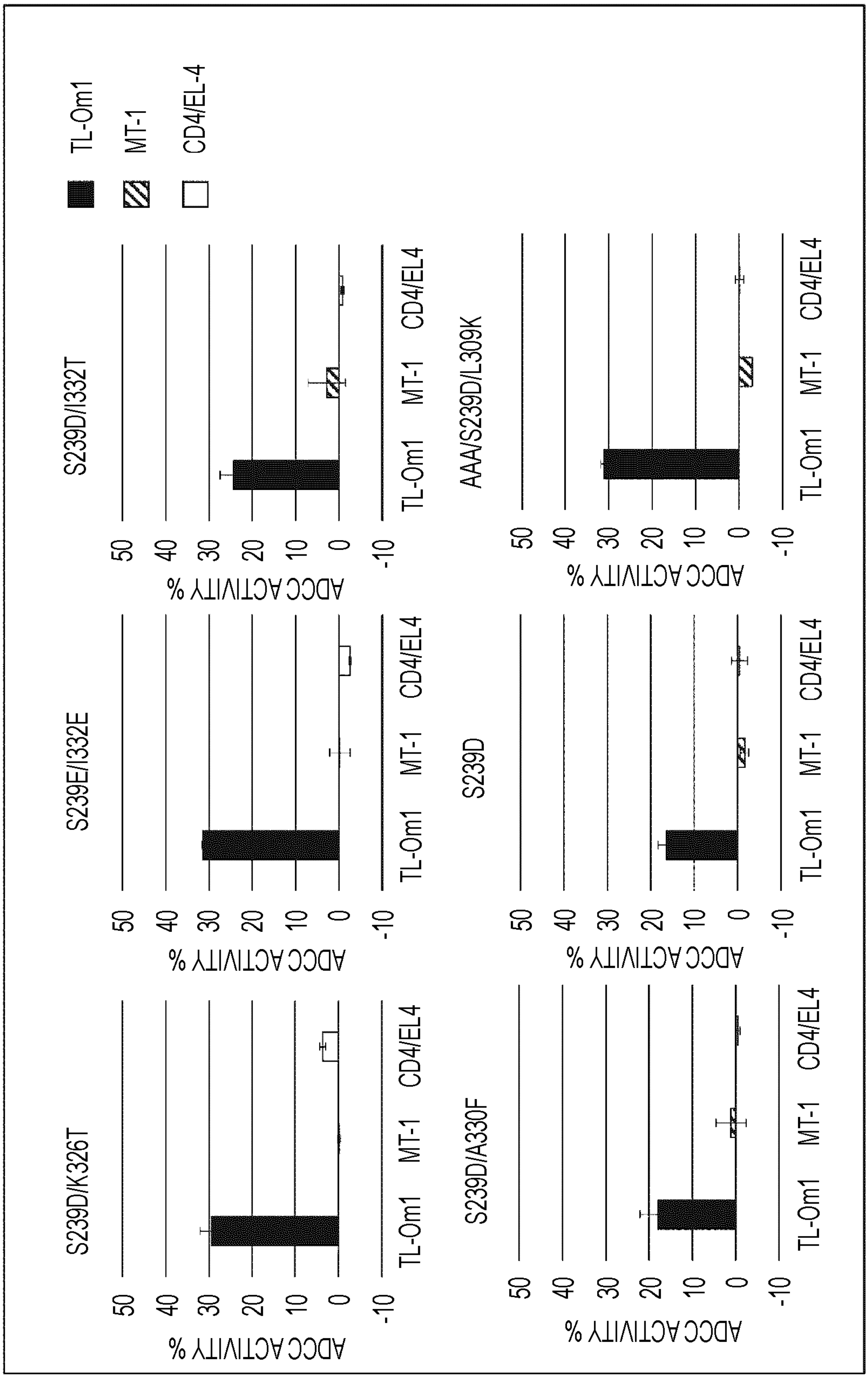
TL-Om1
MT-1
CD4/EL-4
S239D/K326T
S239E/I332E
S239D/I332T
S239D/A330F
S239D
AAA/S239D/L309K
ADCC ACTIVITY %
50
40
30
20
10
0
-10
TL-Om1
MT-1
CD4/EL4

FIG. 18C

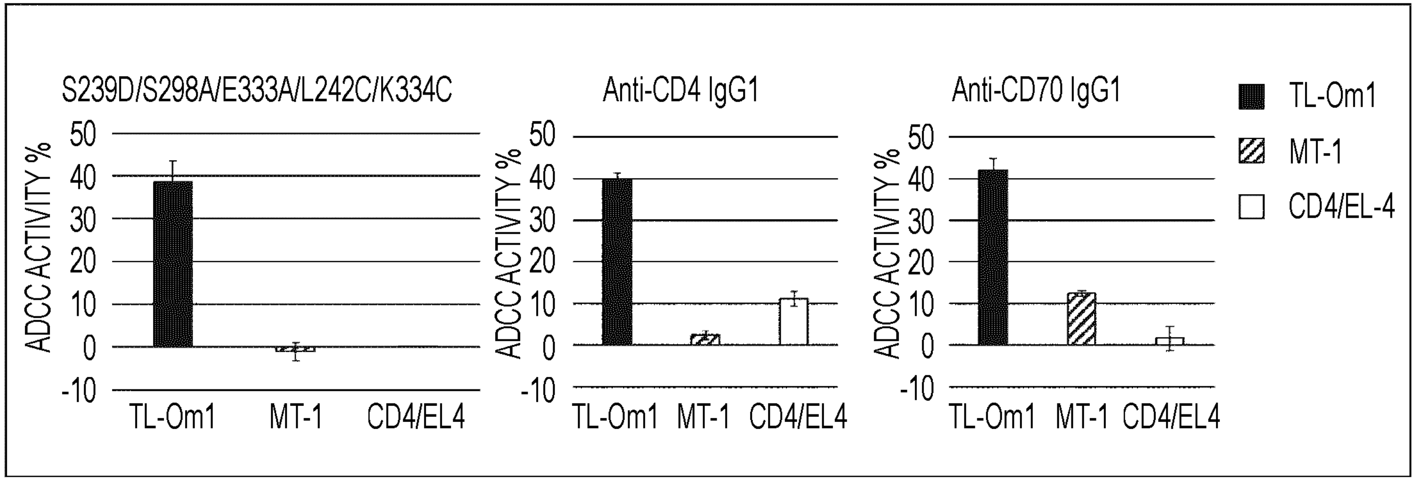
S239D/S298A/E333A/L242C/K334C
Anti-CD4 IgG1
Anti-CD70 IgG1
ADCC ACTIVITY %
50
40
30
20
10
0
-10
TL-Om1
MT-1
CD4/EL4
CD4/EL-4

FIG. 19A

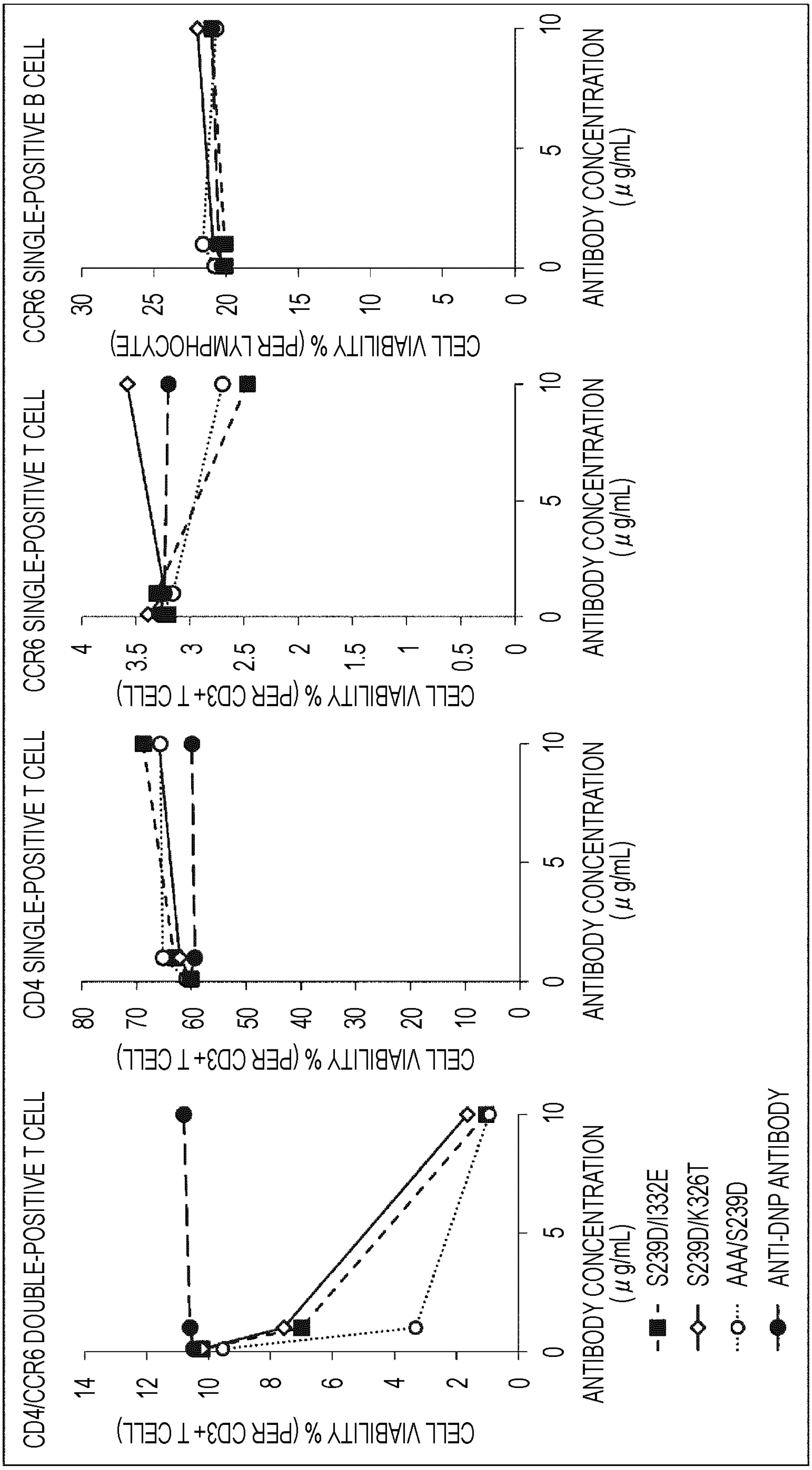
CD4/CCR6 DOUBLE-POSITIVE T CELL
CD4 SINGLE-POSITIVE T CELL
CCR6 SINGLE-POSITIVE T CELL
CCR6 SINGLE-POSITIVE B CELL
CELL VIABILITY % (PER CD3+ T CELL)
CELL VIABILITY % (PER LYMPHOCYTE)
ANTIBODY CONCENTRATION (μg/mL)
S239D/I332E
S239D/K326T
AAA/S239D
ANTI-DNP ANTIBODY

FIG. 19B

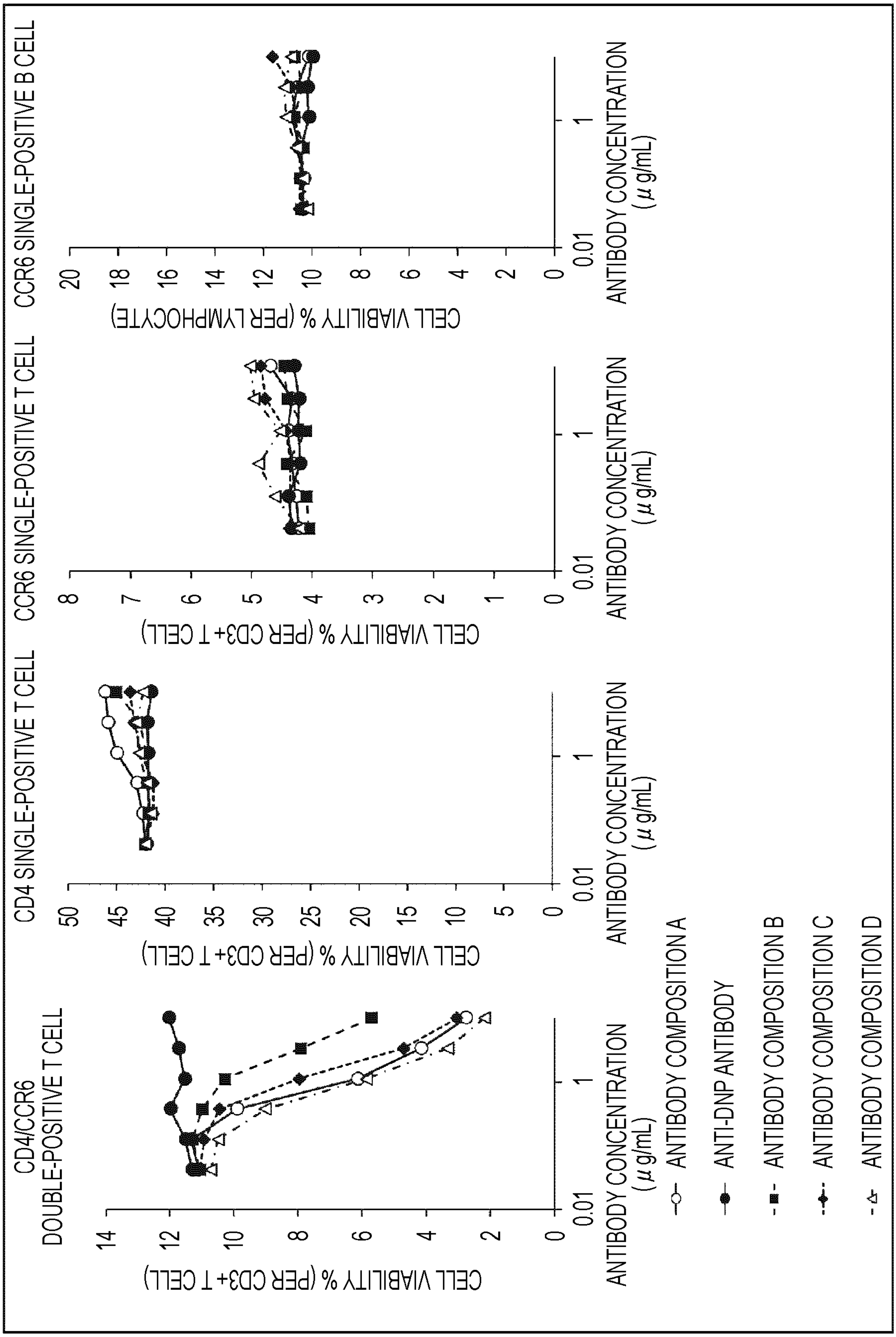
CD4/CCR6
DOUBLE-POSITIVE T CELL
CD4 SINGLE-POSITIVE T CELL
CCR6 SINGLE-POSITIVE T CELL
CCR6 SINGLE-POSITIVE B CELL
CELL VIABILITY % (PER CD3+ T CELL)
CELL VIABILITY % (PER LYMPHOCYTE)
ANTIBODY CONCENTRATION
(μ g/mL)
0.01
1
ANTIBODY COMPOSITION A
ANTI-DNP ANTIBODY
ANTIBODY COMPOSITION B
ANTIBODY COMPOSITION C
ANTIBODY COMPOSITION D

*FIG. 20*

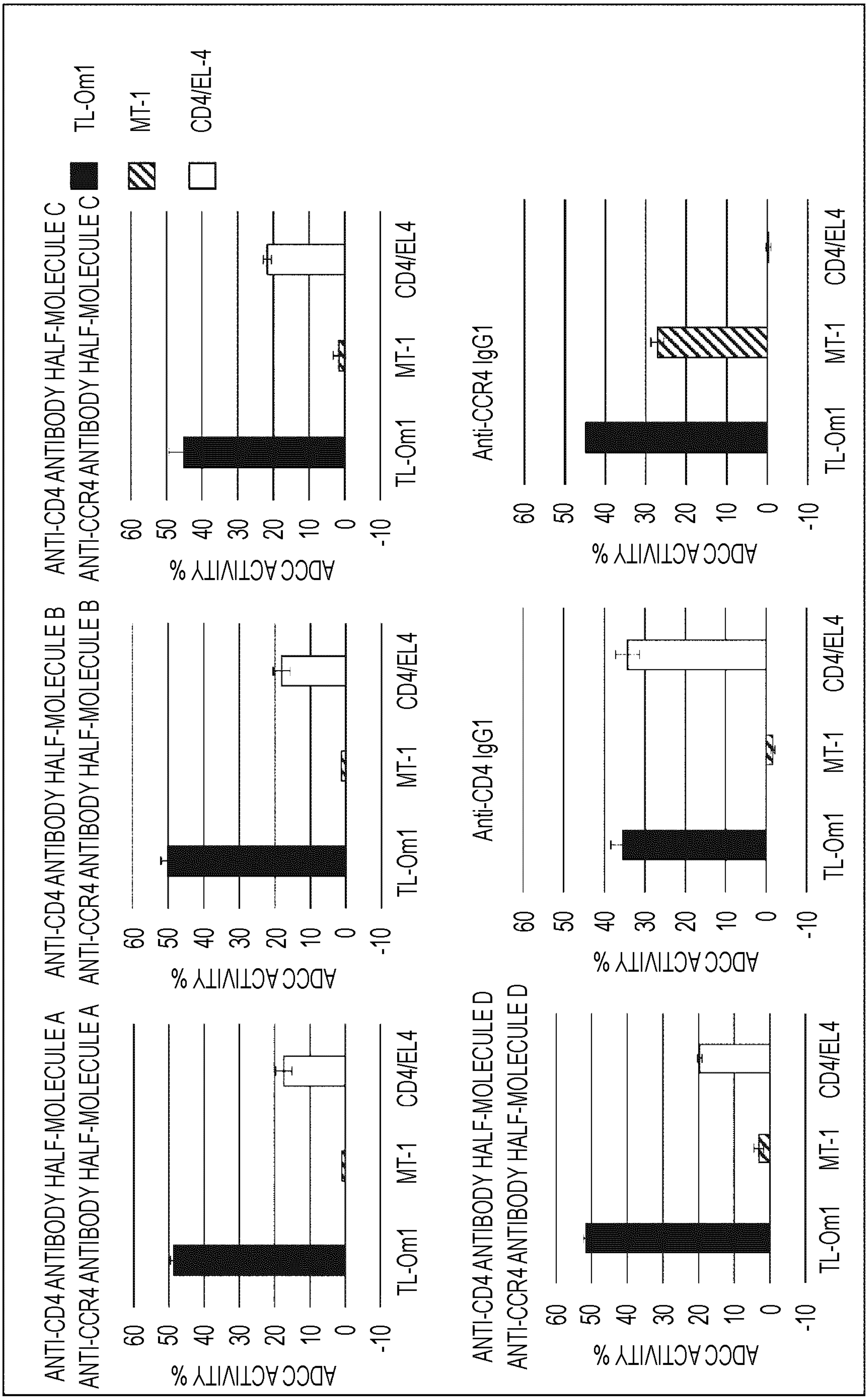
ANTI-CD4 ANTIBODY HALF-MOLECULE A
ANTI-CCR4 ANTIBODY HALF-MOLECULE A
ANTI-CD4 ANTIBODY HALF-MOLECULE B
ANTI-CCR4 ANTIBODY HALF-MOLECULE B
ANTI-CD4 ANTIBODY HALF-MOLECULE C
ANTI-CCR4 ANTIBODY HALF-MOLECULE C
ANTI-CD4 ANTIBODY HALF-MOLECULE D
ANTI-CCR4 ANTIBODY HALF-MOLECULE D
Anti-CD4 IgG1
Anti-CCR4 IgG1
ADCC ACTIVITY %
60
50
40
30
20
10
0
-10
TL-Om1
MT-1
CD4/EL4
CD4/EL-4

FIG. 21

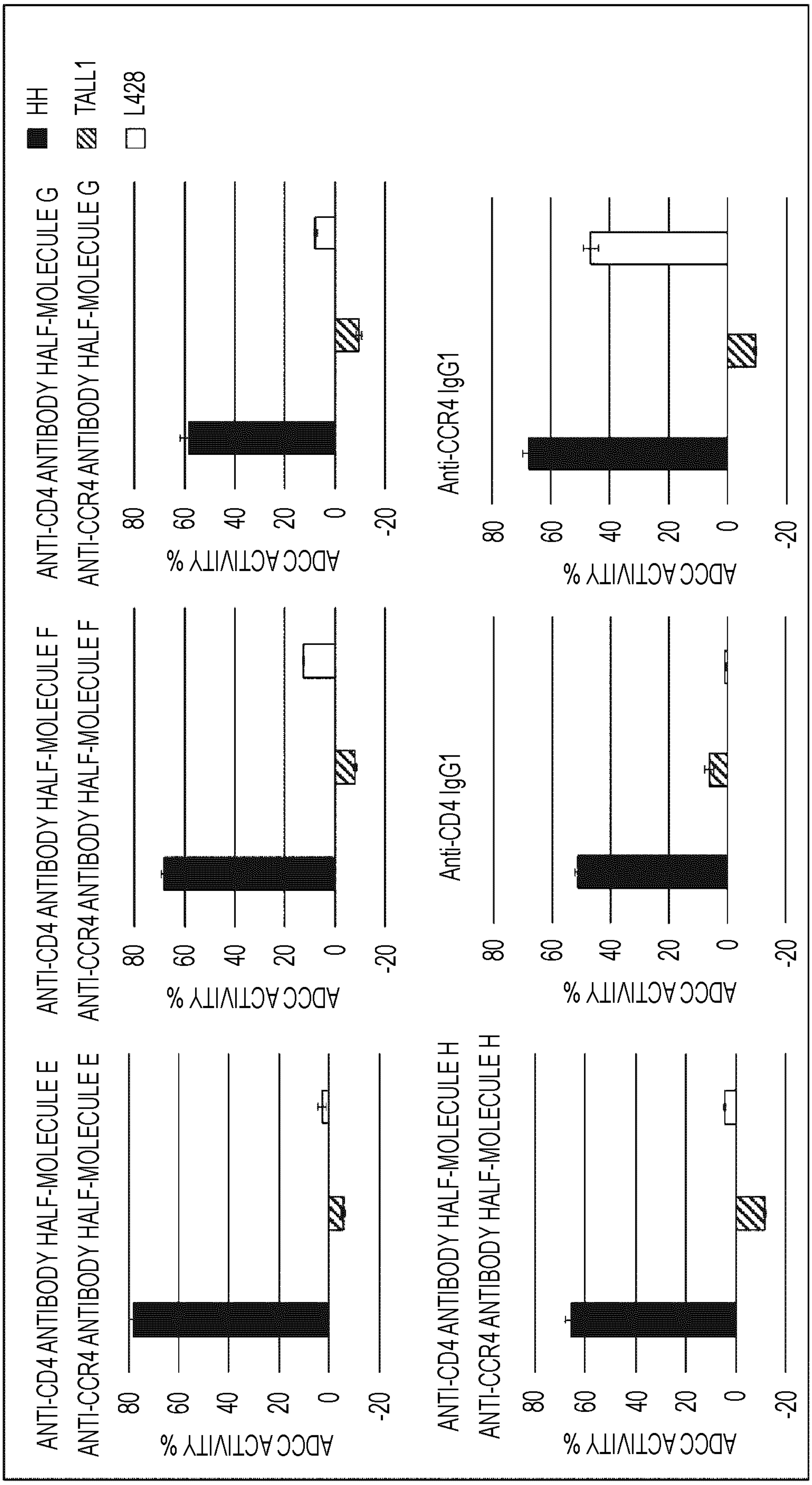

HH
TALL1
L428
ANTI-CD4 ANTIBODY HALF-MOLECULE E
ANTI-CCR4 ANTIBODY HALF-MOLECULE E
ANTI-CD4 ANTIBODY HALF-MOLECULE F
ANTI-CCR4 ANTIBODY HALF-MOLECULE F
ANTI-CD4 ANTIBODY HALF-MOLECULE G
ANTI-CCR4 ANTIBODY HALF-MOLECULE G
ANTI-CD4 ANTIBODY HALF-MOLECULE H
ANTI-CCR4 ANTIBODY HALF-MOLECULE H
Anti-CD4 IgG1
Anti-CCR4 IgG1
ADCC ACTIVITY %
80
60
40
20
0
-20

FIG. 22

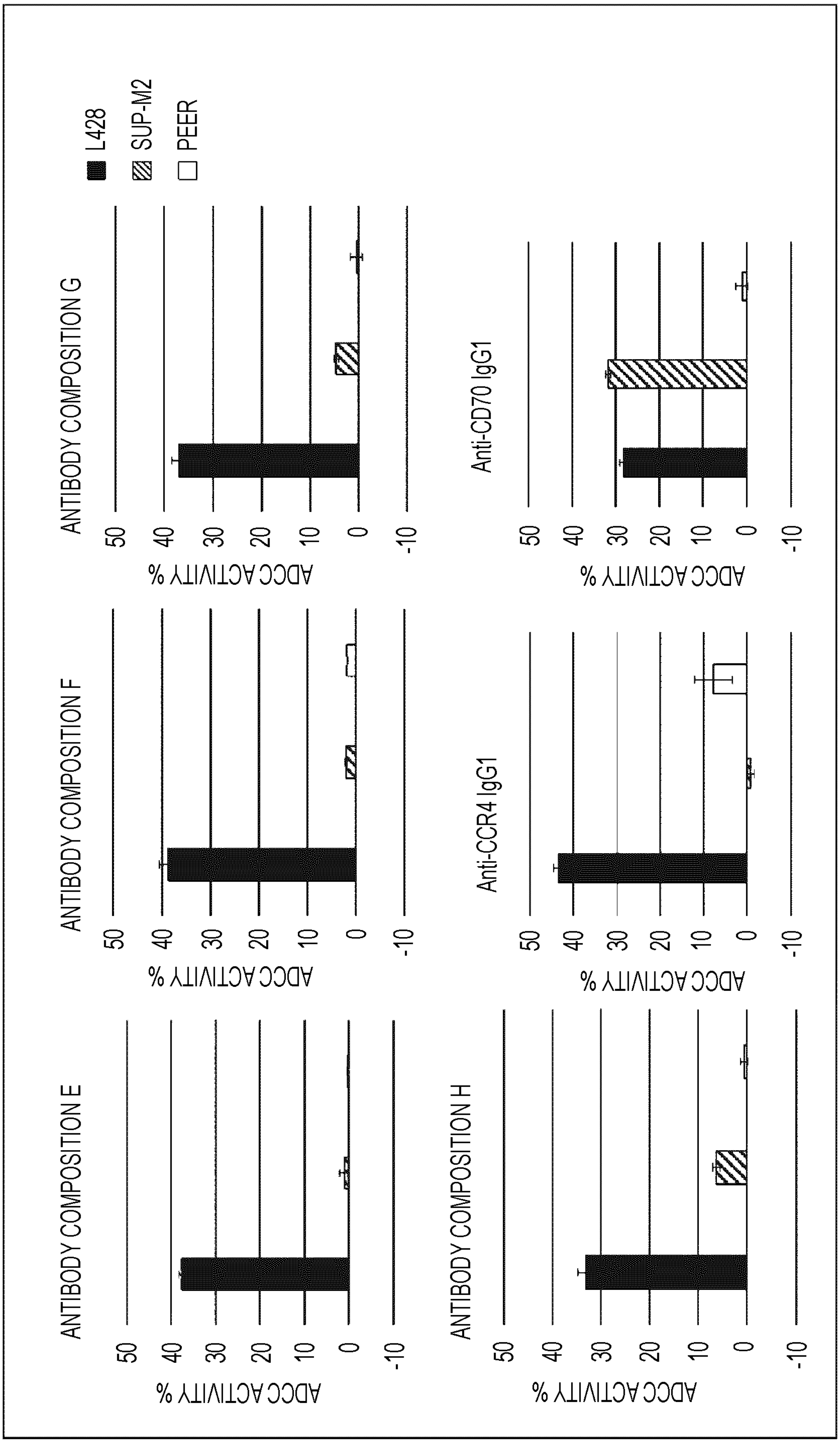
ANTIBODY COMPOSITION E
ANTIBODY COMPOSITION F
ANTIBODY COMPOSITION G
ANTIBODY COMPOSITION H
Anti-CCR4 IgG1
Anti-CD70 IgG1
ADCC ACTIVITY %
50
40
30
20
10
0
-10
L428
SUP-M2
PEER

FIG. 23

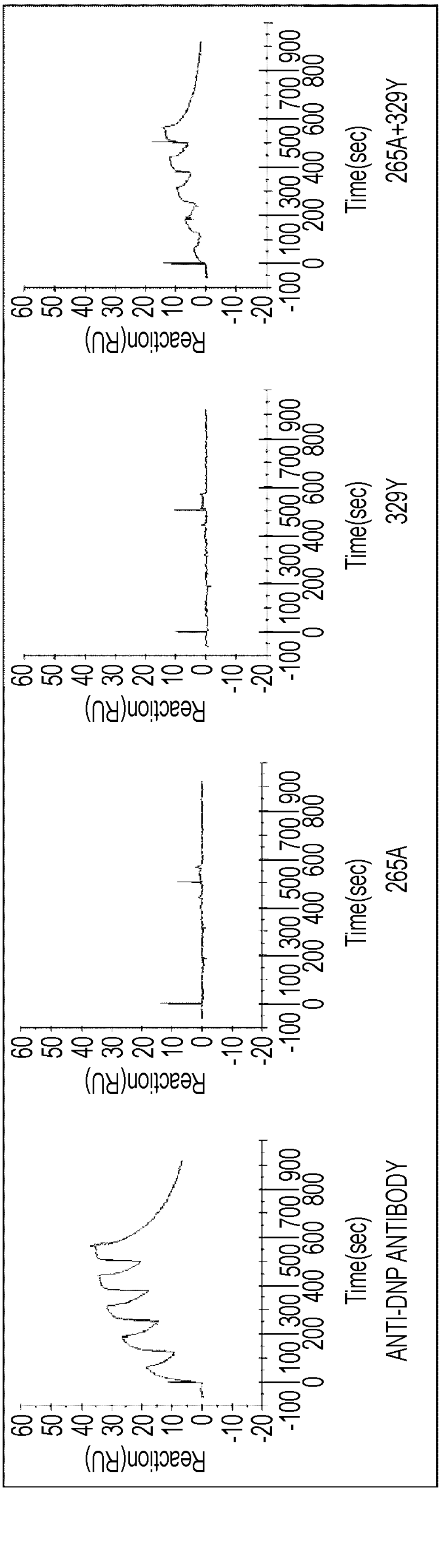
Reaction(RU)
60
50
40
30
20
10
0
-10
-20
-100
0
100
200
300
400
500
600
700
800
900
Time(sec)
ANTI-DNP ANTIBODY
265A
329Y
265A+329Y

## INTERNATIONAL SEARCH REPORT

International application No. PCT/JP2021/014181

A. CLASSIFICATION OF SUBJECT MATTER

C12N 15/13(2006.01)i; A61K 39/395(2006.01)i; A61P 35/00(2006.01)i; A61P 37/02(2006.01)i; A61P 37/08(2006.01)i; A61P 43/00(2006.01)i; C07K 16/00(2006.01)i; C07K 16/18(2006.01)i; C12N 1/15(2006.01)i; C12N 1/19(2006.01)i; C12N 1/21(2006.01)i; C12N 5/10(2006.01)i; C12N 15/63(2006.01)i

FI: C12N15/13 ZNA; C12N15/63 Z; C07K16/18; C12N1/15; C12N1/19; C12N1/21; C12N5/10; A61K39/395 D; A61K39/395 N; A61P35/00; A61P37/02; A61P37/08; C07K16/00; A61P43/00 105

According to International Patent Classification (IPC) or to both national classification and IPC

B. FIELDS SEARCHED

Minimum documentation searched (classification system followed by classification symbols)

C12N15/13; A61K39/395; A61P35/00; A61P37/02; A61P37/08; A61P43/00; C07K16/00; C07K16/18; C12N1/15; C12N1/19; C12N1/21; C12N5/10; C12N15/63

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq; UniProt/GeneSeq

C. DOCUMENTS CONSIDERED TO BE RELEVANT

| Category* | Citation of document, with indication, where appropriate, of the relevant passages | Relevant to claim No. |
|---|---|---|
| Y | WO 2013/002362 A1 (CHUGAI PHARMACEUTICAL CO., LTD.) 03 January 2013 (2013-01-03) claims, examples, paragraphs [0020]-[0030], [0121], [0142] | 9-13 |
| A | claims, examples, paragraphs [0020]-[0030], [0121], [0142] | 1-8, 14-17 |
| Y | WO 2014/104165 A1 (CHUGAI PHARMACEUTICAL CO., LTD.) 03 July 2014 (2014-07-03) claims, examples, paragraphs [0019]-[0029], [0118], [0126] | 9-13 |
| A | claims, examples, paragraphs [0019]-[0029], [0118], [0126] | 1-8, 14-17 |

☒ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 10 June 2021 (10.06.2021) | 22 June 2021 (22.06.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/014181

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2008-511337 A (GENENTECH, INC.) 17 April 2008 (2008-04-17) claims, examples | 9-13 |
| A | claims, examples | 1-8, 14-17 |
| Y | WO 2016/159213 A1 (CHUGAI PHARMACEUTICAL CO., LTD.) 06 October 2016 (2016-10-06) claims, background art | 9-13 |
| A | claims, background art | 1-8, 14-17 |
| A | WO 2018/155611 A1 (CHUGAI PHARMACEUTICAL CO., LTD.) 30 August 2018 (2018-08-30) claims, examples | 1-17 |
| A | JP 2016-509476 A (GENMAB B.V.) 31 March 2016 (2016-03-31) examples 15, 16 | 1-17 |
| A | ESCOBAR-CABRERA, Eric et al., "Asymmetric Fe Engineering for Bispecific Antibodies with Reduced Effector Function", Antibodies, 2017, vol. 6, no. 2, 7, pp. 1-16 abstract | 1-17 |
| P, X | WO 2020/067541 A1 (KYOWA KIRIN CO., LTD.) 02 April 2020 (2020-04-02) claims, examples | 1-17 |

Form PCT/ISA/210 (continuation of second sheet) (January 2015)

**INTERNATIONAL SEARCH REPORT**
Information on patent family members

International application No.
PCT/JP2021/014181

| Patent Documents referred in the Report | Publication Date | Patent Family | Publication Date |
|---|---|---|---|
| WO 2013/002362 A1 | 03 Jan. 2013 | EP 2728002 A1<br>claims, examples, paragraphs [0020]-[0030], [0125], [0148]<br>US 2014/0199294 A1<br>KR 10-2014-0041787 A<br>CN 103827300 A | |
| WO 2014/104165 A1 | 03 Jul. 2014 | EP 2940135 A1<br>claims, examples, paragraphs [0020]-[0030], [0124], [0133]<br>US 2015/0344570 A1<br>KR 10-2015-0097786 A<br>CN 105102618 A | |
| JP 2008-511337 A | 17 Apr. 2008 | US 2006/0204493 A1<br>claims, examples<br>WO 2006/028936 A2<br>EP 1789446 A2 | |
| WO 2016/159213 A1 | 06 Oct. 2016 | US 2018/0057607 A1<br>claims, background art<br>EP 3279216 A1 | |
| WO 2018/155611 A1 | 30 Aug. 2018 | US 2019/0382484 A1<br>claims, examples<br>EP 3586872 A1 | |
| JP 2016-509476 A | 31 Mar. 2016 | US 2015/0353636 A1<br>examples 15, 16<br>WO 2014/108198 A1<br>EP 2943506 A1<br>CN 105229026 A<br>KR 10-2016-0007478 A | |
| WO 2020/067541 A1 | 02 Apr. 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013002362 A
- WO 2018155611 A
- US 20040259150
- WO 2005035586 A
- WO 200231140 A
- WO 0061739 A
- US 20070148165
- US 6737056 B
- US 7297775 B
- US 7317091 B
- JP H322979 A
- JP H2227075 A
- WO 9710354 A
- US 6001358 A
- WO 2010143698 A
- JP 2606856 B
- JP 2517813 B
- JP S63299 A
- WO 2000061739 A
- WO 2003085102 A
- JP H2257891 A
- JP S63309192 A
- WO 0231140 A
- WO 2013005649 A
- WO 2011108502 A
- WO 200703637 A
- WO 9951642 A
- WO 2006020114 A
- WO 2004099249 A
- WO 2004063351 A
- WO 2013118858 A
- WO 2005053741 A
- JP 2020066313 A
- JP 2020181493 A


### Non-patent literature cited in the description


- **CARTER P.** *Nat Rev Cancer,* 2001, vol. 1, 118-29
- **CARTRON G ; DACHEUX L ; SALLES G et al.** *Blood,* 2002, vol. 99, 754-8
- **WENG WK ; LEVY R.** *J Clin Oncol,* 2003, vol. 21, 3940-7
- Monoclonal Antibodies: Principles and Applications. Wiley-Liss, Inc, 1995, 45
- **AALBERSE RC ; SCHUURMAN J.** *Immunology,* 2002, vol. 105, 9-19
- **LABRIJN AF.** *Nat Biotechnol,* 2009, vol. 27, 767-71
- **SONDERMANN P.** *Nature,* 2000, vol. 406, 267-73
- **RADAEV S.** *J Biol Chem,* 2001, vol. 276, 16469-77
- **FERRARA C.** *Proc Natl Acad Sci,* 2011, vol. 108, 12669-74
- **MIZUSHIMA T.** *Genes Cells,* 2011, vol. 16, 1071-80
- **STROHL WR.** *Curr Opin Biotechnol,* 2009, vol. 20, 685-91
- **NIWA R.** *J Pharm Sci,* 2015, 930-41
- **BECK A.** *mAbs,* 2012, vol. 4, 419-25
- **GOEDE V.** *N Engl J Med,* 2014, vol. 370, 1101-10
- **BYRNE H.** *Trends Biotechnol,* 2013, vol. 31, 621-32
- **MAZOR Y.** *MAbs,* 2015, vol. 7, 377-89
- **KABAT et al.** *Sequences of Proteins of Immunological Interest, US Dept. Health and Human Services,* 1991
- **BRINKMANN U et al.** *MABS,* 2017, vol. 9, 182-212
- **FERNANDES CFC et al.** *Frontiers in Immunology,* 2017, vol. 8, 653
- **TOMIZUKA K. et al.** *Proc Natl Acad Sci USA.,* 2000, vol. 97, 722-7
- **WINTER G. et al.** *Annu Rev Immunol.,* 1994, vol. 12, 433-55
- **ROSEN A. et al.** *Nature,* 1977, vol. 267, 52-54
- 36. *Cancer Immunol. Immunother,* 1993, 373
- Monoclonal Antibodies: Principles and Applications. Wiley-Liss, Inc, 1995
- Enzyme Immunoassay. IGAKU-SHOIN Ltd, 1987
- Enzyme Antibody Technique. Gakusai Kikaku, 1985
- *J. Immunol. Methods,* 1997, vol. 200, 121
- *Proc. Natl. Acad. Sci. U. S. A.,* 2000, vol. 97, 9026

- *Cancer Immunol. Immunother.,* 1993, vol. 36, 373
- *Annu. Rev. Immunol.,* 1991, vol. 9, 457-492
- *Chem. Today,* 2003, 65
- *Curr Opin Chem Biol.,* 2000, vol. 6, 645
- *Structure,* 2011, vol. 19 (9), 1274-1282
- *Biochemistry,* 1998, vol. 37, 9266-9273
- Biochemical Experimentation Methods 23-Method for Studying Glycoprotein Sugar Chain. Japan Scientific Societies Press, 1989
- *J. Liq. Chromatogr.,* 1983, vol. 6, 1577
- Molecular Cloning
- Current Protocols in Molecular Biology, Antibodies, A Laboratory Manual. Cold Spring Harbor Laboratory, 1988
- Monoclonal Antibodies: principles and practice. Acad. Press, 1993
- Antibody Engineering, A Practical Approach. IRL Press at Oxford University Press, 1996
- *Cytotechnology,* 1990, vol. 3, 133
- *Nature,* 1987, vol. 329, 840
- *J. Biochemistry,* 1987, vol. 101, 1307
- *Methods in Enzymol.,* 1987, vol. 154 (3
- Molecular Cloning: A Laboratory Manual. Cold Spring Harbor Lab. Press, 1989
- *Current Protocols in Molecular Biology,* (1-34
- *Strategies,* 1992, vol. 5, 58
- *Nucleic Acids Research,* 1989, vol. 17, 9494
- *DNA Cloning: A Practical Approach,* 1985, vol. I, 49
- *Mol. Cell. Biol.,* 1983, vol. 3, 280
- *Gene,* 1985, vol. 33, 103
- *Strategies,* 1992, vol. 5, 81
- *Science,* 1983, vol. 222, 778
- *J. Mol. Biol.,* 1983, vol. 166, 1
- *J. Mol. Biol.,* vol. 16 (118), 1966
- *Gene,* vol. 38 (275), 1985
- **SANGER et al.** *Proc. Natl. Acad. Sci. U. S. A.,* 1977, vol. 74, 5463
- Sequences of Proteins of Immunological Interest. US Dept. Health and Human Services, 1991
- *BIO/TECHNOLOGY,* 1991, vol. 9, 266
- *J. Mol. Biol.,* 1977, vol. 112, 535
- *Protein Engineering,* 1994, vol. 7, 1501
- Methods in Nucleic Acids Res. CRC press, 1991, 283
- Methods in Nucleic Acids Res. CRC press, 1991
- *Proc. Natl. Acad. Sci. USA,* 1987, vol. 84, 7413
- Monoclonal Antibodies-Principles and practice. Academic Press, 1996
- Antibodies-A Laboratory Manual. Cold Spring Harbor Laboratory, 1988
- *Proc. Natl. Acad. Sci. U. S. A.,* 1987, vol. 84, 7413
- Biomanual Series 4-Methods of Gene Transfer, Expression and Analysis (Yodosha). 1994
- *Proc. Natl. Acad. Sci. USA,* 1980, vol. 77, 4216
- *Somatic Cell and Molecular genetics,* 1986, vol. 12, 55
- *Methods Enzymol.,* 1990, vol. 194, 182
- *Proc. Natl. Acad. Sci. U. S. A.,* 1978, vol. 84, 1929
- *J. Bacteriology,* 1983, vol. 153, 163
- *Proc. Natl. Acad. Sci. U. S. A.,* 1978, vol. 75, 1929
- Baculovirus Expression Vectors, A Laboratory Manual. W. H. Freeman and Company, 1992
- *Bio/Technology,* 1988, vol. 6, 47
- *American Journal of Clinical Nutrition,* 1996, vol. 63, 639S
- *American Journal of Clinical Nutrition,* 1996, vol. 63, 627S
- *Bio/Technology,* 1991, vol. 9, 830
- *Tissue Culture,* 1994, 20
- *Tissue Culture,* 1995, vol. 21
- *Trends in Biotechnology,* 1997, vol. 15, 45
- *Nature,* 1970, vol. 227, 680
- Current Protocols in Molecular Biology; Antibodies, A Laboratory manual. Cold Spring Harbor Laboratory, 1988
- *Cancer Immunol. Immunother,* 1993, vol. 36, 373
- *Agric. Biol. Chem.,* 1991, vol. 55 (1), 283-284
- *Anal. Biochem.,* 1988, vol. 171, 73
- Biochemical Experimentation Methods 23-Methods of Studies on Glycoprotein Sugar Chains. Japan Scientific Societies Press, 1989
- *J. Biochem.,* 1984, vol. 95, 197
- Enzyme Antibody Technique. 1985
- *J. Immunol.,* 2002, vol. 169, 5171-80
- *Nature,* 2000, vol. 406, 267-73
- *J Biol Chem,* 2001, vol. 276, 16469-77
- *J. Immunol.,* 2011, vol. 187, 3238-3246
- *J. Immunol.,* 1992, vol. 149, 1779-1787
- *Clin Cancer Res.,* 2005, vol. 11 (8), 3126-3135